# EUROPEAN PATENT SPECIFICATION

(11) **EP 4 031 685 B1**
(45) Date of publication and mention of the grant of the patent: **10.07.2024**
(21) Application number: 20772294.3
(22) Date of filing: 16.09.2020
(51) Int. Cl.: C12Q 1/6883, C12Q 1/6888, A01K 67/00

(54) **IN VITRO FERTILISATION (IVF) EMBRYO VIABILILITY AND QUALITY ASSAY**
TEST FÜR DIE LEBENSFÄHIGKEIT UND QUALITÄT VON IN-VITRO-FERTIERUNGSVERFAHREN (IVF) BEI EMBRYONEN
TEST DE QUALITÉ ET DE VIABILITÉ D'EMBRYONS POUR FÉCONDATION IN VITRO (FIV)

(30) Priority: 18.09.2019 US 201962901891 P
(43) Date of publication of application: 27.07.2022
(73) Proprietor: University of Tartu, 50090 Tartu (EE)
(72) Inventor: FAZELI, Alireza, Sheffield South Yorkshire S10 4BG (GB); GODAKUMARA, Godagedara Kasun Madhuranga, Kandy 20810 (LK); SALUMETS, Andres, 51008 Tartu (EE); DISSANAYAKE, Dissanayake Rallage Keerthie Chulanga, Kandy 20000 (LK); ES-HAGHI, Masoumeh, 70150 Kuopio (FI); TROSIN, Aleksander, 11311 Tallinn (EE)
(74) Representative: Barker Brettell Sweden AB
(86) International application number: PCT/EP2020/075832
(87) International publication number: WO 2021/052996

(56) References cited:
- US-A1- 2019 002 985
- WEIMAR CHARLOTTE H E ET AL: "In-vitro model systems for the study of human embryo-endometrium interact", REPRODUCTIVE BIOMEDICINE ONLINE, vol. 27, no. 5, November 2013 (2013-11-01), pages 461 - 476, XP028762761, ISSN: 1472-6483, DOI: 10.1016/J.RBMO.2013.08.002
- TEKLENBURG GIJS ET AL: "Natural Selection of Human Embryos: Decidualizing Endometrial Stromal Cells Serve as Sensors of Embryo Quality upon Implantation", PLOS ONE, vol. 5, no. 4, 21 April 2010 (2010-04-21), pages e10258, XP093020014, Retrieved from the Internet <URL:https://www.ncbi.nlm.nih.gov/pmc/articles/PMC2858159/pdf/pone.0010258.pdf> DOI: 10.1371/journal.pone.0010258
- HESS A P ET AL: "Decidual stromal cell response to paracrine signals from the trophoblast: amplification of immune and angiogenic modulators", BIOLOGY OF REPRODUCTION, NEW YORK, NY [U.A.] : ACADEM. PRESS, US, vol. 76, no. 1, 1 January 2007 (2007-01-01), pages 102 - 117, XP002586768, ISSN: 0006-3363, [retrieved on 20061004], DOI: 10.1095/BIOLREPROD.106.054791
- KURIAN NOBLE K ET AL: "Extracellular vesicle mediated embryo-endometrial cross talk during implantation and in pregnancy", JOURNAL OF ASSISTED REPRODUCTION AND GENETICS, PLENUM PUBLISHING, US, vol. 36, no. 2, 25 October 2018 (2018-10-25), pages 189 - 198, XP036727768, ISSN: 1058-0468, [retrieved on 20181025], DOI: 10.1007/S10815-018-1343-X
- GIACOMINI ELISA ET AL: "Secretome of in vitro cultured human embryos contains extracellular vesicles that are uptaken by the maternal side", SCIENTIFIC REPORTS, vol. 7, no. 1, 12 July 2017 (2017-07-12), UK, pages 1 - 13, XP055805477, ISSN: 2045-2322, Retrieved from the Internet <URL:https://www.nature.com/articles/s41598-017-05549-w.pdf> DOI: 10.1038/s41598-017-05549-w
- HOMER HAYDEN ET AL: "Review: Embryo- and endometrium-derived exosomes and their potential role in assisted reproductive treatments-liquid biopsies for endometrial receptivity", PLACENTA, vol. 54, 9 December 2016 (2016-12-09), pages 89 - 94, XP085039634, ISSN: 0143-4004, DOI: 10.1016/J.PLACENTA.2016.12.011
- POPOVICI ROXANA M. ET AL: "Gene Expression Profiling of Human Endometrial-Trophoblast Interaction in a Coculture Model", ENDOCRINOLOGY, vol. 147, no. 12, 1 December 2006 (2006-12-01), US, pages 5662 - 5675, XP055805770, ISSN: 0013-7227, Retrieved from the Internet <URL:https://academic.oup.com/endo/article-pdf/147/12/5662/9028802/endo5662.pdf> DOI: 10.1210/en.2006-0916
- ES-HAGHI MASOUMEH ET AL: "Specific trophoblast transcripts transferred by extracellular vesicles affect gene expression in endometrial epithelial cells and may have a role in embryo-maternal crosstalk", CELL COMMUNICATION AND SIGNALING, 14 November 2019 (2019-11-14), England, pages 146 - 146, XP055804954, Retrieved from the Internet <URL:https://biosignaling.biomedcentral.com/track/pdf/10.1186/s12964-019-0448-x.pdf> [retrieved on 20210517], DOI: 10.1186/s12964-019-0448-x

## Description

### TECHNICAL FIELD

The present invention generally relates to *in vitro* fertilization (IVF), and in particular to an assay that can be used to identify and select embryos suitable for uterine transfer and implantation in IVF procedures, and to molecules useful in suppressing embryo rejection in IVF to increase the chance for pregnancy after IVF embryo transfer.

### BACKGROUND

The development of the mammalian embryo into a fully-fledged organism depends critically on its successful implantation into the uterine wall. However, as a non-self-entity, the embryo must avoid rejection by the mother's immune system, necessitating an intricate set of negotiations before pregnancy can occur. Thus, the interaction between the developing embryo and the maternal tract arguably represents the most important diplomatic process in placental mammals. Despite this, very little is known regarding the language in which these negotiations are carried out.

That the female reproductive tract is able to detect and respond to the presence of gametes and embryos is well established and evident in the transcriptomic and proteomic profiles of the oviduct/fallopian tube and endometrial cells, suggesting that some form of signal is transmitted by the embryo. Such signals may exist in a variety of forms as a mean of communication leading to alterations of transcriptomic and epigenomic profiles of the maternal tract.

US 2019/0002985 discloses *in vitro* non-invasive methods and kits for determining the quality of an embryo by determining the level of the cell free nucleic acids.

Biologoy of Reproduction 76: 102-117 (2007) discloses that trophoblasts and maternal decidual stromal cells secrete products that regulate trophoblast differentiation and migration into the maternal endometrium. The secreted products are said to be IL1, TNF, member(s) of the IFN family, as well as IGF2 and perhaps hCG and P₄.

Journal of Assisted Reproduction and Genetics 36: 186-198 (2019) provides a review of extracellular vesicle mediated embryo-endometrial cross talk during implantation and in pregnancy.

Scientific Reports 7: 5210 (2017) discloses that conditioned media from human embryos contain extracellular vesicles, which can be taken up by endometrial epithelial cells. EV exchanges is suggested as an emerging way of communication at the maternal-fetal interface.

Placenta 54: 89-94 (2017) provides a review of embryo- and endometrium-derived exosomes and their potential role in assisted reproductive treatments.

Reproductive BioMedicine Online 27: 461-476 (2017) discusses the principal models used to study early human embryo development and initial stages of implantation *in vitro.*

PLoS ONE 5(4): e10258 (2010) employed a human co-culture model consisting of decidualizing ESCs and single hatched blastocysts to determine the levels of 14 implantation factors secreted by the stromal cells by a multiplex immunoassay. The presence of a developing embryo had no significant effect on decidual secretion, whereas arresting embryos triggered selective inhibition of implantation mediators and immunomodulators.

### SUMMARY

It is a general objective to improve *in vitro* fertilization (IFV) procedure in terms of pregnancy rate achieved after IVF embryo transfer.

A particular objective is to select the most viable embryos suitable for IVF embryo transfer.

Another particular objective is to reduce the risk of embryo implantation failure after IVF embryo transfer.

These and other objectives are met by embodiments disclosed herein.

The present invention is defined in the independent claims. Further embodiments of the invention are defined in the dependent claims.

The present invention is based on evidence of non-contact transfer of embryonic RNA transcripts to endometrium in an *in vitro* embryo-maternal cross-talk model. RNA is taken up by the endometrial cells and the expression of endogenous transcripts is altered as a result. The effect can be seen in endometrial cells treated with embryo derived extracellular vesicles (EVs) or conditioned medium derived from IVF embryos. RNA from EVs derived from human IVF embryos and conditioned culture media from the human IVF embryos have the potential to change the level of endometrial transcripts. Interestingly, only good-prognosis viable embryos, i.e., capable of successful pregnancies, induced the observed effect while non-competent, e.g. degenerated, embryos failed to initiate any changes.

An aspect of the invention relates to a method of predicting outcome of an embryo transfer in an IVF procedure. The method comprises contacting *in vitro* responder cells with extracellular vesicles isolated from an IVF embryo to be transferred into a female subject and/or with a conditioned medium from the IVF embryo. The method also comprises determining, in the responder cells, an amount of at least one RNA transcript induced by the extracellular vesicles and/or the conditioned medium. The method further comprises predicting pregnancy outcome of the embryo transfer based on the determined amount of the at least one RNA transcript by predicting a high likelihood for successful embryo transfer and pregnancy of the female subject based on a significant change in the amount of the at least one RNA transcript relative to a reference level of the at least one RNA transcript, and predicting a low likelihood for successful embryo transfer and pregnancy of the female subject based on a non-significant change in the amount of the at least one RNA transcript relative to the reference level.

Another aspect of the invention relates to a method of determining a quality of an IVF embryo. The method comprises contacting *in vitro* responder cells with extracellular vesicles isolated from the IVF embryo and/or with a conditioned medium from the IVF embryo. The method also comprises determining, in the responder cells, an amount of at least RNA transcript induced by the extracellular vesicles and/or the conditioned medium. The method further comprises determining the quality of the IVF embryo based on the determined amount of the at least one RNA transcript by determining the IVF embryo to be good for intrauterine transfer into a female subject based on a significant change in the amount of the at least one RNA transcript relative to a reference level of the at least one RNA transcript, and determining the IVF embryo to be not good for intrauterine transfer into the female subject based on a non-significant change in the amount of the at least one RNA transcript relative to the reference level.

A further aspect of the invention relates to a method of selecting an embryo for an IVF procedure. The method comprises contacting *in vitro,* for each IVF embryo among *M* potential IVF embryos, responder cells with extracellular vesicles isolated from the IVF embryo and/or a conditioned medium from the IVF embryo, wherein *M* is an integer equal to or larger than two. The method also comprises determining, for each IVF embryo among the *M* potential IVF embryos and in the responder cells, an amount of at least one RNA transcript induced by the extracellular vesicles and/or the conditioned medium. The method further comprises selecting at least one IVF embryo among the *M* potential IVF embryos based on the respective determined amounts of the at least one RNA transcript by selecting the *N* IVF embryo(s) resulting in a largest downregulation of the at least one RNA transcript among the *M* potential IVF embryos, wherein *N* < *M,* or selecting the *N* IVF embryo(s) resulting in a largest upregulation of the at least one RNA transcript among the *M* potential IVF embryos.

### BRIEF DESCRIPTION OF THE DRAWINGS

The embodiments, together with further objects and advantages thereof, may best be understood by making reference to the following description taken together with the accompanying drawings, in which:

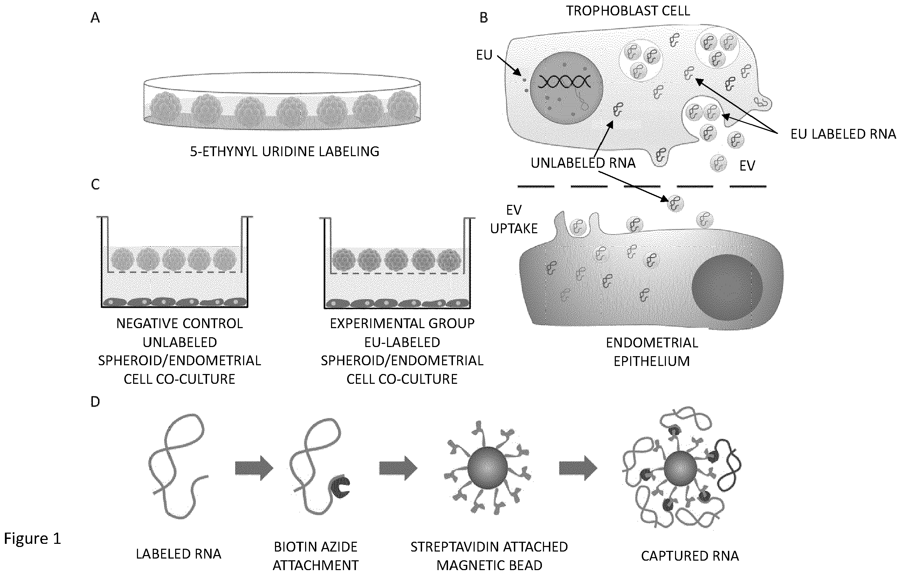

Figure 1 illustrates the bioorthogonal labeling strategy. (A) 5-ethynyl uridine (EU) labeling of trophoblast spheroids. Spheroids were placed in culture media supplemented with EU overnight. (B) Non-contact co-culture of trophoblast spheroids and endometrial cells. EU is incorporated into nascent RNA resulting in EU labeled RNA. RNA is packaged into extracellular vesicles (EVs) and transferred to the endometrial cells through the translucent barrier. EVs containing the labeled RNA are taken up by the endometrial cells. In the endometrial cytoplasm, RNA is released through the degrading EVs' membrane. (C) Experimental setup. Negative control is prepared using unlabeled trophoblast spheroids/endometrial cells. Experimental group consists of EU labeled trophoblast spheroids/endometrial cells. (D) Affinity precipitation procedure. Labeled RNA is attached to biotin azide by click chemistry. Magnetic beads attached to streptavidin are used to selectively precipitate EU labeled RNA.

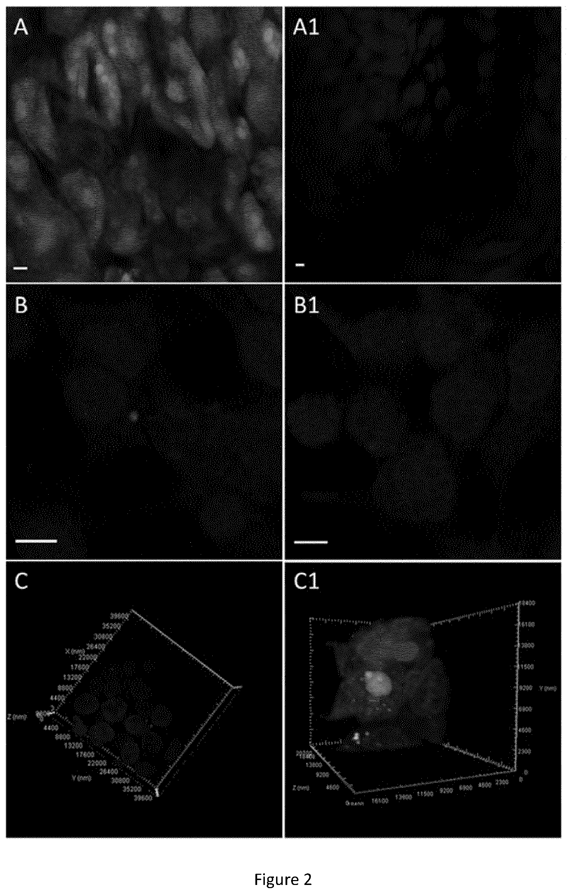

Figure 2 illustrates visualization of 5-ethynyl uridine (EU)-labeled RNA in trophoblast spheroids and endometrial cells. (A) RNA in trophoblast spheroids were labeled with 5-ethynyl uridine (EU) and stained with Alexa azide. Green florescence is evidence of successful labeling. (A1) Unlabeled spheroids (negative control) did not show fluorescent signal. (B) Endometrial cells were stained with Alexa azide after 24 h incubation with labeled spheroids to visualize the transferred transcripts. Green dots in endometrial cells indicate labeled RNA transfer. (B1) Endometrial cells co-incubated with unlabeled spheroids were used as negative controls. Negative control did not exhibit any specific fluorescent signal. (C, C1) 3-dimentional confocal scanning of endometrial cells with cytoplasmic EU labeled RNA with and without cell tracker dye. Scale bar 4 µm.

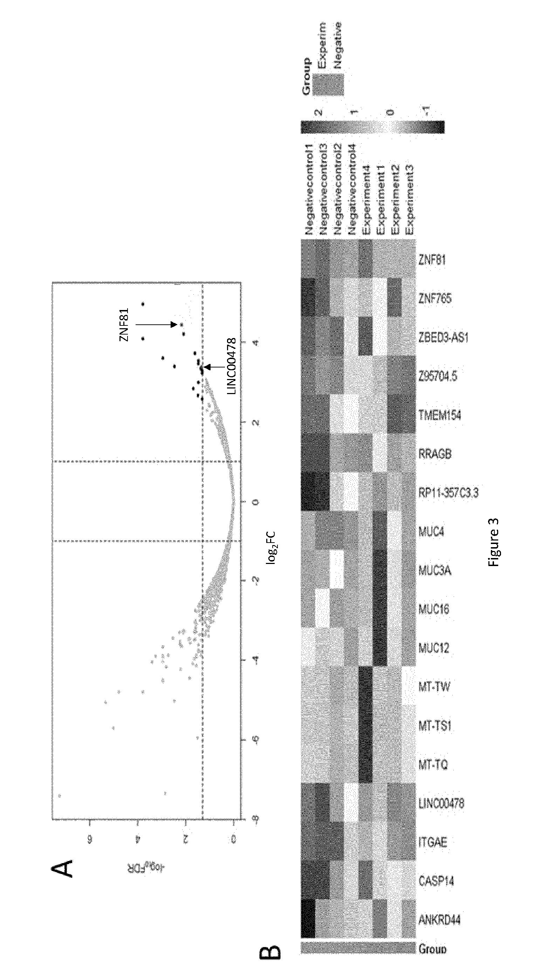

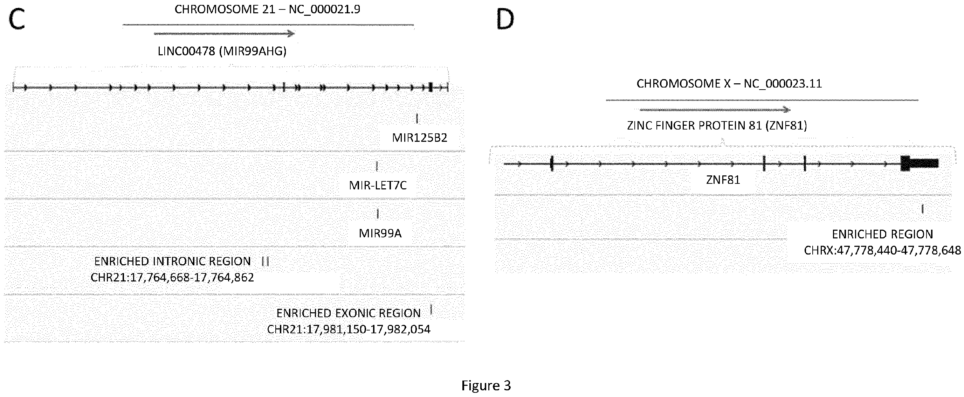

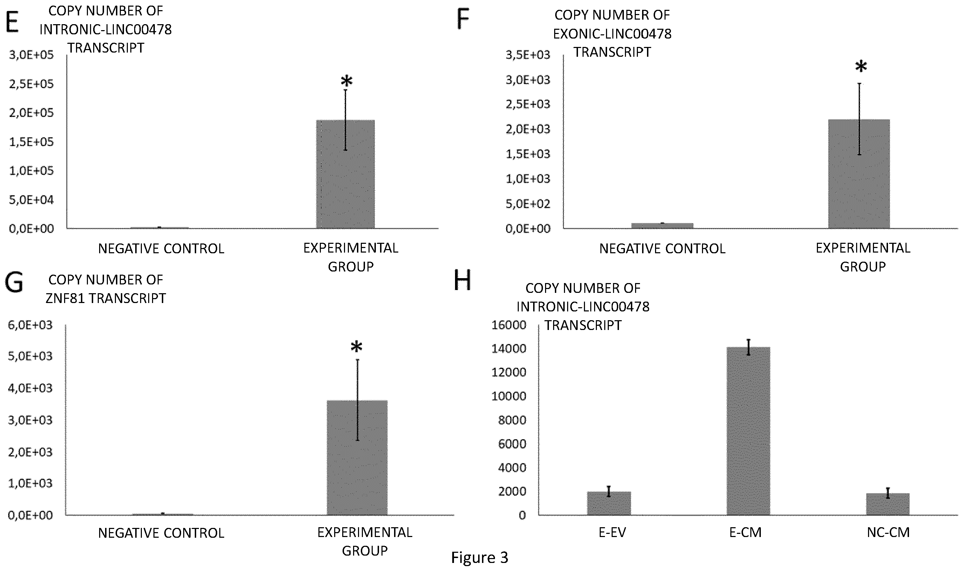

Figure 3 illustrates RNA sequencing of transferred 5-ethynyl uridine (EU)-labeled transcripts (A) Volcano plot from RNA sequencing data of EU-labeled transferred transcripts affinity precipitated from endometrial cells co-incubated with EU-labeled trophoblast spheroids. RNA extracted from endometrial cells co-incubated with unlabeled spheroids was used as negative control. The rate of false discovery is plotted against fold change, demonstrating the 18 putatively transferred transcripts which were significantly enriched in experimental group (black dots). Candidate transferred transcripts were highlighted by arrows (ZNF81 and LINC00478). (B) Heatmap displaying the relative abundances of transcripts enriched in the experimental group compared to the negative control. The values presented on the heatmap are z-scores calculated based on the normalized read counts. Unsupervised hierarchical clustering of samples based on Euclidean distance calculated from presented z-scores is displayed alongside the heatmap. (C) Position of enriched intronic-LINC00478 and exonic-LINC00478 in relation to chromosome 21. (D) Position of enriched ZNF81 in relation to chromosome X. Copy number of EU-labeled (E) Intronic-LINC00478, (F) Exonic-LINC00478 and (G) ZNF81 were measured in endometrial cells co-incubated with EU-labeled trophoblast spheroids (Experimental group) by using qPCR and absolute quantification. Endometrial cells co-incubated with unlabeled trophoblast spheroids were used as a control (Negative control). Data is presented as mean ± SEM. (*) p < 0.05 vs negative control. (H) Presence of intronic-LINC00478 was observed in EU-labeled spheroid/endometrial cell co-culture conditioned media (Experimental group, E-CM) and extracted EVs (Experimental group, E-EV), and in EU-unlabeled spheroid/endometrial cell co-culture conditioned media (Negative control, NC-CM). Exonic-LINC00478 and ZNF81 were not detected in either group. Data is presented as mean ± SEM.

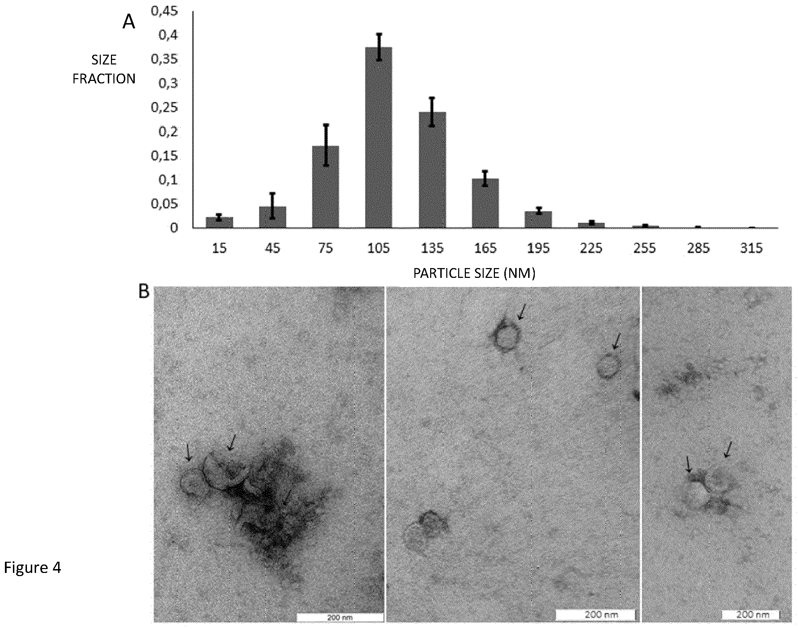

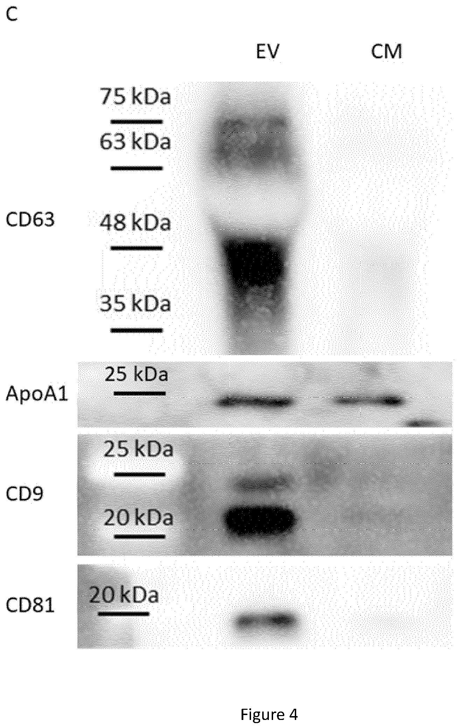

Figure 4 illustrates confirmation of trophoblast spheroid derived nanoparticles as extracellular vesicles (EVs). (A) Nanoparticle tracking analysis (NTA) of trophoblast spheroid derived extracellular vesicles (EVs). Number and size profiles of EVs were analyzed using ZetaView^{™} nanoparticle analyzer. The profile exhibits a typical distribution of particles mostly less than 200 nm. Data is presented as mean ± SEM. (B) The transmission electron microscopy (TEM) for EVs' morphology. EVs visualized after staining in 2% uranyl acetate following by uranyl oxalate and methylcellulose. Scale bar = 200 nm. Classic morphological characteristics, such as uniform shape, clearly discernible lipid bilayers and "cup shape", are observed. (C) Western blot analysis of trophoblast spheroid derived EVs (EV) and trophoblast spheroid conditioned media (CM). Specific protein markers for EVs (CD63, CD9 and CD81) are enriched in EV samples while negative control ApoA-I is not enriched.

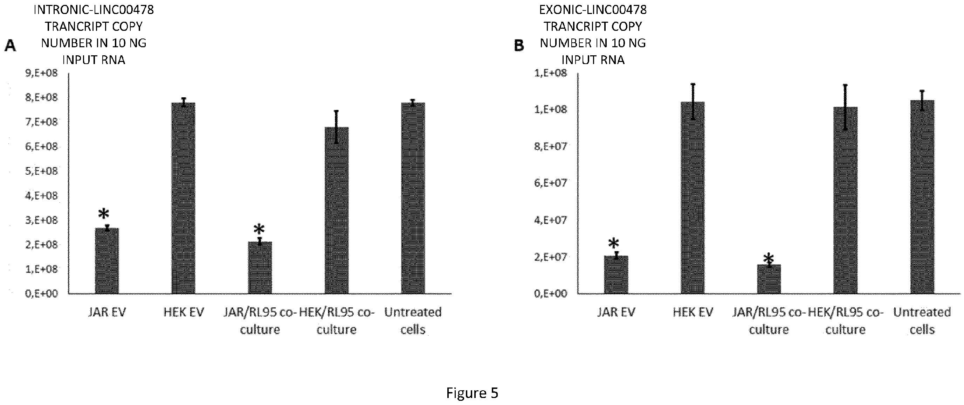

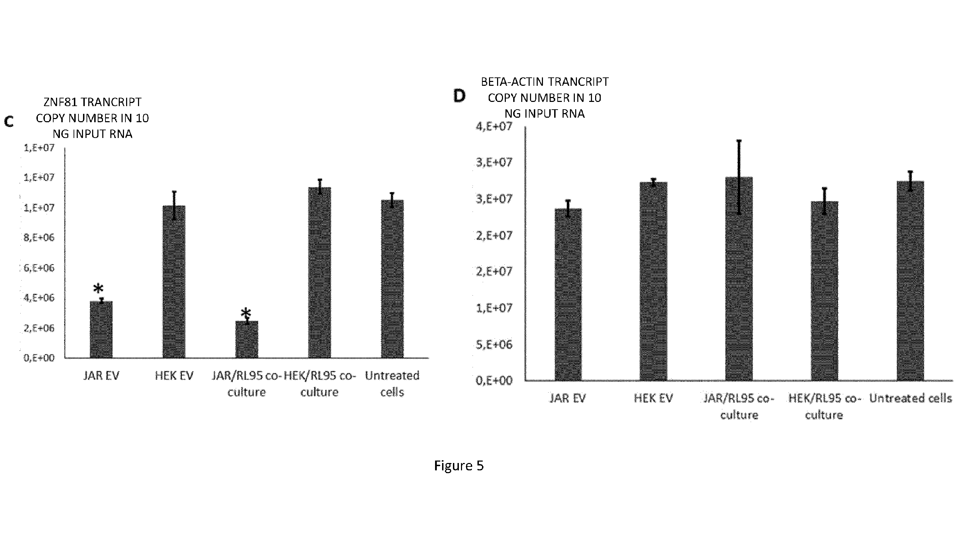

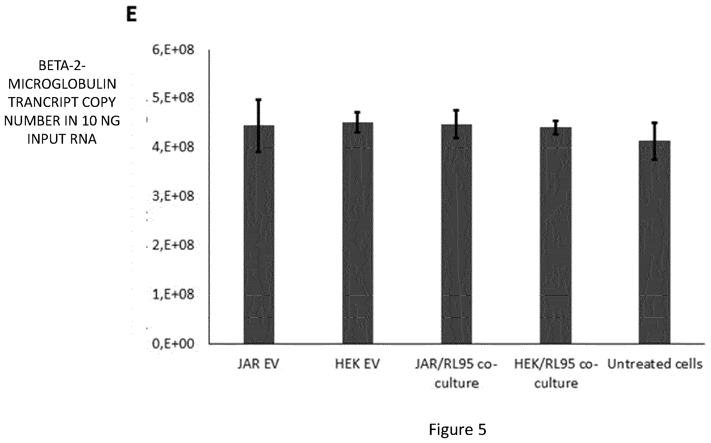

Figure 5 illustrates quantification of transferred and control transcripts' expressions in endometrial cells. Expressions of (A) intronic region of LINC00478, (B) exonic region of LINC00478, (C) ZNF81, (D) beta actin and (E) beta-2-microglobulin in endometrial cells in co-culture with trophoblast spheroids, co-culture with HEK293 spheroids, treated with JAr derived extracellular vesicles (EVs), treated with HEK293 derived EVs and untreated control. Spheroids were co-incubated with endometrial cell monolayer for 24 h. Isolated EVs were incubated with endometrial cells for 24 h. Whole RNA of endometrial cells was quantified using qPCR for expression of transferred/control transcripts. Data is presented as mean ± SEM. (*) p < 0.05 vs untreated control.

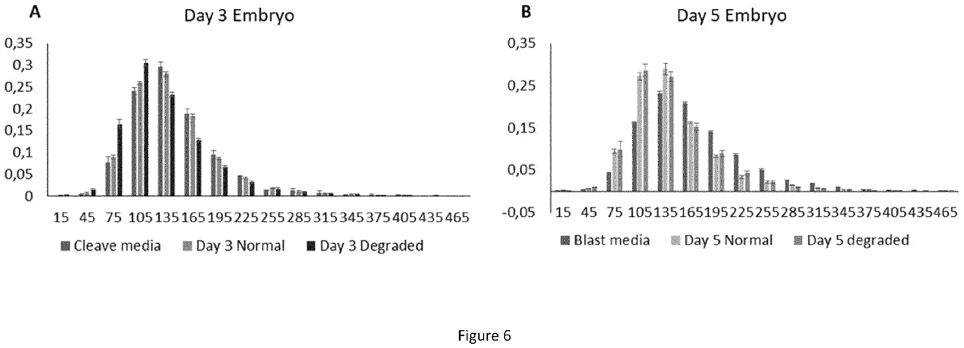

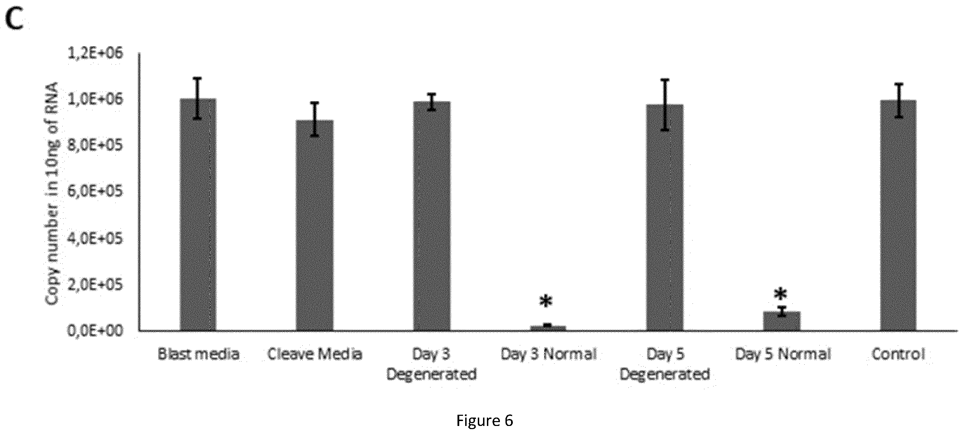

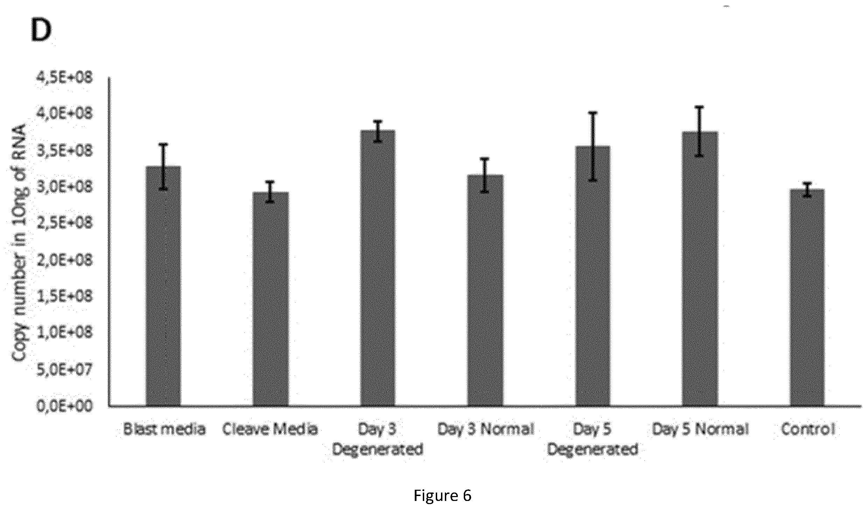

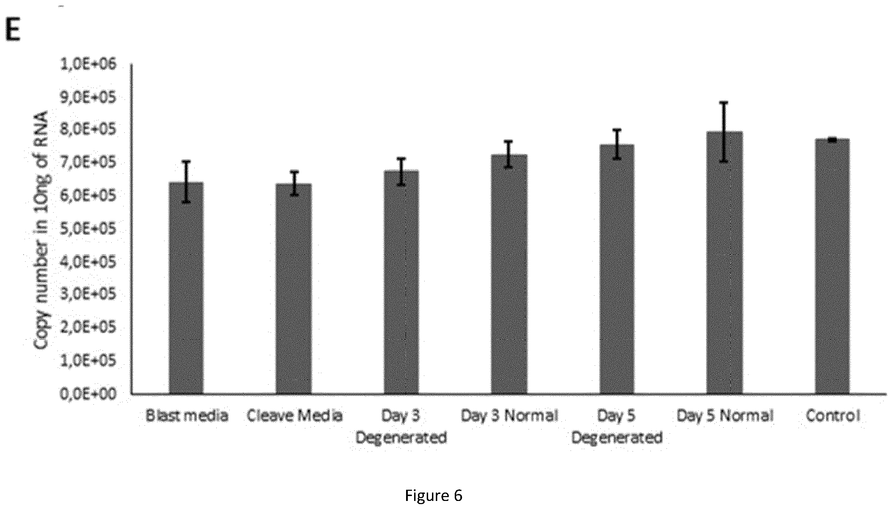

Figure 6 illustrates embryo-derived extracellular vesicles (EVs) alter the expression of specific transcripts in endometrial cells. (A, B) Size profiles of day 3 and day 5 embryo culture media derived nanoparticles strongly resemble a typical size profile of a population of comparable EVs. Gene expressions of (C) ZNF81, (D) beta-2-microglobulin and (E) beta actin in endometrial cells treated with human IVF day 3/5 normal/degenerating embryo-derived EVs, pure (un-used) culture media derived EVs and untreated control. Isolated EVs were incubated with endometrial cells for 24 h and whole RNA of cells was quantified using qPCR. Data is presented as mean ± SEM. (*) p < 0.05 vs untreated control.

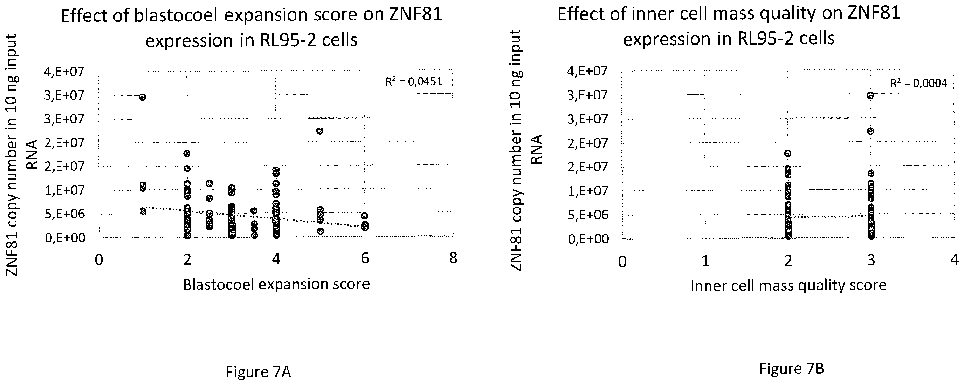

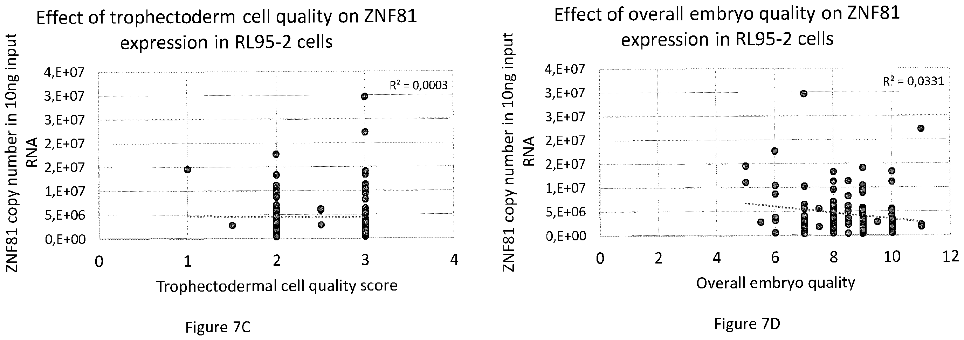

Figure 7 illustrates correlations between endometrial ZNF81 down regulation and embryo quality parameters on day 5. (A) Effect of blastocoel expansion score on *ZNF81* expression in endometrial RL95-2 cells. (B) Effect of inner cell mass quality on *ZNF81* expression in RL95-2 cells. (C) Effect of trophectoderm cell quality on *ZNF81* expression in RL95-2 cells. (D) Effect of overall embryo quality on *ZNF81* expression in RL95-2 cells.

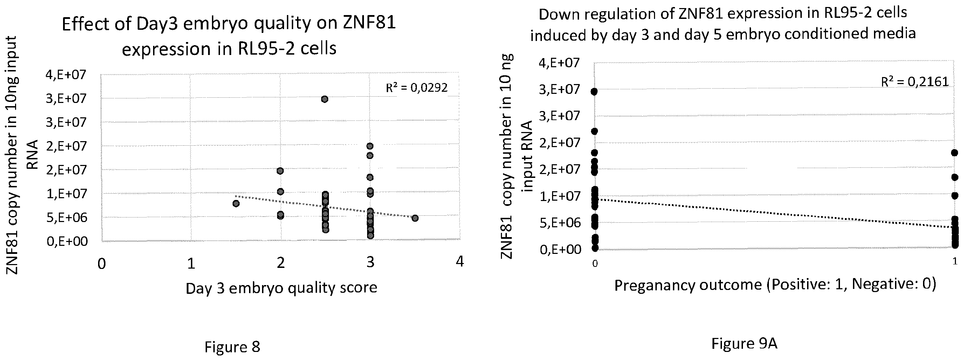

Figure 8 illustrates the effect of Day 3 embryo quality on *ZNF81* expression in RL95-2 cells.

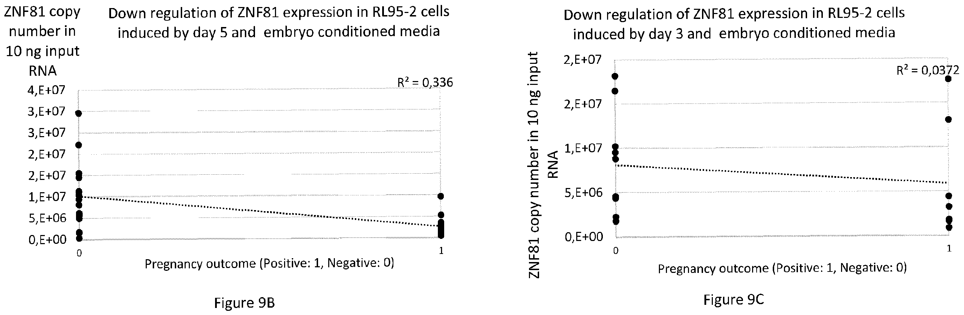

Figure 9 illustrates correlations between the outcome of embryo transfer and embryo conditioned media induced down regulation of *ZNF81* in RL95-2 cells. (A) Day 3 and 5 embryos conditioned media induced *ZNF81* down regulation in RL95-2 cells. (B) Day 5 blastocyst conditioned media induced *ZNF81* down regulation in RL95-2 cells. (C) Day 3 embryo conditioned media induced *ZNF81* down regulation in RL95-2 cells.

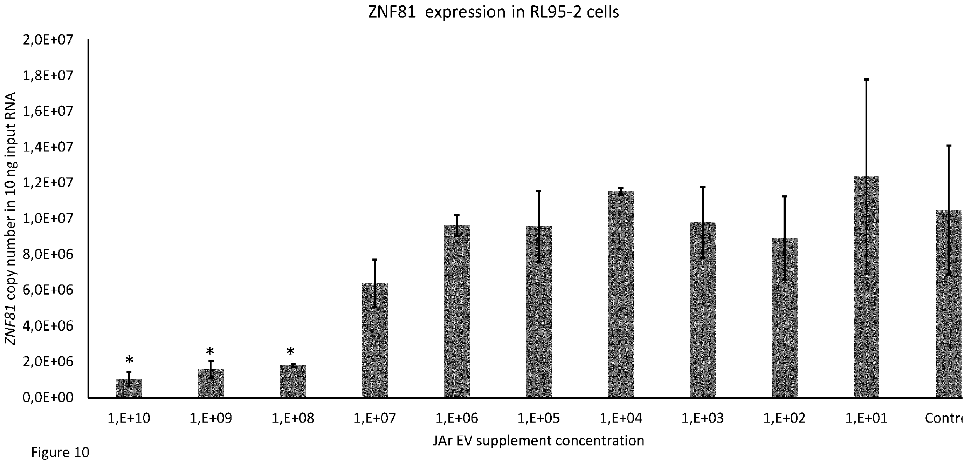

Figure 10 illustrates the amount of EV required to induce the down regulation of *ZNF81* in RL95-2 cells. Different concentrations of EVs were co-incubated with a unit number of RL95-2 cells for 24 hours and the expression of ZNF81 in RL95-2 cells was measured using quantitative PCR.

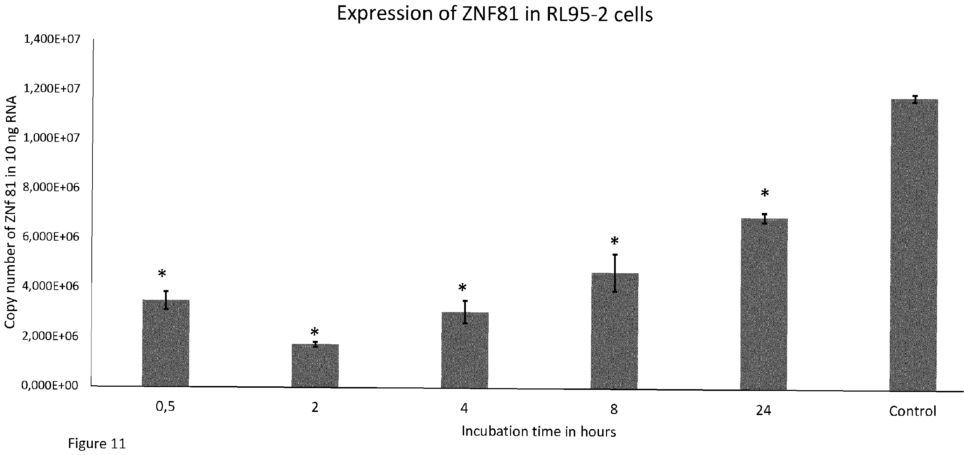

Figure 11 illustrates the duration of time required to induce the down regulation of *ZNF81* in RL95-2 cells. EVs were co-incubated with a unit number of RL95-2 cells for different time periods and the expression of ZNF81 in RL95-2 cells was measured using quantitative PCR.

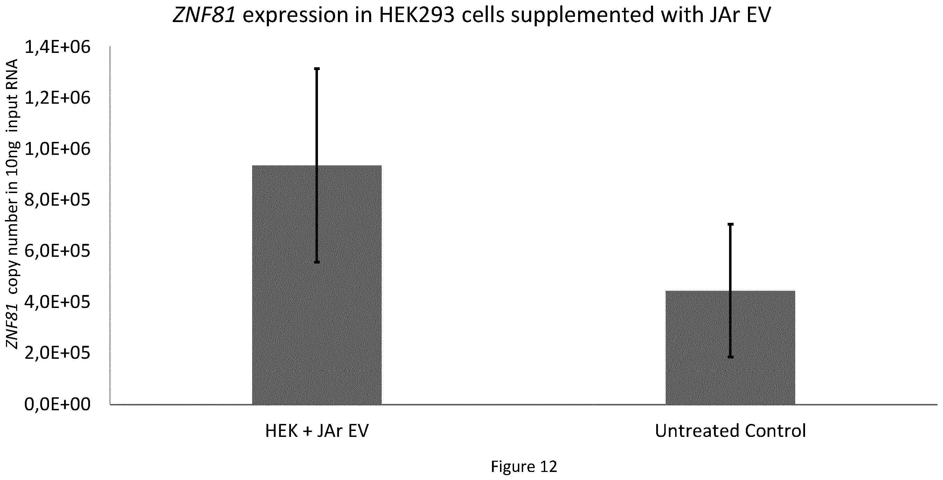

Figure 12 illustrates the effect of JAR EVs on HEK293 cells. 1 × 10⁸ JAR spheroid derived EVs were supplemented to a unit number of HEK293 cells for 24 hours and the expression of *ZNF81* in HEK293 cells was measured using quantitative PCR.

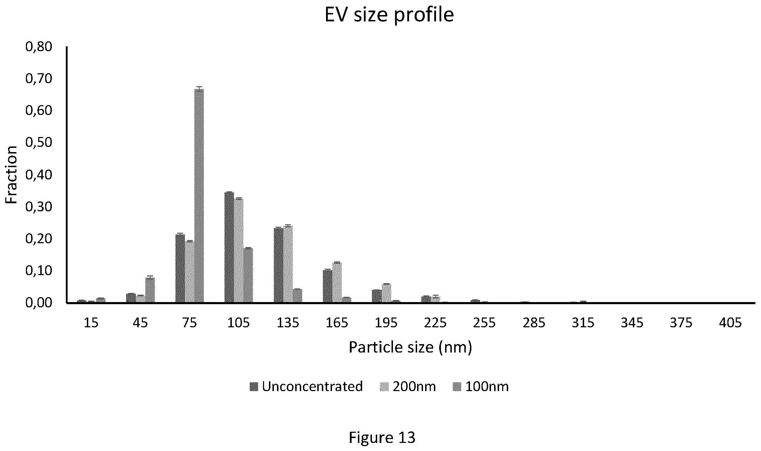

Figure 13 illustrates the results of filtering JAR spheroid derived EVs using 100 nm and 200 nm syringe filters. EV number was measured using nanoparticle tracking analysis. EV number for each length is expressed as a fraction of the whole EV number.

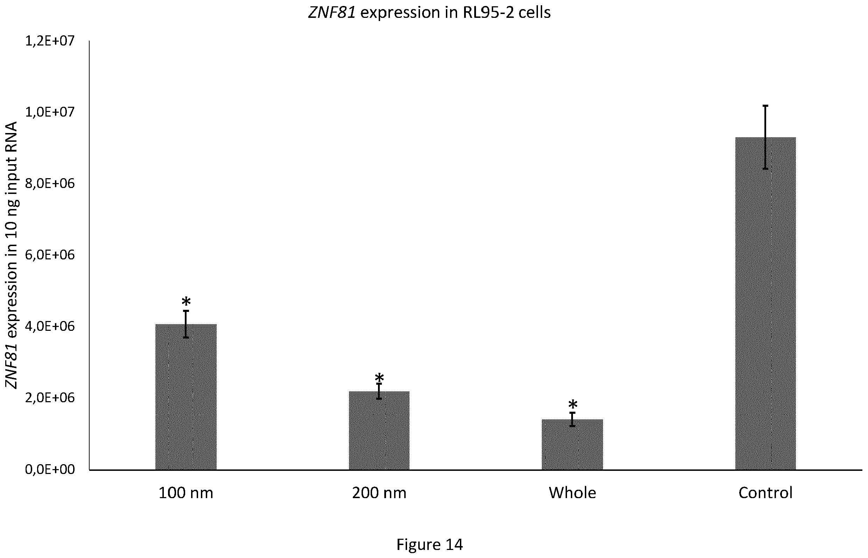

Figure 14 illustrates the effect of filtered JAR EVs on RL95-2 cells. 1 × 10⁸ JAR spheroid derived EVs were supplemented to a unit number of RL95-2 cells for 24 hours and the expression of *ZNF81* in RL95-2 cells was measured using quantitative PCR.

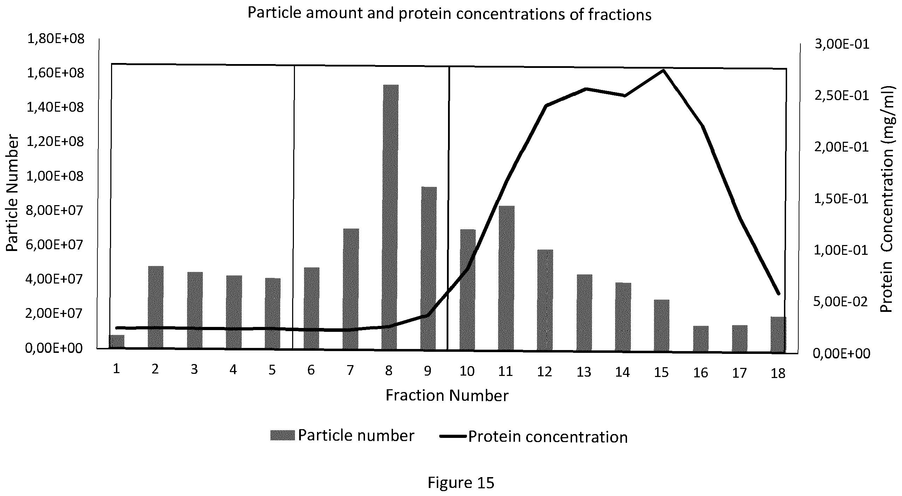

Figure 15 illustrates the distribution of EVs in the size exclusion chromatography fractions. 18 fractions were collected from size exclusion chromatography (fraction size 1 ml). All fractions were analyzed for the particle density using Nanoparticle tracking analysis and the protein concentration using Pierce^{™} modified Lowry protein assay kit (23240, Thermo Scientific, Rockford, IL, USA). Total particle number for each fraction is presented in the bar graph and the protein concentration of each fraction is presented in the line. The fractions can be divided as pre-EV (1-5), EV (6-9) and post-EV (10-18) depending on this result.

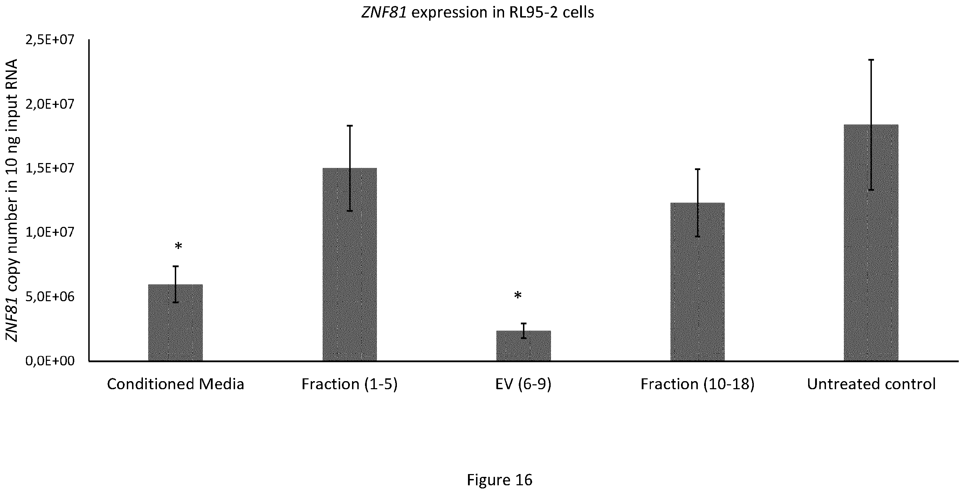

Figure 16 illustrates the effect of pre-EV, EV and post-EV fractions on RL95-2 cells. 1 × 10⁸ JAR spheroid derived "nanoparticles" from each group were supplemented to a unit number of RL95-2 cells for 24 hours and the expression of *ZNF81* in RL95-2 cells was measured using quantitative PCR.

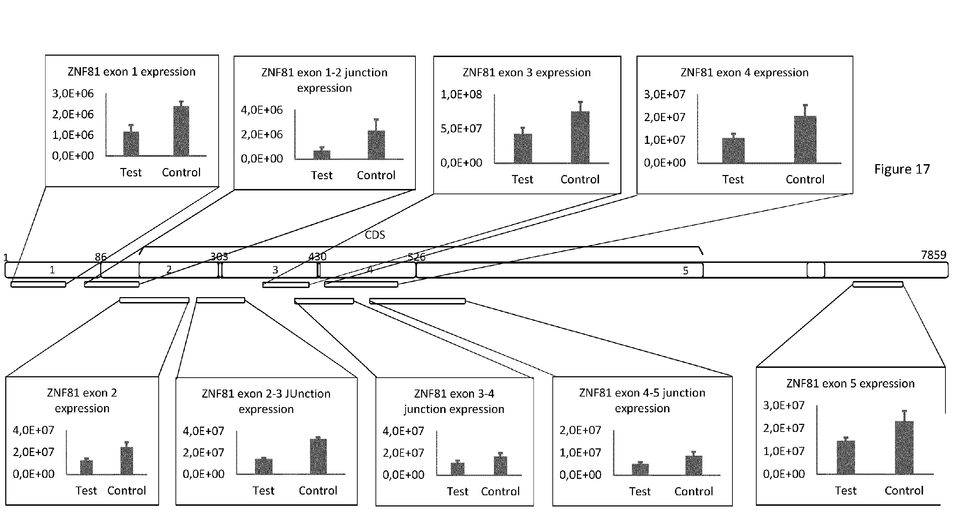

Figure 17 illustrates the *ZNF81* mature mRNA (5' to 3') with its five exons demarcated. Primers were designed to anneal to each exon and each exon-exon junction. RL95-2 cells were co-incubated with Jar spheroid derived EVs (Test) for 24 hours). Control samples were prepared using untreated RL95-2 cells (Control). Expression of each exon and *ZNF81* exon-exon junction in RL95-2 was measured using quantitative PCR.

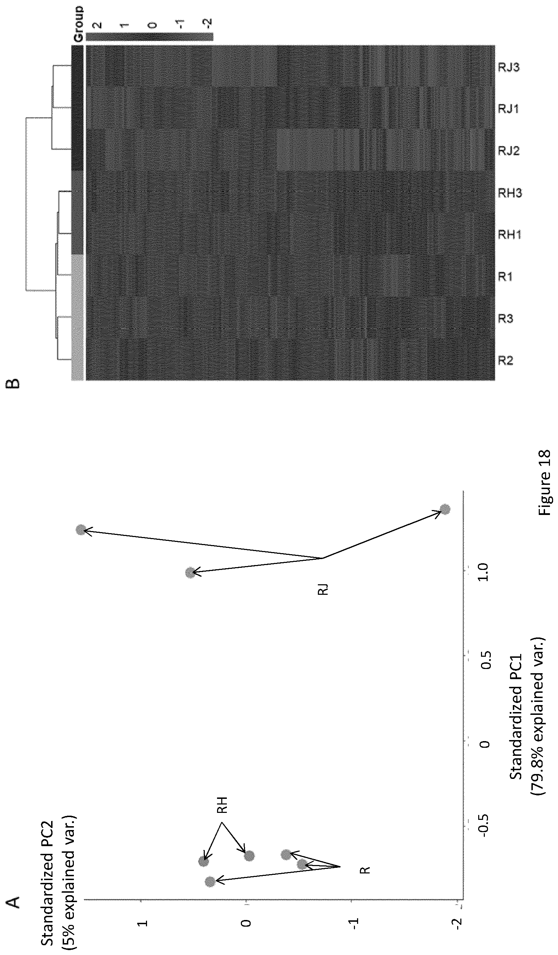

Figure 18 illustrates RL95-2 cells were supplemented with JAr EV and HEK293 EV. Cellular RNA was sequenced and differentially expressed genes (DEG) were determined compared to untreated RL95-2 RNA. Principle component analysis exhibits the significant variance between the JAr EV treated (RJ) and HEK293 EV treated (RH) groups. Untreated Group (R) is also highly dissimilar to JAr EV treated group while being very similar to HEK293 EV treated group (A). Heatmap shows the 1787 DEGs. 1196 significantly upregulated and 591 significantly downregulated genes. Only criterion for significance was FDR < 0.05.

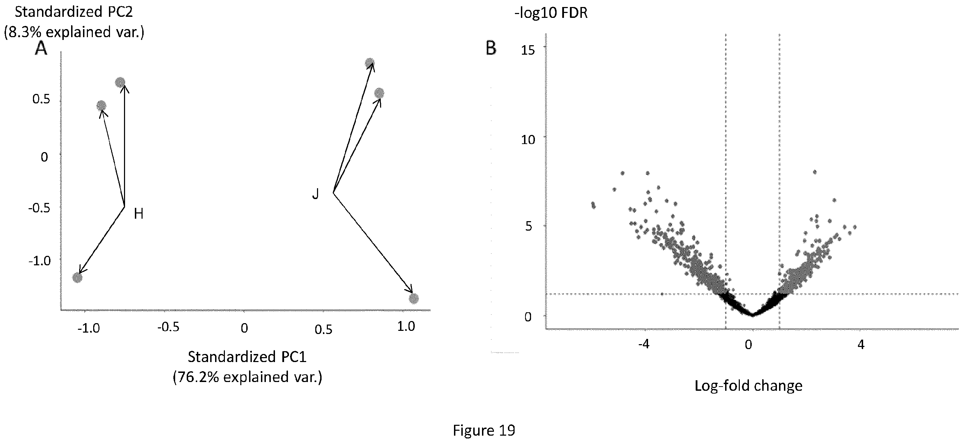

Figure 19 illustrates the contrast between RNA cargo of JAr EV and HEK293 EV. RNA from the EV were isolated and mRNA and miRNA were sequenced separately. Significant variance was observed between JAr EV and HEK293 EV mRNA (A). 400 mRNA were significantly enriched (logFC > 1) in JAr EV while 501 mRNA were significantly depleted (logFC <1) compared to HEK293 EV (B). Only criterion for significance was FDR <0.05.

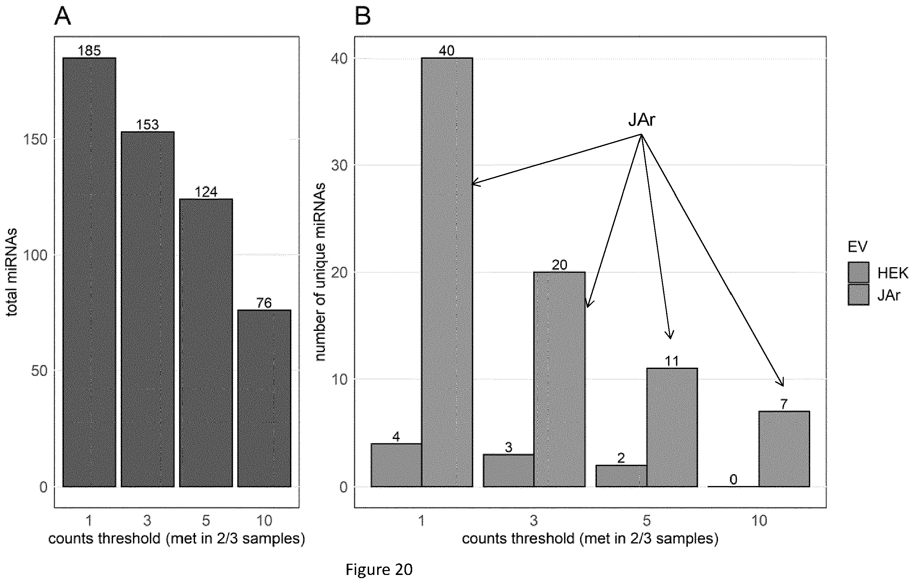

Figure 20 illustrates (A) number of microRNAs detected in at least 2/3 libraries of one of the two EV types at four raw counts thresholds, i.e., at a counts threshold of 10 each miRNA needed to be counted at least 10 times in 2/3 libraries of either HEK or JAr EVs. (B) Numbers of microRNAs considered to be unique to HEK or JAr EVs after passing four raw counts thresholds in at least 2/3 libraries of one of the two EV types. miRNAs were considered unique if they passed the required counts criteria for one EV type but were not detected at all in any of the libraries of the other EV type.

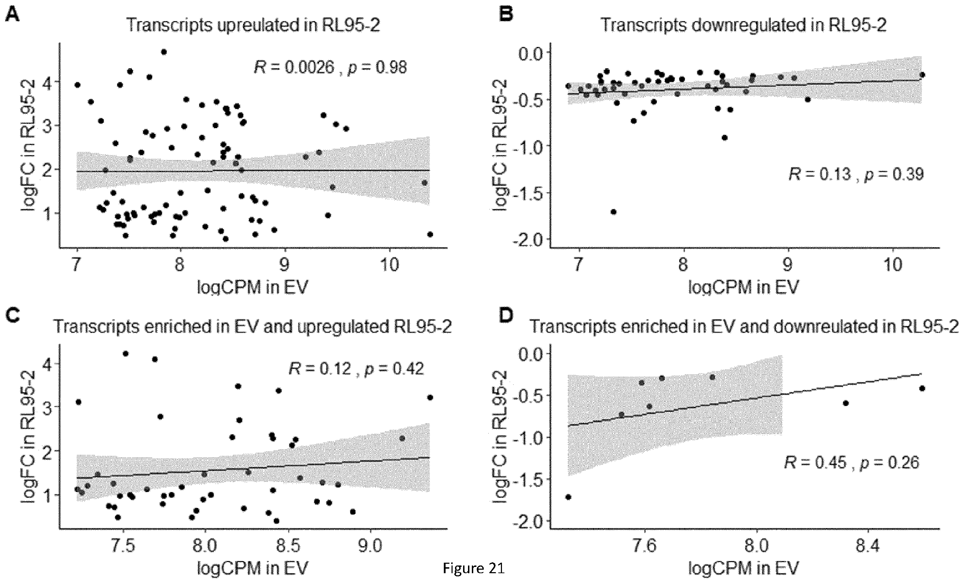

Figure 21 illustrates relationships between DEGs in RL95-2 and the abundance of the same transcripts in JAr EVs. No significant correlation existed in upregulated (A) genes or the downregulated (B) genes. Similarly, no significant correlations exist between genes that were upregulated in RL95-2 and significantly enriched in JAr EVs (C) or the genes that were significantly enriched in JAr EV but down regulated in RL95-2 cells (D). Correlations were done using Pearson's method. Respective coefficients (R) and the p values are presented within each graph. p<0.05 was considered significant. The results suggest that the previously observed down regulation of transferred RNA was not dependent on the abundance of RNA in EVs.

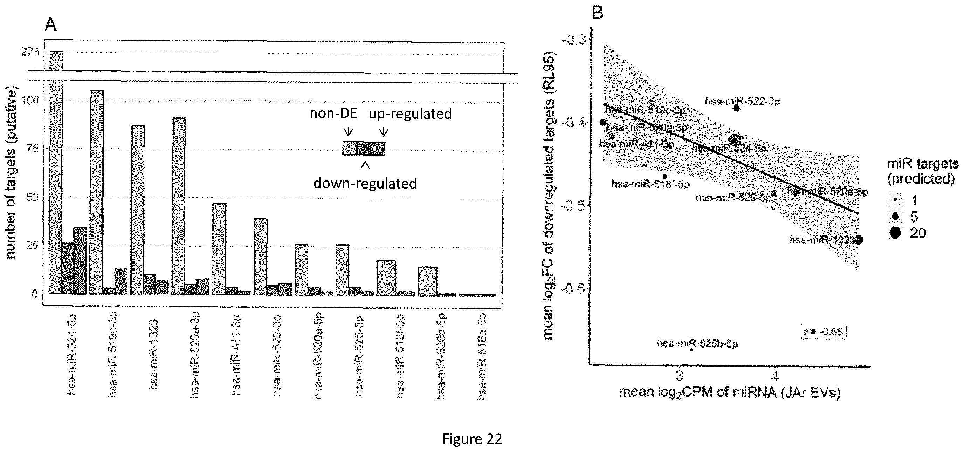

Figure 22 illustrates (A) eleven microRNAs identified as specific to JAr EVs and their corresponding numbers of putative high-confidence (miRDB target score ≥ 90) gene targets present in the RL95-2 gene expression dataset. Numbers of non-differentially expressed, down-regulated and upregulated target genes are shown. (B) relationship between abundance of JAr-specific microRNA in JAr EVs (expressed as mean log₂cpm, derived from three libraries) and the mean log₂FC of downregulated putative high-confidence targets in RL95-2 cells. The number of downregulated putative targets for each miRNA is represented by the point size.

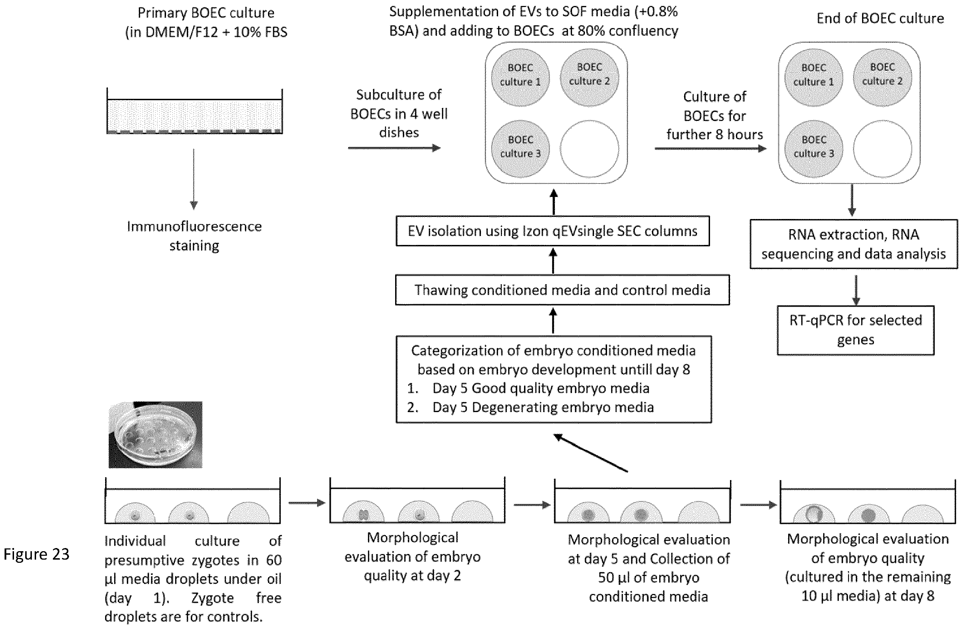

Figure 23 illustrates study design. BOEC: Bovine oviduct epithelial cells, DMEM/F12: Dulbecco's Modified Eagle Medium: Nutrient Mixture F-12, FBS: fetal bovine serum, IVF: *in vitro* fertilization, NTA: nanoparticle tracking analysis, TEM: transmission electron microscopy, EVs: extracellular vesicles.

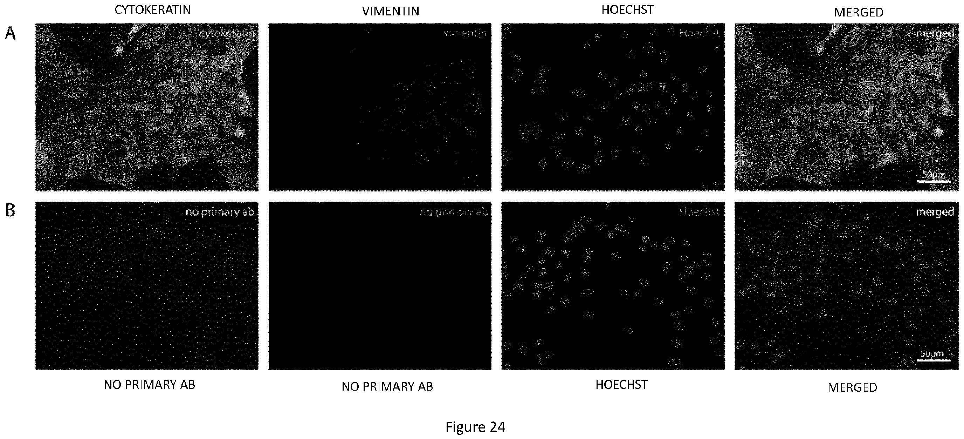

Figure 24 illustrates cytokeratin immunoflurescence staining of cultured BOEC monolayers demonstrating positive staining for the epithelial cell marker cytokeratin and negative staining for the fibroblast marker Vimentin. The cell nuclei were counterstained with Hoechst. (A) BOECs, (B) negative control cells. Magnification was set at 200X. The horizontal bar represents 50 µm.

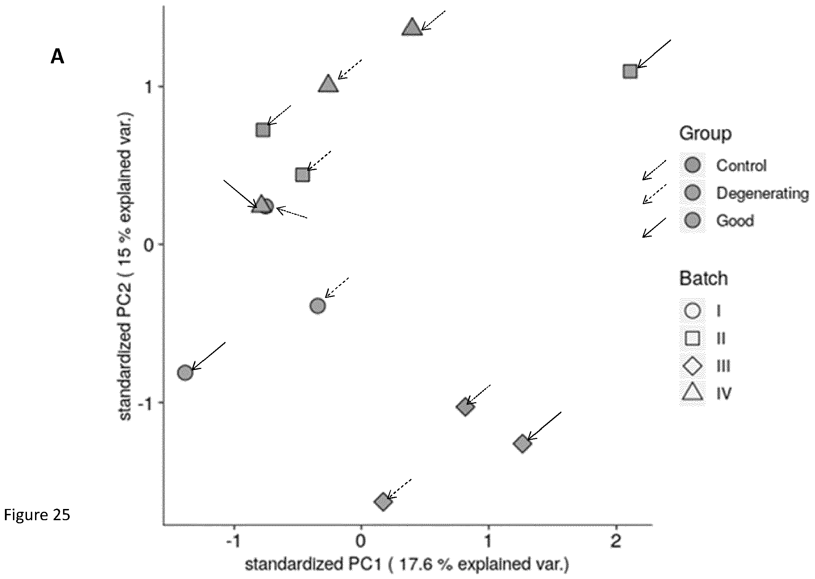

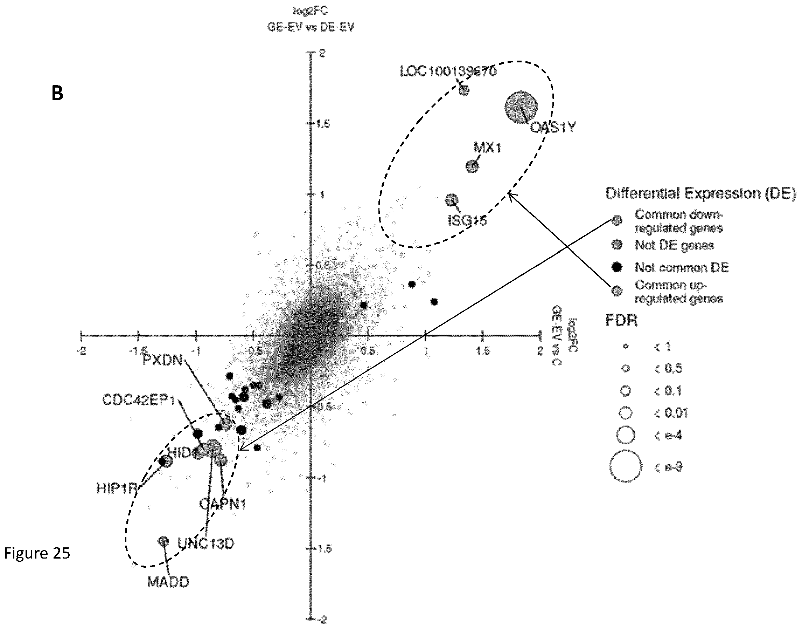

Figure 25 illustrates gene expression profile of the EV-supplemented and control oviductal monolayer culture samples. (A) Two leading principal components of standardised (z-score) counts per million (CPM) values of the expressed genes in the cultured bovine oviductal epithelial cells (BOECs) under control conditions (Control, dotted arrows) and following supplementation with EVs from degenerating embryos (Degenerating, hatched arrows) or EVs from good quality embryos (Good, full arrows). (B) The overlap of differentially expressed genes resulting from differential expression testing between (1) BOECs supplemented with EVs from good quality embryos (GE-EV) and control BOECs (C); (2) GE-EV and BOECs supplemented with EVs from degenerating embryos (DE-EV). The mean false discovery rate (FDR) of the two comparisons is presented on the figure.

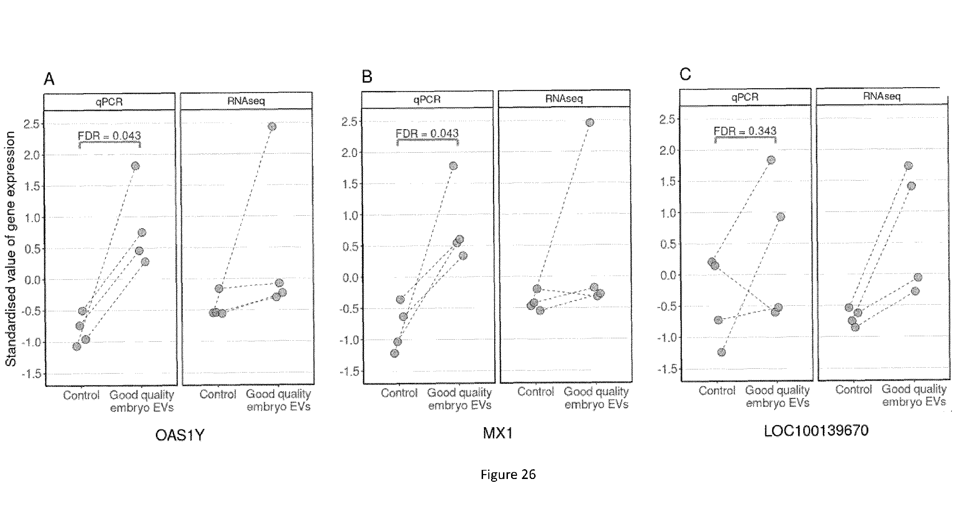

Figure 26 illustrates RT-qPCR based validation of the genes detected as differentially expressed based on RNAseq data. Standardized (z-score) -ΔΔqC and counts per million (CPM) values for the three upregulated genes: (A) OAS1Y, (B) MX1, and (C) LOC100139670. Relative mRNA expression of good quality day 5 bovine embryo-derived EV supplemented BOECs was analyzed with RT-qPCR in the same 4 replicates used for RNA sequencing. Replicates from the same experimental batch are connected by dashed lines on the figures. In the case of the RT-qPCR data, the groups were compared using Mann-Whitney U test with Benjamini-Hochberg Procedure to correct for multiple testing.

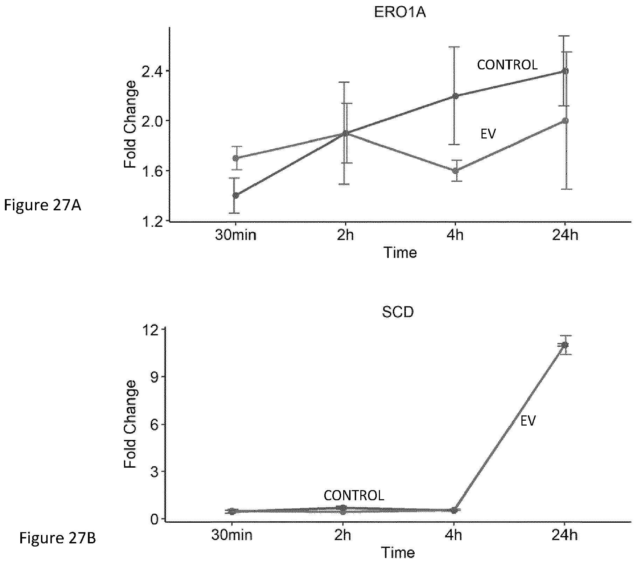

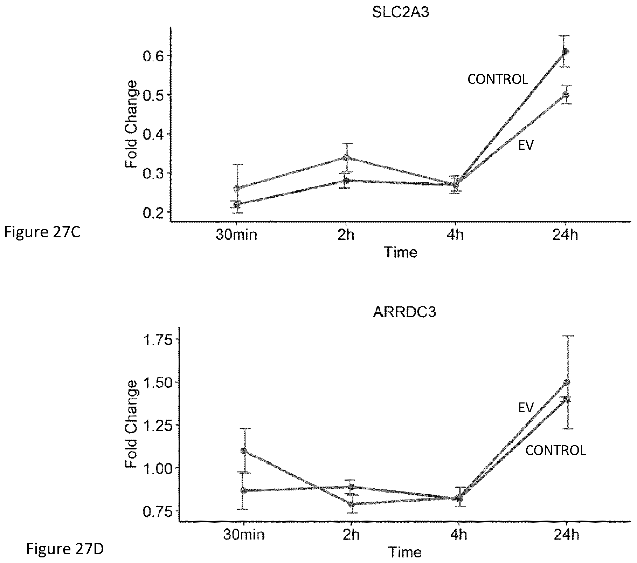

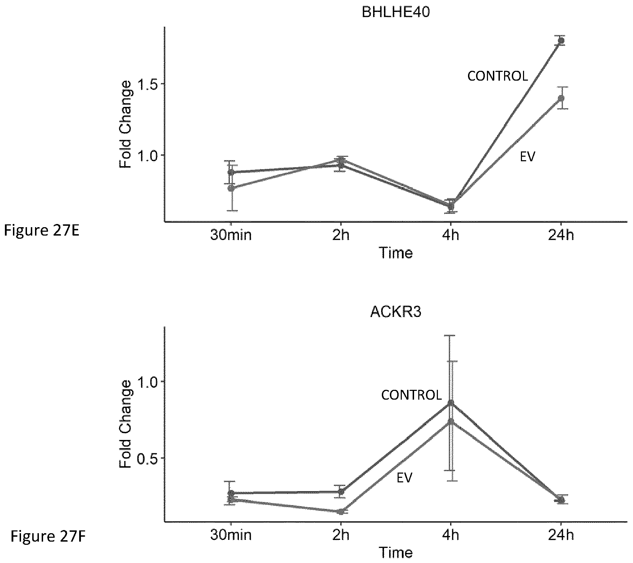

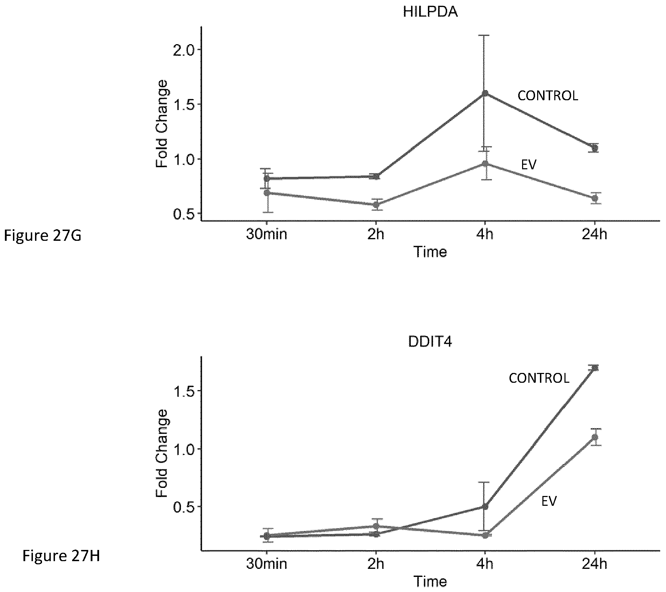

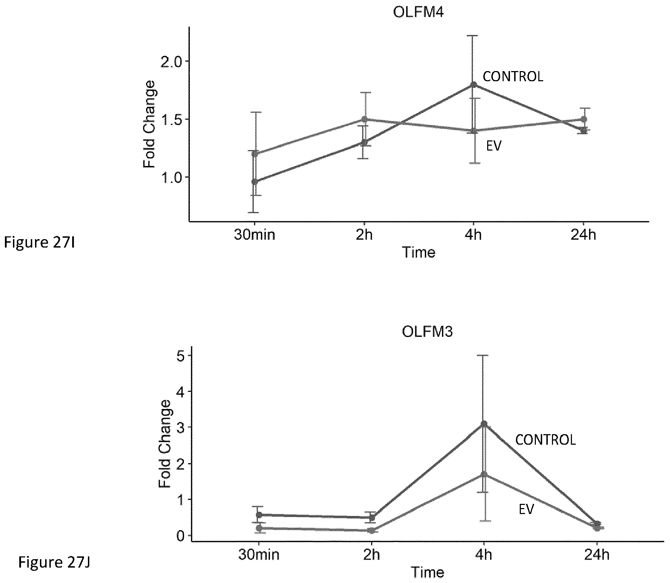

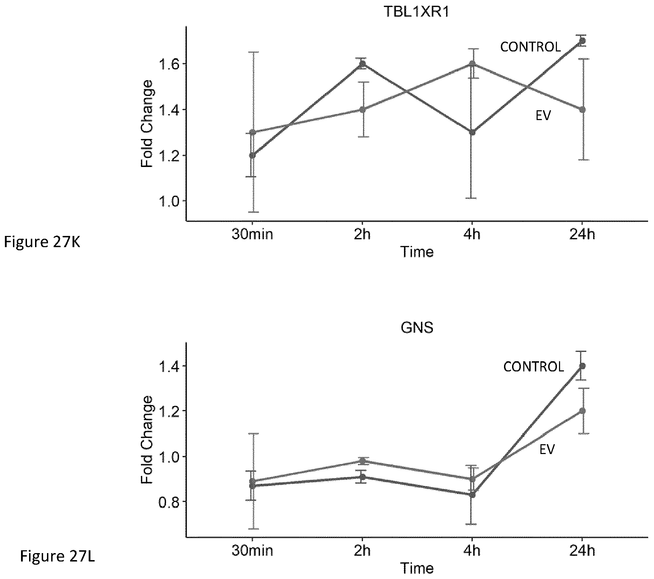

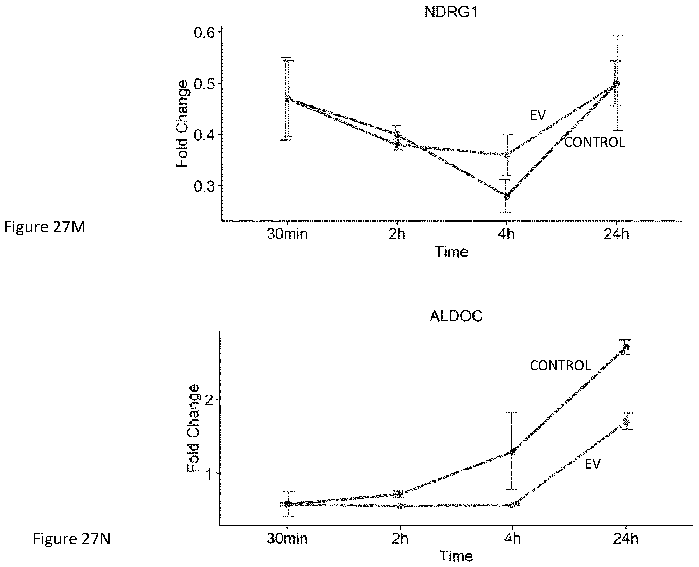

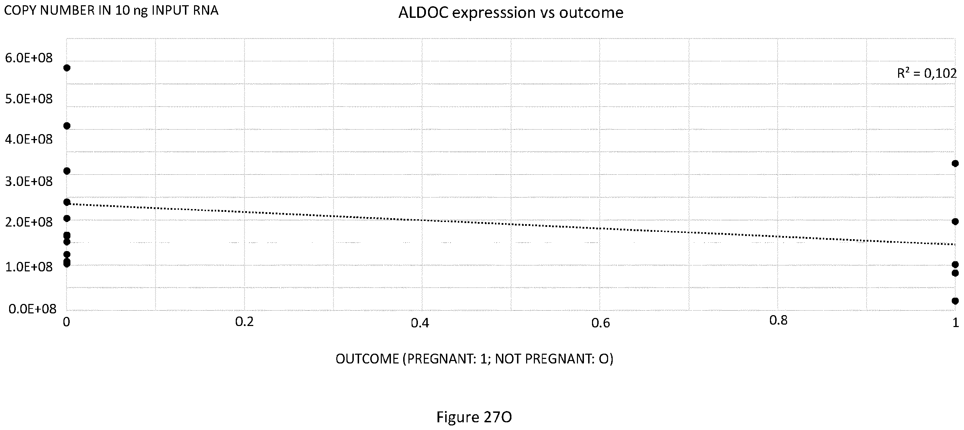

Figure 27 illustrates EV incubation time gradient for various genes in RL95-2 cells. EVs were co-incubated with a unit number of RL95-2 cells for different time periods and the expression of (A) *ERO1A,* (B) *SCD,* (C) *SLC2A3,* (D) *ARRDC3,* (E) *BHLHE40,* (F) *ACKR3,* (G) *HILPDA,* (H) *DDIT4,* (I) *OLFM4,* (J) *OLFM3,* (K) *TBL1XR1,* (L) *GNS,* (M) *NDRG1* and (N) *ALDOC* in RL95-2 cells was measured using quantitative PCR. (O) illustrates correlations between the outcome of embryo transfer and embryo conditioned medium down regulation of *ALDOC* in RL95-2 cells.

### DETAILED DESCRIPTION

Successful establishment of pregnancy hinges on appropriate communication between the embryo and the uterus prior to implantation, but the nature of this communication remains poorly understood. The present invention shows that the endometrium is receptive to embryo-derived signals in the form of ribonucleic acid (RNA). A non-contact co-culture system was used to simulate the conditions of pre-implantation environment of the uterus. Bioorthogonally tagged embryonic RNA were used to track the transferred transcripts to the endometrium. The transferred transcripts were separated from endometrial transcripts and sequenced. Changes in endometrial transcripts were quantified using quantitative polymerase chain reaction (qPCR). Eighteen transcripts as discovered by Next Generation Sequencing (NGS), including three specific transcripts that were further validated using qPCR to be transferred to the endometrial cells. Extracellular vesicles (EVs) were used in this transcript transfer process, as EVs obtained from cultured trophoblast spheroids incubated with endometrial cells induced down-regulation of all the three identified transcripts in endometrial cells. EVs/nanoparticles captured from conditioned culture media of viable embryos, as opposed to degenerating embryos, induced ZNF81 down-regulation in endometrial cells, showing the functional importance of this intercellular communication. This RNA-based communication is of critical importance for: 1) selecting the most viable IVF embryos for intrauterine transfer and 2) suppressing embryo rejection, i.e., increasing embryo implantation, to establish a pregnancy.

An aspect of the invention relates to a method of predicting pregnancy outcome of an embryo transfer in an *in vitro* fertilization (IVF) procedure, also referred to herein as an IVF embryo transfer procedure. The method comprises contacting *in vitro* responder cells with extracellular vesicles (EVs) isolated from an IVF embryo to be transferred into a female subject and/or with a conditioned medium from the IVF embryo. The method also comprises determining, in the responder cells, an amount of at least one ribonucleic acid (RNA) transcript. The method further comprises predicting pregnancy outcome of the embryo transfer based on the determined amount of the at least one RNA transcript.

Experimental data as presented herein indicates that high quality IVF embryos produces EVs and these EVs are released into the medium, in which the IVF embryos are comprised, see Figure 1. The EVs are in turn taken up by the responder cells and cause, therein a modulation in the amount of the at least one RNA transcript. This capability of being able to produce and release EVs that are taken up by responder cells and induce therein a change in the amount of the at least one RNA transcript is limited to high quality IVF embryos. Hence, degenerated IVF embryos of low or poor quality did not have this capability of inducing any significant change in the amount in the responder cells. This means that the ability of inducing such a change in the amount of the at least one RNA transcript in the responder cells following contacting the responder cells with the isolated EVs or the conditioned medium can be assessed or analyzed and used as a predictor of IVF embryo quality and thereby of pregnancy outcome since high quality IVF embryos are more likely to be successfully implanted and lead to pregnancy as compared to low quality IVF embryos.

The EVs released into surroundings can be isolated from the culture medium according to various embodiments. Non-limiting, but illustrative, embodiments of isolating EVs from the culture medium include subjecting the culture medium to one or more centrifugation steps, one or more filtration steps or a combination thereof. For instance, the culture medium could be subject to a first centrifugation step to form a cell pellet and an EV containing supernatant, such as by centrifuging at 400 × g for 10 min. The supernatant can then be subject to one or more centrifugation steps, preferably sequential centrifugation to deplete the cell debris and larger particles, such as a first centrifugation at 4,000 × g for 10 min followed by 10,000 × g for 10 min. The collected supernatant may then optionally be concentrated, such as using a centrifugal filter device, to get an EV concentrate. The EVs may be further isolated from the EV concentration using, for instance, size exclusion chromatography (SEC).

The conditioned medium can also be obtained according to various embodiments. For instance, the culture medium, in which the IVF embryo has been kept, can be subject to one or more centrifugation steps and/or filtration steps to remove cells from the conditioned medium while keeping any EVs produced by the IVF embryo in the conditioned medium.

Conditioned medium or media as used herein relate to medium or media, in which an IVF embryo has been cultured. The medium is preferably a culture medium selected to be adapted to comprise IVF embryos. Any such embryo medium could be used according to the embodiments including, but not limited to, Gibco DMEM/F-12 media, ETS-Embryo medium, artificial embryo culture media, advanced IVF media, etc. The IVF embryo present in the culture medium produces EVs that are released into the culture medium to thereby get a conditioned medium comprising EVs, see Figure 1.

In an embodiment, the isolated EVs comprises at least one RNA transcript and the conditioned medium comprises the at least one RNA transcript, preferably contained in EVs present in the conditioned medium. In a particular embodiment, determining the amount of the at least one RNA transcript comprises determining, in the responder cells, the amount of at least one of the at least one RNA transcript comprised in the isolated EVs and/or the conditioned medium. In this particular embodiment, the isolated EVs or the conditioned medium comprises one or more RNA transcripts, see Figure 1, produced by the IVF embryo and packaged into the EVs. The one or more RNA transcripts from the IVF embryo are taken up by the responder cells and induce therein a regulation in the amount of at least one RNA transcript in the responder cells.

The isolated EVs could comprise *m* different RNA transcripts, wherein *m* is an integer equal to or larger than one. The *m* different RNA transcripts could then induce, when taken up by the responder cells, a change in the amount of *n* different RNA transcripts, wherein *n* is an integer equal to or larger than one. In particular embodiments, *n* may be equal to or different than *m.* Thus, the isolated EVs or conditioned medium may comprise multiple different RNA transcripts and these may, when taken up by the responder cells, induce a change in one or more RNA transcripts, preferably corresponding RNA transcripts in the responder cells.

In more detail, the isolated EVs and conditioned medium may, for instance, contain RNA transcripts A and B. The isolated EVs and conditioned medium, when contacted with the responder cells, may then induce a change in the amount of RNA transcript A in the responder cells, a change in the amount of RNA transcript B in the responder cells or indeed a change in the amounts of both RNA transcript A and RNA transcript B in the responder cells. It is also possible that the isolated EVs and conditioned medium, when contacted with the responder cells, may induce a change in the amount of RNA transcript C in the responder cells even though the isolated EVs and the conditioned medium did not contain any RNA transcript C.

Contacting the responder cells *in vitro* with the isolated EVs or conditioned medium can be performed according to various embodiments. For instance, the isolated EVs may be added to the culture medium, in which the responder cells are kept. In the case of conditioned medium, the conditioned medium may be added to the culture medium, in which the responder cells are kept, or the responder cells may be added to the conditioned medium.

Generally, the concentration of EVs is higher in the isolated EVs as compared to the conditioned medium. However, isolation of EVs from the conditioned medium generally requires more process steps as compared to providing a conditioned medium. Experimental data as presented herein shows that the responder cells can be contacted *in vitro* either with the isolated EVs or the conditioned medium and still achieve a modulation in the at amount of the at least one RNA transcript in the responder cells, which can be determined and used to predict pregnancy outcome.

In an embodiment, determining the amount of the at least one RNA transcript comprises determining an amount of downregulation or upregulation of the at least one RNA transcript in the responder cells induced by the EVs and/or the conditioned medium. In this embodiment, predicting the pregnancy outcome of the embryo transfer comprises predicting the pregnancy outcome of the embryo transfer based on the determined amount of downregulation or upregulation of the at least one RNA transcript.

The isolated EVs and the conditioned medium may, thus, cause a downregulation in the amount of at least one RNA transcript in the responder cells in an embodiment. In another embodiment, the isolated EVs and the conditioned medium may cause an upregulation in the amount of at least one RNA transcript in the responder cells, whereas in a further embodiment, the isolated EVs and the conditioned medium may cause a downregulation in the amount of the at least one RNA transcript in the responder cells and an upregulation in the amount of the at least one RNA transcript in the responder cells.

A downregulation or an upregulation is preferably measured and determined as a fold change (FC) for a given RNA transcript. In such a case, the amount of the RNA transcript is determined in responder cells prior to contacting the responder cells with the isolated EVs or the conditioned medium. The amount of the RNA transcript is then determined in responder cells following contacting the responder cells *in vitro* with the isolated EVs or conditioned medium. The FC can then be determined as the ratio between the two quantities. A FC larger than one indicates an upregulation, a FC smaller than one indicates a downregulation, whereas a FC equal to one or close to one indicates no significant change in the amount of the at least one RNA transcript.

In an embodiment, predicting the pregnancy outcome of embryo transfer comprises predicting a high likelihood for successful embryo transfer and pregnancy of the female subject if the determined amount of the at least one RNA transcript or the determined FC for the at least one RNA transcript is equal to or below a threshold value and predicting a low likelihood for successful embryo transfer and pregnancy of the female subject if the determined amount of the at least one RNA transcript or the determined FC for the at least one RNA transcript is above threshold value.

In another embodiment, predicting the pregnancy outcome of embryo transfer comprises predicting a high likelihood for successful embryo transfer and pregnancy of the female subject if the determined amount of the at least one RNA transcript or the determined FC for the at least one RNA transcript is equal to or above a threshold value and predicting a low likelihood for successful embryo transfer and pregnancy of the female subject if the determined amount of the at least one RNA transcript or the determined FC for the at least one RNA transcript is below threshold value.

A related aspect of the invention defines to a method of predicting pregnancy outcome of an embryo transfer in an *in vitro* fertilization (IVF) procedure. The method comprises contacting *in vitro* responder cells with extracellular vesicles (EVs) isolated from an IVF embryo to be transferred into a female subject and/or with a conditioned medium from the IVF embryo. The method also comprises determining, in the responder cells, an amount of at least one ribonucleic acid (RNA) transcript. In this aspect, a significant change in the amount of the at least one RNA transcript relative to a reference level is indicative of high likelihood for successful embryo transfer and pregnancy of the female subject, whereas a non-significant change in the amount of the at least one RNA transcript relative to the reference level is indicative of low likelihood for successful embryo transfer and pregnancy of the female subject.

Reference level or amount of an RNA transcript as used herein corresponds to the amount of the RNA transcript in the responder cells prior to contacting the responder cells *in vitro* with the isolated EVs and/or the conditioned medium.

Hence, these aspects of the invention are based on that pregnancy outcome of embryo transfer can be predicted based on the capability of EVs isolated from IVF embryos and/or conditioned medium from such IVF embryos to induce modification in the amount of at least one RNA transcript. In more detail, when the EVs and/or conditioned medium induces a comparatively large modification (FC larger than or smaller than one) in the at least one RNA transcript this is an indication of high likelihood for successful embryo transfer for the given IVF embryo and thereby pregnancy of the female subject. Correspondingly, if the EVs and/or conditioned medium from an IVF embryo is not capable of inducing any large modification (FC close to one) in the at least one RNA transcript in the cells, then this is an indication of low or poor likelihood for successful embryo transfer and thereby low or poor likelihood for pregnancy of the female subject.

Another aspect of the invention relates to a method of determining a quality of an *in vitro* fertilization (IVF) embryo, as defined in the appended claims. The method comprises contacting *in vitro* responder cells with extracellular vesicles (EVs) isolated from the IVF embryo and/or with a conditioned medium from the IVF embryo. The method also comprises determining, in the responder cells, an amount of at least one ribonucleic acid (RNA) transcript. The method further comprises determining the quality of the IVF embryo based on the determined amount of the at least one RNA transcript.

A related aspect of the invention defines to a method of determining a quality of an *in vitro* fertilization (IVF) embryo, as defined in the appended claims. The method comprises contacting *in vitro* responder cells with extracellular vesicles (EVs) isolated from the IVF embryo and/or with a conditioned medium from the IVF embryo. The method also comprises determining, in the responder cells, an amount of at least one ribonucleic acid (RNA) transcript. In this aspect, a significant change in the amount of the at least one RNA transcript relative to a reference level is indicative of high quality of the IVF embryo, whereas a non-significant change in the amount of the at least one RNA transcript relative to the reference level is indicative of low or poor quality of the IVF embryo.

In an embodiment, the method comprises determining the IVF embryo to be good for intrauterine transfer into a female subject based on a significant change in the amount of the at least one RNA transcript relative to the reference level and determining the IVF embryo to be not good for intrauterine transfer into the female subject based on a non-significant change in the amount of the at least one RNA transcript relative to the reference level.

In an embodiment, determining the amount of the at least one RNA transcript comprises determining an amount of downregulation or upregulation of the at least one RNA transcript in the responder cells induced by the EVs and/or the conditioned medium. In this embodiment, determining the quality of the IVF embryo comprises determining the quality of the IVF embryo based on the determined amount of downregulation or upregulation of the at least one RNA transcript. In a particular embodiment, the amount of upregulation or downregulation is determined as a fold change for the given RNA transcript.

A further aspect of the invention relates to a method as defined in the appended claims, of selecting an embryo for an *in vitro* fertilization (IVF) procedure. The method comprises contacting *in vitro,* for each IVF embryo among multiple potential IVF embryos, responder cells with extracellular vesicles (EVs) isolated from the IVF embryo and/or a conditioned medium from the IVF embryo. The method also comprises determining, for each IVF embryo among the multiple potential IVF embryos and in the responder cells, an amount of at least one ribonucleic acid (RNA) transcript. The method further comprises selecting at least one IVF embryo among the multiple potential IVF embryos based on the respective determined amounts of the at least one RNA transcript.

In an embodiment, determining the amount of the at least one RNA transcript comprises determining, for each IVF embryo among the multiple IVF embryo, an amount of downregulation or upregulation of the at least one RNA transcript in the responder cells induced by the EVs and/or the conditioned medium. In this embodiment, selecting the at least one IVF embryo comprises selecting at least one IVF embryo among the multiple potential IVF embryos based on the respective determined amounts of downregulation or upregulations of the at least one RNA transcript. In a particular embodiment, the amount of upregulation or downregulation is determined as a fold change for the given RNA transcript.

In a particular embodiment, selecting the at least one IVF embryo comprises selecting the *N* IVF embryos resulting in a largest downregulation of the at least one RNA transcript, such as smallest FC, among *M* potential IVF embryos, wherein *M* < *M* and *M* is an integer equal to or larger than two.

In another particular embodiment, selecting the at least one IVF embryo comprises selecting the *N* IVF embryos resulting in a largest upregulation of the at least one RNA transcript, such as largest FC, among *M* potential IVF embryos, wherein *M* < *M* and *M* is an integer equal to or larger than two.

*M* is an integer equal to or larger than one but smaller than *M.*

High quality IVF embryos that can be selected according to the methods and that increase the likelihood of successful embryo transfer and pregnancy could induce an upregulation of at least one of the RNA transcripts, induce a downregulation of at least one of the RNA transcripts or induce an upregulation of at least one of the RNA transcripts and a downregulation of at least one of the RNA transcripts in the cells, depending on the particular RNA transcript to be measured.

The responder cells that are contacted *in vitro* with the EVs and/or the conditioned medium could be any responder or recipient cells that can be kept in *vitro.* In a particular embodiment, the responder cells are cells of a same species as the female subject and the IVF embryo. The responder cells are preferably of reproductive original, i.e., of reproductive lineage, and are thereby preferably so-called reproductive lineage cells. Such reproductive lineage cells include cells of the female genitals including, but not limited to, cells of the fallopian tubes, ovaries, uterus and/or the vagina. A currently preferred cell type to use in the methods of the invention is endometrial cells. Any endometrial cell or cells could be used according to invention including, but not limited to, human endometrial RL95-2 cells.

In an embodiment, determining the amount of the at least one RNA transcript comprises determining, in the responder cells or for each IVF embryo among the multiple IVF embryos and in the responder cells, the amount of the at least one RNA transcript selected for the group consisting of a mucin 4 *(MUC4)* transcript, a *MUC3A* transcript, a *MUC16* transcript, a *MUC12* transcript, a zinc finger protein 81 (*ZNF81*) transcript, a Ras-related GTP-binding protein B (*RRAGB*) transcript, a mitochondrially encoded tRNA tryptophan (*MT-TW*) transcript, a Z95704.5 transcript, a mitochondrially encoded tRNA serine 1 *(MT-TS1)* transcript, an integrin, alpha E (*ITGAE*) transcript, a RP11-357C3.3 transcript, a transmembrane protein 154 *(TMEM154)* transcript, a caspase 14 *(CASP14)* transcript, a *ZNF765* transcript, a long intergenic non-protein coding RNA 478 (LINC00478) transcript, a mitochondrially encoded tRNA glutamine *(MT-TQ)* transcript, an ankyrin repeat domain-containing protein 44 *(ANKRD44)* transcript, and a zinc finger BED-type containing 3 - antisense RNA 1 *(ZBED3-AS1)* transcript.

In an embodiment, determining the amount of the at least one RNA transcript comprises determining, in the responder cells or for each IVF embryo among the multiple IVF embryos and in the responder cells, the amount of at least one RNA transcript selected among the group consisting of a transcript of an intronic region of LINC00478, a transcript of an exonic region of LINC00478 and a transcript of an exonic region of *ZNF81.*

In a particular embodiment, determining the amount of the at least one RNA transcript comprises determining, in the responder cells or for each IVF embryo among the multiple IVF embryos and in the responder cells, the amount of the at least one RNA transcript comprising an RNA sequence selected from the group consisting of: 
UGAUACAGAAGACUUGAGAUUCUGGAUUGGAGCUUGAUGCCACAAUUUUGGAUGAGAAAUUUG
GAGGUCCUGGAAUAG (SEQ ID NO: 17);
 
UCAAGUUCAGUGUUUGGUUAAAAUACAUACUCAGUAAAUGGUAGCUAUUAUUGUCUUAGUUUAA
GUUAUUGCAAGCAUUAAAAUUAAAUGUUUAGCUACAGACUCAAUCCAGUUUUAAUGUCAUUGUG
UUAAUAAGGCCUCUUAACAUUGAAGCAACAAAGA (SEQ ID NO: 18);
 
AACAGGUCACAAUGGUGGAAUGUCGUCAGCUAAGGCAGGACCUGGCUAUUUGCACUUCUUUUG
UGGAUCUUCAGUUGCUUCA (SEQ ID NO: 19); or
 an RNA sequence complementary to any of SEQ ID NO: 17 to 19.

In another embodiment, determining the amount of the at least one RNA transcript comprises determining, in the responder cells or for each IVF embryo among the multiple IVF embryos and in the responder cells, the amount of at least one RNA transcript selected from the group consisting of an ER oxidoreductin 1 alpha (*ERO1A*) transcript, a stearoyl-CoA desaturase (*SCD)* transcript, a solute carrier family 2, facilitated glucose transporter member 3 (*SLC2A3*) transcript, an arrestin domain containing 3 (*ARRDC3*) transcript, a class E basic helix-loop-helix protein 40 *(BHLHE40)* transcript, an atypical chemokine receptor 3 (*ACKR3*) transcript, a hypoxia inducible lipid droplet-associated (*HILPDA*) transcript, a DNA-damage-inducible transcript 4 *(DDIT4*) transcript, an olfactomedin 4 (*OLFM4*) transcript, an OLFM3 transcript, a F-box-like/WD repeat-containing protein (*TBL1XR1*) transcript, a glucosamine (N-acetyl)-6-sulfatase (*GNS*) transcript, a N-myc downstream regulated 1 (*NDRG1*) transcript and an aldolase C, fructose-bisphosphate (*ALDOC*) transcript, preferably an *ALDOC* transcript.

In a particular embodiment, determining the amount of the at least one RNA transcript comprises determining, in the responder cells or for each IVF embryo among the multiple IVF embryos and in the responder cells, the amount of at least one RNA transcript selected among the group consisting of a transcript of an intronic region of LINC00478, a transcript of an exonic region of LINC00478, a transcript of an exonic region of *ZNF81* and *ALDOC* transcript.

In a further embodiment, determining the amount of the at least one RNA transcript comprises determining, in the responder cells or for each IVF embryo among the multiple IVF embryos and in the responder cells, the amount of at least one RNA transcript selected from the group consisting of an 2'-5' oligoadenylate synthase (*OAS1Y*) transcript, an interferon-induced GTP-binding protein (*MX1*) transcript, an interferon-induced protein with tetratricopeptide repeats 1 (*LOC100139670*) transcript, an interferon-stimulated gene 15 (*ISG15*), an ENSBTAG00000051364 transcript, an ENSBTAG00000053545 transcript, a cytochrome P450, family 1, subfamily A, polypeptide 1 (*CYP1A1*) transcript, an alkB homolog 4, alpha-ketoglutarate dependent dioxygenase (*ALKBH4*) transcript, a MAP kinase-activating death domain protein (*MADD*) transcript, a Huntingtin-interacting protein 1-related protein (*HIP1R*)*,* a chromosome 28 C1 open reading frame 198 (*C28H1orf198*) transcript, a HID1 domain containing (*HID1*) transcript, a Cdc42 effector protein 1 (*CDC42EP1*) transcript, a protein unc-13 homolog D (*UNC13D*) transcript, an aldehyde dehydrogenase 16 family, member A1 (*ALDH16A1*) transcript, a calpain-1 catalytic subunit (CAPN1) transcript, a peroxidasin homolog (*PXDN*)*,* an ENSBTAG00000043565 transcript, a cleavage and polyadenylation specificity factor subunit 1 *(CPSF1)* transcript, a HGH1 homolog (*HGH1*) transcript, a Rho guanine nucleotide exchange factor 2 *(ARHGEF2)* transcript, a laminin subunit beta-3 (LAMB3) transcript, a follistatin-related protein 3 (*FSTL3*) transcript and a rhomboid family member 2 (*RHBDF2*) transcript.

In a particular embodiment, determining the amount of the at least one RNA transcript comprises determining, in the responder cells or for each IVF embryo among the multiple IVF embryos and in the responder cells, the amount of at least one RNA transcript selected from the group consisting of an *OAS1Y* transcript, a *MX1* transcript, a *LOC100139670* transcript, an ISG15 transcript, a *CYP1A1* transcript, an *ALKBH4* transcript, a *MADD* transcript, a *HIP1R* transcript, a *C28H1orf198* transcript, a *HID1* transcript, a *CDC42EP1* transcript, an *UNC13D* transcript, an *ALDH16A1* transcript, a *CAPN1* transcript, a *PXDN* transcript, a *CPSF1* transcript, a *HGH1* transcript, an *ARHGEF2* transcript, a *LAMB3* transcript, a *FSTL3* transcript and a *RHBDF2* transcript.

In another particular embodiment, determining the amount of the at least one RNA transcript comprises determining, in the responder cells or for each IVF embryo among the multiple IVF embryos and in the responder cells, the amount of at least one RNA transcript selected from the group consisting of a *LOC100139670* transcript, an *OAS1Y* transcript, a *MX1* transcript, an *ISG15* transcript, a *MADD* transcript, a *HIP1R* transcript, a *CAPN1* transcript, a *HID1* transcript, a *CDC42EP1* transcript, an *UNC13D* transcript, a *PXDN* transcript, an 1-acyl-sn-glycerol-3-phosphate acyltransferase alpha *(AGPAT1)* transcript, a Bcl-2 homologous antagonist/killer (*BAK1*) transcript, a large neutral amino acids transporter small subunit 2 (*SLC7A8*) transcript and a tissue transglutaminase (*TGM2*) transcript.

The above described RNA transcripts are present in EVs and conditioned medium of IVF embryos and are transferred into the responder cells as shown in Figure 1. Thus, the at least one RNA transcript is advantageously selected from the above described group of RNA transcripts.

Another aspect of the invention is directed towards a method as defined in the appended claims, of determining a quality of an *in vitro* fertilization (IVF) embryo. The method comprises determining an amount of at least one ribonucleic acid (RNA) transcript selected for the group consisting of a mucin 4 *(MUC4)* transcript, a *MUC3A* transcript, a *MUC16* transcript, a *MUC12* transcript, a zinc finger protein 81 *(ZNF81)* transcript, a Ras-related GTP-binding protein B (RRAGB) transcript, a mitochondrially encoded tRNA tryptophan (*MT-TW*) transcript, a Z95704.5 transcript, a mitochondrially encoded tRNA serine 1 *(MT-TS1)* transcript, an integrin, alpha E (*ITGAE*) transcript, a RP11-357C3.3 transcript, a transmembrane protein 154 *(TMEM154)* transcript, a caspase 14 *(CASP14)* transcript, a *ZNF765* transcript, a long intergenic non-protein coding RNA 478 (LINC00478) transcript, a mitochondrially encoded tRNA glutamine *(MT-TQ)* transcript, an ankyrin repeat domain-containing protein 44 *(ANKRD44)* transcript, and a zinc finger BED-type containing 3 - antisense RNA 1 *(ZBED3-AS1)* transcript in extracellular vesicles (EVs) isolated from the IVF embryo and/or in a conditioned medium from the IVF embryo. The method also comprises determining the quality of the IVF embryo based on the determined amount of the at least one RNA transcript.

A further aspect of the invention relates to a method as defined in the appended claims, of selecting an embryo for an *in vitro* fertilization (IVF) procedure. The method comprises determining, for each IVF embryo among multiple potential IVF embryos, a respective amount of at least one ribonucleic acid (RNA) transcript selected for the group consisting of a mucin 4 *(MUC4)* transcript, a *MUC3A* transcript, a *MUC16* transcript, a *MUC12* transcript, a zinc finger protein 81 *(ZNF81)* transcript, a Ras-related GTP-binding protein B (*RRAGB*) transcript, a mitochondrially encoded tRNA tryptophan (*MT-TW*) transcript, a Z95704.5 transcript, a mitochondrially encoded tRNA serine 1 *(MT-TS1)* transcript, an integrin, alpha E (*ITGAE*) transcript, a RP11-357C3.3 transcript, a transmembrane protein 154 *(TMEM154)* transcript, a caspase 14 *(CASP14)* transcript, a *ZNF765* transcript, a long intergenic non-protein coding RNA 478 (LINC00478) transcript, a mitochondrially encoded tRNA glutamine *(MT-TQ)* transcript, an ankyrin repeat domain-containing protein 44 *(ANKRD44)* transcript, and a zinc finger BED-type containing 3 - antisense RNA 1 *(ZBED3-AS1)* transcript in extracellular vesicles (EVs) isolated from the IVF embryo and/or in a conditioned medium from the IVF embryo. The method also comprises selecting at least one IVF embryo among the multiple potential IVF embryos based on the respective amounts of the at least one RNA transcript.

The above described methods are based on the finding that good quality IVF embryos produces RNA transcripts as identified above and package these RNA transcripts into EVs and release them into the surrounding culture medium when kept *in vitro* in a culture medium. Hence, measuring the amount of at least one of the RNA transcript in the isolated EVs and/or in the conditioned medium from the IVF embryo can be used in determining the quality of the IVF embryo and in selecting IVF embryos. Alternatively, the at least one RNA transcript in these aspects of the invention is an RNA transcript from a gene selected from Table 13, Table 24 or among the genes shown in Figures 27A to 27N.

Disclosed, but not part of the claimed invention, is a nucleic acid molecule comprising at least one nucleotide sequence selected from the group consisting of a mucin 4 *(MUC4)* transcript, a complementary deoxyribonucleic acid (cDNA) of the *MUC4* transcript, a *MUC3A* transcript, a cDNA of the *MUC3A* transcript, a *MUC16* transcript, a cDNA of the *MUC16* transcript, a *MUC12* transcript, a cDNA of the *MUC12* transcript, a zinc finger protein 81 *(ZNF81)* transcript, a cDNA of the *ZNF81* transcript, a Ras-related GTP-binding protein B (*RRAGB*) transcript, a cDNA of the RRAGB transcript, a mitochondrially encoded tRNA tryptophan (*MT-TW*) transcript, a cDNA of the *MT-TW* transcript, a Z95704.5 transcript, a cDNA of the Z95704.5 transcript, a mitochondrially encoded tRNA serine 1 *(MT-TS1)* transcript, a cDNA of the *MT-TS1* transcript, an integrin, alpha E (*ITGAE*) transcript, a cDNA of the *ITGAE* transcript, a RP11-357C3.3 transcript, a cDNA of the RP11-357C3.3 transcript, a transmembrane protein 154 *(TMEM154)* transcript, a cDNA of the *TMEM154* transcript, a caspase 14 *(CASP14)* transcript, a cDNA of the *CASP14* transcript, a *ZNF765* transcript, a cDNA of the *ZNF765* transcript, a long intergenic non-protein coding RNA 478 (LINC00478) transcript, a cDNA of the LINC00478 transcript, a mitochondrially encoded tRNA glutamine *(MT-TQ)* transcript, a cDNA of the *MT-TQ* transcript, an ankyrin repeat domain-containing protein 44 *(ANKRD44)* transcript, a cDNA of the *ANKRD44* transcript, a zinc finger BED-type containing 3 - antisense RNA 1 *(ZBED3-AS1)* transcript, and a cDNA of the *ZBED3-AS1* transcript, for use in suppressing rejection of an embryo in an *in vitro* fertilization (IVF) procedure and/or for use in improving embryo implantation of an embryo in an *in vitro* fertilization (IVF) embryo transfer procedure.

In an embodiment, the nucleotide acid molecule comprises at least one nucleotide sequence selected from the group consisting of 
TGATACAGAAGACTTGAGATTCTGGATTGGAGCTTGATGCCACAATTTTGGATGAGAAATTTGGAG
GTCCTGGAATAGG (SEQ ID NO: 14);
 
TCAAGTTCAGTGTTTGGTTAAAATACATACTCAGTAAATGGTAGCTATTATTGTCTTAGTTTAAGTTA
TTGCAAGCATTAAAATTAAATGTTTAGCTACAGACTCAATCCAGTTTTAATGTCATTGTGTTAATAAG
GCCTCTTAACATTGAAGCAACAAAGA (SEQ ID NO: 15);
 
AACAGGTCACAATGGTGGAATGTCGTCAGCTAAGGCAGGACCTGGCTATTTGCACTTCTTTTGTGG
ATCTTCAGTTGCTTCA (SEQ ID NO: 16);
 
UGAUACAGAAGACUUGAGAUUCUGGAUUGGAGCUUGAUGCCACAAUUUUGGAUGAGAAAUUUG
GAGGUCCUGGAAUAG (SEQ ID NO: 17);
 
UCAAGUUCAGUGUUUGGUUAAAAUACAUACUCAGUAAAUGGUAGCUAUUAUUGUCUUAGUUUAA
GUUAUUGCAAGCAUUAAAAUUAAAUGUUUAGCUACAGACUCAAUCCAGUUUUAAUGUCAUUGUG
UUAAUAAGGCCUCUUAACAUUGAAGCAACAAAGA (SEQ ID NO: 18);
 
AACAGGUCACAAUGGUGGAAUGUCGUCAGCUAAGGCAGGACCUGGCUAUUUGCACUUCUUUUG
UGGAUCUUCAGUUGCUUCA (SEQ ID NO: 19); or
 a nucleotide sequence complementary to any of SEQ ID NO; 14 to 19.

Alternatively, the nucleic acid molecule comprises at least one nucleotide sequence corresponding or comprising, such as consisting of, a transcript from a gene selected from Table 13, Table 24 or among the genes shown in Figures 27A to 27N.

In an embodiment, the nucleic acid molecule is comprised in a nanoparticle. In a particular embodiment, the nanoparticle is an extracellular vesicle (EV), preferably an EV having an average diameter within an interval of from 50 nm to 2000 nm, preferably from 75 nm to 165 nm.

This aspect is based on the experimental data indicating that RNA transcripts comprised in EVs produced and released by IVF embryos are taken up, preferably as EVs, by responder cells, such as endomentrial cells, and induce a modification in the amount of the at least one corresponding RNA transcript in the responder cells. Such a modification is in turn seen for high quality IVF embryos that are more likely to be successfully implanted into the uterus of a female subject and result in a successful pregnancy of the female subject. Hence, modification of the at least one RNA transcripts may be an important feature in the communication and interaction between the embryo and maternal tract. Thus, nucleic acid molecules of the invention can be administered and used as a means to achieve the required communication and interaction and to thereby suppress rejection of the embryo in the IVF procedure and improve embryo implantation of the embryo in the IVF embryo transfer procedure.

Disclosed, but not part of the claimed invention, is a transcription modulator for use in suppressing rejection of an embryo in an *in vitro* fertilization (IVF) procedure and/or for use in improving embryo implantation of an embryo in an *in vitro* fertilization (IVF) embryo transfer procedure. The transcription modulator is adapted to modulate transcription of at least one of mucin 4 *(MUC4), MUC3A, MUC16, MUC12,* zinc finger protein 81 *(ZNF81),* Ras-related GTP-binding protein B (*RRAGB*), mitochondrially encoded tRNA tryptophan (*MT-TW*)*,* Z95704.5, mitochondrially encoded tRNA serine 1 *(MT-TS1),* integrin, alpha E *(ITGAE),* RP11-357C3.3, transmembrane protein 154 *(TMEM154),* caspase 14 (*CASP14*)*, ZNF765,* long intergenic non-protein coding RNA 478 (LINC00478), mitochondrially encoded tRNA glutamine *(MT-TQ),* ankyrin repeat domain-containing protein 44 *(ANKRD44),* and zinc finger BED-type containing 3 - antisense RNA 1 *(ZBED3-AS1).*

Alternatively, the transcription modulator is adapted to modulate transcription of at least one gene selected from Table 13, Table 24 or among the genes shown in Figures 27A to 27N.

The transcription modulator is capable of upregulating transcription of the at least one RNA transcript or downregulating transcription of the at least one RNA transcript, depending on the particular RNA transcript.

In a particular embodiment, the transcription modulator is a transcription inhibitor adapted to inhibit transcription of the at least one RNA transcript.

In an embodiment, the transcription inhibitor is adapted to inhibit transcription of at least one of an intronic region of LINC00478, an exonic region of LINC00478 and an exonic region of *ZNF81.* In a particular embodiment, the intronic region of LINC00478 comprises the nucleotide sequence of SEQ ID NO: 16, or a nucleotide sequence complementary to SEQ ID NO: 16. The exonic region of LINC00478 comprises the nucleotide sequence of SEQ ID NO: 15, or a nucleotide sequence complementary to SEQ ID NO: 15. The exonic region of *ZNF81* comprises the nucleotide sequence of SEQ ID NO: 14, or a nucleotide sequence complementary to SEQ ID NO: 14.

In an embodiment, the transcription inhibitor is an antisense polynucleotide comprising a nucleotide sequence complementary to at least a portion of the nucleic acid sequence encoding *MUC4, MUC3A, MUC16, MUC12, ZNF81, RRAGB, MT-TW,* Z95704.5, *MT-TS1, ITGAE,* RP11-357C3.3, *TMEM154, CASP14, ZNF765,* LINC00478, *MT-TQ, ANKRD44,* or *ZBED3-AS1,* or a nucleotide sequence complementary thereto.

In an embodiment, the transcription inhibitor is an RNA interference (RNAi) molecule, preferably an RNAi molecule selected from the group consisting of a micro RNA (miRNA) and a small interfering RNA (siRNA).

The transcription modulator and the nucleic acid molecule as defined above can advantageously be administered to the female subject, such as prior to implantation of the IVF embryo in the uterus, substantially simultaneously with implantation of the IVF embryo and/or following implantation of the IVF embryo. Various administration routes can be used including, but not limited, to intravaginally or intrauterinally administering the transcription inhibitor and/or nucleic acid molecule.

Disclosed, but not part of the claimed invention, is a ribonucleic acid (RNA) molecule consisting of one RNA sequence selected from the group consisting of SEQ ID NO: 17 to 19 and a deoxyribonucleic acid (DNA) molecule consisting of one DNA sequence selected from the group consisting of SEQ ID NO: 14 to 16.

Disclosed, but not part of the claimed invention, is an *in vitro* fertilization (IVF) composition comprising at least one embryo and at least one nucleic acid molecule selected from the group consisting of a mucin 4 *(MUC4)* transcript, a complementary deoxyribonucleic acid (cDNA) of the *MUC4* transcript, a *MUC3A* transcript, a cDNA of the *MUC3A* transcript, a *MUC16* transcript, a cDNA of the *MUC16* transcript, a *MUC12* transcript, a cDNA of the *MUC12* transcript, a zinc finger protein 81 *(ZNF81)* transcript, a cDNA of the *ZNF81* transcript, a Ras-related GTP-binding protein B (RRAGB) transcript, a cDNA of the RRAGB transcript, a mitochondrially encoded tRNA tryptophan (*MT-TW*) transcript, a cDNA of the *MT-TW* transcript, a Z95704.5 transcript, a cDNA of the Z95704.5 transcript, a mitochondrially encoded tRNA serine 1 (*MT-TS1)* transcript, a cDNA of the *MT-TS1* transcript, an integrin, alpha E (*ITGAE*) transcript, a cDNA of the *ITGAE* transcript, a RP11-357C3.3 transcript, a cDNA of the RP11-357C3.3 transcript, a transmembrane protein 154 *(TMEM154)* transcript, a cDNA of the *TMEM154* transcript, a caspase 14 *(CASP14)* transcript, a cDNA of the *CASP14* transcript, a *ZNF765* transcript, a cDNA of the *ZNF765* transcript, a long intergenic non-protein coding RNA 478 (LINC00478) transcript, a cDNA of the LINC00478 transcript, a mitochondrially encoded tRNA glutamine *(MT-TQ)* transcript, a cDNA of the *MT-TQ* transcript, an ankyrin repeat domain-containing protein 44 *(ANKRD44)* transcript, a cDNA of the *ANKRD44* transcript, a zinc finger BED-type containing 3 - antisense RNA 1 *(ZBED3-AS1)* transcript, and a cDNA of the *ZBED3-AS1* transcript.

Alternatively, the at least one nucleic acid molecule comprises a transcript from a gene selected from Table 13, Table 24 or among the genes shown in Figures 27A to 27N.

In an embodiment, the at least one nucleic acid molecule is comprised in a nanoparticle. In a particular embodiment, the nanoparticle is an extracellular vesicle (EV), preferably an EV having an average diameter within an interval of from 50 nm to 2000 nm, preferably from 75 nm to 165 nm.

Disclosed, but not part of the claimed invention, is a method of suppressing rejection of an embryo in an *in vitro* fertilization (IVF) procedure and/or improving embryo implantation of an embryo in an *in vitro* fertilization (IVF) embryo transfer procedure. The method comprises administering, to a female subject, a nucleic acid molecule comprising at least one nucleotide sequence selected from the group consisting of a mucin 4 (*MUC4*) transcript, a complementary deoxyribonucleic acid (cDNA) of the *MUC4* transcript, a *MUC3A* transcript, a cDNA of the *MUC3A* transcript, a *MUC16* transcript, a cDNA of the *MUC16* transcript, a *MUC12* transcript, a cDNA of the *MUC12* transcript, a zinc finger protein 81 *(ZNF81)* transcript, a cDNA of the *ZNF81* transcript, a Ras-related GTP-binding protein B (*RRAGB*) transcript, a cDNA of the RRAGB transcript, a mitochondrially encoded tRNA tryptophan (*MT-TW*) transcript, a cDNA of the *MT-TW* transcript, a Z95704.5 transcript, a cDNA of the Z95704.5 transcript, a mitochondrially encoded tRNA serine 1 *(MT-TS1)* transcript, a cDNA of the *MT-TS1* transcript, an integrin, alpha E *(ITGAE)* transcript, a cDNA of the *ITGAE* transcript, a RP11-357C3.3 transcript, a cDNA of the RP11-357C3.3 transcript, a transmembrane protein 154 *(TMEM154)* transcript, a cDNA of the *TMEM154* transcript, a caspase 14 *(CASP14)* transcript, a cDNA of the *CASP14* transcript, a ZNF765 transcript, a cDNA of the ZNF765 transcript, a long intergenic non-protein coding RNA 478 (LINC00478) transcript, a cDNA of the LINC00478 transcript, a mitochondrially encoded tRNA glutamine *(MT-TQ)* transcript, a cDNA of the *MT-TQ* transcript, an ankyrin repeat domain-containing protein 44 *(ANKRD44)* transcript, a cDNA of the *ANKRD44* transcript, a zinc finger BED-type containing 3 - antisense RNA 1 *(ZBED3-AS1)* transcript, and a cDNA of the *ZBED3-AS1* transcript.

Alternatively, the nucleic acid molecule comprises at least one nucleotide sequence corresponding or comprising, such as consisting of, a transcript from a gene selected from Table 13, Table 24 or among the genes shown in Figures 27A to 27N.

Disclosed, but not part of the claimed invention, is administering the nucleic acid molecule intravaginally or intrauterinally administering the nucleic acid molecule to the female subject.

Disclosed, but not part of the claimed invention, is a method of suppressing rejection of an embryo in an *in vitro* fertilization (IVF) procedure and/or improving embryo implantation of an embryo in an *in vitro* fertilization (IVF) embryo transfer procedure. The method comprises administering, to a female subject, a transcription modulator, such as inhibitor, adapted to modulate, such as inhibit, transcription of at least one of mucin 4 (*MUC4*)*, MUC3A, MUC16, MUC12,* zinc finger protein 81 *(ZNF81),* Ras-related GTP-binding protein B (RRAGB), mitochondrially encoded tRNA tryptophan (*MT-TW*)*,* Z95704.5, mitochondrially encoded tRNA serine 1 *(MT-TS1),* integrin, alpha E (*ITGAE*), RP11-357C3.3, transmembrane protein 154 *(TMEM154),* caspase 14 (*CASP14*), *ZNF765,* long intergenic non-protein coding RNA 478 (LINC00478), mitochondrially encoded tRNA glutamine *(MT-TQ),* ankyrin repeat domain-containing protein 44 *(ANKRD44),* and zinc finger BED-type containing 3 - antisense RNA 1 *(ZBED3-AS1).*

Alternatively, the transcription modulator is adapted to modulate transcription of at least one gene selected from Table 13, Table 24 or among the genes shown in Figures 27A to 27N.

In an example, administering the transcription modulator comprises intravaginally or intrauterinally administering the transcription modulator to the female subject.

The transcription modulator and/or nucleic acid molecule may be administrated in any formulation type suitable for intravaginal or intrauterinal administration. Non-limiting, but illustrative examples, include tablets, hard and soft gelatin capsules, creams, suppositories, pessaries, foams, ointments, gels, films, tampons, vaginal rings, and douches.

Examples of pharmacologically excipients that can be included in the formulation are described in Garg et al., Compendium of Pharmaceutical Excipients for Vaginal Formulations, Pharmaceutical Technology 2001, 25: 14-25, the teachings of which relating to excipients used, approved, or investigated for vaginal formulations in Table II on pages 18-23 is given as examples of suitable excipients.

In an embodiment, the female subject is a female mammalian, and in particular a woman. The embodiments are, however, not limited to humans but can also be applied to non-human mammals including female subjects from mice, cats, dogs, cows, cattle, pigs, sheep, rats, horses, goats, rabbits and guinea pigs.

### EXAMPLES

### EXAMPLE 1

In the current Example, we tracked and captured both coding RNA and non-coding RNA (ncRNA) exchanged in cell-cell communication model using a genetic labeling system based on copper (I)-catalyzed cycloaddition reaction, also known as bioorthogonal click chemistry. Bioorthogonal tagging of metabolites, such as nucleic acids, proteins, glycans and lipids, uniquely enables tracking the tagged substance *in vivo* and *in vitro,* while not disrupting other physiological processes. During neurogenesis, for instance, it is possible to visualize bioorthogonally labeled RNA as it spreads over dendron cells using nascent RNA synthesis in presence of 5-ethynyl uridine (EU). Application of a similar EU-RNA labeling system in the present Example led to the discovery of transcripts transferred from trophoblast/embryo to endometrial cells. Given the well-recognized ethical and technical limitations associated with the study of human embryo-endometrial dialogue *in vivo,* we used an established human *in vitro* implantation model using RL95-2, a human epithelial cell line derived from a moderately differentiated endometrial adenocarcinoma that exhibits pronounced adhesiveness to trophoblast-derived JAr cells. We identified specific trophoblast transcripts that were transferred by extracellular vesicles (EVs) into endometrial RL95-2 cells, leading to the down-regulation of the same transcripts in the co-cultured recipient endometrial cells. Furthermore, EVs/nanoparticles captured from conditioned culture media of viable human IVF embryos down-regulated the expression of at least one of the transcripts in the RL95-2 cells. Interestingly, co-culture of EVs/nanoparticles obtained from the conditioned culture media of degenerating human IVF embryos did not alter the expression of the particular endogenous transcript in RL95-2 cells.

### MATERIALS AND METHODS

### Cell culture and spheroid formation

The human endometrial adenosquamous carcinoma cell line (RL95-2) was obtained from American Type Culture Collection (ATCC CRL-1671, Teddington, UK). RL95-2 was cultured in Dulbecco's Modified Eagles Medium (DMEM 12-604F, Lonza, Verviers, Belgium) supplemented with 1% Penicillin/Streptomycin (P/S, Gibco^{™} 15140122, Bleiswijk, Netherlands), 5 µg/ml Insulin (human recombinant insulin, Gibco, Invitrogen, Denmark), 1% L-glutamine (Sigma, 59202C, Saint Louis, USA) and 10% fetal bovine serum (Gibco^{™}, 10500064) at 37°C in 5% CO₂ atmosphere.

The human choriocarcinoma cell line (JAr) from the first trimester trophoblasts was acquired from ATCC (HTB-144^{™}, Teddington, UK). JAr cells were cultured in a T75 flask in RPMI 1640 media (Gibco, Scotland) supplemented with 10% fetal bovine serum (FBS), 1% L-glutamine and 1% P/S at 5% CO₂ in 37°C. At confluency, JAr cells were washed with Dulbecco's phosphate-buffered saline without Ca²⁺ and Mg²⁺ (DPBS, Verviers, Belgium), harvested using trypsin-ethylenediaminetetraacetic acid (EDTA) (Gibco^{®} Trypsin, New York, USA) and pelleted by centrifugation at 250 × g for 5 minutes. 1 × 10⁶ cells/ml were cultured in 5 ml of supplemented RPMI 1640 medium in 60 mm Petri dishes at 5% CO₂ in 37°C. The cells were kept on a gyratory shaker (Biosan PSU-2T, Riga, Latvia), set at 295 rotations per minute (rpm) for 18 h (Caballero et al. 2013). The viability of produced spheroids was confirmed by Live/dead^{®} viability/cytotoxicity assay kit (Molecular Probes, Eugene, Oregon, USA), according to the manufacturer's instructions. Briefly, a working solution was prepared with the final concentration of 2 µM and 4 µM for calcein AM (acetoxymethyl ester of calcein) and EthD-1 (ethidium homodimer-1), respectively. The working solution was added directly to spheroids and incubated at room temperature ((RT) 20-25°C) for 30 min and the viability of spheroids (majority of cells emitting green fluorescence) was confirmed with florescent microscopy. The multicellular spheroids were used to mimic trophoblast cells *in vitro.*

The human embryo kidney (HEK) 293T cell line was cultured in DMEM/F-12 supplemented with 10% of heat inactivated FBS (Gibco), and 1% L-glutamine (Sigma). All cells were grown in T75 flasks at 37°C in a 5% CO₂ atmosphere. The media was changed every second day until confluence of the cells. One million cells were counted with a haemocytometer and cultured overnight on a gyratory shaker to form multicellular spheroids as described above.

### 5-ethynyl uridine tagging of trophoblast spheroids

Produced spheroids were either used without labeling (based on the particular experimental design) or labeled with 5-ethynyl uridine (EU). For labeling, about 2×10³ spheroids were incubated in 5 ml culture media supplemented with EU at a final concentration of 0.2 mM in 60 mm Petri dishes at 5% CO₂ in 37°C. The spheroids were kept on gyratory shaker (Biosan PSU-2T), set at 295 rpm for 18 h. The day after labeling, spheroids were washed by placing them in a 50 ml tube. The supernatant, including single cells and incomplete spheroids, was removed. Spheroids were re-suspended in 20 ml pre-warmed culture media and after settlement, the supernatant was removed. The washing step was repeated to remove the EU molecules from the spheroid's environment. The labeled spheroids were prepared for co-culture system.

### Non-contact co-culture of trophoblast spheroids with endometrial cells

Endometrial cells were cultured (seeding density 1.25 × 10⁶) in each well of 6-well plate until 60% confluency. For co-incubation of trophoblast spheroids with epithelium, a 0.4 µm membrane insert was inserted in each well (Falcon^{®} Permeable Support for 6 Well Plate with 0.4 µm Translucent High Density PET Membrane). The depth of the insert allowed the membrane to be immersed in the culture media covering the epithelial cells but not in direct contact with the cells (so-called the non-contact co-culture system). Then, approximately 2 × 10³ labeled spheroids were inserted on a 0.4 µm membrane insert in each well of a 6-well plate. The labeled spheroids and endometrial cells were co-incubated in serum-starved media consisted of DMEM (DMEM/F12, Verviers, Belgium v/v 1:1) supplemented with 1% L-glutamine, 1% P/S, transferrin (10 mg/ml; BioReagent, Cat. No. T8158), selenium (25 mg/L; Sigma, Cat. No. 229865), bovine serum albumin (1 mg/ml; HyClone^{™}, Cat. No. SH30574), linoleic acid (4.7 mg/ml; Sigma, Cat. No. L1012) and insulin (5 mg/ml) for 24 hours.

### Total RNA extraction and quality control

Total RNA was extracted from endometrial cell line, conditioned media and EVs by TRIzol Reagent and ethanol precipitation (TRIzol^{®} reagent; Invitrogen). To increase the efficiency of RNA extraction, 2 µl glycogen (UltraPure^{™} Glycogen, Cat. no. 10814-010, Thermo Fisher Scientific, Bleiswijk, Netherlands) was added to the lysis buffer per sample. The RNA pellet was washed three times by 70% ethanol. Quality and quantity of the extracted RNA samples were analyzed by Bioanalyzer Automated Electrophoresis instrument (Agilent technologies, Santa Clara, CA) using Agilent RNA 6000 Pico Kit (Agilent technologies) and Agilent Small RNA kit (Agilent technologies).

### Affinity precipitation of EU-labeled RNA

EU-labeled RNA was affinity precipitated according to the manufacturer's instruction of Click-iT Nascent RNA capture kit (Thermo Fisher Scientific, Waltham, MA; Cat. No. C10365). Briefly, the extracted total RNA from cell lines, conditioned media and/or EVs were biotinylated in click-it reaction mixture with a final concentration of 1 mM biotin azide. The click-it reaction mixture was incubated for 30 min at room temperature while gently mixing using a gyratory shaker with 500 rpm. Biotin-azide (PEG4 carboxamide-6-azidohexanyl biotin) was attached to alkyne reactive group of the EU-labeled RNA using click chemistry. Biotinylated RNA, was incubated with 12 µl MyOne^{™} Streptavidin T1 magnetic Dynabeads^{®} into Click-iT RNA binding buffer for a final volume of 74 µl. The mixture of RNA and bead was incubated in the dark at room temperature for 40 min while mixing using a gyratory shaker, 500 rpm speed to prevent the beads from settling. After biotinylated RNA binding to Dynabeads, beads were washed three times with two wash buffers that were included in the kit (pre-warmed to 65°C), while mixing vigorously with a gyratory shaker at 700 rpm to remove the non-specifically attached RNA. After the last wash, the beads were immobilized by the DynaMag^{™}-2 magnet and wash buffer was completely removed. Beads were re-suspended in 15 µl nuclease free water and were directly used for cDNA synthesis for sequencing and quantitative polymerase chain reaction (qPCR).

### cDNA library preparation and sequencing of captured EU-labeled RNA from endometrial cells

Ovation RNA-Seq System V2 (NuGEN technologies, San Carlos, CA, Cat.No.7102-32) was used for cDNA library synthesis. The manufacturer's protocol was slightly modified to allow single strand cDNA (ssDNA) to be synthesized from on-bead RNA fragments. The modifications were as follows, 2 µl of First Strand Primer Mix was added to 14 µl on-bead RNA fragments and incubated for 5 min at 65°C, followed by cooling on ice for 5 min. Then, 0.5 µl of first strand enzyme mix and 5 µl of first strand buffer mix were added to the mixture resulting in a final volume of 20 µl. The mixture was incubated at 43.5°C for 60 min on an Eppendorf thermomixer (700-800 rpm) to prevent the beads from settling. Finally, the mixture was thermal shocked at 85°C for 10 min and beads were rapidly immobilized by a magnet allowing the collection of cDNA from the supernatant. Ten µl of first strand cDNA was used in the double strand synthesis step. Double strand cDNA synthesis was performed according to NuGEN manufacturer's instructions. cDNA quality was measured by High Sensitivity DNA 1000 Assay Kit (Agilent technologies). Double stranded cDNA was subsequently used for barcoded library preparation. Libraries were prepared using the AB Library Builder^{™} System (Thermo Fisher, Cat. No. 4477598) and Ion Xpress^{™} Plus Fragment Library Kit (Thermo Fisher), according to the manufacturer's instructions. The barcoded libraries were sequenced on two Ion 540^{™} Chips (ThermoFisher Scientific Inc, CA, USA, Cat. No. A27766) with four libraries per chip using the Ion S5 XL sequencer (Thermo Fisher Scientific Inc).

### Differential expression analysis of RNA-seq data

The experimental methods used for detecting transferred transcripts resulted in the selective enrichment of transferred transcripts. This enrichment was quantified by conventional differential expression analysis methods since the measured effect was the alteration in the relative quantity of transcripts in one experimental group compared to another. Sequenced reads were first aligned to the hg19 human reference genome using the Torrent Mapping Alignment Program (TMAP; Thermo Fisher Scientific), using mapping algorithm map4 with default parameters. TMAP is a sequence alignment software optimized specifically for mapping reads produced by Ion Torrent sequencing platforms. Read counts were obtained for 55,766 annotated coding and non-coding genomic elements in the hg19 human reference genome. Differential gene expression analysis of RNA-sequencing (RNA-seq) data was performed using the Generalized Linear Model (GLM) pipeline of edgeR package in R (Robinson et al., 2009; van de Lavoir et al., 2006). The genomic elements failing to surpass counts per million (CPM) cut-off of 0.7 for at least 3 out of 4 samples in at least one of the experimental groups were omitted from further analysis. The threshold CPM ≥ 0.7 translates to 10 aligned reads per genomic element divided by the mean of total sequenced reads of all samples in millions. The differentially expressed transcripts were considered significant if the false-discovery rate (FDR) reported by edgeR was less than or equal to 0.05 (FDR ≤ 0.05). Integrative Genomics Viewer (IGV) was used to inspect the coverage of differentially expressed (enriched) transcripts.

### cDNA synthesis and qPCR analysis for quantification of EU-labeled transferred transcripts

EU-labeled RNAs from the complete conditioned media and EVs were affinity precipitated and the copy number of EU-labeled ZNF81, exonic-LINC00478 and intronic-LINC00478 were quantified. For cDNA synthesis of EU-labeled transferred transcripts, a mixture of random hexamer and oligo (dT) primers was used (SuperScript^{®} VILO^{™} cDNA synthesis kit, 11754 050). For EU-labeled RNA on bead, the cDNA synthesis was performed according to the Click-iT RNA Capture Kit. The primers for transferred transcripts (ZNF81, exonic and intronic-LINC00478) were designed by Beacon designer 8 (PREMIER Biosoft International, Palo Alto, CA) and reads sequences were used as template (Table 1). For quantification of EU-labeled ZNF81 and exonic-LINC00478, cDNA products were amplified in EvaGreen assay system (Solis BioDyne, Tartu, Estonia) with the following program: 95°C for 15 min, followed by 40 cycles of 95°C for 20 s, 60°C for 20 s, and 72°C for 20 s. For melting curve analysis, the fluorescence signals were collected continuously from 65°C to 95°C at 0.05°C per second.

**Table.1 - Primers and sequence information**

| Transcript Name | Primer Sequence (5'-3') | SEQ ID NO: |
|---|---|---|
| ZNF81 | Forward primer: TGATACAGAAGACTTGAGATT | 1 |
| | Reverse primer: TCACAAAGTATTCACATTACC | 2 |
| Exonic LINC00478 | Forward primer: TCAAGTTCAGTGTTTGGTTAA | 3 |
| | Reverse primer: GGCAGAATCGTGAATAGC | 4 |
| Intronic LINC00478 | Forward primer: AACAGGTCACAATGGTGGAATG | 5 |
| | Reverse primer: TGAAGCAACTGAAGATCCACAA | 6 |
| Beta-2-microglobulin | Forward primer: CGGGCATTCCTGAAGCTGA | 7 |
| | Reverse primer: TGGAGTACGCTGGATAGCCT | 8 |
| Beta-actin | Forward primer: GTGCGCCGTTCCGAAAGT | 9 |
| | Reverse primer: ATCATCCATGGTGAGCTGGCG | 10 |
| Synthetic RNA Spike-in (100 bp from | Spike-in Forward primer: TACTGCATCCCGCTCTAC | 11 |
| | Spike-in Reverse primer: CGCTCATCAAGTCGTTCA | 12 |
| | UUGGGCAGAAACCGGGCCCCAACGGUGAC Spike-in RNA sequence: | 13 |
| Isopenicillin N-CoA synthetase) | CGCACCUACUACUGCAUCCCGCUCUACCACGGAACGGGGGGCAUCG CGGCCAUGAACGACUUGAUGAGCGG | |

Table 1 shows specific primers used for qPCR analysis of transferred/control transcripts. Primers were designed using Beacon Designer^{™} (PREMIER Biosoft International, Palo Alto, USA). Primer efficiency was measured using cDNA gradient method. Efficiency in the chosen temperature profile was between 98.7% and 99.2%.

For quantification of EU-labeled intronic-LINC00478, the cDNA product was amplified in EvaGreen master mix, including 5% dimethyl sulfoxide (DMSO) with following real-time touchdown PCR program: starting with 31 cycles of 94°C for 20 s, the decreasing annealing temperature for 20 s, and extension of 72°C for 20 s. The annealing temperature decreased 0.1°C per cycle from 63.6° to 60°C. For melting curve analysis, the fluorescence signals were collected continuously from 65°C to 95°C at 0.05°C per second.

For spike-in and normalizing of candidate transferred transcripts, 100 bp from Isopenicillin N-CoA synthetase gene was used (Biomer.net company, Ulm/Donau, Germany, molecular weight: 32239 g/mol, 100 pmol/µl) (Spike-in synthetic RNA Sequence refer to the Table.1). Synthetic RNA was serially diluted 20 times. For the first serial dilution, 1 µl of synthetic RNA was added to 39 µl RNase-free water to final concentration of 2.5 µM. Serial dilutions were prepared with a dilution factor of 4×. Serial dilutions were reverse-transcribed and amplified using real-time PCR and the cycle threshold (Ct) values of dilutions were plotted against the copy number of transcript. Exponential calibration curve was fitted. In parallel, 1 µl of synthetic transcript was added to the sample during TRIzol RNA extraction and then the Ct of synthetic RNA in this sample was assayed to calculate the RNA extraction efficiency and normalizing factor (Wang et al., 2015).

### Confocal laser scanning and imaging of EU-labeled RNA

The transferred EU-labeled RNAs were tracked by Alexa Fluor 488 azide (Included in Click-iT^{®} RNA Imaging Kit; Invitrogen, C10329). After 24 h co-culture of endometrial cells with EU-labeled spheroids, the conditioned media was removed and the endometrial cells were incubated with pre-warmed cell tracker working solution for 30 min (CellTracker^{™} Deep Red dye; Life Technologies, C34565). After incubation the cells were washed with DPBS, fixed with 4% formaldehyde (Thermo Fisher, GmbH) and permeabilized with 0,1% Triton X-100 in PBS (AppliChem GmbH, Darmstadt, Germany). Next, the EU-labeled RNA was detected using the Click-iT^{®} RNA Imaging Kit (Invitrogen, C10329) according to the kit protocol. Confocal laser scanning microscopy was performed using LSM510 Laser Scanning Confocal Microscope (LSM 510 Duo; Carl Zeiss Microscopy GmbH, Jena, Germany).

### EVs purification and nanoparticle tracking analysis (NTA)

Co-culture EVs were harvested from conditioned media of trophoblast spheroids/endometrial cell co-culture. Three milliliters of conditioned media from each well of 6-well plate dish was collected and 3 µl from RNase inhibitor (Solis BioDyne, Tartu, Estonia) was added to conditioned media. Conditioned media was centrifuged at 400 × g for 10 min. the supernatant was further centrifuged at 4,000 × g for 10 min and the supernatant was further centrifuged at 20,000 × g for 15 min to get rid of cell debris and apoptotic bodies. The supernatant was filtered two times with 0.2 µm filter. To isolate EVs, filtered conditioned media was concentrated to 500 µl with Amicon^{®} Ultra-15 centrifugal filter devices (10 kDa cut-off). EVs were isolated using size exclusion chromatography (SEC). A cross linked 4% agarose matrix of 90 µm beads were used (Sepharose 4 fast flow^{™}, GE HealthCare Bio-Sciences AB, Uppsala, Sweden) in a 30 cm column. Fractions 7-10 (fraction size 1 ml) were collected. Fractions were concentrated using Amicon^{®} Ultra-15 centrifugal filter devices (10 kDa cut-off). Isolated EVs were quantified using NTA (ZetaView, Particle Metrix GmbH, Inning am Ammersee, Germany). When preparing spheroid-derived EVs, conditioned media from 24 h cultures of spheroids in 60 mm dishes were used.

### Collection of human embryo conditioned culture media, EV/Nanoparticles purification and characterization

Experiments with human IVF embryo conditioned culture media were carried out under the ethical approval of Research Ethics Committee of the University of Tartu, approval number 267/T-2. Human embryos were produced by IVF or intracytoplasmic sperm injection (ICSI). They were cultured individually for 17-21 h (day 1) in sequential fertilization media (Sequential Fert^{™}, Origio, Måløv, Denmark), 48 h (day 3) in sequential cleavage stage media (Sequential Cleav^{™}, Origio) and additionally 48 h (day 5) in sequential blastocyst stage media (Sequential Blast^{™}, Origio). At day 3, embryos with equal size blastomeres and no fragmentation were considered as normal. At day 5, embryos with identifiable inner cell mass, trophoblast and blastocyst cavity were considered normal while embryos with degrading cells were considered as degraded. Embryo conditioned media (50 µl) was collected and subjected to low speed spin (400 × g at 10 min followed by 2,000 × g at 10 min). EVs/nanoparticles were isolated from the media using SEC. Namely 8-10 fractions with the volume of 1 ml were collected for further concentration in 10 kDa Amicon^{®} Ultra-15 Centrifugal Filters (Merck Millipore, Burlington, Massachusetts, United States). Concentration of EVs/nanoparticles was measured using NTA (ZetaView).

### Western blot analysis

Purified EVs from trophoblast spheroids were precipitated by adding 200 µl of water, 400 µl of methanol and 100 µl of chloroform to 200 µl of EVs. The solution was vortexed and centrifuged 14,000 × g for 5 min at room temperature. After removing the top layer, precipitated proteins were washed with 400 µl of methanol and centrifuged again. The pellets were air-dried, resuspended in 0.5% SDS and the protein concentrations were determined by Bradford assay. 30 µg of protein were heated for 5 min at 95°C in reducing (for Apo A-I detection) or in non-reducing (for CD63, CD9 and CD81 detection) Laemmli buffer and resolved in 12% sodium dodecyl sulfate-polyacrylamide gel electrophoresis (SDS-PAGE) according to standard protocol. Proteins were transferred onto polyvinylidene difluoride membrane (Thermo Fisher Scientific), followed by blocking in 5% non-fat dry milk in PBS-T (0.05% Tween-20, Thermo Scientific, Michigan, USA) for 1 h at room temperature. Subsequently, membranes were incubated with the primary anti-CD63 (sc-5275, 1:1000, Santa Cruz Biotechnology Inc., Dallas, TX), anti-CD9 (MA1-80307, 1:1000, Thermo Fisher Scientific, Loughborough, UK), anti-Apo A-I (sc-376818, 1:1000, Santa Cruz Biotechnology Inc. Dallas, TX), and anti-CD81 (555675, 1:1000, BD Biosciences, New Jersey, USA) antibodies overnight at 4°C in 5% milk-PBS-T solution and then with horseradish peroxidase conjugated goat anti-mouse secondary antibody (sc-516102, 1:1000, Santa Cruz Biotechnology Inc. Dallas, TX) for 1 h at room temperature. Membranes were washed three times for 5 min in PBS-T after each incubation step. Protein bands were detected using ECL SelectTM Western Blotting Detection Reagent (GE Healthcare, Buckinghamshire, UK) with ImageQuantTM RT ECL Imager (GE Healthcare, Buckinghamshire, UK).

### Electron microscopy

Suspension of EVs was deposited on formvar-carbon-coated 200 mesh copper grids (Agar Scientific, Essex, UK) for transmission electron microscopy (TEM) analysis according to the method described by Thery et al. 2006. Briefly, EVs on grids were fixed in 2% paraformaldehyde (P6148, Sigma-Aldrich, Schnelldorf, Germany) and 1% glutaraldehyde (O 1909-10, Polysciences, Warrington, USA), before being contrasted in uranyl oxalate [mixture of 4% uranyl acetate (21447-25, Polysciences, Warrington, USA) and 0,15 M oxalic acid (75688, Sigma-Aldrich, Schnelldorf, Germany)] and embedded in a mixture of methylcellulose (M6385, Sigma-Aldrich, Schnelldorf, Germany) and uranyl acetate (21447-25, Polysciences, Warrington, USA). Samples were observed with a JEM 1400 transmission electron microscope (JEOL Ltd. Tokyo, Japan) at 80 kV, and digital images were acquired with a numeric camera (Morada TEM CCD camera, Olympus, Germany).

### Statistical analysis

Data were presented as mean ± standard error of mean (SEM). In experiments that warranted statistical analysis for comparison of means, one-way ANOVA was used with appropriate post hoc analysis after testing the homogeneity with Leven's test.

### EXPERIMENTAL DESIGN

### Characterization of transcripts transferred from trophoblast to endometrial cells

To identify the RNA species that originate from trophoblast spheroids and are transferred to the endometrial cells, the trophoblast spheroids were incubated with the endometrial cells in the non-contact co-culture system as described earlier. The experimental group consisted of EU-labeled spheroids while non-EU-labeled spheroids were used as a negative control. After 24 h co-incubation, the transferred EU-labeled transcripts were affinity precipitated from the total RNA obtained from the endometrial cells. The first and second strand cDNA were synthesized and cDNA library was prepared for sequencing of the precipitated EU-labeled RNA as described earlier. Total RNA-seq was conducted with synthesized cDNA from experimental group (n=4) and negative control group (n=4) (Figure 1). The bioinformatics analysis of RNA-seq data and differential expression analysis of the detected transcripts were performed to identify the putatively transferred RNA sequences. After identification of putatively transferred RNA sequences, the presence of the candidate RNA species was confirmed in the endometrial cells by qPCR.

### Identification of the route of transfer of RNA from trophoblast cells to endometrial cells

To illustrate the route of RNA transfer from trophoblast cells to endometrial cells, conditioned media was collected from the EU-labeled trophoblast spheroid/endometrial cell co-culture of 24 h (experimental group). A similar co-culture of unlabeled spheroid/endometrial cells was used as a negative control. Conditioned media from each group was divided into two similar parts by volume. One part was used for EV purification. Total EU-labeled RNA was extracted from both conditioned media and isolated EVs using affinity precipitation. Extracted RNA was quantified for the expression of transferred transcripts by qPCR.

### Visualization of transferred transcripts by confocal microscopy and Alexa Fluor 488 azide

The transferred EU-labeled RNAs were visualized in endometrial cells by Alexa Fluor 488 azide. After 24 h co-incubation of endometrial cells with EU-labeled spheroids, the endometrial cells were stained with Alexa azide and the confocal microscopy imaging was performed on both experimental and negative control group, concurrently.

### The effect of trophoblast spheroid co-culture on expression of specific RNA transcripts in endometrial cells

Approximately 1 × 10³ trophoblast spheroids were co-cultured with 5 × 10⁵ endometrial cells for 24 h in 12 well cell culture plates with 0.4 µm translucent inserts. Total RNA from endometrial cells were isolated and analyzed for the expression of candidate transcripts by qPCR. As controls, endometrial cells co-cultured with HEK293 spheroids and untreated endometrial cells were also analyzed.

### The effect of trophoblast derived EVs on expression of specific RNA transcripts in endometrial cells

To demonstrate the effects of EVs on endometrial transcripts, EVs derived from JAr cells were incubated with endometrial cells in the ratio 50:1. (2.5 × 10⁷ EVs : 5 × 10⁵ cells). EV number was similar to the amount of EVs produced by 1000 trophoblast spheroids in 24 h. Untreated controls were prepared with endometrial cells without EV treatment. Endometrial cells treated with similar concentrations of EVs derived from HEK293 spheroids and untreated endometrial cells were used as negative controls. After 24 h of incubation, the cells were lysed for total RNA extraction. cDNA was prepared and qPCR was performed for candidate transcripts. Beta actin and Beta-2-microglobulin were used as control genes to evaluate the behavior of unaffected genes in endometrial cells (Table 1).

### The effect of human IVF embryo-derived EVs/nanoparticles on specific RNA transcripts from endometrial cells

On day 3 post IVF, conditioned media were collected from 4 embryos that developed normally until day 5 and from 4 embryos that degenerated on day 5. The embryos developed until day 5 and conditioned media were again collected from 4 normal and 4 degenerated embryos. Conditioned media from each group were pooled and EVs/nanoparticles were isolated. EVs/nanoparticles were then supplemented to endometrial cells in 50:1 ratio (1 × 10⁷ EVs/nanoparticles : 2 × 10⁵ cells). Endometrial cells without EVs/nanoparticle treatment were used as negative control. After 24 hours of incubation total RNA was extracted from cells, cDNA was prepared and qPCR was performed for candidate transcripts. Beta actin and beta-2-microglobulin were used as control genes.

### RESULTS

### EU-labeled transcripts were visualized in endometrial cells by confocal microscopy

To identify possible trophoblastic RNA species that are transferred to the endometrial cells, trophoblast-derived JAR spheroids were incubated with the endometrial cells in a non-contact co-culture system. Produced spheroids were either used without labeling (based on the particular experimental design) or labeled with 5-ethynyl uridine (EU). Figure 1 depicts the overall strategy of biorthogonal labeling of trophoblast cells and capture of EU-labeled RNA in the endometrial cell.

Using confocal microscopy, we observed that EU-labeled spheroids exhibited the green fluorescence signal of Alexa 488 in the nuclei and especially in the nucleoli of the spheroids, confirming the successful EU incorporation into RNA while unlabeled control spheroids showed virtually no staining (Figures 2A, 2A1). When incubating endometrial cells with EU-labeled spheroids for 24 hours we could detect single green fluorescent dots in the cytoplasm of the endometrial cells while the overall cytoplasmic staining was low (Figure 2B) indicating the possible transfer of EU labeled RNA from spheroids to endometrial cells. We did not detect any similar concentrated dots with green fluorescence in the endometrial cells co-incubated with unlabeled spheroids (Figure 2B1). The presence of EU-labeled transferred RNA in the cytoplasm of endometrial cells was confirmed by 3-dimensional confocal scanning with and without cell tracker dye (Figures 2C, 2C1).

### Identification of putatively transferred transcripts from trophoblast spheroids to endometrial cells

Trophoblast spheroids with EU labeling (experimental group) were co-incubated with endometrial cells in a non-contact cell culture system to identify the transferred transcripts. Unlabeled spheroids were co-incubated with endometrial cells as a negative control. After 24 hours of incubation, total RNA from endometrial cells were collected and affinity precipitated to capture EU labeled RNA. Captured RNA was used for RNA sequencing (RNA-seq).

The percentage of the EU labeled RNA recovered from the total RNA obtained from cells exposed to EU labeling was calculated to determine the efficiency of EU labeled RNA capturing procedure. In EU labeled spheroids, only 12.66% (± 1.01%) of RNA was precipitated by affinity precipitation procedures.

In endometrial cells co-incubated with labeled JAr spheroids, 2.85% (± 0.45%) of RNA was precipitated. In endometrial cells co-incubated with unlabeled JAr spheroids (negative control), 1.13% (± 0.2%) of RNA was precipitated. The results indicated that approximately 35% of the supposedly EU labeled precipitated RNA might be unlabeled and non-specifically captured by the magnetic beads.

RNA-seq yielded on average 13.5 million reads per sample with average read length of 178 base pairs. The proportion of base pairs exceeding Phred quality score of 20 (base call confidence ≥ 99%) was 0.81 ± 0.01 (mean of all samples ± SD). The results of read alignment to the hg19 human reference genome varied extensively between the samples with alignment percentage ranging from 31 to 91%. This did not, however, have a major effect on the group averages, as the average alignment percentages were 51% and 55% for the experimental and control group, respectively.

Differential expression (DE) analysis, showed statistically significant enrichment of eighteen genomic elements in the endometrial cells. These elements were presumed to be transferred transcripts from trophoblast cells to endometrial cells (Figures 3A, 3B, Table 2). The alignments of individual reads to the 18 genomic elements of interest were visually inspected using Integrative Genomics Viewer (IGV), to estimate the full sequences of potentially transferred transcripts. This enabled the exclusion of genomic elements, for which the counted reads were presumed to be originating from random RNA fragments not specifically enriched but rather representing the random noise of the EU-labeled RNA capturing process.

**Table. 2 - Putatively transferred transcripts**

| | Gene | logFC | logCPM | LR | PValue | FDR |
|---|---|---|---|---|---|---|
| 1 | MUC4 | 4.962 | 1.84E+00 | 2.76E+01 | 1.50E-07 | 1.64E-04 |
| 2 | MUC3A | 4.09E+00 | 3.69E+00 | 2.73E+01 | 1.72E-07 | 1.64E-04 |
| 3 | MUC16 | 3.59E+00 | 3.57E+00 | 2.20E+01 | 2.68E-06 | 1.12E-03 |
| 4 | MUC12 | 3.40E+00 | 2.98E+00 | 1.93E+01 | 1.12E-05 | 3.41E-03 |
| 5 | ZNF81 | 4.43E+00 | -2.96E-01 | 1.75E+01 | 2.93E-05 | 6.97E-03 |
| 6 | RRAGB | 4.22E+00 | -7.88E-02 | 1.69E+01 | 3.87E-05 | 8.32E-03 |
| 7 | MT-TW | 2.84E+00 | 3.89E+00 | 1.48E+01 | 1.21E-04 | 2.13E-02 |
| 8 | Z95704.5 | 3.72E+00 | 1.20E-01 | 1.42E+01 | 1.67E-04 | 2.48E-02 |
| 9 | MT-TS1 | 2.67E+00 | 5.02E+00 | 1.31E+01 | 2.91E-04 | 3.29E-02 |
| 10 | ITGAE | 3.54E+00 | 9.15E-02 | 1.29E+01 | 3.30E-04 | 3.48E-02 |
| 11 | RP11-357C3.3 | 2.98E+00 | 1.85E+00 | 1.29E+01 | 3.33E-04 | 3.48E-02 |
| 12 | TMEM154 | 3.45E+00 | 4.12E-01 | 1.28E+01 | 3.40E-04 | 3.48E-02 |
| 13 | CASP14 | 3.35E+00 | 4.68E-01 | 1.22E+01 | 4.87E-04 | 4.33E-02 |
| 14 | ZNF765 | 3.31E+00 | 5.09E-01 | 1.20E+01 | 5.26E-04 | 4.45E-02 |
| 15 | LINC00478 | 3.38E+00 | -1.14E-01 | 1.18E+01 | 5.87E-04 | 4.69E-02 |
| 16 | MT-TQ | 2.56E+00 | 7.00E+00 | 1.16E+01 | 6.63E-04 | 4.85E-02 |
| 17 | ANKRD44 | 3.22E+00 | 7.80E-01 | 1.15E+01 | 6.78E-04 | 4.85E-02 |
| 18 | ZBED3-AS1 | 3.29E+00 | -1.13E-01 | 1.15E+01 | 6.98E-04 | 4.85E-02 |

Table 2 shows the 18 putatively transferred transcripts identified using glmLRT function of edgeR package. RNA sequencing was carried out using RNA affinity precipitated from endometrial cells co-incubated for 24 hours with EU labeled trophoblast spheroids. Endometrial cells co-incubated with a similar number of unlabeled trophoblast spheroids were used as a negative control.

The genomic sequences were considered to be specifically enriched when the alignment of reads originating from random RNA fragments were aligned to specific sequences and were: i) detected in at least three biological repeats out of four in the experimental group and ii) were not detected in any of the negative control samples. Only three candidate transcripts passed these stringent selection criteria: an intronic-non-coding region and an exonic-coding region, originating from LINC00478 locus of chromosome 21 (Figure 3C) and one exonic region from ZNF81 gene (Figure 3D). These transcripts were selected for further analysis.

The presence of EU-labeled intronic-LINC00478 (Figure 3E), exonic-LINC00478 (Figure 3F) and ZNF81 (Figure 3G) were also confirmed in endometrial cells by qPCR after 24 h co-incubation and there was a significant difference between the experimental group and the negative control group. Sanger sequencing of qPCR products confirmed the sequences of the candidate transcripts (Table 3).

**Table 3 - Sequences of transferred transcripts**

| Transcript | Sanger Sequence | SEQ ID NO: |
|---|---|---|
| ZNF81 | TGATACAGAAGACTTGAGATTCTGGATTGGAGCTTGATGCCACAA TTTTGGATGAGAAATTTGGAGGTCCTGGAATAGG | 14 |
| Exonic LINC00478 | TCAAGTTCAGTGTTTGGTTAAAATACATACTCAGTAAATGGTAGCTATT ATTGTCTTAGTTTAAGTTATTGCAAGCATTAAAATTAAATGTTTAGCTAC AGACTCAATCCAGTTTTAATGTCATTGTGTTAATAAGGCCTCTTAACATT GAAGCAACAAAGA | 15 |
| Intronic LINC00478 | AACAGGTCACAATGGTGGAATGTCGTCAGCTAAGGCAGGACCTGGC TATTTGCACTTCTTTTGTGGATCTTCAGTTGCTTCA | 16 |

Expression of transferred transcripts was quantified using qPCR. Products of qPCR were purified using column purification (MinElute PCR Purification Kit, Qiagen, No 28004) and sequenced using Sanger method. The results are shown in Table 3.

### EU-labeled intronic-LINC00478 transcript was detected in conditioned co-culture media

Conditioned media was collected from EU labeled spheroid/endometrial cell co-culture (experimental group) and unlabeled spheroid/endometrial cell co-culture (negative control). Half of the conditioned media from each group was used to extract EVs. Whole RNA of the condition media and EVs were extracted and subjected to affinity precipitation. Precipitated RNA was analyzed for the presence of candidate transcripts using qPCR.

The presence of EU-labeled intronic-LINC00478 transcript in conditioned media was confirmed by qPCR (Figure 3H). Copy number of this transcript was significantly higher in RNA extracted from complete conditioned media (including free RNA, RNA bound to proteins and RNA in EVs) compared to the RNA extracted from EVs. The conditioned media of the negative control also exhibited the presence of a small copy number of (7 times less than that of the experimental group) intronic-LINC00478 transcript. The presence of EU-labeled exonic-LINC00478 transcript or EU-labeled ZNF81 transcript was not detected in conditioned media or in EVs via our qPCR assay conditions due to the low copy numbers present in the samples.

### Trophoblast spheroid derived nanoparticles were confirmed as EVs using nanoparticle tracking analysis (NTA), electron microscopy and Western blot analysis

Conditioned media from trophoblast spheroids were collected and nanoparticles were isolated using sequential centrifugation and size exclusion liquid chromatography (SEC). Isolated particles were characterized using NTA, Western blotting for EV specific proteins and electron microscopy.

NTA revealed a population of particles largely under 200 nm with majority of the particles in 75 -135 nm range (Figure 4A). Electron microscopy showed uniform particles of less than 200 nm with identifiable lipid bilayer membranes, circular cross section and characteristic "cup shape" (Figure 4B).

Western blot analysis showed that EVs' specific protein markers CD63, CD9 and CD81 were enriched in trophoblast spheroid derived EVs compared to trophoblast spheroid conditioned culture media, while apolipoprotein A-I (a negative marker for EV) was not enriched (Figure 4C).

### Transferred transcripts were significantly down regulated in endometrium

Endometrial cells were co-incubated with trophoblast spheroids and HEK293 spheroids in separate groups. Similar numbers of endometrial cells were supplemented with trophoblast spheroid derived EVs and HEK293 spheroid derived EV in separate groups. HEK293 spheroids and HEK293 derived EVs were used as a negative control along with untreated endometrial cells. After 24 h of co-incubation, endometrial cell RNA was analyzed for the expression of candidate transcripts using qPCR.

The three transferred transcripts showed significant down-regulation in endometrial cells co-cultured with trophoblast spheroids compared to untreated controls and endometrial cells co-cultured with HEK293 spheroids. Transferred transcripts were also significantly down-regulated in endometrial cells treated with trophoblast derived EVs compared to untreated controls and endometrial cells treated with HEK293 derived EVs (Figures 5A, 5B, 5C). Control genes (beta-actin and beta-2-microglobulin) did not show a significant change of gene expression between the groups (Figures 5D, 5E).

### Embryo derived EV/nanoparticles alter the expression of specific transcripts in endometrial cells

Conditioned media was collected from both viable and degenerating human embryos at day 3 and day 5 post IVF. EVs were isolated from conditioned media and supplemented to endometrial cells. After 24 h of EV supplemented incubation, whole RNA from endometrial cells were collected and analyzed for the expression of candidate genes by qPCR.

The size profile of nanoparticles derived from embryo conditioned media (Figures 6A, 6B) exhibits the characteristics of a typical EV population. EVs derived from both day 3 and 5 normal quality embryos induced a significant down-regulation of ZNF81 transcript (Figure 6C). EVs derived from day 3 and 5 degenerating embryos did not induce similar change in the expression of ZNF81. Control genes (beta-actin and beta-2-microglobulin) did not show a significant change of gene expression between the groups (Figures 6D, 6E).

### DISCUSSION

A new paradigm has arisen in the scientific literature, pointing to the transfer of genetic material as an important mediator of the process of cell-to-cell communication. There is evidence of plant cells using non-coding RNA (ncRNA) to communicate within and between the cells. These examples are not limited to communication between the members of one species. Inter-species and inter-kingdom communication using ncRNA is also evident. A recent example is the case of miRNAs from the parasitic plant *Cuscuta campestris* targeting host messenger RNAs in the host plants and changing the transcription profile of the host plant. Plants use ncRNA to fight fungal infections by inhibiting fungal growth. In the human context, ncRNA is also likely to play a major role in intercellular communication. A well-known example is the communication and exchange of genetic material involving cancerous cells metastasizing to different tissues. It seems that cancerous cells are capable of signaling the cells of distant tissues, resulting in the remodeling of those tissues to better support metastatic tumor growth. The signals conveyed by cancerous cells seem to be in the form of ncRNA.

In nearly all these scenarios, ncRNAs seem to be transferred from one cell to another. Thereafter, the transferred material acts upon gene expression regulation in the recipient cells and changes the transcriptomic profile of them. The consequences of such communication would lead to alterations in the function and physiology of the cells, and ultimately may even result in the occurrence of disease or in the case of reproduction may affect conception and maintenance of the pregnancy. There is evidence of the exchange of miRNA between the pre-implantation embryo and the endometrium (Cuman et al., 2015) and vice versa (Vilella et al., 2015). Exchanged ncRNA could perform a number of functions in the target cells. Considering the lack of immune response towards embryo, which should be identified as "non-self', from the maternal immune system, one such function could be the modification of maternal immune response. Indeed, there are evidence of maternal immune system treating the embryo as a "temporary self' and assume "immune ignorance" (Trowsdale and Betz, 2006; Lynch et al., 2009; Smárason et al., 1993). Initiation and regulation of such unique immune response could be due to epigenetic modification caused by transferred genetic material by the developing embryo.

In the present Example we used biorthogonal click chemistry to track trophoblastic RNA and its uptake by endometrial cells. Compared to other enzyme dependent labeling solutions such as 5-bromouridine (BrU), 5-iodouridine (IU), or 5-fluorouridine (FU), which rely on indirect immunofluorescence, EU has a significant advantage to be compatible to be used in Click-chemistry and downstream applications requiring affinity precipitation of labeled RNA (Dvo 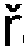 áčková and Fajkus, 2018). However, the efficiency of tagging is around one nucleotide in 35, which is not significantly different from the other labeling methods. Another important factor causing approximately 35% non-specifically captured unlabeled RNA in our investigation is the problems associated with RNA recovery using affinity precipitation protocols.

In the current Example, the origins of three transcripts were identified to be transferred from embryonal to endometrial cells: an intronic-non-coding region and an exonic-coding region, originating from LINC00478, and an exonic-coding region originating from ZNF81 gene (Table 2). In the case of transcripts originating from LINC00478, Dfam v2.0 software showed that this transcript matches with LTR7B family (ERV1 endogenous retrovirus super family) (Hubley et al., 2016). Open reading frame prediction demonstrated that 5 kbp upstream of this region might be a considerable potential for endogenous retrovirus protein. The regulatory role of endogenous retroviruses elements in development of human pre-implantation embryo has been strongly emphasized (Goke et al., 2015). It has been demonstrated that LTR7B and LTR7Y are enriched in the eight-cell/morula and blastocyst stage embryos, respectively. LTR7 copies can produce specific class of long non-coding RNA (IncRNA) (Kelley and Rinn, 2012) and in human embryonic stem cells they are involved in the regulatory network of pluripotency (Lu et al., 2014). Specific class of ncRNA can also be produced from endogenous retrovirus ERV9, activating the transcription of erythropoiesis genes (Hu et al., 2017). These elements can be horizontally transferred via EVs during intercellular communication. For instance, it has been confirmed that the RNA sequence of retrotransposon from human ERVs can be packaged into the EVs and transferred and spread during tumorigenesis (Balaj et al., 2011). In addition, the protein products of endogenous retroviral elements (such as envelope glycoprotein syncytin-2) are essential for early embryo and placenta development during implantation and these proteins are transferred by exosomes and are up-taken by endometrial cells (Lokossou et al., 2014; Vargas et al., 2014; Soygur et al., 2016).

We were not able to precipitate measurable amounts of ZNF81 transcript from EU labeled spheroid derived EVs due to the low efficiency of EU labeled RNA capture system. It has been shown that zinc-finger protein family can cooperate with transposable elements to form an epigenetic regulatory network (Imbeault et al., 2017; Trono et al., 2016; Berg, 1993). In the case of ZNF81, it is believed that this protein has the potential binding site for LINE elements (long interspersed nuclear elements) involved in regulation of many gene expression regulatory networks (Imbeault et al., 2017).

In all the three identified transferred transcripts, the endogenous expression of the same transcripts in the endometrial cells was significantly down-regulated after JAr cell or JAr cell-derived EVs' co-culture (Figure 5). Down-regulation of gene expression in target cells has been observed in the context of intercellular communication in different cell types (Lloret-Llinares et al., 2018; Syed et al., 1997). RNA-mediated gene expression down-regulation could be achieved using one of the several pathways, such as post-transcriptional gene silencing, co-suppression, quelling, and RNA interference (RNAi) (Travella et al., 2009). Recent investigations have postulated that negative feedback mechanisms are utilized by IncRNA to regulate self-expression (Tian et al., 2018; Yan et al., 2018; Jiang et al., 2018). LncRNAs are capable of increasing or decreasing self-expression or the expressions of specific target genes by interacting with chromatin-modifying complexes to modulate the epigenetic landscape of chromatin (Derrien et al., 2012; Quinn et al., 2016). The effect of RNA transfer on endogenous RNA down-regulation observed in the current Example is likely achieved by the RNA-mediated gene expression regulation. However, the possible involvement of RNA-independent mechanism cannot also be entirely excluded due to the heterogeneous nature of EV cargo.

One of the main criticisms of assisted reproduction has been its high tendency to cause multiple births. To avoid the issue, single embryo transfer is often practiced. Selecting the best embryo for transfer is important in single embryo transfer procedures (Bromer et al., 2008). Until very recent past, the selection was done using morphological criteria, such as the number of blastomeres, the absence of multinucleation, early cleavage to the two-cell stage, a low percentage of cell fragments in embryos, the blastocoelic cavity expansion and the cohesiveness and number of the inner cell mass and trophectodermal cells (Gardner et al., 2000; Sakkas et al., 2001). Despite of the evolution of the selection criteria for IVF embryos, the rate of live birth remains as low as 30% (Wang et al., 2011). Protein biomarkers from culture media (soluble human leukocyte antigen-G (sHLA-G) and ubiquitin) (Wang et al., 2004; Sher et al., 2005) and cumulus cell transcriptomic markers (cyclooxygenase 2 (COX2), steroidogenic acute regulatory protein (STAR), and pentraxin 3) have been proposed as tools for embryo selection (Feuerstein et al., 2007; Zhang et al., 2005; Rødgaard et al., 2015) without major improvement in the embryo implantation rate.

EVs isolated from conditioned culture media of IVF embryos as early as on day 3 after fertilization have the potential to be used as non-invasive biomarkers for embryo selection. In the current Example we provide evidence that EVs/nanoparticles isolated from embryo conditioned culture media can induce a measurable effect on endometrial cells and the effect is only seen when using conditioned media from morphologically good-quality embryos as opposed to degenerating embryos. Although the minimal requirements for EV studies require NTA, Western blot analysis of EV specific proteins and electron microscopy as per International Society for Extracellular Vesicles (ISEV) guidelines (Théry et al., 2019), due to the low number of particles isolated from single embryo culture media, Western blot analysis are currently not feasible in this context. However, with the NTA results, it could be argued that these nanoparticles are highly likely to constitute EVs. In the current Example, endometrial ZNF81 expression was significantly down-regulated after EV-co-incubation originating from good-prognosis day 3/5 IVF embryos. To the contrary, the EVs from poor prognosis IVF embryos were unable to initiate any changes of endometrial cells. We therefore suggest that the EV-based method could be used as a non-invasive tool or assay for selecting high-quality IVF embryos for transfer.

In conclusion, we present the evidence of non-contact transfer of embryonic RNA transcripts to endometrium in an *in vitro* embryo-maternal cross-talk model. RNA is taken up by the endometrial cells and the expression of endogenous transcripts is altered as a result. The effect can be seen in endometrial cells treated with EVs derived from IVF embryos suggesting that the RNA is transferred through EVs. EVs derived from human IVF embryos also have the potential to change the endometrial transcripts.

Interestingly, only good-prognosis IVF embryos induced the observed effect while degenerated IVF embryos failed to initiate any changes.

### EXAMPLE 2

### MATERIALS AND METHODS

### Cell culture

The human endometrial adenosquamous carcinoma cell line (RL95-2) was obtained from American Type Culture Collection (ATCC CRL-1671, Teddington, UK). RL95-2 was cultured in Dulbecco's Modified Eagles Medium (DMEM 12-604F, Lonza, Verviers, Belgium) supplemented with 1% Penicillin/Streptomycin (P/S, Gibco^{™} 15140122, Bleiswijk, Netherlands), 5 µg/ml Insulin (human recombinant insulin, Gibco, Invitrogen, Denmark), 1% L-glutamine (Sigma, 59202C, Saint Louis, USA) and 10% FBS (Gibco^{™}, 10500064) at 37°C in 5% CO₂ atmosphere.

### EV depletion of fetal bovine serum (FBS)

Extracellular vesicles in FBS were depleted using the protocol previously published Kornilov et al. 2018. Briefly, FBS was ultra-filtered using Amicon ultra-15 centrifugal filters (100 kDa) for 55 min at 3,000 × g in room temperature. The flow through was collected and filtered with 0.22 µm syringe filters for sterilization before using in cell culture. EV depleted complete media was prepared by supplementing Dulbecco's Modified Eagles Medium with 10% EV depleted FBS, 1% Penicillin/Streptomycin, 5 µg/ml Insulin and 1% L-glutamine.

### Total RNA extraction and quality control

Total RNA was extracted from endometrial cell line by TRIzol Reagent and isopropanol precipitation (TRIzol^{®} reagent; Invitrogen). To increase the efficiency of RNA extraction, 15 µg glycogen (UltraPure^{™} Glycogen, Cat. no. 10814-010, Thermo Fisher Scientific, Bleiswijk, Netherlands) was added to the aqueous phase of the sample in the precipitation step. The RNA pellet was washed three times by 75% ethanol. RNA was quantified using Qubit^{™} RNA HS Assay Kit (Q32852, ThermoFisher scientific). Quality of the extracted RNA samples was analyzed by Bioanalyzer Automated Electrophoresis instrument (Agilent technologies, Santa Clara, CA) using Agilent RNA 6000 nano Kit (Agilent technologies).

### cDNA synthesis and qPCR analysis

Gene expression of *ZNF81* was analyzed using SYBR green based quantitative PCR. cDNA synthesis was carried out using FIREScript RT cDNA Synthesis mix^{™} with Oligo (dT) and random primers (06-20-00100, Solis BioDyne, Tartu, Estonia) using the following program: Primer annealing 25°C for 10 min, reverse transcription 50°C for 60 min and enzyme inactivation 85°C for 5 min.

The primers for candidate transcript *(ZNF81)* were designed by Beacon designer 8 (PREMIER Biosoft International, Palo Alto, CA). Primer sequences (Forward primer: TGATACAGAAGACTTGAGATT (SEQ ID NO: 1) and Reverse primer: TCACAAAGTATTCACATTACC (SEQ ID NO: 2)). cDNA products were amplified using HOT FIREPol^{®} EvaGreen^{®} qPCR SuperMix (08-36-00001, Solis BioDyne, Tartu, Estonia) in QuantStudio 12K Flex^{™} real time PCR system. Following program was used: enzyme activation 95°C for 15 min followed by 40 cycles of 95°C for 20 s, 60°C for 20 s, and 72°C for 20 s. For melting curve analysis, the fluorescence signals were collected continuously from 65°C to 95°C at 0.05°C per second.

For spike-in and normalizing of candidate transferred transcripts, 100 bp from Isopenicillin N-CoA synthetase gene was used (Biomer.net company, Ulm/Donau, Germany, molecular weight: 32239 g/mol, 100 pmol/µl). Spike-in RNA sequence: UUGGGCAGAAACCGGGCCCCAACGGUGACCGCACCUACU ACUGCAUCCCGCUCUACCACGGAACGGGGGGCAUCGCGGCCAUGAACGACUUGAUGAGCGG (SEQ ID NO: 13). Spike-in Forward primer: TACTGCATCCCGCTCTAC (SEQ ID NO: 11). Spike-in Reverse primer: CGCTCATCAAGTCGTTCA (SEQ ID NO: 12). Synthetic RNA was serially diluted 20 times. For the first serial dilution, 1 µl of synthetic RNA was added to 39 µl RNase-free water to final concentration of 2.5 µM. Serial dilutions were prepared with a dilution factor of 4×. Serial dilutions were reverse-transcribed and amplified using real-time PCR and the cycle threshold (Ct) values of dilutions were plotted against the copy number of transcript. Exponential calibration curve was fitted. In parallel, 1 µl of synthetic transcript was added to the sample during TRIzol RNA extraction and then the Ct of synthetic RNA in this sample was assayed to calculate the RNA extraction efficiency and normalizing factor. Spike-in RNA expression Ct values were used as a scale to calculate absolute *ZNF81* expression.

### Collection of embryo culture media

Experiments with human IVF embryo conditioned culture media were carried out under the ethical approval of Research Ethics Committee of the University of Tartu, approval number 267/T-2. Human embryos were produced by IVF or intracytoplasmic sperm injection (ICSI). They were cultured individually until transfer or vitrification in SAGE-1 (CooperSurgical, Trumbull, Connecticut, United States) or Continuous Single Culture Complete (CSCC, 90164, Fujifilm Irvine Scientific, Santa Ana, California, United States) media. Conditioned media was collected and frozen in -80°C until supplementation.

### EXPERIMENTAL DESIGN

### Quantification of ZNF81 expression in RL95-2 cells supplemented with human embryo conditioned media

RL95-2 cells were seeded into 48 well cell culture plates (3 × 10 ⁴ cells/well) and cultured until 80% confluence in nearly 36 hours of culture. After the confluency, the medium was replaced with 180 µl of EV depleted conditioned media supplemented with 20 µl of embryo conditioned media. Cells were incubated for 24 h in the supplemented media. After incubation, 1 × 10⁵ cells from each well were lyzed and RNA was collected. RNA was measured using Nanodrop^{™} 2000c spectrophotometer (Thermo Fisher Scientific, Waltham, Massachusetts, United States). The amount of collected RNA was 103.48 ng/µl (± 13.31 ng/µl), 1 µg of which was used to produce 20 µl volume of cDNA (50 ng/µl final cDNA concentration). cDNA was diluted 5× (10 ng/µl final concentration) for qPCR experiments. *ZNF81* expression was measured using qPCR. Basel *ZNF81* expression in RL95-2 cells were measured using similarly processed untreated RL95-2 cells.

### Assigning quantitative values for embryo quality parameters

Embryos were graded morphologically using the system introduced by Gardner et al., 2000. The letter grades in the scoring system were replaced by numeric grades as shown in Table 4 - 7.

**Table 4 - Quantitative values for blastocoel expansion**

| Characteristic | Numeric grade |
|---|---|
| The fluid filled cavity takes up less than half the space of embryo | 1 |
| The fluid filled cavity takes up more than half the space of embryo | 2 |
| The blastocyst cavity has expanded into the entire volume of the embryo pressing the trophectoderm cells up tightly against the inside of the zona | 3 |
| Expanded blastocyst, where the blastocyst has increased beyond the original volume of the embryo and caused the zona pellucida "shell" to become super thin | 4 |
| Embryo has breached the zona pellucida and is hatching out of its shell | 5 |
| Embryo is completely hatched | 6 |

**Table 5 - Quantitative values for inner cell mass quality**

| Characteristic | Letter grade | Numeric grade |
|---|---|---|
| Many cells, tightly packed | A | 3 |
| Several cells, loosely packed | B | 2 |
| Very few cells | C | 1 |

**Table 6 - Quantitative values for trophectoderm quality**

| Characteristic | Letter grade | Numeric grade |
|---|---|---|
| Many cells, forming a cohesive layer | A | 3 |
| Few cells, forming a loose layer | B | 2 |
| Very few large cells | C | 1 |

**Table 7 - Quantitative values for Day 3 embryo (Starting score 3.5)**

| Characteristic | Ideal level | Penalty of non-ideal characteristics |
|---|---|---|
| Number of blastomeres | 8 blastomeres | -0.5 |
| Blastomere size | Equal sized blastomeres | -0.5 |
| Embryo shape | Spherical embryo | -0.5 |
| Fragmentation | No fragmentation | 10-25% fragmentation -0.5 |
| | | 25-50% fragmentation -1.0 |
| | | >50% fragmentation -1.5 |
| Multinuclearity | No multinuclearity | -0.5 |

Correlations between *ZNF81* down regulation and embryo quality parameters and pregnancy outcomes were calculated using the Spearman's rank-order correlation.

### RESULTS

Basal *ZNF81* expression level in 10 ng RNA from RL95-2 cells was 1.4 × 10⁷ (± 1.86 × 10⁶). The results from embryo conditioned media treated cells indicate that there were some significant correlations between day 5 blastocyst quality and conditioned media induced down regulation of ZNF81 in RL95-2 cells (Figures 7A to 7D). The Spearman's rank order correlation coefficient was -0.167 (p < 0.02) for the effect of blastocoel expansion score on *ZNF81* expression in RL95-2 cells (Figure 7A). The Spearman's rank order correlation coefficient was -0.04 (p = 0.31) for the effect of inner cell mass quality on *ZNF81* expression in RL95-2 cells (Figure 7B). The Spearman's rank order correlation coefficient was -0.099 (p = 0.119) for the effect of trophectoderm cell quality on *ZNF81* expression in RL95-2 cells (Figure 7C). The Spearman's rank order correlation coefficient was -0.204 (p < 0.01) for the effect of overall embryo quality on *ZNF81* expression in RL95-2 cells (Figure 7D).

The results indicate that there was a significant correlation between day 3 embryo quality and conditioned media induced down regulation of ZNF81 in RL95-2 cells (Figure 8). The Spearman's rank order correlation coefficient was -0.349 (p < 0.01) for the effect of day 3 embryo quality on *ZNF81* expression in RL95-2 cells.

The correlation between the pregnancy outcome of embryo transfer and embryo conditioned media induced down regulation of ZNF81 in RL95-2 cells are shown in Figures 9A to 9C. The Spearman's rank order correlation coefficient was -0.53 (p < 0.00001) for day 3 and 5 embryos conditioned media induced *ZNF81* down regulation in RL95-2 cells (Figure 9A). The Spearman's rank order correlation coefficient was -0.625 (p < 0.001) for day 5 blastocyst conditioned media induced *ZNF81* down regulation in RL95-2 cells (Figure 9B). The Spearman's rank order correlation coefficient was -0.365 (p <0.05) for day 3 embryo conditioned media induced *ZNF81* down regulation in RL95-2 cells (Figure 9C).

Table 8 provides information of day 5 and 3 single transferred embryos pregnancy prediction for 56 embryos. The quality of the embryos was tested in terms of quantification of *ZNF81* expression in RL95-2 cells supplemented with conditioned media from the embryos. An embryo was classified as having a good quality for transfer if the conditioned media from the same embryo was able to reduce *ZNF81* copy number down to 4 million copies in 10 ng of input RNA from RL95-2 cells, otherwise the embryo was determined as a poor quality embryo for transfer.

**Table 8 - Pregnancy prediction for day 5 and 3 single transferred embryos based on ZNF81 down-regulation in RL95-2 cells.**

| | | Pregnancy outcome | | Total |
|---|---|---|---|---|
| | | Pregnant | Not pregnant | |
| Test outcome | Good for transfer | 19 | 5 | 24 |
| | Not good for transfer | 6 | 26 | 32 |
| | Total | 25 | 31 | 56 |

The chance of implantation after embryo transfer was 45 %. The specificity of the test was 26 / (26 + 5) × 100 = 84 % and the sensitivity of the test was 19 / (19 + 6) × 100 = 76 %. The positive predictive value of the test was 19 / (19 + 5) × 100 = 79 % and the negative predictive value of the test was 26 / (6 + 26) × 100 = 81%.

If the embryo transfers are done based on the test outcome, the implantation rate of 79 % after a single embryo transfer would be achieved, i.e., nearly double the success rate without selecting embryos based on the *ZNF81* copy number testing.

Figures 27A to 27N illustrate gene expression of selected genes in RL95-2 cells when treated with Jar EVs. Figure 28O illustrates the ability of predicting the outcome of embryo transfer and pregnancy using the reporter gene *ALDOC.* Table 9 provides information of single transferred embryos pregnancy prediction for 17 embryos. The quality of the embryos was tested in terms of quantification of *ALDOC* expression in RL95-2 cells supplemented with conditioned media from the embryos. An embryo was classified as having a good quality for transfer if the conditioned media from the same embryo was able to reduce *ALDOC* copy number from 100 million copies in 10 ng of input RNA down to 4 million copies in 10 ng of input RNA from RL95-2 cells, otherwise the embryo was determined as a poor quality embryo for transfer.

**Table 9 - Pregnancy prediction for transferred embryos based on ALDOC down-regulation in RL95-2 cells.**

| | | Pregnancy outcome | | Total |
|---|---|---|---|---|
| | | Pregnant | Not pregnant | |
| Test outcome | Good for transfer | 2 | 3 | 5 |
| | Not good for transfer | 0 | 12 | 12 |
| | Total | 2 | 15 | 17 |

The chance of implantation after embryo transfer was 12 %. The specificity of the test was 12 / (12 + 3) × 100 = 80 % and the sensitivity of the test was 2 / (2 + 0) × 100 = 100 %. The positive predictive value of the test was 2 / (2 + 3) × 100 = 40 % and the negative predictive value of the test was 12 / (12 + 0) × 100 = 100%.

If the embryo transfers are done based on the test outcome using *ALDOC* copy number, the success rate would increase more than three times after a single embryo transfer.

### EXAMPLE 3

### MATERIALS AND METHODS

### Cell culture and spheroid formation

The human endometrial adenosquamous carcinoma cell line (RL95-2) was obtained from American Type Culture Collection (ATCC CRL-1671, Teddington, UK). RL95-2 was cultured in Dulbecco's Modified Eagles Medium (DMEM 12-604F, Lonza, Verviers, Belgium) supplemented with 1% Penicillin/Streptomycin (P/S, Gibco^{™} 15140122, Bleiswijk, Netherlands), 5 µg/ml Insulin (human recombinant insulin, Gibco, Invitrogen, Denmark), 1% L-glutamine (Sigma, 59202C, Saint Louis, USA) and 10% fetal bovine serum (Gibco^{™}, 10500064) at 37°C in 5% CO₂ atmosphere.

The human choriocarcinoma cell line (JAr) from the first trimester trophoblasts was acquired from ATCC (HTB-144^{™}, Teddington, UK). JAr cells were cultured in a T75 flask in RPMI 1640 media (Gibco, Scotland) supplemented with 10% FBS, 1% L-glutamine and 1% P/S at 5% CO₂ in 37°C. At confluency, JAr cells were washed with Dulbecco's phosphate-buffered saline without Ca²⁺ and Mg²⁺ (DPBS, Verviers, Belgium), harvested using trypsin-EDTA (Gibco^{®} Trypsin, New York, USA) and pelleted by centrifugation at 250 × g for 5 minutes. 1 × 10⁶ cells/ml were cultured in 5 ml of supplemented RPMI 1640 medium in 60 mm Petri dishes at 5% CO₂ in 37°C. The cells were kept on a gyratory shaker (Biosan PSU-2T, Riga, Latvia), set at 295 rotations per minute (rpm) for 18 h. The viability of produced spheroids was confirmed by Live/dead^{®} viability/cytotoxicity assay kit (Molecular Probes, Eugene, Oregon, USA), according to the manufacturer's instructions. The multicellular spheroids were used to mimic trophoblast cells *in vitro.*

The human embryo kidney (HEK) 293T cell line was cultured in DMEM/Low glucose medium supplemented with 10% of heat inactivated FBS (Gibco), and 1% L-glutamine (Sigma). All cells were grown in 100 mm dishes at 37°C in a 5% CO₂ atmosphere. The media was changed every second day until confluence of the cells. One million cells were counted with a haemocytometer and cultured overnight on a gyratory shaker to form multicellular spheroids as described above.

### EV depletion of FBS

Extracellular vesicles in FBS were depleted using the protocol published by Kornilov et al. 2018. Briefly, FBS was ultra-filtered using Amicon ultra-15 centrifugal filters (100 kDa) for 55 min at 3,000 × g in room temperature. The flow through was collected and filtered with 0.22 syringe filters for sterilization before using in cell culture.

### EVs purification and nanoparticle tracking analysis (NTA)

Multicellular spheroids were cultured for 24 hours in media supplemented with EV depleted FBS. Conditioned media was collected and centrifuged at 400 × g for 10 minutes. The supernatant was collected and further centrifuged at 4,000 × g for 10 minutes. The supernatant was further centrifuged at 10,000 × g for 10 minutes. Sequential centrifugation was used to deplete the cell debris and larger particles. Collected supernatant was concentrated to 500 µl with Amicon^{®} Ultra-15 centrifugal filter devices (10 kDa cut-off). EVs were isolated using size exclusion chromatography (SEC). A cross linked 4% agarose matrix of 90 µm beads were used (Sepharose 4 fast flow^{™}, GE HealthCare Bio-Sciences AB, Uppsala, Sweden) in a 30 cm column. Fractions 7-10 (fraction size 1 ml) were collected. Fractions were concentrated using Amicon^{®} Ultra-15 centrifugal filter devices (10 kDa cut-off). Isolated EVs were quantified using NTA (ZetaView, Particle Metrix GmbH, Inning am Ammersee, Germany).

### Western blot analysis

Purified EVs from trophoblast spheroids were precipitated by adding 200 µl of water, 400 µl of methanol and 100 µl of chloroform to 200 µl of EVs. The solution was vortexed and centrifuged 14,000 × g for 5 min at room temperature. After removing the top layer, precipitated proteins were washed with 400 µl of methanol and centrifuged again. The pellets were air-dried, resuspended in 0.5% SDS and the protein concentrations were determined by Bradford assay. 30 µg of protein were heated for 5 min at 95°C in reducing (for Apo A-I detection) or in non-reducing (for CD63, CD9 and CD81 detection) Laemmli buffer and resolved in 12% SDS-PAGE according to standard protocol. Proteins were transferred onto polyvinylidene difluoride membrane (Thermo Fisher Scientific), followed by blocking in 5% non-fat dry milk in PBS-T (0,05% Tween-20, Thermo Scientific, Michigan, USA) for 1 h at room temperature. Subsequently, membranes were incubated with the primary anti-CD63 (sc-5275, 1:1000, Santa Cruz Biotechnology Inc., Dallas, TX), anti-CD9 (MA1-80307, 1:1000, Thermo Fisher Scientific, Loughborough, UK), anti-Apo A-I (sc-376818, 1:1000, Santa Cruz Biotechnology Inc. Dallas, TX), and anti-CD81 (555675, 1:1000, BD Biosciences, New Jersey, USA) antibodies overnight at 4°C in 5% milk-PBS-T solution and then with horseradish peroxidase conjugated goat anti-mouse secondary antibody (sc-516102, 1:1000, Santa Cruz Biotechnology Inc. Dallas, TX) for 1 h at room temperature. Membranes were washed three times for 5 min in PBS-T after each incubation step. Protein bands were detected using ECL SelectTM Western Blotting Detection Reagent (GE Healthcare, Buckinghamshire, UK) with ImageQuantTM RT ECL Imager (GE Healthcare, Buckinghamshire, UK).

### Electron microscopy

Suspension of EVs was deposited on formvar-carbon-coated 200 mesh copper grids (Agar Scientific, Essex, UK) for transmission electron microscopy (TEM) analysis according to the method described by Thery et al. 2006 Briefly, EVs on grids were fixed in 2% paraformaldehyde (P6148, Sigma-Aldrich, Schnelldorf, Germany) and 1% glutaraldehyde (O 1909-10, Polysciences, Warrington, USA), before being contrasted in uranyl oxalate [mixture of 4% uranyl acetate (21447-25, Polysciences, Warrington, USA) and 0,15 M oxalic acid (75688, Sigma-Aldrich, Schnelldorf, Germany)] and embedded in a mixture of methylcellulose (M6385, Sigma-Aldrich, Schnelldorf, Germany) and uranyl acetate (21447-25, Polysciences, Warrington, USA). Samples were observed with a JEM 1400 transmission electron microscope (JEOL Ltd. Tokyo, Japan) at 80 kV, and digital images were acquired with a numeric camera (Morada TEM CCD camera, Olympus, Germany).

### EV filtration

Syringe filters were used to filter isolated EVs. 0.1 µm and 0.22 µm filters were used. Filters were primed using nuclease free water before filtration. Filtrates were quantified using NTA (ZetaView, Particle Metrix GmbH, Inning am Ammersee, Germany).

### Total RNA extraction and quality control

Total RNA was extracted from endometrial cell line by TRIzol Reagent and isopropanol precipitation (TRIzol^{®} reagent; Invitrogen). To increase the efficiency of RNA extraction, 15 µg glycogen (UltraPure^{™} Glycogen, Cat. no. 10814-010, Thermo Fisher Scientific, Bleiswijk, Netherlands) was added to the aqueous phase of the sample in the precipitation step. The RNA pellet was washed three times by 75% ethanol. RNA was quantified using Qubit^{™} RNA HS Assay Kit (Q32855, ThermoFisher Scientific, Waltham, Massachusetts, United States). Quality of the extracted RNA samples was analyzed by Bioanalyzer Automated Electrophoresis instrument (Agilent technologies, Santa Clara, CA) using Agilent RNA 6000 Nano kit (Agilent technologies).

### cDNA synthesis and qPCR analysis

Gene expressions of *ZNF81* and the house keeping genes Beta-actin, Beta-2-microglobulin and Glyceraldehyde 3-phosphate dehydrogenase (*GAPDH*) were analyzed using SYBR green based quantitative PCR. cDNA synthesis was carried out using FIREScript RT cDNA Synthesis mix^{™} with Oligo (dT) and random primers (06-20-00100, Solis BioDyne, Tartu, Estonia) using the following program: Primer annealing 25°C for 10 min, reverse transcription 50°C for 60 min and enzyme inactivation 85°C for 5 min (Veriti^{™} Thermal Cycler, Applied Biosystems, Foster City, California, United States).

The primers for candidate transcripts were designed by Beacon designer 8 (PREMIER Biosoft International, Palo Alto, CA) (Table 1). cDNA products were amplified using HOT FIREPol^{®} EvaGreen^{®} qPCR SuperMix (08-36-00001, Solis BioDyne, Tartu, Estonia) in QuantStudio 12K Flex^{™} real time PCR system. Following program was used: Enzyme activation 95°C for 15 min followed by 40 cycles of 95°C for 20 s, 60°C for 20 s, and 72°C for 20 s. For melting curve analysis, the fluorescence signals were collected continuously from 65°C to 95°C at 0.05°C per second.

For spike-in and normalizing of candidate transferred transcripts, 100 bp from Isopenicillin N-CoA synthetase gene was used (Biomer.net company, Ulm/Donau, Germany, molecular weight: 32239 g/mol, 100 pmol/µl). Spike-in RNA sequence: UUGGGCAGAAACCGGGCCCCAACGGUGACCGCACCUACU ACUGCAUCCCGCUCUACCACGGAACGGGGGGCAUCGCGGCCAUGAACGACUUGAUGAGCGG (SEQ ID NO: 13). Spike-in Forward primer: TACTGCATCCCGCTCTAC (SEQ ID NO: 11). Spike-in Reverse primer: CGCTCATCAAGTCGTTCA (SEQ ID NO: 12). Synthetic RNA was serially diluted 20 times. For the first serial dilution, 1 µl of synthetic RNA was added to 39 µl RNase-free water to final concentration of 2.5 µM. Serial dilutions were prepared with a dilution factor of 4×. Serial dilutions were reverse-transcribed and amplified using real-time PCR and the cycle threshold (Ct) values of dilutions were plotted against the copy number of transcript. Exponential calibration curve was fitted. In parallel, 1 µl of synthetic transcript was added to the sample during TRIzol RNA extraction and then the Ct of synthetic RNA in this sample was assayed to calculate the RNA extraction efficiency and normalizing factor. Spike-in RNA expression Ct values were used as a scale to calculate absolute copy number of the target genes expressed.

### Statistical analysis

Data were presented as mean ± standard error of mean (SEM). In experiments that warranted statistical analysis for comparison of means, one-way ANOVA was used with appropriate post hoc analysis after testing the homogeneity with Leven's test.

### EXPERIMENTAL DESIGN

### Conformation of spheroid derived nanoparticles as EVs

Nanoparticles were characterized using nanoparticle tracking analysis, electron microscopy and western blot analysis to confirm their nature as EVs.

### Quantification of the number of EVs required to down regulate ZNF81 expression in RL95-2 cells

RL95-2 cells were seeded into 12 well plates (1 × 10⁵ cells per well). At 80% confluency, the culture media was replaced with EV depleted complete medium supplemented with various concentrations of JAr spheroid derived EVs. JAr EVs were diluted using EV depleted complete culture medium to gain final concentrations of 1 × 10¹¹ EV/ml, 1 × 10¹⁰ EV/ml, 1 × 10⁹ EV/ml, 1 × 10⁸ EV/ml, 1 × 10⁷ EV/ml, 1 × 10⁶ EV/ml, 1 × 10⁵ EV/ml, 1 × 10⁴ EV/ml. Untreated cells were used as negative controls. EVs were supplemented to the RL95-2 cells and incubated for 24 hours. After incubation, cells were lyzed using trypsin EDTA and counted using automated cell counter (Bio-Rad-TC10^{™}, Bio-Rad Laboratories, Hercules, California, United States). Whole RNA from 5 × 10⁵ cells from each well was extracted and analyzed for the expression of *ZNF81* using qPCR.

### Characterization of the timeline of EV induced ZNF81 down regulation in RL95-2 cells

RL95-2 cells were seeded into 12 well plates (1 × 10⁵ cells per well). At 80% confluency, the culture media was replaced with 200 µl of EV depleted complete medium supplemented with JAr spheroid derived EVs (final concentration; 1 × 10⁸ EV/ml). Cells were incubated for 30 min, 1 hr, 2 hr, 4 hr, 6 hr and 24 hr separately. Identically prepared RL95-2 cells without EV supplementation were incubated similar lengths of times and used as negative controls. After incubation, whole RNA from 5 × 10⁵ cells from each well was extracted and analyzed for the expression of *ZNF81* using qPCR.

### EV receiver cell specificity of JAr EV induced ZNF81 down regulation

HEK293 cells and RL95-2 cells were seeded into 12 well plates (1 × 10⁵ cells per well) and incubated until 80% confluency with daily media changes. Then, the culture media was replaced with 200 µl of EV depleted complete medium supplemented with JAr spheroid derived EVs (final concentration; 1 × 10⁸ EV/ml). After 24 h incubation, whole RNA from 5 × 10⁵ cells from each well was extracted and analyzed for the expression of *ZNF81* using qPCR.

### Characterization of the relationship between supplemented EV size and the EV induced ZNF81 down regulation in RL95-2 cells

Isolated EVs were filtered using 0.1 µm and 0.2 µm syringe filters in separate groups. Size profile of the filtrates was analyzed using NTA. Filtered EVs were supplemented to 80% confluent RL95-2 cell monolayers in 12 well plates and cultured in EV depleted media (EV concentration; 1 × 10⁸ EV/ml). After 24 h incubation, whole RNA from 5 × 10⁵ cells were collected and analyzed for the expression of *ZNF81* using qPCR.

### Characterization of the contribution of non-EV fractions of JAr spheroid conditioned media to the EV induced ZNF81 down regulation in RL95-2 cells

Characterization of the functionality of pre-EV and post-EV fractions collected from SEC was used to establish EVs as the mode of communication between the trophoblast spheroids and the endometrial epithelial cells. Conditioned media was collected in 18 fractions of 1 ml. Particle concentration of each fraction was analyzed using NTC. Protein concentration of each fraction was quantified using Pierce^{™} modified Lowry protein assay kit (23240, Thermo Scientific, Rockford, IL, USA). Fractions 1-5 were considered to be pre-EV. Fractions 6-9 contained EVs and fractions 10-18 were considered to be post-EV depending on the particle and protein concentrations of each fraction. EVs from each group (pre-EV, EV and post-EV) were supplemented to RL95-2 cells in EV depleted media (1 × 10⁸ EV/ml concentration). After 24 h of incubation, total RNA from 5 × 10⁵ cells were collected and analyzed for the expression of *ZNF81* using qPCR.

### Identifying the down regulated regions of ZNF81

Primers were prepared for the five exons and the exon-exon junctions of the mature mRNA of *ZNF81* (Table 10). Expression of the specific regions of the mRNA was measured in RL95-2 cells incubated in EV depleted medium supplemented with JAr EVs (1 × 10⁸ EV/ml concentration). After 24 h of incubation, total RNA from 5 × 10⁵ cells were collected and analyzed for the expression of different regions of *ZNF81* using qPCR.

**Table 10 - primer sequences**

| Primer name | Primer sequence (5'-3') | SEQ ID NO: |
|---|---|---|
| ZNF81-F | TGATACAGAAGACTTGAGATT | 1 |
| ZNF81-R | TCACAAAGTATTCACATTACC | 2 |
| Z-1-F | GAAGCGGCTGCGGTTCTC | 20 |
| Z-1-R | TGAACGTCGAATCCTCCTGACAAC | 21 |
| Z-1-2-F | GGATGTGGAGAGTTCTTGGA | 22 |
| Z-1-2-R | GCTGGGGTCAGAAGGAAG | 23 |
| Z-2-F | CTTGGAGTCTCTGCGGAG | 24 |
| Z-2-R | GGCTTTCTTGCTGACAACTT | 25 |
| Z-2-3-F | GTGCCTGTGAGGTATCAGTGTC | 26 |
| Z-2-3-R | GCGTCTTTGAGTAGAGTCCAGTTG | 27 |
| Z-3-F | GAGGATGTGACTGTGGACTT | 28 |
| Z-3-R | GCAGGTGGCTGTAGTTCT | 29 |
| Z-3-4-F | GGCAGCAACTGGACTCTACT | 30 |
| Z-3-4-R | TCGAACCCCACTGAGAGC | 31 |
| Z-4-F | AGTTCCTAAACCAGAGGTCATC | 32 |
| Z-4-R | GGCTTCCCCTTCCAATGT | 33 |
| Z-4-5-F | GTCATCTTCAAGTTGGAGCAAGGA | 34 |
| Z-4-5-R | ATTTCCCATCTGAACAGCTCTGAT | 35 |
| Z-5-F | CAGTGGATGACTATGGAGAAGA | 36 |
| Z-5-R | TACAGCAGGAAGGAAGATGAG | 37 |

### RESULTS

### Amount of JAr spheroid derived EVs required to down regulate ZNF81 in RL95-2 cells

A gradient of JAr spheroid derived EVs were supplemented to a unit number of RL95-2 cells and incubated for 24 h in EV depleted medium. After the incubation, whole RNA from 5 × 10⁵ cells from each well was extracted and analyzed for the expression of *ZNF81* using qPCR. Cells supplemented with higher than 1 × 10⁸ JAr spheroid derived EVs exhibited a significant down regulation (p < 0.05) while cells supplemented with lower amounts of JAr spheroid derived EVs did not show a significant down regulation compared to untreated negative controls. Number of Jar spheroid derived EVs required to induce a significant down regulation in RL95-2 cells can be calculated as 200 EVs per cells (Figure 10).

### Timeline of EV induced ZNF81 down regulation in RL95-2 cells

JAr spheroid derived EVs were co-incubated with RL95-2 cells for varying time limits. After each incubation, whole RNA from EV treated cells and untreated controls were isolated and analyzed for the expression of *ZNF81* using qPCR. Statistically significant down regulations were observed in *ZNF81* expression as early as 30 minutes after EV supplementation. Lowest *ZNF81* expression was observed in the 2-hour incubation group (Figure 11).

### JAr EV induced down regulation of ZNF81 expression is absent in HEK293 cells

HEK293 cells were co-incubated with 1 × 10⁸ JAR spheroid derived EVs for 24 hours. Whole RNA of the treated HEK293 cells and the untreated controls were analyzed for *ZNF81* expression using qPCR. There was no down regulation of *ZNF81* expression in EV treated HEK293 cells compared to untreated control. *ZNF81* in treated cells were up regulated (Figure 12).

### Size of the extracellular vesicle is not a significant factor in JAr spheroid derived EV induced down regulation of ZNF81 in RL95-2 cells

JAr spheroid derived EVs were filtered using 100 nm and 200 nm syringe filters separately (Figure 13). Filtered EVs and unfiltered EVs were supplemented to RL95-2 cells and co incubated for 24 h in EV depleted medium. Whole RNA from treated cells and untreated controls were extracted and analyzed for the expression of *ZNF81.* Both filtered EVs and un-filtered EVs were able to down regulate *ZNF81* expression in RL95-2 cells significantly (P < 0. 05) compared to the untreated control. There were no significant differences between the treatment groups (Figure 14).

### Down regulation of ZNF81 in RL95-2 cells co-incubated with JAr spheroid conditioned media is due to extracellular vesicles

RL95-2 cells were co incubated with concentrated JAr spheroid conditioned media, pre-EV fraction of the conditioned media, EV fraction and the post EV fraction separately for 24 hours (Figure 15). In RL95-2 RNA, only the concentrated conditioned media and the EV fraction were able to induce a significant (p< 0.01) down regulation compared to the untreated control (Figure 16).

### The whole mature ZNF81 mRNA is down regulated in JAr EV induced ZNF81 down regulation in RL95-2 cells

Primers were prepared for the five exons and the exon-exon junctions of the mature mRNA of *ZNF81.* Expression of the specific regions of the mRNA was measured in RL95-2 cells incubated in EV depleted medium supplemented with JAr EVs (1 × 10⁸ EV/ml concentration). After 24 h of incubation, total RNA from 5 × 10⁵ cells were collected and analysed for the expression of different regions of *ZNF81* using qPCR. All the regions analyzed exhibited a down regulation compared to control samples (Figure 17).

### EXAMPLE 4

In the current Example, the hypothesis that embryonic RNA, packaged in EVs, are capable of significantly altering the endometrial transcriptome to induce a favorable environment for implantation was investigated. The RNA cargo of trophoblast EVs was characterized together with the effect they would induce on receptive endometrium while demonstrating that the effects are unique to embryonic EV. Human choriocarcinoma cell line JAr in 3D spheroidal form was used as an analogue for the trophoblast and the RL95-2 cell line was used as an analogue for the mid-secretary receptive endometrium. The model is a well-established tool to study the trophoblast spheroids (embryo like structures) attachment to endometrial cells. Both cell lines are reported to be superior to other available cell types in mimicking the characteristics of pre-implantation embryo and the receptive endometrium.

### MATERIALS AND METHODS

### Cell culture and spheroid formation

The human endometrial adenosquamous carcinoma cell line (RL95-2) was obtained from American Type Culture Collection (ATCC CRL-1671, Teddington, UK). RL95-2 was cultured in Dulbecco's Modified Eagles Medium (DMEM 12-604F, Lonza, Verviers, Belgium) supplemented with 1% Penicillin/Streptomycin (P/S, Gibco^{™} 15140122, Bleiswijk, Netherlands), 5 µg/ml Insulin (human recombinant insulin, Gibco, Invitrogen, Denmark), 1% L-glutamine (Sigma, 59202C, Saint Louis, USA) and 10% fetal bovine serum (Gibco^{™}, 10500064) at 37°C in 5% CO₂ atmosphere.

The human choriocarcinoma cell line (JAr) from the first trimester trophoblasts was acquired from ATCC (HTB-144^{™}, Teddington, UK). JAr cells were cultured in a T75 flask in RPMI 1640 media (Gibco, Scotland) supplemented with 10% FBS, 1% L-glutamine and 1% P/S at 5% CO₂ in 37°C. At confluency, JAr cells were washed with Dulbecco's phosphate-buffered saline without Ca²⁺ and Mg²⁺ (DPBS, Verviers, Belgium), harvested using trypsin-EDTA (Gibco^{®} Trypsin, New York, USA) and pelleted by centrifugation at 250 × g for 5 minutes. 1 × 10⁶ cells/ml were cultured in 5 ml of supplemented RPMI 1640 medium in 60 mm Petri dishes at 5% CO₂ in 37°C. The cells were kept on a gyratory shaker (Biosan PSU-2T, Riga, Latvia), set at 295 rotations per minute (rpm) for 18 h. The multicellular spheroids were used to mimic trophoblast cells *in vitro.*

The human embryo kidney (HEK) 293T cell line was cultured in DMEM supplemented with 10% of heat inactivated FBS (Gibco), and 1% L-glutamine (Sigma). All cells were grown in T75 flasks at 37°C in a 5% CO₂ atmosphere. The media was changed every second day until confluence of the cells. One million cells were counted with a haemocytometer and cultured overnight on a gyratory shaker to form multicellular spheroids as described above.

### Preparation of EV depleted medium

EV depleted FBS was produced using the ultrafiltration method described by Kornilov et al. 2018. Briefly, the FBS was filtered using Amicon ultra-15 centrifugal filters (100 kDa) at 3000 × g for 55 minutes. This method removed 90% of the nanoparticles from the FBS. The filtered FBS was used as a 10% supplementation for all the cell type specific complete culture media described above to prepare the EV depleted complete media.

### EVs purification and characterization

EVs were harvested from conditioned media of spheroid culture. Conditioned media was then centrifuged at 400 × g for 10 min. the supernatant was further centrifuged at 4,000 × g for 10 min and the supernatant was further centrifuged at 20,000 g for 15 min to get rid of cell debris and apoptotic bodies. To isolate EVs, conditioned media was concentrated to 500 µl with Amicon^{®} Ultra-15 centrifugal filter devices (10 kDa cut-off). RNase inhibitor (1u/µl, Recombinant RNasin^{®}, Promega corp., 2800, Woods Hollow Road, Madison, WI) was added to conditioned media to protect EV RNA during the isolation process. EVs were isolated using size exclusion chromatography (SEC). A cross linked 4% agarose matrix of 90 µm beads were used (Sepharose 4 fast flow^{™}, GE HealthCare Bio-Sciences AB, Uppsala, Sweden) in a 30 cm column. Fractions 7-10 (fraction size 1 ml) were collected. Fractions were concentrated using Amicon^{®} Ultra-15 centrifugal filter devices (10 kDa cut-off). Isolated EVs were characterized following the protocols described in Es-Haghi et al. 2019. Briefly, EVs were quantified by nano particle tracking analysis using ZetaView (Particle Metrix GmbH, Inning am Ammersee, Germany). Surface proteome of the isolated EVs were analyzed using western blot for standard EV markers, CD63, CD81 and CD9. Morphology of the EVs was observed using transmission electron microscopy.

### Whole RNA extraction and quality control

Whole RNA was extracted from cells and EVs by TRIzol Reagent and isopropanol precipitation (TRIzol^{®} reagent; Invitrogen). To increase the efficiency of RNA extraction, 20 µg glycogen (UltraPure^{™} Glycogen, Cat. no. 10814-010, Thermo Fisher Scientific, Bleiswijk, Netherlands) was added to the lysis buffer per sample. The RNA pellet was washed three times by 75% ethanol. Quality and quantity of the extracted RNA samples were analyzed by Bioanalyzer Automated Electrophoresis instrument (Agilent technologies, Santa Clara, CA) using Agilent RNA 6000 pico kit (Agilent technologies).

### cDNA Library preparation and mRNA sequencing

RNA sequencing libraries were generated using multiplexing capacity of Smart-seq2 methodology with slight modifications (Picelli et al. 2014). Instead of single cells, 20 ng of total RNA was used for cDNA synthesis and 10 cycles of PCR for pre-amplification. KAPA HiFi DNA polymerase was replaced with Phusion High-Fidelity DNA Polymerase (Thermo Scientific) compatible with the original protocol. 2 µL of diluted cDNA was applied to dual-index library preparation using Illumina Nextera XT DNA Sample Preparation Kit (FC-131-1024). Ampure XP beads (Beckman Coulter) were used for all clean-up steps and for size selection 200-700 bp. All samples were pooled into single library by equal concentration and sequenced on Illumina NextSeq500 using High Output Flow Cell v2.5 (single-end, 75 bp).

### Processing, alignment, and quantification of RNAseq reads

The quality of raw reads was assessed using FASTQC v0.11.8 47. Trimmomatic v0.39 was used for read trimming and removal of adaptor sequences using the following parameters: LEADING:20 SLIDINGWINDOW:4:15 ILLUMINACLIP: *:1:30:15 MINLEN:25.

Reads were aligned to the hg19 human reference genome. The alignment was performed using HISAT2 48 with default parameters and with the inclusion of splice site information derived from the corresponding Ensembl *H. sapiens* annotation file (Homo_sapiens. GRCh38.97). The EV RNA samples yielded relatively low percentage of genes mapped to the genome. In HEK293 EV, 3.08% of 6820518 total alignments were successfully assigned on average. In JAr EVs, 4.48% of 4672213 total alignments were successfully assigned on average. In RL95-2 cells treated with JAr EVs, 5747968 reads were aligned on average and 32.39% of which were successfully assigned to the genome. Number of average aligned reads and percentage of successfully assigned reads were 5282088 (56.84%) in Runtreated RL95-2 cells and 4974678 (54.94%) in RL95-2 cells treated with HEK293 EVs. Gene-level read counts were obtained using featureCounts 49 with default parameters, using the Ensembl *H. sapiens* annotation file (Homo_sapiens. GRCh38.97) for genomic feature annotations. Genes with at least 10 counts for all the samples in at least one of the experimental groups were retained in the analysis.

### Differential gene expression analysis

Differential expression (DE) analysis was carried out in R version 3.6.1 using the edgeR package version 3.26.8 50. Tagwise dispersion estimates were obtained based on the trended dispersions, and statistical comparisons were performed using a generalized linear model followed by likelihood ratio tests, also accounting for the experiment batch. We considered the differential expression of genes (DEG) with a false discovery rate (FDR) ≤ 0.05 to be statistically significant.

Gene set enrichment analysis (GSEA), and pathway over-representation analysis was conducted using the clusterProfiler package (Yu et al. 2012) and Reactome Pathway database annotations (Yu et al. 2016). GSEA was used for full gene lists obtained from DE analysis that were ranked by -log₁₀p × log₂FC, where p denotes unadjusted p-values and FC the fold-change. Pathway over-representation analysis was used in the case of intersected gene lists. Obtained results were considered to be statistically significant at FDR ≤ 0.05.

Principal components were calculated using prcomp function from the Stats package and visualized using the ggplot2 package (Wickham 2016). The pheatmap package (Kolde 2019) was used for heatmap visualization with hierarchical clustering based on Euclidean distance.

### RNA extraction for miRNA sequencing

The EVs were sorted into 100 µl of RLT buffer (Qiagen) and proceeded for RNA extraction. Briefly, 100 µl of RLT buffer with sorted EVs is mixed with 2 µl of pellet paint (Merck Millipore), vortexed briefly, 19 µl of 3 M Sodium Acetate (pH 5.5) and 300 µl of 100% ethanol is added and vortexed briefly and incubated at +4°C overnight. The contents were then centrifuged at 16,000 × g for 15 min at 4°C and carefully the supernatant is discarded without disturbing the pellet. The pellet was washed twice with fresh 1 ml of 80% ethanol and air dried. The pellet is resuspended in 10 µl of RNAse free water and stored at -80°C till further use.

### Small RNA library Construction and data analysis

The small RNA transcriptome library was constructed for the different concentrations of EV's and HEK cells as described in Faridani et al. 2016 and Hagemann-Hensen et al. 2018 using 3 µl of extracted whole RNA from EV's and HEK cells. The amplified libraries were then purified using AMPure XP beads with 1:0.7 ratio of sample to beads as per the manufacturer protocol and eluted in 10 µl of RNAse free water. DNA quantification was done using Qubit HS DNA analysis (Thermo Scientific) and QC was performed on Bioanalyzer 2100 station (Agilent). 5ng of DNA from each sample is pooled and sequenced 1×100 bp using Illumina Novaseq platform (National Genomics Infrastructure, SciLifeLab, Sweden).

The initial data analysis was performed on the Partek Flow bioinformatics software (Partek Inc, USA). Briefly, all the fastq files were screened and the contaminating reads from the mitochondrial DNA and ribosomal DNA were removed. The UMI's were removed from the sequences and appended to the read names for later analysis. Adapters and poly A sequences were removed from the reads and the trimmed reads were aligned to human genome Hg38 using Bowtie 2 aligner with a seed length of 10 and seed mismatch of 1 nt. Post alignment the UMI were deduplicated and the reads were quantified to Hg38 miRBase mature microRNAs version 22 deduplicated.

### Identification of putative JAr-specific microRNAs and their putative targets in RL95 cells

To identify putative JAr EV-specific miRNAs, we examined miRNA alignment counts from three sRNAseq libraries derived from JAr EVs (total genome-mapped reads: 1359431) alongside three derived from HEK EVs (total genome-mapped reads: 1912942). The dataset was filtered to retain miRNAs which were detected in at least 2/3 libraries of either of JAr or HEK EVs. We subsequently counted the number of miRNAs which were detected above raw counts thresholds of 1, 3, 5, and 10 in the required number of samples. For downstream analysis, miRNAs were considered specific to JAr EVs if they were represented by at least five counts in 2/3 JAr EV libraries while not being detected at all in any of the HEK EV libraries.

A list of all predicted target transcripts from miRDB (Chen & Wang 2019) was obtained. These were filtered to retain only high-confidence targets (those with a target score of 90 or higher). REFSEQ transcript IDs were converted to ENSEMBL gene IDs to obtain a list of predicted miR targets at the gene level. We were thus able to identify putative miRNA targets in the RL95-2 gene expression dataset by matching the ENSEMBL IDs. We subsequently counted the number of putative targets within the RL95-e gene expression dataset that were down-regulated, up-regulated, and non-DE for each miRNA.

Focusing on down-regulated putative targets, we then sought to ascertain whether the abundance of a given miRNA corresponded with the extent of repression of downregulated targets. We obtained the mean counts per million (cpm) value for each miRNA in JAr EVs and the mean log₂FC of downregulated putative target genes for each miRNA. We then performed a weighted Pearson's correlation using the weights package, in which each miRNA was weighted according to the number of downregulated targets.

### EXPERIMENTAL DESIGN

### Investigating the RNA cargo of extracellular vesicles

JAr and HEK293 cells were cultured and spheroids were formed according to the methods and conditions described above in this Example. Approximately 1 × 10⁵ spheroids were prepared from each cell type. Once the spheroids were fully formed, they were transferred into 60 mm dishes containing 5 ml EV depleted culture media (5000 spheroids per dish). Spheroids were incubated in a slow rotating gyratory shaker for 24 hours to stop the spheroids from losing the structural cohesion. After incubation, conditioned media (approximately 100 ml) were collected and EVs were isolated. After removing the EVs used for supplementation, remaining EVs (approximately 1 × 10¹² EVs per each sample) were subjected to RNA extraction and mRNA seq was performed. Samples were prepared in three different days for EV supplementation and mRNA seq. Samples used for miRNA seq were prepared separately. 1 × 10⁷ - 1 × 10⁸ EVs were used for miRNA sequencing for each sample.

### Determining the effects of JAr and HEK293 cell derived EV on RL95-2 cellular transcriptome

Endometrial analogue (RL95-2) cells were cultured in 12 well plates until 80% confluency using the culture methods and conditions described above. At the desired confluency, growth media was removed and 1 × 10⁸ EVs derived from trophoblast analogue (JAr) and non-reproductive cellular spheroid (HEK293) cells, were added to the RL95-2 cell monolayer separately in an EV depleted supplementation media. Controls were prepared using untreated RL95-2 cells cultured in EV depleted media. Cells were incubated for 24 hours. After incubation, the media was removed and cellular RNA was collected for sequencing. The experiment was done in three different days to prepare the three samples.

### RESULTS

### JAr cell derived EVs induced significantly differentiated gene expression in RL95-2 cells while HEK293 cell derived EVs failed to induce a similar effect

JAr cell spheroid derived EVs and HEK293 cell spheroid derived EVs were supplemented to RL95-2 cell monolayers separately and incubated for 24 h. Control samples were prepared using untreated RL95-2 cells. After incubation, the cellular RNA was extracted and sequenced for mRNA expression. Differential expression was calculated with reference to untreated control (R). The principle component analysis shows the clustering of biological samples. RL95-2 cells treated with JAr spheroid derived EVs (RJ) is clearly separated from the untreated control (R) and the RL95-2 cells treated with HEK293 spheroid derived EV (RH) indicating high variance between the groups (Figure 18A). There is very limited variance between the untreated RL95-2 cells and RL95-2 cells treated with HEK EV indicating that there was none or very minute effect on RL95-2 cells from HEK293 derived EVs. The unsupervised heatmap, shows the relatively high number of significantly upregulated genes in RJ group (1166, see Annex A) and the down regulated genes (588, see Annex B) compared to the untreated RL95-2 cells. The similarity between the groups R and RH is also apparent (Figure 18B). We decided to exclude the group RH2 from analysis due to the high degree of outlier characteristics. The DE analysis data suggested that JAr spheroid derived EVs can induce significant changes in in RL95-2 transcriptome while the HEK293 derived EVs lack that capability.

### JAr spheroid derived EVs carry distinct mRNA cargo compared to HEK293 cell derived EVs

JAr and HEK293 spheroid conditioned media were used to isolate EVs using size exclusion chromatography. EV RNA was extracted and the mRNA cargo of the EVs was sequenced. After alignment, data was visualized using the integrated genome viewer (IGV). Both JAr and HEK293 EV derived mRNA were found to be highly fragmented. Exon spanning reads were sparse. Abundance of mRNA was quantified as counts per million after normalization. Enrichment of mRNA was calculated by contrasting the abundance of JAr EV mRNA to the abundance of HEK293 EV mRNA. The PCA plot exhibited a substantial variance between the JAr EV mRNA and HEK293 EV mRNA (Figure 19A). 400 mRNA were significantly enriched (logFC > 1) in JAr EV while 501 mRNA were significantly depleted (logFC < 1) compared to HEK293 EV (Figure 19B). The mRNA cargo of each EV type appears to be significantly different from each other.

### JAr spheroid derived EVs carry distinct miRNA cargo compared to HEK293 cell derived EVs

JAr EVs were also distinguished from HEK EVs according to their microRNA content. The miRNA filtering criteria influenced both the total number of microRNAs detected in either of the two EV types examined (Figure 20A) and the number of miRNAs which were unique to either JAr or HEK EVs (Figure 20B). When considering a read count threshold of five which had to be met in 2/3 libraries within a given group (JAr or HEK), 11 microRNAs were detected only in JAr EVs while only two were detected only in HEK EVs. These 11 microRNAs were subsequently taken for further analysis of their target genes.

### Pathway analysis

Gene set enrichment analysis (GSEA), and pathway over-representation analysis was conducted using the clusterProfiler package (Yu et al. 2012) and Reactome Pathway database annotations (Yu et al. 206). Some of the more significantly enriched pathways are listed in the Table 11.

**Table 11 - Pathways enriched by the effect of JAr EVs on RL95-2 cells. Normalized Enrichment Score (NES) and False Discovery Rate (FDR) indicate the significance of the enrichment**

| **Reactome ID** | **Description** | **NES** | **FDR** |
|---|---|---|---|
| R-HSA-372790 | Signaling by GPCR | 1.267 | 0.010 |
| R-HSA-388396 | GPCR downstream signaling | 1.289 | 0.010 |
| R-HSA-1474228 | Degradation of the extracellular matrix | 1.479 | 0.010 |
| R-HSA-1474244 | Extracellular matrix organization | 1.448 | 0.010 |
| R-HSA-1474290 | Collagen formation | 1.490 | 0.010 |
| R-HSA-216083 | Integrin cell surface interactions | 1.549 | 0.010 |
| R-HSA-3000171 | Non-integrin membrane-ECM interactions | 1.456 | 0.011 |
| R-HSA-3000157 | Laminin interactions | 1.568 | 0.019 |
| R-HSA-3000178 | ECM proteoglycans | 1.441 | 0.051 |

Extracellular matrix (ECM) organization and signaling by GPCR are the two major pathways enriched by JAR EVs effect of RL95-2 cells. Other significantly enriched pathways are major events of the two parent pathways. One of the two events of Signaling by GPCR (R-HSA-372790) is significantly enriched and 6 of the 11 events of ECM organization (R-HSA-1474244) are significantly enriched.

### Relationship between the abundance of mRNA in EV and the expression of the same gene in EV receiver cell

Here we have compared the abundance of an mRNA in EV (logCPM) and the expression of the same gene in the receiver cell (logFC) to test the relationship between the uptaken RNA and the expression in receiver cell. There was no significant correlation between the abundance of a transcript in EV and the fold change of the same gene in cells (Figure 21A, 21B). Similarly, there were no such correlations in the subsets of mRNA that were significantly enriched in EV (Figure 21C, 21D). The expression of a gene in target cells appear to be independent from the amount of similar transcript carried in the EV.

### miRNA abundance in JAr EVs correlates with fold change of downregulated target genes in RL95-2 cells

For the 11 JAr-specific miRNAs, a total of 1188 high-confidence putative gene targets were identified from miRDB applying a target score cutoff of 90. Of these, 744 were present within the RL95-2 gene expression dataset. Only a small proportion of these were differentially expressed, with 53 of them down-regulated and 68 of them up-regulated. Although more putative targets were up-regulated than down-regulated, putative miRNA targets constituted a higher percentage of total down-regulated genes (9%) compared to both total up-regulated (5.8%) and total non-differentially expressed genes (6.4%). Furthermore, six out of the eleven miRNAs had a greater number of targets which were down-regulated than up-regulated, while only four had a greater number of up-regulated than down-regulated targets (Figure 22A). hsa-miR-524-5p had the largest number of putative targets represented in the expression dataset, the 26 down-regulated targets of which constituted 4.4% of the total down-regulated genes.

Although the number of miRNAs examined was low, the mean log₂FC of down-regulated target genes displayed a moderate negative correlation with the abundance of a given miRNA in JAr EVs (weighted Pearson's correlation, r = -0.65, p = 0.041; Figure 22B). The most abundant JAr-specific miR was has-miR-1323, the down-regulated targets of which had the lowest log₂FC of all miRNAs except for hsa-miR-526b-5p, which had only one down-regulated target.

We also examined whether any down-regulated genes constituted high-confidence predicted targets of multiple miRNAs. In this regard, we found only two down-regulated genes that were the putative targets of at least three JAr-specific miRNAs: ATF2 (predicted target of hsa-miR-524-5p, hsa-miR-520a-5p, and hsa-miR-525-5p) and SPTSSA (predicted target of hsa-miR-524-5p, hsa-miR-526b-5p, and hsa-miR-1323), respectively.

### DISCUSSION

In the current Example, the effect of JAr spheroid derived EV (an analogue for pre-implantation embryo) and HEK293 spheroid derived EVs (a cell line of non-reproductive origin) on RL95-2 cells (an analogue for mid-secretary receptive endometrium) was studied.

JAr EVs induced substantial alterations to the RL95-2 transcriptome. Interestingly, HEK293 EV failed to induce any significant (FDR<0.05) alterations to the RL95-2 transcriptome (Figure 18). This compelling effect could be attributed to the differences of EV cargo of JAr and HEK293 EVs. HEK293 EVs, being derived from a cell type not of the reproductive lineage, were used as a control to investigate the specificity of JAr derived EVs in inducing transcriptomic changes in RL95-2. Since the JAr / RL95-2 model was used to mimic the pre-implantation uterine microenvironment in this Example, we could deduce that the transcriptomic changes induced by EVs are not random, but a purposeful process specific to a biological phenomenon, namely embryo maternal communication in this instance.

The purposeful nature of the JAr EV effect on RL95-2 cells is more apparent while considering the pathways enriched by the DEGs (Table 11). Majority of the participants of the extracellular matrix (ECM) organization pathway (R-HSA-1474244) were enriched by the genes in the RL95-2 cells treated with JAr EVs. ECM remodeling is a critical morphological and biochemical alteration the endometrium undergoes in preparation for the implantation. It promotes and stabilizes the embryo adhesion while protects the underlying stromal cell layer form over invasion by the extravillous trophoblast. Major participants of the pathway such as laminin interactions (R-HSA-3000157), integrin cell surface interactions (R-HSA-216083), non-integrin membrane-ECM interactions (R-HSA-3000171) are all implicated in endometrial modifications in the window of implantation.

The pathway of signaling by G-Protein couple receptor (GPCR) was significantly enriched (R-HSA-372790). GPCRs are the largest family of transmembrane receptors accounting for 4% of the coding regions of the human genome. They are known to bind a highly diverse set of ligands perform biological functions ranging from sight and olfactory senses to immune regulation. They also act as receptors for a number of ligands known to alter the endometrial microenvironment during the window of implantation, such as hCG, prostaglandin E2, cytokines and progesterone. Downstream signaling of GPCR (R-HSA-388396) pathway is also significantly enriched by the DEGs. These downstream pathways are secondary messengers that modify the endometrial morphology to facilitate implantation. For example, phosphatidylinositol 3-kinase/protein kinase B (PI3K/Akt), which regulates cell growth and survival, is reported to be involved in endometrial migration which is crucial in embryo attachment and invasion. Evidence from the enriched pathways led to notion that the transcriptomic changes induced by JAr EVs on RL95-2 cells is not only directly modifying the endometrium for imminent implantation, but also modifying and priming the membrane receptors for further reception of embryonic signals such as hormones and cytokines.

We investigated the nature of the EV populations derived from JAr and HEK293 cells to decipher the mechanism of EV induced transcriptomic changes. Identification of the RNA cargo of the two types of EVs by sequencing mRNA and miRNA was the first step of the investigation. There were significant (FOR < 0.05) differences between mRNA found in JAr and HEK293 EVs indicating that the two EV populations are distinct from each other (Figure 19). Using IGV to visualize the aligned reads, we observed that the RNA in both the samples was highly fragmented despite the efforts taken to protect RNA from external RNases during processing. Reads aligning to more than one exon were very sparse implying the near absence of large fragments. It should be pointed out that the library preparation system we have used was heavily biased towards polyadenylated RNA. Given the low number of aligned reads in EVs compared to cells, it could be stated that EV RNA, at least in the observed samples, contain non or very limited amount of intact mRNA.

In addition to mRNA, JAr EVs differed from HEK293 EVs in their microRNA composition. miRNAs are regulators of gene expression which uses multiple mechanisms to inhibit, destabilize and cleave transcripts. There are about 600 miRNA identified and characterized which target about 60% of the genes in humans. It is a well-documented fact that miRNA play a vital role in in cell-to-cell communication. Numerous studies present evidence that miRNAs are involved in EV mediated intercellular signaling. When applying a reasonable counts threshold, we identified eleven microRNAs in JAr EVs which were not detected in HEK293 EVs. Interestingly, while substantially more JAr-specific miRNAs could be detected lower counts thresholds, relaxing the counts threshold did not substantially influence the number of HEK-specific EVs, suggesting that the majority of miRNAs present in HEK EVs are indeed also present in JAr EVs. Meanwhile, given our use of robust filtering criteria based on both read count and consideration of replicate samples, we reasoned that the 11 microRNAs taken for downstream target analysis could reasonably be considered as `JAr-specific' relative to HEK EVs. Although the ability to detect differences between miRNA profiles of EV types is highly influenced by technical factors such as read depth and filtering criteria to detect miRNAs, multiple reports nevertheless corroborate the presence of contrasting miRNA profiles in populations of EVs isolated from different cell types and biological fluids. These differences could aid in interpreting the observed EV induced effects of RL95-2 transcriptome.

Considering the mechanisms of EV induced transcriptomic changes, most of the studies have followed the miRNA mediated gene expression regulation hypothesis which posits that miRNA transported by EV are uptaken by the target cell and proceeds to regulate the mRNA expression. As shown herein, this method of action might not be exclusive to miRNA by tracking embryonic RNA (mRNA and IncRNA) from embryo to the endometrium through EVs. There was an appreciable effect in the endometrium due to this transfer, namely, similar cellular transcripts were significantly down regulated. In the current Example, using the cargo mRNA seq data and the DEG data from cellular RNA, we observed that the abundance of a transcript in EVs does not correlate (R= 0.0026, p>0.05) with the gene expression of the same transcript in the target cell after the transfer (Figure 21A, 21B). This finding was true for both up-regulated (Figure 21C) and down-regulated (Figure 21D) transcripts in the EVs. Simply put, there is no correlation between the abundance of EV mRNA and the respective gene expression in target cells.

The function of mRNA in fragmented state is not a well understood phenomenon. There are reports of mRNA performing the same regulatory functions as the long non-coding RNA (IncRNA), which includes structural regulation, transcription control, translation control, miRNA sponging, elongation control, guiding epigenetic enzymes such as the polycomb repressive complex 2 (PRC2) and possibly RNA degradation by STAU1 mediated decay. Therefore, it can be hypothesized that, even in the fragmented form, EV mRNA may perform a regulatory function in the target cell. The lack of correlation between EV mRNA abundance and corresponding gene expression changes suggests that the phenomenon identified in previous Examples is specific to a limited number of genes using one or more of the identified mechanisms of mRNA-based gene regulation.

Although we hypothesized that some gene expression changes may be putatively linked with microRNAs present in JAr EVs, the finding that the majority of gene expression changes constituted up-regulation suggests that canonical microRNA-induced silencing is not the primary mode of action by which JAr EVs modulate gene expression changes in target cells. Indeed, the 11 microRNAs identified as JAr-specific had high-confidence putative targets among both down-regulated and up-regulated genes in RL95-2 cells. However, in the absence of any miRNA-induced silencing, we would expect the relative proportions of down-regulated and up-regulated putative targets to reflect the relative proportions of overall up- and down-regulation. However, this was not the case. Indeed, most JAr-specific miRNAs had more high-confidence predicted targets that were down-regulated than upregulated, and miRNA targets constituted a higher proportion of down-regulated genes compared to either up-regulated or non-DE genes, despite there being twice as many up-regulated than down-regulated genes overall. Furthermore, the log₂FC of downregulated putative targets negatively correlated with the abundance of miRNA detected in JAr EVs, with the putative targets of the most abundant JAr-specific miRNA (has-miR-1312) showing the strongest decrease in mean log₂FC. Collectively, these observations suggest that at least some of the downregulation was a direct result of canonical miRNA silencing. On the contrary to EV mRNA, the abundance of miRNA in the EVs significantly correlated with the logFC of the down-regulated targets genes in RL95-2 cell (Figure 22B), leading to the deduction that some of the DE seen in the RL95-2 cells treated with JAr EVs was due to the actions of miRNA. Observed disparity between the two types of EV RNA abundance and the DE of the cellular RNA could be due to the mode of regulation used by the two types of EV RNA and the extent of mRNA-based gene regulation transpired in this communication event.

Majority of the observed DE cannot be explained as a direct result of either mRNA or miRNA transported via EVs. Considering the relatively small copy number of RNA transported in EVs, attributing the substantial degree of DE to direct results of EV RNA intervention would be illogical. However, the role played by EVs as the agent of the effect is unmistakable.

In conclusion, trophoblast derived EVs, through transcriptomic alterations, were able to induce an environment favorable for implantation in the endometrium by remodeling the extracellular matrix and increasing the GPCR mediated signaling which would facilitate further embryo maternal communication. This effect was unique to the trophoblast spheroid derived EVs compared to EVs from a non-reproductive source. Differences of the effects could be due to the distinct RNA cargo of the EVs from the two sources. miRNA based post-transcriptional regulation is, at least partially, responsible for the induced transcriptomic alteration.

### EXAMPLE 5

Embryo-derived extracellular vesicles (EVs) may play a role in mediating the embryo-maternal dialogue at the oviduct during the pre-implantation period of embryonic development, potentially carrying signals reflecting embryo quality. This Example aimed to investigate the effects of bovine embryo-derived EVs on the gene expression of bovine oviductal epithelial cells (BOECs), and whether these effects are dependent on embryo quality. Presumptive zygotes were cultured individually *in vitro* in droplets of regular culture media till day 8 while evaluating their development. Conditioned media samples were collected at day 5 and pooled based on embryo development as good quality embryo media (conditioned by embryos that developed to blastocysts by day 8) and degenerating embryo media (embryos that degenerated after cleavage by day 2). EVs were isolated by size exclusion chromatography and supplemented to primary BOEC monolayer cultures to evaluate the effects of embryo-derived EV supplementation on their gene expression profile. Gene expression was quantified by both RNA sequencing and RT-qPCR. A total of 7 upregulated and 18 downregulated genes were detected in the good quality embryo-derived EV supplemented BOECs compared to the control. The upregulated genes included classical interferon-induced genes, such as OAS1Y, MX1, and ISG15. In contrast, only one differentially expressed gene was detected in BOECs in response to EVs derived from degenerating embryo media. The results show that these effects could be linked to EV-mediated communication, therefore, demonstrating that embryo-derived EVs are involved in embryo-maternal communication at the oviduct. Moreover, the observed oviductal responses were dependent on the embryo quality, indicating that this system could be used as an indirect method to evaluate the embryo quality.

### MATERIALS AND METHODS

### In vitro embryo production (IVP)

All chemicals were purchased from Sigma-Aldrich/Merck (Germany/USA), unless otherwise stated. The production of Bovine embryos was carried out as described by Nõmm et al. 2019 with modifications. Ovaries of cattle (*Bos taurus*)*,* recovered from the local slaughterhouse, were transported to the laboratory in 0.9% sterile NaCl solution within 4 h after the sacrifice at ~32-37°C and washed twice in fresh 0.9% NaCl. Cumulus-oocyte complexes (COCs) were aspirated from ovarian follicles with a diameter of 2-8 mm, using a vacuum pump (Minitüb GmbH, Germany). Quality code 1 COCs were selected, washed and *in vitro* matured (IVM) in groups of 50 in 500 µl of IVM-medium (supplemented with 0.8% fatty acid-free BSA fraction V) in 4-well plates (Nunc, Roskilde, Denmark) by incubating at 38.5°C with 5% CO₂ in humidified air for 22-24 h.

For *in vitro* fertilization (IVF) of matured oocytes, frozen-thawed semen was used. Thawed sperms were washed and diluted to the final concentration of 1 × 10⁶ motile sperms per ml. The sperms and COCs were co-incubated in groups of 50 in 500 µl of Fert-TALP media in 4-well plates at 38.5°C with 5% CO₂ in humidified air for 18-20 h.

Cumulus cells were detached from the presumptive zygotes by vortexing, and the denuded presumptive zygotes were cultured individually in 60 µl droplets of modified Synthetic Oviduct Fluid with amino acids and myo-inositol (SOFaaci) containing 0.8% BSA under mineral oil at 38.5°C, 5% CO₂ and 90% N₂ with humidified air for eight days. Embryos were morphologically evaluated at 2, 5, and 8 days post-fertilization, and the developmental stages and embryo quality were recorded as previously described in Bo & Mapletoft 2013. The 3 distinct development stages were: cleavage, morula, and blastocyst stage. In parallel, culture media samples were incubated as droplets for 5 days without embryos and labeled as "Day 5 control".

### Collection of the embryo conditioned media and isolation of EVs

Conditioned media samples (50 µl) were collected at day 5 (morula stage) post-fertilization from individually cultured bovine embryos. Following the collection of conditioned media, the embryos were continuously cultured in the remaining 10 µl culture media droplet upto day 8. The collected conditioned and control media samples were stored at -80°C until isolation of EVs.

The collected conditioned media samples were retrospectively categorized, based on the morphological evaluation of the embryos on days 2, 5, and 8 post-fertilization and the samples relevant to the study were identified. Media conditioned by embryos that developed to morula by day 5 and subsequently developed to blastocysts by day 8 (hereafter referred as "Day 5 good quality embryo media") and media conditioned by embryos that cleaved by day 2, but subsequently degenerated (hereafter referred as "Day 5 bad quality embryo media") were used as embryo conditioned media.

Samples belonging to "Day 5 good quality embryo media" (n = 40), "Day 5 bad quality embryo media" (n = 40), and "Day 5 control media" (n = 40) were thawed and pooled according to their category. Despite it is unlikely that pre-implantation embryos introduce dead cells or larger particles such as apoptotic bodies to the conditioned media due to its zona pellucida (ZP), sequential centrifugation was used to get rid of such potential cells or bigger particles as such particles could affect the EV purification by size exclusion chromatography method.

Pooled conditioned media and control media samples were subjected to double centrifugation steps. Initially, the samples were centrifuged at 400 × g for 10 min at 4°C to remove any dead cells and debris, and the supernatants were transferred to fresh tubes. The collected supernatants were centrifuged at 2,000 × g for 10 min to remove any apoptotic bodies. After centrifugation, the supernatants were transferred to new tubes and concentrated to 150 µl by centrifuging at 3,200 × g for 40 minutes at 4°C. The concentrated media samples were subjected to isolation of EVs.

Isolation of EVs was carried out using qEVsingle size exclusion chromatography columns (qEVsingle/70nm by Izon Sciences, UK, product code SP2). The columns were vertically mounted in a holder and equilibrated by running through 10 ml of fresh filtered (0.2 µm) elution buffer (DPBS). Then, the samples (150 µl of prepared media) were loaded to the top of the column, and fraction (200 µl) collection was initiated immediately. When the samples levelled with the upper column filter, the columns were topped up with the elution buffer. The first 5 fractions (total of 1000 µl, which was the void volume) were collected together and discarded. Fractions 6-9 (total of 800 µl) were collected and pooled as EVs elute in these fractions should there be any in the sample. The EV elutes were concentrated and adjusted to a final volume of ~220 µl by centrifuging at 3,200 g for 20 min. While 20 µl of the concentrated sample was used for the measurement of size and the concentrations of EVs by nanoparticle tracking analyzer-ZetaView^{®} as described previously (Dissanayake et al. 2020), the remaining 200 µl was aliquoted into 50 µl fractions and stored at -80°C till used for supplementation to BOECs monolayer cultures.

### Primary bovine oviductal epithelial cell culture

Bovine oviducts, with attached ovaries, were obtained from the slaughterhouse and transported in normal saline at 37°C within 4 hours from animal slaughter and sample collection. The selection of an oviduct from a single cow in the post-ovulatory stage of the estrous cycle was based on the ipsilateral ovary showing an ovulation site (Day 0-3 post-ovulation). The selected oviduct was washed with wash solution I (DPBS supplemented with 1% Amphotericin B and 1% Penicillin/Streptomycin) and dissected free of connective tissue. The isthmus part and the ampullary parts were separated. Oviductal mucosa was carefully expelled by squeezing the oviduct with a sterile glass slide, and the cells were retrieved and transferred into a conical tube containing washing medium II (DPBS supplemented with 5% fetal bovine serum (FBS), 1% Amphotericin B and 1% Penicillin/Streptomycin). BOECs were washed twice in wash media II by centrifuging at 180 × g for 2 minutes at 4°C. The final cell pellet was dissolved in 5 ml of culture media (DMEM/F12 media supplemented with 10% fetal bovine serum (FBS), 1% Amphotericin B and 1% Penicillin/Streptomycin) and seeded in a 10 cm culture dish in a final volume of 10 ml media and culture in a 5% CO₂ incubator at 38°C. After 72 hours, cells were checked, and the media was changed subsequently every 48 hours. When the cells reached 80% confluency, the cells were passaged once and cultured to increase the cell population. Cells were trypsinized and frozen in freezing media (DMEM/F12, 20% FBS, and 10% DMSO) in separate aliquots in Liquid Nitrogen till the extended BOEC culture.

### Extended BOEC culture and supplementation of embryo-derived EVs

One frozen vial of BOECs (ampullary region) was thawed at a time, and the cells were cultured in Petri dishes (100 mm) in DMEM/F12 media supplemented with 10% FBS in a humidified atmosphere with 5% CO₂ at 38.8°C. After 48 hours of culture, the BOEC monolayer was trypsinized and sub-cultured in 4-well plates (Nunc, Roskilde, Denmark) by adding 50,000 cells per well. Once the monolayer reached 80% confluency, the media was removed and washed once with synthetic oviductal fluid media (SOF) supplemented with 0.8% BSA. Then SOF media (450 µl) supplemented with 0.8% BSA and 50 µl of either thawed EVs isolated from embryo conditioned media or nanoparticles (NPs) isolated from control media were mixed and added to the relevant monolayer culture. After the supplementation, the BOEC monolayers were incubated for further 8 hours at 38.5°C with 5% CO₂ and 90% N₂ in humidified air. This experiment was carried out 4 times using BOECs cultured on 4 separate days (four technical replicates). Figure 23 illustrates the experiment design.

### RNA extraction, sequencing library preparation, and RNA sequencing

After the BOEC culture, the conditioned media were discarded, and the RNA extraction was carried out by guanidinium thiocyanate-phenol-chloroform RNA extraction method. In brief, 300 µl of guanidinium thiocyanate (Qiagen^{®} reagent; Invitrogen) was added to each monolayer and left at room temperature for 10 minutes. After mixing thoroughly by pipetting, the samples were transferred to sterile microcentrifuge tubes, and 150 µl of Chloroform was added. After vortexing for 15 s and incubating at room temperature for 3 min, the samples were centrifuged at 12,000 × g for 15 min at 4°C. Of the 3 layers formed, the aqueous phase was transferred to a new tube, and an equal volume of isopropyl alcohol was added. In order to increase the RNA yield, 20 µg (1 µl) of glycogen (UltraPure^{™} Glycogen, Cat. no. 10814-010, Thermo Fisher Scientific, Bleiswijk, Netherlands) was added. The sample was incubated at RT for 10 min and centrifuged at 12,000 × g for 30 min at 4°C. The precipitated RNA pellet was washed thrice with 500 µl of 70% ethanol by centrifuging at 12,000 × g for 5 min at 4°C. The pellet was air-dried and diluted in 20 µl of nuclease-free water by incubating at 70°C for 10 min. RNA was quantified using Qubit^{™} RNA HS Assay Kit (Q32852, ThermoFisher scientific), and the quality was determined by Bioanalyzer Automated Electrophoresis instrument (Agilent Technologies, Santa Clara, CA) using Agilent RNA 6000 nano Kit (Agilent technologies).

RNA sequencing libraries were generated using multiplexing capacity of Smart-seq2 methodology (Picelli et al. 2014) with slight modifications. Instead of single cells, we used 20 ng of total RNA for cDNA synthesis and 10 cycles of PCR for pre-amplification. We replaced KAPA HiFi DNA polymerase with Phusion High-Fidelity DNA Polymerase (Thermo Scientific) compatible with the original protocol. Two µL of diluted cDNA was applied to dual-index library preparation using Illumina Nextera XT DNA Sample Preparation Kit (FC-131-1024). Ampure XP beads (Beckman Coulter) were used for all clean-up steps and for size selection 200-700 bp. All 12 samples were pooled into single library mix by equal concentration and sequenced on Illumina NextSeq500 using High Output Flow Cell v2.5 (single-end, 75 bp).

### Read alignment and differential gene expression analysis

FASTQC v0.11.8 was used to assess the quality of raw reads prior to subsequent processing (Andrews 2010). Reads were trimmed and adaptor sequences were removed using Trimmomatic v0.39 (Bolger et al. 2014) with the following parameters: LEADING:20 SLIDINGWINDOW:4:15 ILLUMINACLIP: adaptor_file.fa:1:30:15 MINLEN:25 (Bolger et al. 2014). Next, the reads remaining in the analysis were aligned to ARS-UCD1.2 B. taurus genome assembly. HISAT2 (Kim et al. 2019) with default parameters was used for read alignment with the inclusion of splice site annotations obtained from the corresponding genome assembly annotation file version 1.2.97 (Kim et al. 2019). Reads were counted at the gene level, with feature Counts by counting only uniquely mapping reads with a mapping quality score (MAPQ) ≤ 8 (Liao et al. 2014). Genes with at least 10 counts for three of the four samples in at least one of the experimental groups were subjected to subsequent differential expression testing.

Differential expression analysis was carried out in R version 3.6.1 using the edgeR package version 3.26.8 (Robinson et al. 2009). Statistical comparisons were performed using a generalized linear model followed by likelihood ratio tests, also accounting for the experiment batch. Due to the small sample size and high variability among the samples, trended dispersion without tagwise shrinkage was used in order to increase the sensitivity of the model and yield a more extensive list of putative candidate genes to be further validated. We considered the differential expression of genes with a false discovery rate (FDR) ≤ 0.05 to be statistically significant while noting the inflated probability of false-positive results due to the omission of tagwise dispersion estimates.

Gene set enrichment analysis (GSEA) conducted using the clusterProfiler package (Yu et al. 2012) and pathway annotations from KEGG Pathway database. GSEA was used for full gene lists resulting from differential expression analysis that were ranked by -log₁₀p × log₂FC, where p represents unadjusted p-values and FC the fold-change. Obtained results were considered to be statistically significant at FDR ≤ 0.05.

Principal components were calculated using the prcomp function from the Stats package (Team 2019) in R. All graphs were constructed using the ggplot2 package (Wickham 2016).

### Quantitative real-time PCR (RT-qPCR) validation

Using the same RNA samples, that were used for RNAseq, the expression levels of genes of interest were validated with RT-qPCR. Designing the primers was carried out using NCBI primer blast (https://www.ncbi.nlm.nih.gov/tools/primer-blast/, last accession 2019.11.13) and the primer quality was further tested with Integrated Genome Technologies-IDT^{™} (https://www.idtdna.com/pages, last accession 2019.11.13) (Table 12). Gene exon-exon junctions were included in the primer design. Primers were purchased from Microsynth AG, Wolfurt, Austria. Reverse transcription of RNA was carried out using FIRESript RT cDNA Synthesis mix^{™} with Oligo (dT) and random primers (06-20-00100, SolisBiodyne, Tartu, Estonia). Using RT-qPCR, the tested gene transcripts were quantified using HOT FIREPol^{®} EvaGreen^{®} qPCR Supermix (08-36-00001, SolisBiodyne, Tartu, Estonia), on Quantstudio 12K Flex^{™} real-time PCR system, with following settings: enzyme activation 95°C for 15 min; denaturation - 95°C for 20 s, 40 cycles; annealing - 57°C for 20 s, 40 cycles; extension - 72°C for 20 s, 40 cycles. Mann-Whitney U Test was used as the statistical test to compare qPCR-derived gene expression values. The obtained p-values were adjusted for multiple testing by the Benjamini-Hochberg Procedure and the resulting false discovery rate (FDR) values are reported. In order to compare the qPCR-derived gene expression values with RNAseq results, the gene expression values were standardized (z-score) within genes, i.e., the mean and standard deviation of expression values were calculated individually for each gene. All of the aforementioned calculations were performed in R.

**Table 12 - Primer sequences used for RT-qPCR analysis**

| Gene | Primer sequence | SEQ ID NO: | Product size (bp) |
|---|---|---|---|
| OAS1Y | FW-ATGTGTCGCCCCAAGAACAC | 38 | 96 |
| | REW-CTCCTCCGTGGAACTGGATTC | 39 | |
| MX1 | FW-GGAAGTGAAGATATGGAGTCCAAGA | 40 | 82 |
| | REW-AGTCGATGAGGTCAATGCAGG | 41 | |
| LOC100139670 | FW-TCGCCATAATGAGGAGGGAATTA | 42 | 127 |
| | REW-AAATCCAGCCCCAACAGAGTT | 43 | |
| B2M | FW-AAGGATGGCTCGCTTCGTG | 44 | 84 |
| | REW-ATCTTTGGAGGACGCTGGATG | 45 | |
| GAPDH | FW-TGTCAAGCTCATTTCCTGGTACG | 46 | 134 |
| | REW-GAACTCTTCCTCTCGTGCTCC | 47 | |
| TGFB2 | FW-GACACTCAGCACAGTAGGGTTC | 48 | 84 |
| | REW-ATCTTGGGACACGCAGCAAG | 49 | |

| | | | |
|---|---|---|---|
| FW- forward primer, REW- reverse primer | | | |

### Immunofluorescence analysis of BOECs

BOECs grown on coverslips were first fixed with 4% paraformaldehyde for 10 min at room temperature and then with cold methanol for 10 min on ice. After blocking cells with 4% normal goat serum for 1 hour at room temperature, the cells were incubated with anti-Cytokeratin (C2562, 1:250, Sigma-Aldrich) and anti-Vimentin (PLA0199, 1:250, Sigma-Aldrich, USA) primary antibodies in blocking buffer for 1 hour at room temperature. Negative control cells were incubated in blocking buffer without primary antibodies. Next, cells were incubated with Alexa Fluor 488 goat anti-mouse and Alexa Fluor 594 goat anti-rabbit secondary antibodies (A11029 and A11012, respectively, both 1:500, Invitrogen, Thermo Fisher Scientific, Eugene, USA) in blocking buffer for 45 minutes in the dark at room temperature. After incubation, the nuclei were counterstained with Hoechst 33342 (1:2000, Thermo Fisher Scientific) for 3 minutes, and the coverslips were mounted with Fluorescence Mounting Medium (Dako, Denmark) and visualized under an epifluorescence microscope.

### RESULTS

### Characterization of primary bovine oviductal epithelial cells - Immunofluorescence staining

The epithelial nature of the cultured BOECs was determined with cytokeratin immunofluorescence staining as a specific marker for epithelial cells, for which the cultured BOECs tested positive (Figure 24). Moreover, they were negative for the specific fibroblast marker Vimentin, indicating the absence of fibroblast contamination in the BOECs. Despite the cells having been passaged thrice at the time of EV supplementation, to obtain the adequate number of cells to carry out the supplementation in four replicates on different days, the cells had not gone through the epithelial-mesenchymal transition (EMT). No specific staining for cytokeratin was observed in the negative control. Cells had retained their epithelial cell heterogeneity and displayed a polygonal shape.

### Supplementation of embryo-derived EVs to the BOEC monolayer culture

During the supplementation of EVs/NPs, each BOEC monolayer culture was supplemented with EVs isolated from 10 individually cultured bovine embryos or control media samples. Based on the quantification of EVs by NTA, this was counted as approximately 7.99 × 108, 6.48 × 108, and 6.81 × 108 per well for good quality embryo-derived EVs, bad quality embryo-derived EVs and NPs isolated from Day 5 control media respectively. The methodology used for EV purification was same as methodology used previously (Dissanayake et al. 2020). This methodology will result in purification of well characterized EVs secreted by individually cultured bovine embryos.

### RNAseq and differential gene expression analysis and RT-qPCR

Messenger RNA sequencing yielded 6.0 ± 0.6 million reads (mean ± SD) per sample. Following quality control procedures and read filtering, 99.0 ± 0.1 percent of the reads remained in the analysis and were aligned to the *B*. *taurus* genome assembly. Genome alignment resulted in 95.4 ± 1.1 percent mapping rate. Uniquely aligned reads were summarized at the gene level, and after removing genes considered not to be expressed in any of the experimental groups, 10 412 genes remained in the analysis and were subjected to differential expression testing. No apparent outliers were detected among the samples. However, a considerable degree of inter-group and intra-group variation was observed in the overall gene expression profile of the samples of oviductal monolayer cultures (Figure 25A).

The comparison between BOECs supplemented with good quality embryo-derived EVs and the control group BOECs resulted in 7 upregulated genes and 18 downregulated genes. Of the 7 upregulated genes, 4 were found to be interferon-induced genes (ISG-15, MX1, OAS1Y, LOC100139670) (Table 13). The comparison between the degenerating embryo-derived EV-supplemented BOECs and the control group BOECs yielded only a single, uncharacterized gene that was differentially expressed (ENSBTAG00000051364, log₂FC = 0.83, FDR = 0.046).

**Table 13 - List of differentially expressed genes (DEGs) in BOECs stimulated by the supplementation of good quality embryo-derived EVs compared with control BOECs (FOR < 0.05)**

| **Gene name** | **Log₂FC** | **FC** | **FDR** | **Putative Function** |
|---|---|---|---|---|
| OAS1Y | 1.83 | 3.55 | 4.35e-13 | Immune response, anti-viral |
| MX1 | 1.40 | 2.6 | 0.0004 | Antiviral, apoptosis |
| LOC100139670 | 1.33 | 2.51 | 0.0404 | Unknown |
| ISG15 | 1.23 | 2.34 | 3.93e-06 | Protein modification |
| ENSBTAG00000051364* | 1.07 | 2.09 | 2.28e-06 | Unknown |
| ENSBTAG00000053545* | 0.88 | 1.84 | 0.0012 | Unknown |
| CYP1A1 | 0.46 | 1.37 | 0.0067 | Metabolism of endogenous substrates |
| ALKBH4 | -1.29 | 0.40 | 0.0118 | Transcription regulation |
| MADD | -1.28 | 0.41 | 0.0456 | Cell proliferation, survival and death |
| HIP1R | -1.25 | 0.42 | 1.12e-06 | Support early stages of endocytosis |
| C28H1orf198 | -0.98 | 0.50 | 3.11e-05 | Unknown |
| HID1 | -0.97 | 0.51 | 4.38e-05 | Unknown |
| CDC42EP1 | -0.93 | 0.52 | 8.69e-05 | Organization of the actin cytoskeleton |
| UNC13D | -0.85 | 0.55 | 2.78e-06 | Innate immune response |
| ALDH16A1 | -0.80 | 0.57 | 0.0393 | Oxidoreductase activity |
| CAPN1 | -0.78 | 0.58 | 0.0118 | Cytoskeletal remodeling and signal transduction |
| PXDN | -0.74 | 0.59 | 9.08e-05 | Extracellular matrix formation |
| ENSBTAG00000043565* | -0.70 | 0.61 | 0.0114 | Unknown |
| CPSF1 | -0.68 | 0.62 | 0.0337 | 3-prime processing of pre-mRNAs |
| HGH1 | -0.65 | 0.63 | 0.0473 | Unknown |
| ARHGEF2 | -0.63 | 0.64 | 0.0309 | Cell cycle regulation and innate immune response |
| LAMB3 | -0.58 | 0.66 | 0.0015 | Cell signaling |
| FSTL3 | -0.57 | 0.67 | 0.0162 | Transcriptional regulation |
| RHBDF2 | -0.49 | 0.71 | 0.0162 | Cell survival, proliferation, migration and inflammation |
| MYC | -0.45 | 0.73 | 0.0212 | Transcription factor |

| | | | | |
|---|---|---|---|---|
| Log₂FC- log₂ fold change, FC - fold change, FDR - false discovery rate, *Genes without an assigned gene name are labeled with Ensembl symbol. | | | | |

Comparison of the good quality embryo-derived EV-supplemented BOECs and degenerating embryo-derived EV-supplemented BOECs resulted in 4 upregulated genes and 11 down-regulated genes (Table 14). Similar to good quality embryo-derived EV-supplemented group and the control group BOECs, the upregulated genes included interferon-induced ISG-15, MX1, OAS1Y and LOC100139670 (Figure 25B).

**Table 14 - List of differentially expressed genes (DEGs) in BOECs stimulated by the supplementation of good quality embryo-derived EVs compared to BOECs supplemented with degenerating embryo-derived EVs (FOR < 0.05)**

| **Gene name** | **Log₂FC** | **FC** | **FDR** | **Putative Function** |
|---|---|---|---|---|
| LOC100139670 | 1.73 | 3.31 | 0.0039 | Unknown |
| OAS1Y | 1.61 | 3.05 | 1.37e-09 | Immune response, anti-viral |
| MX1 | 1.19 | 2.28 | 0.0115 | Anti-viral, Induction of apoptosis |
| ISG15 | 0.95 | 1.93 | 0.0039 | Protein modification |
| MADD | -1.45 | 0.36 | 0.0081 | Cell proliferation, survival and death |
| HIP1R | -0.88 | 0.54 | 0.0119 | Support early stages of endocytosis |
| CAPN1 | -0.87 | 0.54 | 0.0039 | Cytoskeletal remodeling and signal transduction |
| HID1 | -0.82 | 0.56 | 0.0058 | Unknown |
| CDC42EP1 | -0.80 | 0.57 | 0.0061 | Organization of the actin cytoskeleton |
| AGPAT1 | -0.79 | 0.57 | 0.0061 | Cell metabolism |
| UNC13D | -0.79 | 0.57 | 8.87e-05 | Cytolysis and regulation of immune system. |
| BAK1 | -0.66 | 0.62 | 0.0287 | Role in the mitochondrial apoptosis |
| PXDN | -0.62 | 0.65 | 0.0081 | Extracellular matrix formation |
| SLC7A8 | -0.47 | 0.72 | 0.0039 | Molecular transport |
| TGM2 | -0.43 | 0.74 | 0.0212 | Protein modification |

| | | | | |
|---|---|---|---|---|
| Log₂FC- log₂ fold change, FC - fold change, FDR - false discovery rate, Genes that are upregulated and down-regulated are separated by a line. | | | | |

The GSEA with KEGG pathway annotations based on the results of differential expression tests did not result in any significantly enriched pathways, nor were any of the pathways among the top results relevant in the context of the biological system being investigated in this study.

RT-qPCR based validation was conducted with the three genes (OAS1Y, MX1, and LOC100139670) that implied the most relevance in the context of this system, based on previously published studies in this field. The expression levels of the three genes were quantified in BOECs that were supplemented with good quality embryo-derived EVs and in the control group BOECs. These three genes displayed a similar trend of upregulation as observed based on the RNAseq data (Figure 26A-26C), thus adding more confidence to these genes being upregulated in BOECs in our experimental system as the result of supplementation with good quality embryo-derived EVs. Two of the genes - OAS1Y and MX1 - were detected to be significantly upregulated (FOR ≤ 0.05, Mann-Whitney U test, Benjamini-Hochberg Procedure correction) based on the RT-qPCR data.

### DISCUSSION

This Example investigated whether embryo-derived EVs are involved in mediating this embryo-maternal dialogue at the oviduct, and if the oviductal response depends on the quality of the embryo. The results show that the supplementation with EVs isolated from media conditioned by good quality embryos could induce specific transcriptional changes in the primary bovine oviductal epithelial cells (BOEC), which were not detected when BOECs were supplemented with EVs isolated from media conditioned by degenerating embryos.

The genes upregulated in the BOECs in response to supplementation with good quality day 5 embryo-derived EVs included ISG-15, MX1, OAS1Y, LOC100139670, all of which are classically known as interferon-stimulated genes (ISGs) or belong to the interferon tau (IFN-τ) pathway. These genes are known to be upregulated in response to IFN-τ, a type 1 interferon, secreted by the trophoblast cells in days 13-21 days of bovine pregnancy. IFN-τ, widely known as a pregnancy recognition signal, inhibits the expression of oxytocin receptors and the synthesis of PGF2α, and prevents the breakdown of the corpus luteum (luteolysis) and maintains the pregnancy. Although Hensen et al. reported that IFN-τ first appears on day 15 of pregnancy in bovine endometrium (Hansen et al. 1997), Talukder et al. showed that IFN-τ is secreted by 16-cell stage bovine embryos (day 4) when co-cultured with BOECs, but not by 16-cell stage embryos cultured alone (Talukder et al. 2018). This Example suggests that EVs secreted by day 5 good quality bovine embryos, which are 16-cell stage (morula), could carry biomolecules such as IFN-τ that induce transcriptomic changes in the maternal tract. Moreover, the supplementation of NPs isolated from culture media to the control BOEC culture in the current study, while supplementing EVs isolated from embryo conditioned media to the experimental BOEC cultures, verifies that those signals that altered the gene expression of experimental BOEC cultures were originated from embryos.

Interestingly, this Example shows that the expression of the genes ISG-15, MX1, OAS1 in BOECs is induced in the presence of EVs isolated from good quality embryos, but without the embryos themselves. This suggests that the upregulation of these genes in the oviduct may be mediated by EVs secreted by pre-blastulation embryos.

ISG-15 plays a vital role in the innate immune response to viral infection. It acts either by its conjugation to a target protein (ISGylation) or by its action as a free or unconjugated protein. With reference to embryo-maternal communication, this protein may ligate to and regulate proteins responsible for the release of prostaglandin F2-alpha (PGF), which prevents the breakdown of corpus luteum. The proteins encoded by Myxovirus resistance (MX) genes also undergo ISGylation, and their level of expression in the endometrium increases during implantation. MX genes have at least 2 isoforms, MX1 and MX2 and are known to be involved in the suppression viruses such as influenza virus and vesicular stomatitis virus. The OAS1 (including OAS1Y) gene family is engaged in the immune response and the defense response to viruses. They are key effectors in innate cellular antiviral response and act via the OAS1-RNaseL antiviral pathway. They sense exogenous nucleic acid and activate endoribonuclease L (RNAseL), which degrades viral RNA. Moreover, RNAseL is involved in other cellular events such as apoptosis and cell growth. According to current evidence, EVs show resemblance to viruses, in particular retroviruses, in several regards such as morphology, biogenesis, and endocytosis mechanisms. Thus, the finding that non-self EVs induce transcriptional responses in recipient cells may resemble the response to viral infection presents an additional intriguing parallel.

In contrast, LOC100139670, though categorized as an ISG, has not been fully characterized. Thus, its function remains to be identified. While we obtain validation of the upregulation of OAS1Y and MX1 genes by RT-qPCR quantification, it did not confirm the upregulation of LOC100139670 observed based on RNAseq data.

Of the down-regulated genes in BOECs due to the supplementation of good quality embryo-derived EVs, UNC13D and ARHGEF2 are found to be involved in immune response. UNC13D alias Munc13-4 is a protein-coding gene and known to be involved in innate immune response and neutrophil degranulation. ARHGEF2, which encodes GEF-H1, is involved in epithelial barrier permeability, antigen presentation, cytokinesis, cell cycle regulation, and innate immune response. Immune regulation of the maternal tract is vital during the pre- and post-implantation phases of embryonic development as embryos, being foreign entities, should overcome the immune rejection by the mother. Most of the other downregulated genes are involved in different cellular activities such as cell survival and proliferation (MADD, RHBDF2), transcription regulation (ALKBH4, FSTL3), and cell signaling (CAPN1, LAMB3).

It remains unclear why supplementation of BOECs with EVs produced by further degenerating embryos could not induce a notable change in the gene expression profile of the BOECs. It is possible that degenerating/arrested pre-implantation embryos are not recognized by the maternal tract because they may lack the production of specific type of EVs produced by the healthy embryos to induce positive maternal responses leading to implantation. Cellular events taking place in good quality embryos and degenerating embryos are substantially different, and in order to develop properly, the early embryo must exhibit specific gene expression patterns in a temporally controlled manner. Thus, the detection of RNA or protein in EVs originating from specific genes would be reflective of the embryo developing properly, and this may constitute the 'signal' that is communicated during the embryo-maternal communication.

The results of this Example indicate that pre-implantation embryos do indeed exhibit molecular signatures of developmental potential, which are portrayed in the surface molecules or molecular cargo of EVs depending on the embryo quality. EV surface molecules and/or EV cargo molecules may be used as potential biomarkers indicative of the embryo quality, which, if properly utilized, could lead to advancements in assisted reproductive technology (ART) in both humans and animals with regard to embryo selection. This could possibly supplement the current morphology-based evaluation of embryo quality, enabling embryologists to select the best embryo for uterine transfer during ART.

The topmost potentially differentially expressed genes that were identified in this Example, were subsequently successfully confirmed by RT-qPCR based validation. Moreover, the differential expression of these genes was also supported by results of previous studies conducted in comparable *in vitro* and *in vivo* systems.

In conclusion, the results of this study indicate that embryo-derived EVs are capable of altering the gene expression of primary BOECs, and these effects appear to be dependent on the quality of the embryos. This supports the notion that maternal tissues are capable of sensing the quality of embryos, which may help to determine the decision of whether to invest resources in pregnancy or not. Furthermore, this observed effect of embryo-derived EVs on BOECs could serve as a non-invasive method of evaluating the embryo quality.

The applicant informs that the project leading to this application has received funding from the European Union's Horizon 2020 research and innovation program under the grant agreement No. 668989.

The embodiments described above are to be understood as a few illustrative examples of the present invention.

### ANNEX A - upregulated genes

| **ENSEMBL** | **SYMBOL** | **logFC** | **logCPM** | **F** | **PValue** | **FDR** |
|---|---|---|---|---|---|---|
| ENSG00000007174 | DNAH9 | 3.392926 | 3.116801 | 103.3405 | 1.08E-11 | 5.64E-09 |
| ENSG00000205592 | MUC19 | 3.302214 | 3.089374 | 106.9985 | 6.94E-12 | 3.80E-09 |
| ENSG00000228340 | MIR646HG | 3.137381 | 3.208208 | 88.50507 | 7.34E-11 | 2.91E-08 |
| ENSG00000228340 | LOC729296 | 3.137381 | 3.208208 | 88.50507 | 7.34E-11 | 2.91E-08 |
| ENSG00000169894 | MUC3A | 3.135979 | 3.279708 | 100.3202 | 1.57E-11 | 7.72E-09 |
| ENSG00000140538 | NTRK3 | 3.013209 | 3.105198 | 90.12219 | 5.89E-11 | 2.42E-08 |
| ENSG00000143520 | FLG2 | 3.004586 | 3.575034 | 100.8222 | 1.61E-11 | 7.72E-09 |
| ENSG00000184226 | PCDH9 | 2.897203 | 3.16731 | 84.151 | 1.35E-10 | 4.85E-08 |
| ENSG00000198838 | RYR3 | 2.875721 | 3.148824 | 85.33498 | 1.14E-10 | 4.23E-08 |
| ENSG00000229140 | CCDC26 | 2.795048 | 3.93768 | 110.582 | 6.25E-12 | 3.78E-09 |
| ENSG00000229140 | LINC00977 | 2.795048 | 3.93768 | 110.582 | 6.25E-12 | 3.78E-09 |
| ENSG00000229140 | LINC00976 | 2.795048 | 3.93768 | 110.582 | 6.25E-12 | 3.78E-09 |
| ENSG00000181143 | MUC16 | 2.768334 | 4.193989 | 104.0955 | 1.47E-10 | 4.98E-08 |
| ENSG00000143341 | HMCN1 | 2.69509 | 3.249308 | 79.9613 | 2.48E-10 | 7.50E-08 |
| ENSG00000138759 | FRAS1 | 2.672629 | 3.057146 | 69.34662 | 1.29E-09 | 2.43E-07 |
| ENSG00000196628 | TCF4 | 2.601879 | 3.312495 | 83.0231 | 1.59E-10 | 5.21E-08 |
| ENSG00000091513 | TF | 2.577132 | 3.028069 | 60.69404 | 5.64E-09 | 6.75E-07 |
| ENSG00000149970 | CNKSR2 | 2.553363 | 3.146782 | 63.95668 | 3.18E-09 | 4.35E-07 |
| ENSG00000156113 | KCNMA1 | 2.542799 | 3.278612 | 72.43645 | 7.83E-10 | 1.67E-07 |
| ENSG00000254166 | PCAT2 | 2.339799 | 3.345718 | 58.50873 | 8.37E-09 | 9.17E-07 |
| ENSG00000121904 | CSMD2 | 2.120402 | 3.203093 | 51.39283 | 3.25E-08 | 2.15E-06 |
| ENSG00000235257 | ITGA9-AS1 | 2.119028 | 3.031402 | 37.74882 | 7.05E-07 | 2.32E-05 |
| ENSG00000163531 | NFASC | 2.113014 | 3.652656 | 68.55665 | 1.46E-09 | 2.59E-07 |
| ENSG00000285219 | LOC100506207 | 2.110098 | 3.670751 | 67.55089 | 1.73E-09 | 2.84E-07 |
| ENSG00000115590 | IL1R2 | 1.954498 | 3.548029 | 58.40314 | 8.53E-09 | 9.26E-07 |
| ENSG00000141837 | CACNA1A | 1.940663 | 3.531316 | 51.96715 | 2.90E-08 | 2.02E-06 |
| ENSG00000039139 | DNAH5 | 1.915167 | 3.18015 | 37.70313 | 6.40E-07 | 2.15E-05 |
| ENSG00000181722 | ZBTB20 | 1.835979 | 3.10426 | 41.58297 | 2.60E-07 | 1.09E-05 |
| ENSG00000171435 | KSR2 | 1.810873 | 3.164905 | 35.12719 | 1.20E-06 | 3.51E-05 |
| ENSG00000182389 | CACNB4 | 1.796717 | 4.105539 | 68.07751 | 1.58E-09 | 2.64E-07 |
| ENSG00000107614 | TRDMT1 | 1.79318 | 3.101134 | 35.5366 | 1.08E-06 | 3.28E-05 |
| ENSG00000214900 | LINC01588 | 1.784938 | 3.570428 | 45.81657 | 1.03E-07 | 5.18E-06 |
| ENSG00000270344 | NA | 1.775449 | 3.047627 | 32.59226 | 2.28E-06 | 5.94E-05 |
| ENSG00000183091 | NEB | 1.763847 | 3.748907 | 49.55831 | 4.71E-08 | 2.79E-06 |
| ENSG00000164199 | ADGRV1 | 1.739617 | 3.53241 | 41.67391 | 2.55E-07 | 1.07E-05 |
| ENSG00000160145 | KALRN | 1.71956 | 3.231648 | 34.51463 | 1.40E-06 | 3.96E-05 |
| ENSG00000131018 | SYNE1 | 1.692309 | 3.301367 | 39.26614 | 4.43E-07 | 1.59E-05 |
| ENSG00000214944 | ARHGEF28 | 1.691396 | 3.322956 | 31.50142 | 3.03E-06 | 7.41E-05 |
| ENSG00000109339 | MAPK10 | 1.668854 | 3.626073 | 42.20666 | 2.26E-07 | 9.80E-06 |
| ENSG00000151320 | AKAP6 | 1.665563 | 3.13625 | 34.55364 | 1.38E-06 | 3.94E-05 |
| ENSG00000154556 | SORBS2 | 1.64637 | 3.236821 | 27.50245 | 8.99E-06 | 0.000186 |
| ENSG00000065534 | MYLK | 1.640357 | 3.34659 | 30.65812 | 3.79E-06 | 8.88E-05 |
| ENSG00000158220 | ESYT3 | 1.614745 | 3.078848 | 26.46481 | 1.21E-05 | 0.000243 |
| ENSG00000138829 | FBN2 | 1.600942 | 3.309437 | 29.77116 | 4.81E-06 | 0.000109 |
| ENSG00000186205 | MTARC1 | 1.585928 | 3.882167 | 49.55281 | 4.71E-08 | 2.79E-06 |
| ENSG00000153721 | CNKSR3 | 1.583342 | 3.226478 | 28.25423 | 7.28E-06 | 0.000154 |
| ENSG00000281344 | NA | 1.574907 | 6.070966 | 159.9516 | 3.37E-14 | 4.84E-11 |
| ENSG00000133392 | MYH11 | 1.566703 | 3.153242 | 22.55765 | 4.63E-05 | 0.000795 |
| ENSG00000248932 | LOC100507291 | 1.561441 | 3.281902 | 29.17223 | 5.66E-06 | 0.000125 |
| ENSG00000227036 | LINC00511 | 1.551885 | 3.634418 | 35.15698 | 1.19E-06 | 3.49E-05 |
| ENSG00000227036 | LINC00673 | 1.551885 | 3.634418 | 35.15698 | 1.19E-06 | 3.49E-05 |
| ENSG00000126091 | ST3GAL3 | 1.551298 | 3.802482 | 36.32483 | 8.93E-07 | 2.80E-05 |
| ENSG00000138411 | HECW2 | 1.549118 | 3.16319 | 27.23034 | 9.71E-06 | 0.0002 |
| ENSG00000133401 | PDZD2 | 1.526381 | 3.095219 | 22.9181 | 3.49E-05 | 0.000615 |
| ENSG00000020129 | NCDN | 1.52446 | 3.289476 | 29.41981 | 5.29E-06 | 0.000119 |
| ENSG00000204792 | LINC01291 | 1.501004 | 3.24226 | 23.61258 | 2.79E-05 | 0.000504 |
| ENSG00000187775 | DNAH17 | 1.48559 | 3.011659 | 16.55016 | 0.000357 | 0.004472 |
| ENSG00000198959 | TGM2 | 1.467645 | 6.744324 | 181.72 | 5.72E-15 | 1.10E-11 |
| ENSG00000227486 | NA | 1.45257 | 3.00481 | 19.51266 | 0.000102 | 0.001542 |
| ENSG00000174469 | CNTNAP2 | 1.448114 | 3.375213 | 28.73445 | 6.38E-06 | 0.000138 |
| ENSG00000153815 | CMIP | 1.445864 | 3.061293 | 21.34809 | 5.63E-05 | 0.000935 |
| ENSG00000224086 | NA | 1.435893 | 3.869824 | 29.63782 | 6.47E-06 | 0.000139 |
| ENSG00000137502 | RAB30 | 1.43024 | 3.869124 | 33.12016 | 1.99E-06 | 5.29E-05 |
| ENSG00000067798 | NAV3 | 1.427483 | 4.20985 | 46.92752 | 8.11E-08 | 4.26E-06 |
| ENSG00000279159 | NA | 1.423725 | 4.031356 | 39.37831 | 4.31E-07 | 1.55E-05 |
| ENSG00000115525 | ST3GAL5 | 1.404073 | 3.266165 | 25.60868 | 1.55E-05 | 0.000301 |
| ENSG00000116396 | KCNC4 | 1.402053 | 3.613963 | 25.76807 | 1.57E-05 | 0.000306 |
| ENSG00000165914 | TTC7B | 1.376604 | 3.514246 | 28.11887 | 7.56E-06 | 0.000159 |
| ENSG00000186409 | CCDC30 | 1.369643 | 3.297067 | 21.84747 | 4.81E-05 | 0.000822 |
| ENSG00000268222 | NA | 1.367297 | 3.302971 | 18.11781 | 0.000216 | 0.002903 |
| ENSG00000031081 | ARHGAP31 | 1.361765 | 3.138691 | 16.63134 | 0.000289 | 0.003713 |
| ENSG00000198626 | RYR2 | 1.361563 | 3.3483 | 19.95222 | 0.000103 | 0.001557 |
| ENSG00000197892 | KIF13B | 1.345446 | 3.616198 | 23.88482 | 2.58E-05 | 0.000472 |
| ENSG00000262879 | LOC101927060 | 1.333165 | 3.165561 | 21.24529 | 5.81E-05 | 0.000957 |
| ENSG00000196814 | MVB12B | 1.329064 | 3.018097 | 18.83966 | 0.000127 | 0.001865 |
| ENSG00000132694 | ARHGEF11 | 1.328634 | 3.303762 | 16.5882 | 0.000295 | 0.003785 |
| ENSG00000120875 | DUSP4 | 1.327972 | 3.86457 | 31.33335 | 3.17E-06 | 7.65E-05 |
| ENSG00000150967 | ABCB9 | 1.312397 | 3.35598 | 21.74027 | 4.97E-05 | 0.000842 |
| ENSG00000186472 | PCLO | 1.303459 | 3.458733 | 23.03478 | 3.33E-05 | 0.000589 |
| ENSG00000279738 | NA | 1.296156 | 3.174794 | 20.396 | 7.62E-05 | 0.001205 |
| ENSG00000116584 | ARHGEF2 | 1.283408 | 3.434069 | 17.56045 | 0.0002 | 0.002736 |
| ENSG00000099139 | PCSK5 | 1.282995 | 3.024728 | 16.65394 | 0.000267 | 0.003488 |
| ENSG00000268089 | GABRQ | 1.279534 | 3.044119 | 17.13422 | 0.000226 | 0.003017 |
| ENSG00000188549 | CCDC9B | 1.272241 | 3.666837 | 21.05668 | 6.90E-05 | 0.001108 |
| ENSG00000169247 | SH3TC2 | 1.268867 | 4.309515 | 31.94747 | 2.92E-06 | 7.20E-05 |
| ENSG00000076555 | ACACB | 1.267316 | 3.640376 | 23.19111 | 3.18E-05 | 0.000566 |
| ENSG00000134245 | WNT2B | 1.265193 | 3.364944 | 21.20906 | 5.88E-05 | 0.000963 |
| ENSG00000187792 | ZNF70 | 1.263426 | 3.113486 | 16.94753 | 0.000241 | 0.003185 |
| ENSG00000182585 | EPGN | 1.242995 | 3.611937 | 24.24355 | 2.31E-05 | 0.000429 |
| ENSG00000036448 | MYOM2 | 1.241963 | 3.440025 | 18.25376 | 0.000156 | 0.002222 |
| ENSG00000168702 | LRP1B | 1.22945 | 3.149623 | 14.66817 | 0.000544 | 0.006293 |
| ENSG00000148343 | MIGA2 | 1.220384 | 3.496884 | 19.78439 | 9.30E-05 | 0.00143 |
| ENSG00000244405 | ETV5 | 1.211245 | 3.585424 | 23.37619 | 3.00E-05 | 0.000538 |
| ENSG00000114739 | ACVR2B | 1.208171 | 3.164935 | 18.04094 | 0.000166 | 0.00234 |
| ENSG00000088538 | DOCK3 | 1.192004 | 3.208277 | 17.17647 | 0.000223 | 0.002991 |
| ENSG00000253438 | PCAT1 | 1.188423 | 3.647879 | 18.00908 | 0.000191 | 0.002639 |
| ENSG00000232973 | CYP1B1-AS1 | 1.185223 | 3.148644 | 16.59599 | 0.000273 | 0.003552 |
| ENSG00000141068 | KSR1 | 1.171323 | 3.310959 | 13.99125 | 0.00071 | 0.007812 |
| ENSG00000245275 | SAP30L-AS1 | 1.155336 | 3.341069 | 17.79 | 0.000181 | 0.002515 |
| ENSG00000251136 | LOC101929709 | 1.146105 | 3.040084 | 13.07687 | 0.000986 | 0.010237 |
| ENSG00000248538 | LOC157273 | 1.143357 | 4.728186 | 39.87672 | 3.84E-07 | 1.42E-05 |
| ENSG00000248538 | LOC101929128 | 1.143357 | 4.728186 | 39.87672 | 3.84E-07 | 1.42E-05 |
| ENSG00000078114 | NEBL | 1.141733 | 3.238651 | 13.65056 | 0.000794 | 0.00858 |
| ENSG00000197951 | ZNF71 | 1.141166 | 3.032405 | 15.34749 | 0.000425 | 0.005127 |
| ENSG00000184949 | FAM227A | 1.140839 | 3.013873 | 10.65701 | 0.002723 | 0.022727 |
| ENSG00000203709 | NA | 1.135971 | 3.582961 | 17.12496 | 0.000227 | 0.003023 |
| ENSG00000226688 | ENTPD1-AS1 | 1.130218 | 3.042457 | 15.26849 | 0.000437 | 0.005247 |
| ENSG00000129566 | TEP1 | 1.129025 | 3.810184 | 21.91427 | 4.71E-05 | 0.000806 |
| ENSG00000179532 | DNHD1 | 1.128811 | 3.875434 | 21.15309 | 5.98E-05 | 0.000979 |
| ENSG00000185019 | UBOX5 | 1.118073 | 3.386512 | 14.15109 | 0.00066 | 0.007373 |
| ENSG00000167548 | KMT2D | 1.102196 | 3.543467 | 16.17416 | 0.000317 | 0.004024 |
| ENSG00000113448 | PDE4D | 1.100521 | 3.850231 | 21.45967 | 5.43E-05 | 0.000908 |
| ENSG00000148814 | LRRC27 | 1.100162 | 3.119701 | 13.10012 | 0.000977 | 0.01018 |
| ENSG00000178607 | ERN1 | 1.099936 | 3.286921 | 14.90238 | 0.0005 | 0.005876 |
| ENSG00000109321 | AREG | 1.099825 | 4.349304 | 26.14107 | 1.33E-05 | 0.000266 |
| ENSG00000168016 | TRANK1 | 1.097737 | 3.826573 | 21.31398 | 5.69E-05 | 0.000941 |
| ENSG00000121454 | LHX4 | 1.094434 | 3.07645 | 10.31658 | 0.002993 | 0.024404 |
| ENSG00000245149 | RNF139-AS1 | 1.091994 | 3.059067 | 13.32609 | 0.000897 | 0.00951 |
| ENSG00000066117 | SMARCD1 | 1.089795 | 3.602212 | 14.43882 | 0.000616 | 0.006949 |
| ENSG00000155657 | TTN | 1.085452 | 5.482456 | 58.29386 | 8.71E-09 | 9.31E-07 |
| ENSG00000054690 | PLEKHH1 | 1.063597 | 3.003864 | 12.55546 | 0.001205 | 0.012007 |
| ENSG00000231312 | MAP4K3-DT | 1.061889 | 3.076052 | 13.49739 | 0.000841 | 0.00903 |
| ENSG00000122870 | BICC1 | 1.059754 | 3.594705 | 14.62561 | 0.000553 | 0.006355 |
| ENSG00000171914 | TLN2 | 1.057813 | 3.650684 | 16.06342 | 0.000329 | 0.004152 |
| ENSG00000183044 | ABAT | 1.055691 | 3.042604 | 13.38393 | 0.000878 | 0.009364 |
| ENSG00000280138 | NA | 1.046262 | 3.48657 | 16.32076 | 0.000301 | 0.003851 |
| ENSG00000157388 | CACNA1D | 1.045828 | 3.712502 | 15.08013 | 0.000475 | 0.005633 |
| ENSG00000287299 | LOC101927741 | 1.045107 | 3.130155 | 12.02109 | 0.001483 | 0.014193 |
| ENSG00000173065 | FAM222B | 1.04467 | 3.653801 | 15.56953 | 0.000393 | 0.004819 |
| ENSG00000131061 | ZNF341 | 1.044302 | 3.056365 | 10.7051 | 0.002508 | 0.021354 |
| ENSG00000212719 | LINC02693 | 1.039185 | 3.63581 | 16.9351 | 0.000243 | 0.003195 |
| ENSG00000248905 | FMN1 | 1.038957 | 3.086676 | 11.78925 | 0.001625 | 0.015206 |
| ENSG00000137221 | TJAP1 | 1.023637 | 3.611923 | 14.49147 | 0.000581 | 0.006619 |
| ENSG00000184640 | SEPTIN9 | 1.020055 | 3.570277 | 13.14208 | 0.00097 | 0.010112 |
| ENSG00000175592 | FOSL1 | 1.020017 | 4.110383 | 18.59057 | 0.000145 | 0.002089 |
| ENSG00000134369 | NAV1 | 1.019959 | 4.721173 | 34.42665 | 1.43E-06 | 4.03E-05 |
| ENSG00000128512 | DOCK4 | 1.019647 | 3.696579 | 18.67326 | 0.000134 | 0.001951 |
| ENSG00000250903 | NA | 1.019283 | 4.213406 | 22.26379 | 4.22E-05 | 0.000731 |
| ENSG00000112624 | BICRAL | 1.017955 | 3.088573 | 10.01176 | 0.003335 | 0.026473 |
| ENSG00000279110 | NA | 1.01612 | 3.37003 | 9.702601 | 0.004276 | 0.032063 |
| ENSG00000166450 | PRTG | 1.006115 | 3.169686 | 10.34068 | 0.002911 | 0.02397 |
| ENSG00000182795 | C1orf116 | 1.00365 | 4.343889 | 20.19487 | 8.67E-05 | 0.001347 |
| ENSG00000196405 | EVL | 0.999526 | 3.366835 | 13.72208 | 0.000773 | 0.008393 |
| ENSG00000134531 | EMP1 | 0.995097 | 3.950567 | 15.7206 | 0.000379 | 0.004691 |
| ENSG00000175115 | PACS1 | 0.988466 | 3.239726 | 11.28669 | 0.001984 | 0.017757 |
| ENSG00000179240 | GVQW3 | 0.982081 | 3.341513 | 11.30767 | 0.001967 | 0.017663 |
| ENSG00000124788 | ATXN1 | 0.979467 | 3.450929 | 11.16744 | 0.002081 | 0.018397 |
| ENSG00000054392 | HHAT | 0.971902 | 3.483839 | 12.38188 | 0.001288 | 0.012656 |
| ENSG00000136104 | RNASEH2B | 0.967986 | 4.739971 | 28.40208 | 6.99E-06 | 0.000149 |
| ENSG00000283646 | LINC02009 | 0.96778 | 3.295226 | 11.92517 | 0.00154 | 0.014628 |
| ENSG00000079805 | DNM2 | 0.967764 | 4.141157 | 18.54632 | 0.00014 | 0.002025 |
| ENSG00000144857 | BOC | 0.965184 | 3.851243 | 12.79797 | 0.001193 | 0.011935 |
| ENSG00000154127 | UBASH3B | 0.961873 | 4.735911 | 29.24411 | 5.55E-06 | 0.000123 |
| ENSG00000154237 | LRRK1 | 0.958581 | 4.405205 | 22.3205 | 4.15E-05 | 0.00072 |
| ENSG00000164663 | USP49 | 0.957207 | 3.129476 | 8.71392 | 0.005778 | 0.040615 |
| ENSG00000103248 | MTHFSD | 0.952857 | 4.080166 | 18.1464 | 0.00016 | 0.002274 |
| ENSG00000070371 | CLTCL1 | 0.951577 | 4.183432 | 19.296 | 0.000109 | 0.001639 |
| ENSG00000236144 | TMEM147-AS1 | 0.951293 | 3.447825 | 12.30528 | 0.001327 | 0.01296 |
| ENSG00000140545 | MFGE8 | 0.950945 | 3.112188 | 10.17032 | 0.003123 | 0.025279 |
| ENSG00000146858 | ZC3HAV1L | 0.948535 | 3.195808 | 9.440695 | 0.004236 | 0.031802 |
| ENSG00000161405 | IKZF3 | 0.947214 | 3.010864 | 9.623325 | 0.003922 | 0.029896 |
| ENSG00000185024 | BRF1 | 0.945983 | 3.410365 | 12.40401 | 0.001277 | 0.012569 |
| ENSG00000181649 | PHLDA2 | 0.944088 | 3.868127 | 11.01983 | 0.002485 | 0.021186 |
| ENSG00000278535 | DHRS11 | 0.940851 | 3.291792 | 11.10136 | 0.002137 | 0.018848 |
| ENSG00000143772 | ITPKB | 0.939894 | 4.368372 | 22.87228 | 3.50E-05 | 0.000617 |
| ENSG00000196218 | RYR1 | 0.939466 | 3.137597 | 11.00537 | 0.002221 | 0.019402 |
| ENSG00000075213 | SEMA3A | 0.939158 | 3.606363 | 11.80706 | 0.001613 | 0.015124 |
| ENSG00000156650 | KAT6B | 0.938035 | 4.909554 | 30.98134 | 3.47E-06 | 8.26E-05 |
| ENSG00000150990 | DHX37 | 0.93597 | 3.43253 | 12.72478 | 0.001128 | 0.01142 |
| ENSG00000143368 | SF3B4 | 0.933207 | 4.391141 | 10.301 | 0.005448 | 0.038825 |
| ENSG00000183023 | SLC8A1 | 0.93176 | 3.389106 | 11.2845 | 0.001985 | 0.017758 |
| ENSG00000285280 | LOC105371664 | 0.931034 | 3.418209 | 9.833314 | 0.003592 | 0.028011 |
| ENSG00000008086 | CDKL5 | 0.923055 | 3.257572 | 9.462514 | 0.004197 | 0.031593 |
| ENSG00000102452 | NALCN | 0.916716 | 3.710627 | 13.34394 | 0.000891 | 0.009463 |
| ENSG00000280109 | PLAC4 | 0.915954 | 3.069632 | 9.008995 | 0.005089 | 0.036864 |
| ENSG00000130158 | DOCK6 | 0.914772 | 3.645661 | 14.04944 | 0.000684 | 0.007563 |
| ENSG00000121964 | GTDC1 | 0.911672 | 3.787896 | 14.37748 | 0.000606 | 0.006862 |
| ENSG00000250305 | TRMT9B | 0.911533 | 3.306205 | 11.23071 | 0.002029 | 0.018019 |
| ENSG00000171132 | PRKCE | 0.911406 | 3.460006 | 10.80941 | 0.002404 | 0.020591 |
| ENSG00000231527 | FAM27C | 0.909303 | 3.990616 | 16.95468 | 0.000241 | 0.003181 |
| ENSG00000231527 | LOC105379444 | 0.909303 | 3.990616 | 16.95468 | 0.000241 | 0.003181 |
| ENSG00000112659 | CUL9 | 0.905716 | 3.970991 | 15.54651 | 0.000396 | 0.004843 |
| ENSG00000173068 | BNC2 | 0.904391 | 3.892094 | 16.57882 | 0.000275 | 0.003561 |
| ENSG00000112541 | PDE10A | 0.89949 | 3.122806 | 9.183278 | 0.004724 | 0.03459 |
| ENSG00000112541 | LINC00473 | 0.89949 | 3.122806 | 9.183278 | 0.004724 | 0.03459 |
| ENSG00000234444 | ZNF736 | 0.89549 | 3.78234 | 13.53836 | 0.000828 | 0.008908 |
| ENSG00000089022 | MAPKAPK5 | 0.892233 | 3.551444 | 9.032445 | 0.005113 | 0.036989 |
| ENSG00000075702 | WDR62 | 0.891282 | 5.571262 | 33.17404 | 2.34E-06 | 6.03E-05 |
| ENSG00000151240 | DIP2C | 0.889596 | 3.152612 | 9.192363 | 0.004706 | 0.034523 |
| ENSG00000188825 | LINC00910 | 0.888913 | 3.451673 | 11.5688 | 0.001773 | 0.016377 |
| ENSG00000116649 | SRM | 0.88821 | 3.891398 | 10.78952 | 0.002584 | 0.021904 |
| ENSG00000008710 | PKD1 | 0.887559 | 3.109909 | 8.626408 | 0.006001 | 0.041685 |
| ENSG00000078269 | SYNJ2 | 0.886959 | 3.680981 | 12.12494 | 0.001424 | 0.013718 |
| ENSG00000159433 | STARD9 | 0.885974 | 4.377538 | 17.99 | 0.000169 | 0.002369 |
| ENSG00000173706 | HEG1 | 0.882623 | 3.595439 | 12.19606 | 0.001385 | 0.013429 |
| ENSG00000103044 | HAS3 | 0.882588 | 5.018797 | 28.39407 | 7.01E-06 | 0.000149 |
| ENSG00000172915 | NBEA | 0.879636 | 3.676825 | 12.80873 | 0.001093 | 0.011142 |
| ENSG00000177675 | CD163L1 | 0.878922 | 3.24519 | 9.537852 | 0.004065 | 0.030807 |
| ENSG00000100418 | DESI1 | 0.877332 | 3.733736 | 11.27042 | 0.001997 | 0.017817 |
| ENSG00000069424 | KCNAB2 | 0.875441 | 4.423834 | 21.03443 | 6.21E-05 | 0.001014 |
| ENSG00000172534 | HCFC1 | 0.872923 | 5.833863 | 35.19712 | 1.63E-06 | 4.51E-05 |
| ENSG00000100150 | DEPDC5 | 0.868971 | 4.530042 | 21.72117 | 5.00E-05 | 0.000846 |
| ENSG00000109819 | PPARGC1A | 0.861778 | 3.699967 | 12.18106 | 0.001393 | 0.013484 |
| ENSG00000156103 | MMP16 | 0.855695 | 3.432306 | 9.783311 | 0.003668 | 0.028429 |
| ENSG00000196730 | DAPK1 | 0.853912 | 3.402518 | 10.3335 | 0.00292 | 0.024002 |
| ENSG00000173599 | PC | 0.853151 | 3.424332 | 10.05863 | 0.00327 | 0.026162 |
| ENSG00000135905 | DOCK10 | 0.852521 | 3.607301 | 10.90193 | 0.002316 | 0.020044 |
| ENSG00000104142 | VPS18 | 0.85037 | 3.557875 | 9.982141 | 0.003376 | 0.026763 |
| ENSG00000215190 | GUSBP1 | 0.850303 | 3.943743 | 14.62687 | 0.000552 | 0.006355 |
| ENSG00000215190 | LINC00680 | 0.850303 | 3.943743 | 14.62687 | 0.000552 | 0.006355 |
| ENSG00000148841 | ITPRIP | 0.849729 | 3.443381 | 8.896692 | 0.00534 | 0.038131 |
| ENSG00000132359 | RAP1GAP2 | 0.844969 | 3.936721 | 13.55864 | 0.000822 | 0.008849 |
| ENSG00000143842 | SOX13 | 0.841225 | 3.726391 | 11.98415 | 0.001505 | 0.014376 |
| ENSG00000141449 | GREB1L | 0.839728 | 3.403712 | 9.580524 | 0.003993 | 0.030359 |
| ENSG00000169429 | CXCL8 | 0.83925 | 3.631689 | 10.9888 | 0.002236 | 0.019513 |
| ENSG00000074755 | ZZEF1 | 0.838924 | 4.45593 | 19.8858 | 8.99E-05 | 0.001392 |
| ENSG00000166473 | PKD1L2 | 0.838585 | 4.115132 | 13.95453 | 0.000708 | 0.007799 |
| ENSG00000086015 | MAST2 | 0.836517 | 4.114994 | 12.555 | 0.001205 | 0.012007 |
| ENSG00000136895 | GARNL3 | 0.833756 | 3.531993 | 9.912236 | 0.003476 | 0.02729 |
| ENSG00000124762 | CDKN1A | 0.83345 | 4.044975 | 13.93844 | 0.000713 | 0.007822 |
| ENSG00000124574 | ABCC10 | 0.82974 | 4.488797 | 17.4855 | 0.000201 | 0.002737 |
| ENSG00000247809 | NR2F2-AS1 | 0.829323 | 3.5067 | 10.27294 | 0.002993 | 0.024404 |
| ENSG00000165185 | KIAA1958 | 0.825255 | 3.349473 | 8.710346 | 0.005787 | 0.040623 |
| ENSG00000164506 | STXBP5 | 0.821933 | 3.185614 | 8.320365 | 0.006857 | 0.046094 |
| ENSG00000141664 | ZCCHC2 | 0.820095 | 3.17725 | 8.183943 | 0.00728 | 0.048324 |
| ENSG00000108344 | PSMD3 | 0.817878 | 3.639108 | 10.96035 | 0.002262 | 0.019678 |
| ENSG00000233184 | LOC102606465 | 0.816532 | 3.888383 | 12.75853 | 0.001114 | 0.011299 |
| ENSG00000175344 | CHRNA7 | 0.815781 | 3.430172 | 10.36936 | 0.002877 | 0.02374 |
| ENSG00000144893 | MED12L | 0.812721 | 3.243608 | 8.591574 | 0.006092 | 0.042206 |
| ENSG00000203879 | GDI1 | 0.809082 | 4.304873 | 12.4866 | 0.001267 | 0.0125 |
| ENSG00000137309 | HMGA1 | 0.808245 | 3.821643 | 10.2832 | 0.002981 | 0.024334 |
| ENSG00000178950 | GAK | 0.806109 | 4.977982 | 18.39076 | 0.000154 | 0.002198 |
| ENSG00000083290 | ULK2 | 0.804448 | 3.59621 | 10.886 | 0.002331 | 0.020112 |
| ENSG00000144645 | OSBPL10 | 0.8019 | 4.113496 | 12.48926 | 0.001236 | 0.012244 |
| ENSG00000173064 | HECTD4 | 0.800628 | 4.794829 | 21.28402 | 5.74E-05 | 0.000948 |
| ENSG00000170946 | DNAJC24 | 0.798884 | 3.88282 | 11.52421 | 0.001804 | 0.016604 |
| ENSG00000110274 | CEP164 | 0.798806 | 3.361128 | 8.520701 | 0.006283 | 0.043148 |
| ENSG00000135083 | CCNJL | 0.798215 | 3.407209 | 8.785385 | 0.005602 | 0.039585 |
| ENSG00000165271 | NOL6 | 0.796421 | 5.660654 | 33.45732 | 1.83E-06 | 4.93E-05 |
| ENSG00000136854 | STXBP1 | 0.795289 | 3.811022 | 11.94849 | 0.001526 | 0.014529 |
| ENSG00000102805 | CLN5 | 0.792796 | 3.80766 | 9.897534 | 0.003497 | 0.027401 |
| ENSG00000157168 | NRG1 | 0.791104 | 4.772856 | 18.39511 | 0.000147 | 0.00212 |
| ENSG00000164989 | CCDC171 | 0.790517 | 3.818904 | 9.22763 | 0.004636 | 0.034161 |
| ENSG00000144724 | PTPRG | 0.787887 | 4.469686 | 15.5752 | 0.000392 | 0.004815 |
| ENSG00000081026 | MAGI3 | 0.787786 | 3.589341 | 8.73458 | 0.005727 | 0.040319 |
| ENSG00000186283 | TOR3A | 0.787083 | 4.156651 | 12.19467 | 0.001386 | 0.013429 |
| ENSG00000243156 | MICAL3 | 0.786356 | 4.452452 | 14.48455 | 0.000582 | 0.006623 |
| ENSG00000154358 | OBSCN | 0.784977 | 5.481003 | 29.97446 | 4.55E-06 | 0.000104 |
| ENSG00000184708 | EIF4ENIF1 | 0.783927 | 4.069814 | 10.18272 | 0.003162 | 0.025525 |
| ENSG00000257335 | MGAM | 0.783073 | 3.806783 | 9.217881 | 0.004655 | 0.034215 |
| ENSG00000144824 | PHLDB2 | 0.781266 | 5.159348 | 22.59529 | 3.81E-05 | 0.000669 |
| ENSG00000173085 | COQ2 | 0.774458 | 4.440238 | 12.11477 | 0.001542 | 0.014636 |
| ENSG00000095637 | SORBS1 | 0.767634 | 4.485164 | 14.82565 | 0.000514 | 0.006023 |
| ENSG00000152767 | FARP1 | 0.767089 | 4.111704 | 12.5166 | 0.001223 | 0.012157 |
| ENSG00000172046 | USP19 | 0.766236 | 3.560024 | 8.310062 | 0.006888 | 0.046174 |
| ENSG00000226479 | TMEM185B | 0.762529 | 4.991627 | 22.33675 | 4.13E-05 | 0.000718 |
| ENSG00000157064 | NMNAT2 | 0.761318 | 3.782907 | 9.914985 | 0.003472 | 0.027277 |
| ENSG00000155846 | PPARGC1B | 0.757598 | 4.246909 | 12.26086 | 0.00135 | 0.013153 |
| ENSG00000069020 | MAST4 | 0.757403 | 4.296768 | 11.57158 | 0.0018 | 0.016578 |
| ENSG00000185070 | FLRT2 | 0.757345 | 4.53222 | 11.24854 | 0.002185 | 0.019186 |
| ENSG00000198198 | SZT2 | 0.752762 | 4.852234 | 17.57334 | 0.000195 | 0.002675 |
| ENSG00000171552 | BCL2L1 | 0.749505 | 4.988052 | 16.45764 | 0.000289 | 0.003713 |
| ENSG00000160298 | C21orf58 | 0.747981 | 4.127825 | 11.97425 | 0.001511 | 0.01442 |
| ENSG00000181026 | AEN | 0.74694 | 5.509309 | 24.57222 | 2.10E-05 | 0.000393 |
| ENSG00000077782 | FGFR1 | 0.74316 | 4.568833 | 11.58053 | 0.001894 | 0.017222 |
| ENSG00000050820 | BCAR1 | 0.740758 | 4.170367 | 9.787258 | 0.003713 | 0.028683 |
| ENSG00000127511 | SIN3B | 0.736053 | 3.569106 | 8.791598 | 0.005587 | 0.039503 |
| ENSG00000182010 | RTKN2 | 0.732654 | 3.966117 | 10.40139 | 0.002839 | 0.023514 |
| ENSG00000275066 | SYNRG | 0.731638 | 4.275074 | 11.25695 | 0.002007 | 0.017872 |
| ENSG00000154917 | RAB6B | 0.73056 | 3.854927 | 9.776551 | 0.003678 | 0.02846 |
| ENSG00000196924 | FLNA | 0.730231 | 7.75282 | 20.16142 | 0.001761 | 0.01632 |
| ENSG00000100403 | ZC3H7B | 0.729153 | 4.144889 | 11.56712 | 0.001774 | 0.016377 |
| ENSG00000106012 | IQCE | 0.727462 | 3.550563 | 8.14286 | 0.007413 | 0.04898 |
| ENSG00000105221 | AKT2 | 0.726099 | 3.8711 | 8.517054 | 0.006293 | 0.043191 |
| ENSG00000184254 | ALDH1A3 | 0.725802 | 5.582842 | 14.60422 | 0.001176 | 0.011809 |
| ENSG00000160949 | TONSL | 0.723239 | 4.065544 | 9.447299 | 0.004224 | 0.031755 |
| ENSG00000176170 | SPHK1 | 0.723101 | 4.266597 | 11.53031 | 0.0018 | 0.016578 |
| ENSG00000138162 | TACC2 | 0.721375 | 5.229617 | 19.53247 | 0.000101 | 0.001534 |
| ENSG00000163638 | ADAMTS9 | 0.720839 | 3.703008 | 8.268061 | 0.007016 | 0.046922 |
| ENSG00000107554 | DNMBP | 0.719579 | 4.703063 | 15.54084 | 0.000397 | 0.004843 |
| ENSG00000089280 | FUS | 0.719087 | 5.555402 | 13.70865 | 0.001612 | 0.015124 |
| ENSG00000176155 | CCDC57 | 0.717868 | 4.725904 | 15.81034 | 0.00036 | 0.004499 |
| ENSG00000157193 | LRP8 | 0.7172 | 4.71176 | 14.65395 | 0.000547 | 0.006317 |
| ENSG00000185630 | PBX1 | 0.716613 | 4.305888 | 12.08081 | 0.001449 | 0.013913 |
| ENSG00000089159 | PXN | 0.713687 | 5.401961 | 20.36908 | 7.77E-05 | 0.001226 |
| ENSG00000129667 | RHBDF2 | 0.713135 | 4.840924 | 15.47416 | 0.000406 | 0.00494 |
| ENSG00000175471 | MCTP1 | 0.711845 | 4.023511 | 9.779971 | 0.003673 | 0.028449 |
| ENSG00000106443 | PHF14 | 0.705884 | 4.241938 | 11.19506 | 0.002058 | 0.018222 |
| ENSG00000002587 | HS3ST1 | 0.705737 | 3.916025 | 8.834998 | 0.005484 | 0.038935 |
| ENSG00000166444 | DENND2B | 0.70495 | 3.612054 | 8.158653 | 0.007362 | 0.04878 |
| ENSG00000196220 | SRGAP3 | 0.703589 | 4.801312 | 17.30678 | 0.000213 | 0.002883 |
| ENSG00000113389 | NPR3 | 0.702188 | 4.077423 | 10.56172 | 0.002659 | 0.022293 |
| ENSG00000160216 | AGPAT3 | 0.700529 | 4.124605 | 9.735109 | 0.003742 | 0.028834 |
| ENSG00000131797 | CLUHP3 | 0.696811 | 3.792825 | 8.685972 | 0.005848 | 0.040895 |
| ENSG00000178038 | ALS2CL | 0.696556 | 6.004413 | 28.53176 | 6.74E-06 | 0.000145 |
| ENSG00000101901 | ALG13 | 0.696006 | 4.875342 | 14.30942 | 0.000621 | 0.006996 |
| ENSG00000077157 | PPP1R12B | 0.69395 | 5.018724 | 17.28921 | 0.000215 | 0.002897 |
| ENSG00000128159 | TUBGCP6 | 0.692973 | 4.06586 | 9.774637 | 0.003681 | 0.02846 |
| ENSG00000178971 | CTC1 | 0.690196 | 5.03871 | 17.74003 | 0.000184 | 0.002548 |
| ENSG00000188211 | NCR3LG1 | 0.689611 | 3.999289 | 9.143516 | 0.004805 | 0.03509 |
| ENSG00000119720 | NRDE2 | 0.689171 | 4.593521 | 13.12629 | 0.000968 | 0.010101 |
| ENSG00000140853 | NLRC5 | 0.683287 | 4.350655 | 11.34593 | 0.001937 | 0.017505 |
| ENSG00000125779 | PANK2 | 0.681813 | 3.857446 | 8.317338 | 0.006866 | 0.046107 |
| ENSG00000129933 | MAU2 | 0.675221 | 4.437696 | 11.06629 | 0.002167 | 0.019058 |
| ENSG00000133065 | SLC41A1 | 0.671048 | 5.038111 | 15.63244 | 0.000384 | 0.004742 |
| ENSG00000166900 | STX3 | 0.670554 | 4.700227 | 13.24661 | 0.000924 | 0.009747 |
| ENSG00000107099 | DOCK8 | 0.669695 | 4.195985 | 10.81748 | 0.002396 | 0.020554 |
| ENSG00000144040 | SFXN5 | 0.669208 | 4.380351 | 10.02517 | 0.003316 | 0.026407 |
| ENSG00000110090 | CPT1A | 0.666656 | 4.504531 | 12.08032 | 0.001449 | 0.013913 |
| ENSG00000139645 | ANKRD52 | 0.664179 | 4.417684 | 10.60375 | 0.002614 | 0.022055 |
| ENSG00000179818 | PCBP1-AS1 | 0.663801 | 4.914773 | 15.82886 | 0.000358 | 0.004474 |
| ENSG00000110888 | CAPRIN2 | 0.662387 | 4.052728 | 8.307813 | 0.006895 | 0.046192 |
| ENSG00000030110 | BAK1 | 0.657161 | 6.716863 | 19.00186 | 0.000537 | 0.006242 |
| ENSG00000178921 | PFAS | 0.653371 | 6.133833 | 13.98359 | 0.001914 | 0.017353 |
| ENSG00000171877 | FRMD5 | 0.652474 | 3.93506 | 8.277851 | 0.006986 | 0.046749 |
| ENSG00000029534 | ANK1 | 0.650778 | 4.597271 | 10.98404 | 0.00224 | 0.019536 |
| ENSG00000177494 | ZBED2 | 0.647458 | 5.279648 | 17.04134 | 0.000234 | 0.003094 |
| ENSG00000186185 | KIF18B | 0.645371 | 5.793475 | 10.7645 | 0.005275 | 0.037803 |
| ENSG00000100280 | AP1B1 | 0.645115 | 6.175008 | 14.71427 | 0.001463 | 0.014023 |
| ENSG00000123144 | TRIR | 0.64438 | 4.330824 | 8.646836 | 0.005948 | 0.041367 |
| ENSG00000125454 | SLC25A19 | 0.643582 | 4.20195 | 9.492934 | 0.004143 | 0.031274 |
| ENSG00000177570 | SAMD12 | 0.639157 | 4.413426 | 10.25256 | 0.003019 | 0.024574 |
| ENSG00000031823 | RANBP3 | 0.633476 | 4.374926 | 10.06936 | 0.003256 | 0.026064 |
| ENSG00000115355 | CCDC88A | 0.631986 | 4.621741 | 12.49939 | 0.001231 | 0.012207 |
| ENSG00000104290 | FZD3 | 0.63073 | 4.440949 | 10.32706 | 0.002927 | 0.024002 |
| ENSG00000102858 | MGRN1 | 0.628556 | 4.476654 | 9.518535 | 0.004099 | 0.031018 |
| ENSG00000013573 | DDX11 | 0.627707 | 5.214564 | 14.63574 | 0.000551 | 0.006347 |
| ENSG00000078061 | ARAF | 0.626589 | 5.083043 | 13.329 | 0.000896 | 0.009508 |
| ENSG00000132382 | MYBBP1A | 0.626498 | 6.816121 | 13.25852 | 0.004045 | 0.030691 |
| ENSG00000072609 | CHFR | 0.617445 | 4.504938 | 9.283214 | 0.004528 | 0.033471 |
| ENSG00000072121 | ZFYVE26 | 0.617241 | 4.854375 | 12.79759 | 0.001097 | 0.01117 |
| ENSG00000049759 | NEDD4L | 0.616551 | 6.720973 | 38.91457 | 4.81E-07 | 1.70E-05 |
| ENSG00000168528 | SERINC2 | 0.612304 | 5.258889 | 17.14458 | 0.000226 | 0.003013 |
| ENSG00000183495 | EP400 | 0.611262 | 4.544397 | 10.33633 | 0.002916 | 0.023995 |
| ENSG00000172137 | CALB2 | 0.601885 | 6.782513 | 31.87823 | 2.74E-06 | 6.80E-05 |
| ENSG00000170921 | TANC2 | 0.601072 | 4.588822 | 10.37224 | 0.002874 | 0.023729 |
| ENSG00000123384 | LRP1 | 0.599472 | 4.437991 | 9.187899 | 0.004715 | 0.034544 |
| ENSG00000108846 | ABCC3 | 0.595199 | 5.753591 | 20.26988 | 7.94E-05 | 0.001251 |
| ENSG00000127311 | HELB | 0.594168 | 4.216758 | 8.444173 | 0.006496 | 0.044215 |
| ENSG00000120709 | FAM53C | 0.592656 | 5.350333 | 13.72222 | 0.000773 | 0.008393 |
| ENSG00000120709 | LOC100128966 | 0.592656 | 5.350333 | 13.72222 | 0.000773 | 0.008393 |
| ENSG00000108669 | CYTH1 | 0.589463 | 4.614618 | 9.016623 | 0.005072 | 0.036767 |
| ENSG00000149639 | SOGA1 | 0.588955 | 5.65304 | 18.87389 | 0.000125 | 0.001846 |
| ENSG00000137200 | CMTR1 | 0.586897 | 4.550074 | 8.798769 | 0.00557 | 0.03943 |
| ENSG00000129657 | SEC14L1 | 0.585792 | 5.060008 | 11.71421 | 0.001673 | 0.0156 |
| ENSG00000073910 | FRY | 0.583417 | 4.654944 | 8.484369 | 0.006406 | 0.04381 |
| ENSG00000181222 | POLR2A | 0.578642 | 6.769057 | 15.96056 | 0.001036 | 0.010687 |
| ENSG00000228794 | LINC01128 | 0.57731 | 4.38052 | 8.533723 | 0.006247 | 0.043063 |
| ENSG00000228794 | LOC107984850 | 0.57731 | 4.38052 | 8.533723 | 0.006247 | 0.043063 |
| ENSG00000168542 | COL3A1 | 0.574922 | 4.647006 | 10.32943 | 0.002925 | 0.024002 |
| ENSG00000162840 | NA | 0.573944 | 6.254229 | 24.05358 | 2.45E-05 | 0.000452 |
| ENSG00000164171 | ITGA2 | 0.573184 | 5.594685 | 17.23971 | 0.000218 | 0.00293 |
| ENSG00000162139 | NEU3 | 0.569558 | 5.186929 | 13.63815 | 0.000797 | 0.008612 |
| ENSG00000002834 | LASP1 | 0.568967 | 7.032988 | 24.55607 | 3.95E-05 | 0.00069 |
| ENSG00000105676 | ARMC6 | 0.566075 | 5.754276 | 17.07996 | 0.000231 | 0.003056 |
| ENSG00000012232 | EXTL3 | 0.565392 | 5.51185 | 12.83572 | 0.001135 | 0.01147 |
| ENSG00000158125 | XDH | 0.564822 | 5.448783 | 14.76329 | 0.000526 | 0.006144 |
| ENSG00000139318 | DUSP6 | 0.564257 | 5.509393 | 14.72984 | 0.000532 | 0.00621 |
| ENSG00000183696 | UPP1 | 0.56062 | 6.370426 | 24.47983 | 2.15E-05 | 0.000402 |
| ENSG00000115107 | STEAP3 | 0.557025 | 5.336775 | 14.26268 | 0.000632 | 0.007104 |
| ENSG00000065413 | ANKRD44 | 0.556857 | 4.601437 | 8.656534 | 0.005923 | 0.041244 |
| ENSG00000170100 | ZNF778 | 0.556135 | 5.2926 | 13.93818 | 0.000713 | 0.007822 |
| ENSG00000150995 | ITPR1 | 0.556075 | 4.630069 | 8.737927 | 0.005718 | 0.040305 |
| ENSG00000005884 | ITGA3 | 0.552843 | 6.156591 | 16.54327 | 0.000313 | 0.003998 |
| ENSG00000126012 | KDM5C | 0.547251 | 5.289635 | 11.41951 | 0.001881 | 0.017152 |
| ENSG00000131089 | ARHGEF9 | 0.545715 | 4.887533 | 9.412161 | 0.004287 | 0.032104 |
| ENSG00000141956 | PRDM15 | 0.543935 | 4.680284 | 8.843067 | 0.005465 | 0.038828 |
| ENSG00000137843 | PAK6 | 0.542409 | 6.271427 | 20.26293 | 7.96E-05 | 0.001252 |
| ENSG00000137843 | BUB1B-PAK6 | 0.542409 | 6.271427 | 20.26293 | 7.96E-05 | 0.001252 |
| ENSG00000148396 | SEC16A | 0.541497 | 6.109106 | 15.90125 | 0.00039 | 0.004812 |
| ENSG00000089154 | GCN1 | 0.539634 | 7.751337 | 15.13658 | 0.002327 | 0.020095 |
| ENSG00000156463 | SH3RF2 | 0.537305 | 6.459028 | 21.41827 | 5.50E-05 | 0.000917 |
| ENSG00000053747 | LAMA3 | 0.535059 | 9.176628 | 47.36216 | 7.40E-08 | 3.98E-06 |
| ENSG00000165323 | FAT3 | 0.532238 | 4.846223 | 10.05472 | 0.003276 | 0.026165 |
| ENSG00000196914 | ARHGEF12 | 0.531161 | 4.757692 | 9.085621 | 0.004925 | 0.035901 |
| ENSG00000234545 | FAM133B | 0.529933 | 4.870466 | 9.142144 | 0.004808 | 0.03509 |
| ENSG00000196878 | LAMB3 | 0.52958 | 8.518278 | 22.22464 | 0.000275 | 0.003565 |
| ENSG00000151131 | C12orf45 | 0.528342 | 5.064489 | 11.37567 | 0.001914 | 0.017353 |
| ENSG00000081760 | AACS | 0.526802 | 5.124034 | 8.228577 | 0.007446 | 0.049115 |
| ENSG00000177084 | POLE | 0.522776 | 5.640852 | 14.67976 | 0.000542 | 0.006283 |
| ENSG00000047188 | YTHDC2 | 0.522441 | 5.060549 | 10.32692 | 0.002928 | 0.024002 |
| ENSG00000110104 | CCDC86 | 0.520569 | 5.829279 | 15.28333 | 0.000435 | 0.005225 |
| ENSG00000160193 | WDR4 | 0.519147 | 5.487731 | 13.21186 | 0.000937 | 0.009846 |
| ENSG00000100726 | TELO2 | 0.517519 | 5.212166 | 8.333873 | 0.007133 | 0.047535 |
| ENSG00000166348 | USP54 | 0.514507 | 5.141346 | 10.59702 | 0.002621 | 0.022055 |
| ENSG00000100888 | CHD8 | 0.513046 | 5.600553 | 11.41225 | 0.001925 | 0.017422 |
| ENSG00000133812 | SBF2 | 0.51106 | 5.1121 | 9.682221 | 0.003826 | 0.029341 |
| ENSG00000140465 | CYP1A1 | 0.509817 | 9.477467 | 45.1674 | 1.18E-07 | 5.77E-06 |
| ENSG00000141252 | VPS53 | 0.503619 | 5.1538 | 9.962047 | 0.003404 | 0.026914 |
| ENSG00000173517 | PEAK1 | 0.503461 | 4.992737 | 9.667296 | 0.00385 | 0.029467 |
| ENSG00000196549 | MME | 0.503158 | 5.16784 | 10.03372 | 0.003304 | 0.026342 |
| ENSG00000269821 | KCNQ1OT1 | 0.498006 | 6.602448 | 23.80301 | 2.64E-05 | 0.000481 |
| ENSG00000109111 | SUPT6H | 0.496086 | 5.546089 | 8.573254 | 0.006782 | 0.045754 |
| ENSG00000163545 | NUAK2 | 0.492436 | 5.652399 | 11.75263 | 0.001648 | 0.015402 |
| ENSG00000106852 | LHX6 | 0.492395 | 5.225209 | 10.3752 | 0.00287 | 0.023717 |
| ENSG00000110047 | EHD1 | 0.492001 | 6.3946 | 16.87687 | 0.00025 | 0.003273 |
| ENSG00000130175 | PRKCSH | 0.488297 | 5.339026 | 10.07699 | 0.003246 | 0.026046 |
| ENSG00000135318 | NT5E | 0.481041 | 5.183933 | 9.405028 | 0.0043 | 0.032138 |
| ENSG00000127564 | PKMYT1 | 0.478346 | 6.028277 | 9.461203 | 0.005211 | 0.037535 |
| ENSG00000160613 | PCSK7 | 0.478156 | 5.764587 | 10.31672 | 0.003068 | 0.024906 |
| ENSG00000126883 | NUP214 | 0.477183 | 5.974062 | 13.68049 | 0.000785 | 0.008501 |
| ENSG00000108591 | DRG2 | 0.476186 | 5.014306 | 8.590833 | 0.006094 | 0.042206 |
| ENSG00000140350 | ANP32A | 0.475418 | 5.379627 | 10.16158 | 0.003134 | 0.025352 |
| ENSG00000196922 | NA | 0.471138 | 5.210636 | 9.717793 | 0.00377 | 0.028985 |
| ENSG00000052749 | RRP12 | 0.469947 | 6.656343 | 20.00273 | 8.66E-05 | 0.001347 |
| ENSG00000112159 | MDN1 | 0.469334 | 5.122865 | 8.137586 | 0.00743 | 0.049065 |
| ENSG00000144136 | SLC20A1 | 0.468151 | 8.413299 | 38.74269 | 5.00E-07 | 1.76E-05 |
| ENSG00000006327 | TNFRSF12A | 0.467101 | 7.639627 | 29.73172 | 4.86E-06 | 0.00011 |
| ENSG00000137642 | SORL1 | 0.466879 | 5.531106 | 8.194064 | 0.007622 | 0.049988 |
| ENSG00000103197 | TSC2 | 0.466766 | 5.451911 | 11.02358 | 0.002205 | 0.019314 |
| ENSG00000072786 | STK10 | 0.466364 | 5.712965 | 10.4253 | 0.00282 | 0.023423 |
| ENSG00000188636 | RTL6 | 0.456448 | 5.64824 | 10.60233 | 0.002615 | 0.022055 |
| ENSG00000135763 | URB2 | 0.448034 | 6.545857 | 15.74262 | 0.000369 | 0.004584 |
| ENSG00000100258 | LMF2 | 0.445596 | 5.604623 | 10.49731 | 0.00273 | 0.022759 |
| ENSG00000125148 | MT2A | 0.443251 | 10.51156 | 36.05142 | 9.55E-07 | 2.94E-05 |
| ENSG00000184677 | ZBTB40 | 0.441608 | 5.788999 | 10.42102 | 0.002817 | 0.02341 |
| ENSG00000116455 | WDR77 | 0.439892 | 6.085941 | 13.40102 | 0.000872 | 0.009321 |
| ENSG00000147459 | DOCK5 | 0.436884 | 6.269706 | 14.89415 | 0.000501 | 0.005881 |
| ENSG00000122390 | NAA60 | 0.435675 | 5.746127 | 8.876426 | 0.005431 | 0.038731 |
| ENSG00000142002 | DPP9 | 0.434345 | 6.221654 | 12.96498 | 0.001029 | 0.010655 |
| ENSG00000140829 | DHX38 | 0.433712 | 6.238549 | 15.0111 | 0.00048 | 0.005677 |
| ENSG00000048342 | CC2D2A | 0.431558 | 5.683454 | 10.50984 | 0.002716 | 0.022705 |
| ENSG00000065618 | COL17A1 | 0.431352 | 7.300478 | 21.43206 | 5.48E-05 | 0.000915 |
| ENSG00000132470 | ITGB4 | 0.421441 | 8.047804 | 13.70401 | 0.001925 | 0.017422 |
| ENSG00000105953 | OGDH | 0.419801 | 6.539436 | 11.76574 | 0.00177 | 0.016377 |
| ENSG00000226887 | ERVMER34-1 | 0.418552 | 5.946015 | 10.98087 | 0.002243 | 0.019546 |
| ENSG00000196923 | PDLIM7 | 0.418374 | 5.650075 | 9.695284 | 0.003805 | 0.02922 |
| ENSG00000004478 | FKBP4 | 0.417412 | 6.471219 | 12.00187 | 0.001527 | 0.014529 |
| ENSG00000159842 | ABR | 0.412191 | 5.888348 | 9.291868 | 0.004511 | 0.033391 |
| ENSG00000055070 | SZRD1 | 0.4097 | 6.396536 | 10.04746 | 0.003541 | 0.027693 |
| ENSG00000189280 | GJB5 | 0.401055 | 5.537195 | 8.339118 | 0.006801 | 0.04581 |
| ENSG00000115946 | PNO1 | 0.399411 | 5.851975 | 9.475172 | 0.004174 | 0.031487 |
| ENSG00000070814 | TCOF1 | 0.397659 | 7.890197 | 21.21852 | 5.86E-05 | 0.000963 |
| ENSG00000107862 | GBF1 | 0.396131 | 6.708318 | 11.48722 | 0.001965 | 0.017656 |
| ENSG00000084112 | SSH1 | 0.390019 | 5.665817 | 8.209672 | 0.007198 | 0.047885 |
| ENSG00000116127 | ALMS1 | 0.388339 | 5.977911 | 9.90652 | 0.003484 | 0.027336 |
| ENSG00000026508 | CD44 | 0.386774 | 8.110291 | 22.06266 | 4.50E-05 | 0.000774 |
| ENSG00000117143 | UAP1 | 0.386399 | 6.644075 | 13.95402 | 0.000708 | 0.007799 |
| ENSG00000172927 | MYEOV | 0.386045 | 6.767449 | 13.31286 | 0.000902 | 0.00954 |
| ENSG00000035681 | NSMAF | 0.384519 | 5.936037 | 10.02195 | 0.003321 | 0.026416 |
| ENSG00000185219 | ZNF445 | 0.381504 | 5.830901 | 8.915163 | 0.005298 | 0.037924 |
| ENSG00000127603 | MACF1 | 0.380112 | 6.510674 | 13.12776 | 0.000967 | 0.010101 |
| ENSG00000127603 | KIAA0754 | 0.380112 | 6.510674 | 13.12776 | 0.000967 | 0.010101 |
| ENSG00000167658 | EEF2 | 0.374243 | 7.634038 | 20.53682 | 7.29E-05 | 0.001158 |
| ENSG00000068654 | POLR1A | 0.36943 | 6.538896 | 10.44589 | 0.002788 | 0.023221 |
| ENSG00000129255 | MPDU1 | 0.363589 | 6.25908 | 10.15485 | 0.003143 | 0.025405 |
| ENSG00000196235 | SUPT5H | 0.360475 | 6.105851 | 8.156001 | 0.00737 | 0.048809 |
| ENSG00000196396 | PTPN1 | 0.357243 | 6.343311 | 8.52985 | 0.006267 | 0.043091 |
| ENSG00000100401 | RANGAP1 | 0.354415 | 7.601007 | 13.6066 | 0.000883 | 0.009411 |
| ENSG00000117525 | F3 | 0.350392 | 7.584139 | 18.22625 | 0.000156 | 0.002222 |
| ENSG00000118971 | CCND2 | 0.343979 | 9.440093 | 23.04367 | 3.32E-05 | 0.000589 |
| ENSG00000132768 | DPH2 | 0.335527 | 6.67611 | 11.02114 | 0.002207 | 0.019319 |
| ENSG00000148843 | PDCD11 | 0.331135 | 6.677477 | 9.919737 | 0.003465 | 0.027277 |
| ENSG00000137801 | THBS1 | 0.324836 | 9.429536 | 17.7542 | 0.000183 | 0.002539 |
| ENSG00000150687 | PRSS23 | 0.321949 | 6.405218 | 8.962076 | 0.005192 | 0.037471 |
| ENSG00000107984 | DKK1 | 0.321709 | 6.717819 | 8.47371 | 0.006418 | 0.043866 |
| ENSG00000138772 | ANXA3 | 0.320738 | 6.692347 | 8.913415 | 0.005302 | 0.037929 |
| ENSG00000145934 | TENM2 | 0.317272 | 7.066348 | 11.65958 | 0.00171 | 0.015889 |
| ENSG00000167601 | AXL | 0.311702 | 6.570089 | 8.601456 | 0.006066 | 0.042062 |
| ENSG00000155850 | SLC26A2 | 0.307447 | 7.793365 | 13.65964 | 0.000791 | 0.008559 |
| ENSG00000126457 | PRMT1 | 0.304819 | 7.2069 | 8.355929 | 0.007085 | 0.047246 |
| ENSG00000215012 | RTL10 | 0.299618 | 6.713047 | 8.773522 | 0.005631 | 0.039739 |
| ENSG00000080608 | PUM3 | 0.280958 | 6.918153 | 8.418898 | 0.006568 | 0.044644 |
| ENSG00000053372 | MRTO4 | 0.278312 | 7.040617 | 9.049643 | 0.005001 | 0.036412 |
| ENSG00000112759 | SLC29A1 | 0.267248 | 7.258904 | 9.4688 | 0.004186 | 0.031551 |
| ENSG00000058085 | LAMC2 | 0.259531 | 10.90697 | 14.61094 | 0.000556 | 0.006367 |
| ENSG00000109971 | HSPA8 | 0.21997 | 11.07232 | 9.387258 | 0.004332 | 0.032297 |
| ENSG00000185344 | ATP6V0A2 | 1.57097 | 2.998878 | 23.34823 | 3.03E-05 | 0.000542 |
| ENSG00000076641 | PAG1 | 1.354114 | 2.996371 | 19.36019 | 0.000107 | 0.001613 |
| ENSG00000287778 | NA | 0.919339 | 2.992766 | 8.675043 | 0.005876 | 0.041025 |
| ENSG00000105738 | SIPA1L3 | 0.964535 | 2.992501 | 10.05649 | 0.003273 | 0.026165 |
| ENSG00000102755 | FLT1 | 2.00402 | 2.984404 | 37.07368 | 7.45E-07 | 2.41E-05 |
| ENSG00000103150 | MLYCD | 1.772339 | 2.983067 | 33.00734 | 2.05E-06 | 5.42E-05 |
| ENSG00000186487 | MYT1L | 2.586858 | 2.977636 | 60.45106 | 5.89E-09 | 6.91E-07 |
| ENSG00000197301 | HMGA2-AS1 | 1.081916 | 2.976133 | 12.95465 | 0.001033 | 0.010687 |
| ENSG00000116793 | PHTF1 | 1.110773 | 2.975983 | 12.46957 | 0.001245 | 0.012327 |
| ENSG00000248323 | LUCAT1 | 1.589638 | 2.971742 | 26.61101 | 1.16E-05 | 0.000235 |
| ENSG00000142798 | HSPG2 | 1.488723 | 2.971457 | 16.5499 | 0.000324 | 0.004103 |
| ENSG00000154310 | TNIK | 1.252266 | 2.968273 | 18.76142 | 0.00013 | 0.001909 |
| ENSG00000185278 | ZBTB37 | 1.122884 | 2.965695 | 13.83738 | 0.00074 | 0.008092 |
| ENSG00000078237 | TIGAR | 1.166799 | 2.956869 | 12.57192 | 0.001197 | 0.011962 |
| ENSG00000236859 | NIFK-AS1 | 0.94913 | 2.955921 | 8.255143 | 0.007056 | 0.047101 |
| ENSG00000179361 | ARID3B | 0.901727 | 2.955861 | 8.081292 | 0.007617 | 0.049988 |
| ENSG00000142920 | AZIN2 | 1.567852 | 2.951296 | 26.46581 | 1.21E-05 | 0.000243 |
| ENSG00000071242 | RPS6KA2 | 1.159272 | 2.946835 | 12.79227 | 0.0011 | 0.011183 |
| ENSG00000069702 | TGFBR3 | 1.408667 | 2.946351 | 18.31235 | 0.000151 | 0.002172 |
| ENSG00000091879 | ANGPT2 | 1.026675 | 2.943052 | 10.60696 | 0.00261 | 0.022055 |
| ENSG00000272168 | NA | 2.873501 | 2.942424 | 62.75457 | 4.69E-09 | 5.93E-07 |
| ENSG00000166147 | FBN1 | 2.817369 | 2.941841 | 65.63432 | 2.39E-09 | 3.52E-07 |
| ENSG00000188807 | TMEM201 | 1.011447 | 2.935772 | 10.93012 | 0.002289 | 0.019886 |
| ENSG00000164053 | ATRIP | 1.047342 | 2.93558 | 11.02913 | 0.0022 | 0.019301 |
| ENSG00000164053 | ATRIP-TREX1 | 1.047342 | 2.93558 | 11.02913 | 0.0022 | 0.019301 |
| ENSG00000134824 | FADS2 | 1.289157 | 2.93081 | 15.71399 | 0.000373 | 0.004616 |
| ENSG00000042429 | MED17 | 1.619053 | 2.930398 | 28.67999 | 6.47E-06 | 0.000139 |
| ENSG00000069188 | SDK2 | 1.202677 | 2.929247 | 15.7146 | 0.000373 | 0.004616 |
| ENSG00000042781 | USH2A | 3.275614 | 2.926267 | 78.49145 | 3.08E-10 | 8.34E-08 |
| ENSG00000015133 | CCDC88C | 1.385981 | 2.918579 | 20.57174 | 7.20E-05 | 0.001146 |
| ENSG00000238197 | PAXBP1-AS1 | 0.906656 | 2.915212 | 8.222689 | 0.007157 | 0.047673 |
| ENSG00000073849 | ST6GAL1 | 1.687441 | 2.912938 | 28.03021 | 7.75E-06 | 0.000163 |
| ENSG00000139998 | RAB15 | 1.265723 | 2.907012 | 16.07963 | 0.000327 | 0.004133 |
| ENSG00000170456 | DENND5B | 1.231867 | 2.905378 | 13.62482 | 0.000801 | 0.008648 |
| ENSG00000183020 | AP2A2 | 1.065462 | 2.904363 | 10.07336 | 0.003251 | 0.026057 |
| ENSG00000148357 | HMCN2 | 2.021186 | 2.903765 | 34.06936 | 1.56E-06 | 4.37E-05 |
| ENSG00000237356 | NA | 1.721526 | 2.902633 | 28.47976 | 6.84E-06 | 0.000146 |
| ENSG00000188827 | SLX4 | 1.308925 | 2.897237 | 17.77768 | 0.000181 | 0.002521 |
| ENSG00000187240 | DYNC2H1 | 1.864722 | 2.893248 | 33.37175 | 1.87E-06 | 5.03E-05 |
| ENSG00000253394 | LINC00534 | 2.922787 | 2.891403 | 69.26103 | 1.30E-09 | 2.43E-07 |
| ENSG00000183914 | DNAH2 | 2.530651 | 2.887956 | 55.28449 | 1.53E-08 | 1.36E-06 |
| ENSG00000251364 | LOC100506258 | 0.893774 | 2.885999 | 8.713138 | 0.00578 | 0.040615 |
| ENSG00000184144 | CNTN2 | 2.169261 | 2.876739 | 38.41683 | 5.76E-07 | 1.95E-05 |
| ENSG00000154229 | PRKCA | 1.369168 | 2.876509 | 18.06905 | 0.000164 | 0.002323 |
| ENSG00000130349 | MTRES1 | 1.118025 | 2.874513 | 10.30622 | 0.002953 | 0.024191 |
| ENSG00000140848 | CPNE2 | 0.944948 | 2.874093 | 9.355397 | 0.004391 | 0.032651 |
| ENSG00000105357 | MYH14 | 1.57743 | 2.869301 | 24.23835 | 2.32E-05 | 0.000429 |
| ENSG00000113594 | LIFR | 1.330266 | 2.868739 | 15.74593 | 0.000369 | 0.004583 |
| ENSG00000245248 | NA | 1.306787 | 2.86676 | 16.58514 | 0.000274 | 0.003557 |
| ENSG00000181220 | ZNF746 | 1.584481 | 2.85878 | 24.72987 | 2.00E-05 | 0.000376 |
| ENSG00000213949 | ITGA1 | 1.403842 | 2.856416 | 16.14566 | 0.00032 | 0.00406 |
| ENSG00000280434 | NA | 2.175002 | 2.853113 | 35.28855 | 1.59E-06 | 4.43E-05 |
| ENSG00000259343 | NA | 1.488091 | 2.849734 | 21.6653 | 5.09E-05 | 0.000859 |
| ENSG00000110693 | SOX6 | 1.424731 | 2.847605 | 17.40365 | 0.000206 | 0.002801 |
| ENSG00000279249 | NA | 2.625375 | 2.847561 | 67.00461 | 1.89E-09 | 3.07E-07 |
| ENSG00000118997 | DNAH7 | 2.595779 | 2.847226 | 56.27943 | 1.27E-08 | 1.16E-06 |
| ENSG00000166436 | TRIM66 | 1.146144 | 2.844984 | 11.91653 | 0.001545 | 0.014641 |
| ENSG00000227500 | SCAMP4 | 1.11735 | 2.844476 | 11.59209 | 0.001756 | 0.016293 |
| ENSG00000165164 | CFAP47 | 0.90719 | 2.844319 | 8.566703 | 0.006159 | 0.042599 |
| ENSG00000226674 | TEX41 | 3.11258 | 2.841709 | 80.15552 | 2.41E-10 | 7.48E-08 |
| ENSG00000149485 | FADS1 | 1.538201 | 2.839029 | 21.66037 | 5.10E-05 | 0.000859 |
| ENSG00000187391 | MAGI2 | 3.208939 | 2.838957 | 79.59474 | 2.61E-10 | 7.71E-08 |
| ENSG00000141519 | CCDC40 | 0.901834 | 2.832988 | 8.416382 | 0.006575 | 0.044648 |
| ENSG00000186868 | MAPT | 0.99156 | 2.832118 | 9.915005 | 0.003472 | 0.027277 |
| ENSG00000254101 | LINC02055 | 3.374374 | 2.828206 | 68.49323 | 2.06E-09 | 3.25E-07 |
| ENSG00000146592 | CREB5 | 1.589135 | 2.827697 | 23.73975 | 2.69E-05 | 0.000489 |
| ENSG00000008300 | CELSR3 | 1.073042 | 2.824399 | 12.72383 | 0.001129 | 0.01142 |
| ENSG00000152582 | SPEF2 | 0.998532 | 2.823804 | 8.682141 | 0.005858 | 0.040938 |
| ENSG00000106070 | GRB10 | 1.121185 | 2.822956 | 11.94312 | 0.001529 | 0.014537 |
| ENSG00000165124 | SVEP1 | 1.644584 | 2.817843 | 25.64544 | 1.53E-05 | 0.000299 |
| ENSG00000198947 | DMD | 2.465203 | 2.814063 | 60.18971 | 6.17E-09 | 7.10E-07 |
| ENSG00000127663 | KDM4B | 1.279474 | 2.813882 | 15.29839 | 0.000433 | 0.005202 |
| ENSG00000089250 | NOS1 | 1.267732 | 2.813679 | 14.41871 | 0.000596 | 0.006766 |
| ENSG00000114841 | DNAH1 | 1.950969 | 2.809652 | 36.35502 | 8.86E-07 | 2.79E-05 |
| ENSG00000145990 | GFOD1 | 0.960224 | 2.809518 | 8.410759 | 0.006591 | 0.044705 |
| ENSG00000250072 | SH3TC2-DT | 1.706981 | 2.808914 | 18.61741 | 0.000181 | 0.002515 |
| ENSG00000215156 | NA | 1.45077 | 2.807046 | 19.84569 | 9.11E-05 | 0.001405 |
| ENSG00000277693 | NA | 1.338983 | 2.805264 | 18.92128 | 0.000124 | 0.001826 |
| ENSG00000203666 | EFCAB2 | 1.264438 | 2.803658 | 12.40871 | 0.001288 | 0.012656 |
| ENSG00000224660 | SH3BP5-AS1 | 1.22162 | 2.802542 | 14.10789 | 0.000669 | 0.007436 |
| ENSG00000166535 | A2ML1 | 0.986973 | 2.802145 | 8.97573 | 0.005162 | 0.037322 |
| ENSG00000179406 | LINC00174 | 0.979441 | 2.801985 | 9.025324 | 0.005054 | 0.036723 |
| ENSG00000164796 | CSMD3 | 2.462078 | 2.80148 | 57.46569 | 1.01E-08 | 9.97E-07 |
| ENSG00000103264 | FBXO31 | 1.053471 | 2.800848 | 8.427315 | 0.006571 | 0.044644 |
| ENSG00000245750 | DRAIC | 2.711919 | 2.791313 | 52.72927 | 2.50E-08 | 1.81E-06 |
| ENSG00000245750 | LINC00593 | 2.711919 | 2.791313 | 52.72927 | 2.50E-08 | 1.81E-06 |
| ENSG00000245750 | PCAT29 | 2.711919 | 2.791313 | 52.72927 | 2.50E-08 | 1.81E-06 |
| ENSG00000142621 | FHAD1 | 1.385366 | 2.783793 | 17.46914 | 0.000202 | 0.002746 |
| ENSG00000136531 | SCN2A | 1.493389 | 2.782776 | 21.71271 | 5.02E-05 | 0.000847 |
| ENSG00000198216 | CACNA1E | 2.664501 | 2.782567 | 68.16032 | 1.56E-09 | 2.64E-07 |
| ENSG00000154175 | ABI3BP | 1.29836 | 2.782098 | 15.5943 | 0.000389 | 0.004802 |
| ENSG00000246695 | NA | 1.34368 | 2.781055 | 14.72298 | 0.000535 | 0.006231 |
| ENSG00000186088 | GSAP | 1.321705 | 2.780789 | 16.19687 | 0.000314 | 0.004 |
| ENSG00000158321 | AUTS2 | 2.159234 | 2.777397 | 37.16467 | 7.29E-07 | 2.37E-05 |
| ENSG00000162105 | SHANK2 | 2.338191 | 2.768751 | 52.64686 | 2.54E-08 | 1.81E-06 |
| ENSG00000136828 | RALGPS1 | 0.995359 | 2.768225 | 9.540033 | 0.004062 | 0.030799 |
| ENSG00000137573 | SULF1 | 1.450627 | 2.759214 | 19.23905 | 0.000111 | 0.001667 |
| ENSG00000137103 | TMEM8B | 2.192271 | 2.7576 | 44.33506 | 1.41E-07 | 6.77E-06 |
| ENSG00000064309 | COON | 1.055454 | 2.745604 | 10.81078 | 0.002403 | 0.020591 |
| ENSG00000269473 | NA | 1.154945 | 2.745568 | 9.140613 | 0.005169 | 0.037352 |
| ENSG00000128849 | CGNL1 | 1.977808 | 2.74281 | 31.43282 | 3.08E-06 | 7.50E-05 |
| ENSG00000155849 | ELMO1 | 1.506311 | 2.740588 | 18.45262 | 0.000146 | 0.002104 |
| ENSG00000150471 | ADGRL3 | 1.638069 | 2.740197 | 25.22405 | 1.73E-05 | 0.000333 |
| ENSG00000155275 | TRMT44 | 1.601102 | 2.738953 | 20.8369 | 6.62E-05 | 0.001074 |
| ENSG00000213694 | S1PR3 | 1.69693 | 2.738692 | 18.00812 | 0.000225 | 0.003011 |
| ENSG00000213694 | C9orf47 | 1.69693 | 2.738692 | 18.00812 | 0.000225 | 0.003011 |
| ENSG00000171219 | CDC42BPG | 1.183034 | 2.736751 | 11.45269 | 0.001856 | 0.017016 |
| ENSG00000198624 | CCDC69 | 1.110145 | 2.73606 | 11.5046 | 0.001818 | 0.016721 |
| ENSG00000175170 | NA | 1.084862 | 2.73595 | 10.70065 | 0.002513 | 0.021377 |
| ENSG00000158805 | ZNF276 | 1.204559 | 2.735708 | 11.90678 | 0.001551 | 0.014661 |
| ENSG00000157890 | MEGF11 | 1.874755 | 2.73123 | 28.68429 | 6.47E-06 | 0.000139 |
| ENSG00000140015 | KCNH5 | 1.760047 | 2.730784 | 25.91751 | 1.41E-05 | 0.00028 |
| ENSG00000233012 | NA | 1.828364 | 2.727792 | 21.96527 | 5.53E-05 | 0.000921 |
| ENSG00000107282 | APBA1 | 1.134764 | 2.72404 | 12.6905 | 0.001143 | 0.011513 |
| ENSG00000237667 | LINC01115 | 1.037429 | 2.721687 | 9.585817 | 0.003984 | 0.030311 |
| ENSG00000249738 | LOC285626 | 1.406625 | 2.717632 | 14.28004 | 0.0007 | 0.007716 |
| ENSG00000163297 | ANTXR2 | 1.805742 | 2.717143 | 19.89933 | 0.000125 | 0.001846 |
| ENSG00000183117 | CSMD1 | 3.125153 | 2.716263 | 53.3523 | 6.47E-08 | 3.61E-06 |
| ENSG00000112964 | GHR | 1.188902 | 2.714941 | 11.66195 | 0.001708 | 0.015887 |
| ENSG00000237638 | NA | 1.386417 | 2.714675 | 18.07821 | 0.000164 | 0.00232 |
| ENSG00000135636 | DYSF | 1.319157 | 2.714628 | 14.61522 | 0.000555 | 0.006363 |
| ENSG00000103343 | ZNF174 | 1.129208 | 2.714101 | 11.68234 | 0.001695 | 0.015772 |
| ENSG00000103510 | KAT8 | 1.029079 | 2.711943 | 8.506239 | 0.006323 | 0.043317 |
| ENSG00000115295 | CLIP4 | 1.070284 | 2.710577 | 9.414295 | 0.004283 | 0.032096 |
| ENSG00000174640 | SLCO2A1 | 1.904206 | 2.706587 | 32.43983 | 2.37E-06 | 6.07E-05 |
| ENSG00000174640 | C3orf36 | 1.904206 | 2.706587 | 32.43983 | 2.37E-06 | 6.07E-05 |
| ENSG00000198691 | ABCA4 | 1.776937 | 2.705882 | 29.13743 | 5.71E-06 | 0.000126 |
| ENSG00000110436 | SLC1A2 | 3.290907 | 2.7057 | 75.10654 | 5.15E-10 | 1.24E-07 |
| ENSG00000081479 | LRP2 | 3.457251 | 2.704839 | 76.26711 | 4.31E-10 | 1.10E-07 |
| ENSG00000247199 | LOC102546294 | 1.416759 | 2.704656 | 16.43054 | 0.000289 | 0.003713 |
| ENSG00000242759 | NA | 2.606314 | 2.700236 | 56.92965 | 1.12E-08 | 1.07E-06 |
| ENSG00000108187 | PBLD | 1.055136 | 2.699847 | 9.247511 | 0.004597 | 0.033895 |
| ENSG00000175820 | CCDC168 | 2.151232 | 2.697869 | 37.17361 | 7.27E-07 | 2.37E-05 |
| ENSG00000253452 | NA | 3.703371 | 2.69415 | 97.27922 | 2.30E-11 | 1.06E-08 |
| ENSG00000273079 | GRIN2B | 3.414278 | 2.693655 | 83.86629 | 1.40E-10 | 4.90E-08 |
| ENSG00000128656 | CHN1 | 1.486908 | 2.691628 | 17.99943 | 0.000168 | 0.002364 |
| ENSG00000085872 | CHERP | 1.239277 | 2.689447 | 11.38394 | 0.001973 | 0.017701 |
| ENSG00000150672 | DLG2 | 2.491638 | 2.688066 | 51.67848 | 3.07E-08 | 2.09E-06 |
| ENSG00000165995 | CACNB2 | 2.078544 | 2.684576 | 34.94002 | 1.26E-06 | 3.63E-05 |
| ENSG00000123243 | ITIH5 | 3.265204 | 2.681188 | 86.03416 | 1.03E-10 | 3.97E-08 |
| ENSG00000188897 | LOC400499 | 2.378427 | 2.673065 | 46.36146 | 9.14E-08 | 4.69E-06 |
| ENSG00000106078 | COBL | 1.734923 | 2.670478 | 24.76098 | 1.98E-05 | 0.000373 |
| ENSG00000073605 | GSDMB | 1.890789 | 2.669874 | 22.79578 | 4.84E-05 | 0.000825 |
| ENSG00000177614 | PGBD5 | 1.563793 | 2.6677 | 21.30796 | 5.70E-05 | 0.000942 |
| ENSG00000145416 | MARCHF1 | 1.224986 | 2.667525 | 11.39912 | 0.001949 | 0.017567 |
| ENSG00000110427 | KIAA1549L | 1.287906 | 2.667148 | 15.49384 | 0.000403 | 0.00491 |
| ENSG00000119121 | TRPM6 | 1.304828 | 2.666527 | 15.40401 | 0.000417 | 0.005039 |
| ENSG00000235706 | DICER1-AS1 | 0.952403 | 2.664716 | 8.255198 | 0.007056 | 0.047101 |
| ENSG00000066044 | ELAVL1 | 1.088875 | 2.663939 | 9.915285 | 0.003471 | 0.027277 |
| ENSG00000019582 | CD74 | 1.009349 | 2.663582 | 8.610408 | 0.006043 | 0.04195 |
| ENSG00000139971 | ARMH4 | 1.040242 | 2.663473 | 8.509711 | 0.006313 | 0.043277 |
| ENSG00000179583 | CIITA | 1.472982 | 2.655965 | 18.69057 | 0.000133 | 0.001942 |
| ENSG00000146426 | TIAM2 | 1.298517 | 2.655836 | 13.78368 | 0.000755 | 0.008224 |
| ENSG00000148677 | ANKRD1 | 1.148218 | 2.653914 | 10.02442 | 0.003317 | 0.026407 |
| ENSG00000155393 | HEATR3 | 1.144287 | 2.652952 | 10.62514 | 0.002591 | 0.021931 |
| ENSG00000148053 | NTRK2 | 2.069869 | 2.649051 | 33.47736 | 1.82E-06 | 4.91E-05 |
| ENSG00000074621 | SLC24A1 | 1.097665 | 2.641075 | 9.677679 | 0.003833 | 0.029358 |
| ENSG00000196951 | SCOC-AS1 | 2.549937 | 2.638992 | 47.34452 | 7.43E-08 | 3.98E-06 |
| ENSG00000270641 | TSIX | 2.390345 | 2.638404 | 35.99471 | 1.58E-06 | 4.40E-05 |
| ENSG00000188039 | NWD1 | 2.255679 | 2.637942 | 37.1122 | 7.38E-07 | 2.39E-05 |
| ENSG00000251629 | LINC02241 | 3.925141 | 2.632851 | 92.30508 | 4.40E-11 | 1.94E-08 |
| ENSG00000004660 | CAMKK1 | 1.415474 | 2.631526 | 17.1088 | 0.000228 | 0.003034 |
| ENSG00000079482 | OPHN1 | 1.147085 | 2.631237 | 11.44727 | 0.00186 | 0.01703 |
| ENSG00000148339 | SLC25A25 | 1.343742 | 2.631089 | 14.61875 | 0.000554 | 0.006361 |
| ENSG00000237187 | NR2F1-AS1 | 3.073974 | 2.629237 | 66.50294 | 2.06E-09 | 3.25E-07 |
| ENSG00000164038 | SLC9B2 | 1.185837 | 2.629038 | 10.61072 | 0.002606 | 0.022044 |
| ENSG00000146063 | TRIM41 | 0.982254 | 2.628016 | 8.465184 | 0.006437 | 0.043941 |
| ENSG00000215483 | LINC00548 | 2.513864 | 2.626301 | 49.90628 | 4.38E-08 | 2.63E-06 |
| ENSG00000215483 | LINC00598 | 2.513864 | 2.626301 | 49.90628 | 4.38E-08 | 2.63E-06 |
| ENSG00000127124 | HIVEP3 | 2.16728 | 2.624519 | 30.96202 | 3.63E-06 | 8.56E-05 |
| ENSG00000230461 | PROX1-AS1 | 1.402302 | 2.620546 | 17.79192 | 0.000181 | 0.002515 |
| ENSG00000042832 | TG | 3.465374 | 2.620504 | 78.91395 | 2.89E-10 | 8.12E-08 |
| ENSG00000261799 | NA | 1.29312 | 2.619089 | 11.90708 | 0.001551 | 0.014661 |
| ENSG00000227115 | LINC01630 | 3.298203 | 2.617967 | 78.41378 | 3.12E-10 | 8.34E-08 |
| ENSG00000116147 | TNR | 2.528246 | 2.616205 | 41.65623 | 3.10E-07 | 1.23E-05 |
| ENSG00000239445 | NA | 3.080371 | 2.616035 | 70.11456 | 1.14E-09 | 2.25E-07 |
| ENSG00000186615 | KTN1-AS1 | 1.113327 | 2.615939 | 10.52123 | 0.002703 | 0.022616 |
| ENSG00000115423 | DNAH6 | 2.665331 | 2.615098 | 57.81385 | 9.51E-09 | 9.68E-07 |
| ENSG00000242808 | SOX2-OT | 2.598277 | 2.614631 | 53.74411 | 2.05E-08 | 1.62E-06 |
| ENSG00000224699 | NA | 2.132321 | 2.611337 | 32.45735 | 2.36E-06 | 6.07E-05 |
| ENSG00000163596 | ICA1L | 1.788382 | 2.611028 | 25.83527 | 1.45E-05 | 0.000286 |
| ENSG00000112137 | PHACTR1 | 1.575139 | 2.609185 | 21.98614 | 4.60E-05 | 0.000792 |
| ENSG00000180998 | GPR137C | 1.303252 | 2.60704 | 13.4622 | 0.000852 | 0.009142 |
| ENSG00000251192 | ZNF674 | 1.003143 | 2.603635 | 8.521937 | 0.00628 | 0.043148 |
| ENSG00000186416 | NKRF | 1.02407 | 2.602684 | 8.901065 | 0.00533 | 0.038096 |
| ENSG00000259905 | PWRN1 | 2.497334 | 2.602587 | 41.78417 | 2.63E-07 | 1.10E-05 |
| ENSG00000259905 | PWRN3 | 2.497334 | 2.602587 | 41.78417 | 2.63E-07 | 1.10E-05 |
| ENSG00000134376 | CRB1 | 2.280348 | 2.601023 | 39.81403 | 3.90E-07 | 1.44E-05 |
| ENSG00000176406 | RIMS2 | 3.241882 | 2.594664 | 78.96873 | 2.87E-10 | 8.12E-08 |
| ENSG00000171962 | DRC3 | 1.17154 | 2.592776 | 11.02714 | 0.002201 | 0.019301 |
| ENSG00000129204 | USP6 | 2.902887 | 2.591069 | 62.78676 | 3.90E-09 | 5.15E-07 |
| ENSG00000183486 | MX2 | 2.596498 | 2.58949 | 43.05671 | 2.28E-07 | 9.80E-06 |
| ENSG00000163792 | TCF23 | 1.664723 | 2.585043 | 21.62444 | 5.16E-05 | 0.000867 |
| ENSG00000105556 | MIER2 | 1.807173 | 2.583307 | 21.82183 | 4.85E-05 | 0.000825 |
| ENSG00000280739 | EIF1B-AS1 | 2.380081 | 2.579446 | 41.06669 | 2.92E-07 | 1.16E-05 |
| ENSG00000147251 | DOCK11 | 0.994337 | 2.566788 | 8.702773 | 0.005806 | 0.040719 |
| ENSG00000205356 | TECPR1 | 1.341148 | 2.559806 | 10.92659 | 0.002525 | 0.021463 |
| ENSG00000137501 | SYTL2 | 1.595662 | 2.558068 | 15.00704 | 0.000533 | 0.006217 |
| ENSG00000162241 | SLC25A45 | 1.103742 | 2.555048 | 10.87688 | 0.002339 | 0.020171 |
| ENSG00000006071 | ABCC8 | 2.82019 | 2.554894 | 55.74638 | 1.40E-08 | 1.27E-06 |
| ENSG00000284966 | NA | 1.146221 | 2.554843 | 8.802303 | 0.005828 | 0.040778 |
| ENSG00000158445 | KCNB1 | 2.386605 | 2.550105 | 42.06452 | 2.33E-07 | 9.90E-06 |
| ENSG00000255248 | MIR100HG | 3.479231 | 2.543337 | 73.84252 | 6.27E-10 | 1.41E-07 |
| ENSG00000168453 | HR | 1.129107 | 2.541185 | 10.95856 | 0.002263 | 0.019678 |
| ENSG00000177181 | RIMKLA | 1.00504 | 2.541088 | 8.537497 | 0.006237 | 0.043039 |
| ENSG00000205559 | CHKB-DT | 1.058837 | 2.540452 | 8.6744 | 0.005878 | 0.041025 |
| ENSG00000280011 | NA | 2.499334 | 2.54035 | 40.5819 | 3.37E-07 | 1.31E-05 |
| ENSG00000236539 | NA | 2.729202 | 2.539862 | 55.22895 | 1.54E-08 | 1.37E-06 |
| ENSG00000177576 | C18orf32 | 1.075559 | 2.539795 | 8.530385 | 0.006257 | 0.043063 |
| ENSG00000130561 | SAG | 2.559896 | 2.539677 | 39.00798 | 6.78E-07 | 2.25E-05 |
| ENSG00000158683 | PKD1L1 | 2.721113 | 2.538517 | 50.06755 | 4.24E-08 | 2.56E-06 |
| ENSG00000107736 | CDH23 | 2.093275 | 2.538155 | 17.81249 | 0.000584 | 0.006643 |
| ENSG00000206077 | ZDHHC11B | 2.148767 | 2.537353 | 34.95225 | 1.25E-06 | 3.63E-05 |
| ENSG00000234494 | SP2-AS1 | 1.562895 | 2.531194 | 15.56221 | 0.000401 | 0.004893 |
| ENSG00000110171 | TRIM3 | 1.123217 | 2.529396 | 8.939881 | 0.005242 | 0.037664 |
| ENSG00000168280 | KIF5C | 1.016346 | 2.528751 | 8.159061 | 0.00736 | 0.04878 |
| ENSG00000101680 | LAMA1 | 3.214179 | 2.527656 | 69.23899 | 1.31E-09 | 2.43E-07 |
| ENSG00000279080 | NA | 2.634891 | 2.525196 | 43.46502 | 1.71E-07 | 7.94E-06 |
| ENSG00000116117 | PARD3B | 1.936295 | 2.522207 | 31.54551 | 2.99E-06 | 7.34E-05 |
| ENSG00000280007 | NA | 1.484264 | 2.518815 | 18.71389 | 0.000132 | 0.001932 |
| ENSG00000167037 | SGSM1 | 1.146491 | 2.518776 | 10.85754 | 0.002358 | 0.020285 |
| ENSG00000083067 | TRPM3 | 3.238423 | 2.514657 | 65.59698 | 2.40E-09 | 3.52E-07 |
| ENSG00000249669 | NA | 2.973466 | 2.514304 | 57.58659 | 9.92E-09 | 9.84E-07 |
| ENSG00000206579 | XKR4 | 3.216232 | 2.513417 | 46.56545 | 2.18E-07 | 9.60E-06 |
| ENSG00000143631 | FLG | 2.212804 | 2.510074 | 41.42861 | 2.69E-07 | 1.10E-05 |
| ENSG00000185920 | PTCH1 | 2.170072 | 2.50873 | 34.53809 | 1.39E-06 | 3.94E-05 |
| ENSG00000157601 | MX1 | 1.190105 | 2.503832 | 10.34753 | 0.002906 | 0.023945 |
| ENSG00000106415 | GLCCI1 | 1.281867 | 2.503454 | 12.50042 | 0.00123 | 0.012207 |
| ENSG00000119139 | TJP2 | 1.40337 | 2.502826 | 12.68792 | 0.001193 | 0.011935 |
| ENSG00000145362 | ANK2 | 2.925094 | 2.500752 | 47.54948 | 1.12E-07 | 5.57E-06 |
| ENSG00000204131 | NHSL2 | 2.890036 | 2.499807 | 51.71679 | 3.05E-08 | 2.09E-06 |
| ENSG00000204131 | FLJ44635 | 2.890036 | 2.499807 | 51.71679 | 3.05E-08 | 2.09E-06 |
| ENSG00000189056 | RELN | 2.924919 | 2.499648 | 52.63124 | 2.55E-08 | 1.81E-06 |
| ENSG00000062282 | DGAT2 | 2.112042 | 2.495922 | 31.12323 | 3.35E-06 | 8.00E-05 |
| ENSG00000205930 | NA | 1.727496 | 2.49451 | 23.62718 | 2.78E-05 | 0.000502 |
| ENSG00000205420 | KRT6A | 1.816313 | 2.49365 | 25.40963 | 1.64E-05 | 0.000317 |
| ENSG00000168918 | INPP5D | 1.370019 | 2.491135 | 14.97295 | 0.000487 | 0.005744 |
| ENSG00000021826 | CPS1 | 1.147082 | 2.489082 | 10.12059 | 0.003188 | 0.025641 |
| ENSG00000163359 | COL6A3 | 3.317132 | 2.488984 | 64.83512 | 2.73E-09 | 3.84E-07 |
| ENSG00000257261 | NA | 1.481972 | 2.488744 | 16.17739 | 0.000316 | 0.004024 |
| ENSG00000084710 | EFR3B | 1.097291 | 2.488624 | 9.021282 | 0.005062 | 0.03674 |
| ENSG00000247081 | LOC105369147 | 2.804786 | 2.487479 | 48.65737 | 5.75E-08 | 3.29E-06 |
| ENSG00000163395 | IGFN1 | 2.762375 | 2.487036 | 37.15649 | 2.76E-06 | 6.82E-05 |
| ENSG00000236790 | LINC00299 | 2.820739 | 2.485828 | 62.0886 | 4.40E-09 | 5.63E-07 |
| ENSG00000236432 | MFF-DT | 2.377803 | 2.48336 | 36.67022 | 8.21E-07 | 2.62E-05 |
| ENSG00000149294 | NCAM1 | 2.2067 | 2.482549 | 33.58151 | 1.77E-06 | 4.83E-05 |
| ENSG00000116852 | KIF21B | 2.007022 | 2.48206 | 34.09936 | 1.55E-06 | 4.35E-05 |
| ENSG00000227252 | NA | 1.496717 | 2.478163 | 19.21738 | 0.000112 | 0.001677 |
| ENSG00000198756 | COLGALT2 | 1.389148 | 2.476382 | 12.93082 | 0.001043 | 0.010728 |
| ENSG00000090674 | MCOLN1 | 1.678894 | 2.476039 | 17.39789 | 0.000207 | 0.002804 |
| ENSG00000242512 | LINC01206 | 3.315865 | 2.475393 | 69.53977 | 1.25E-09 | 2.43E-07 |
| ENSG00000198590 | C3orf35 | 1.120237 | 2.475197 | 9.597378 | 0.003965 | 0.030184 |
| ENSG00000257176 | LOC100506606 | 1.166061 | 2.473262 | 8.900289 | 0.005332 | 0.038096 |
| ENSG00000158486 | DNAH3 | 3.169265 | 2.472793 | 66.09496 | 2.21E-09 | 3.39E-07 |
| ENSG00000249816 | LINC00964 | 2.676482 | 2.47151 | 46.81942 | 8.30E-08 | 4.34E-06 |
| ENSG00000154262 | ABCA6 | 2.490778 | 2.470279 | 42.30281 | 2.21E-07 | 9.71E-06 |
| ENSG00000245498 | LOC100507283 | 1.634047 | 2.466515 | 20.62322 | 7.09E-05 | 0.001132 |
| ENSG00000133083 | DCLK1 | 1.285676 | 2.464509 | 12.75518 | 0.001115 | 0.011303 |
| ENSG00000163491 | NEK10 | 1.622534 | 2.464509 | 18.97026 | 0.000123 | 0.001819 |
| ENSG00000099954 | CECR2 | 1.077774 | 2.463482 | 9.726347 | 0.003756 | 0.0289 |
| ENSG00000143786 | CNIH3 | 1.247279 | 2.463307 | 12.35315 | 0.001303 | 0.012776 |
| ENSG00000148219 | ASTN2 | 1.33592 | 2.46271 | 14.70558 | 0.000537 | 0.006242 |
| ENSG00000099992 | TBC1D10A | 1.140009 | 2.461455 | 10.91446 | 0.002304 | 0.019986 |
| ENSG00000139351 | SYCP3 | 1.118838 | 2.461092 | 9.558949 | 0.004029 | 0.030615 |
| ENSG00000157423 | HYDIN | 3.165517 | 2.459657 | 61.59099 | 4.81E-09 | 6.01E-07 |
| ENSG00000130508 | PXDN | 3.072152 | 2.45917 | 65.12522 | 2.60E-09 | 3.69E-07 |
| ENSG00000286786 | NA | 2.236619 | 2.458349 | 26.01506 | 2.22E-05 | 0.000413 |
| ENSG00000050438 | SLC4A8 | 2.57374 | 2.457277 | 51.55008 | 3.15E-08 | 2.12E-06 |
| ENSG00000223960 | CHROMR | 2.295198 | 2.455913 | 40.19831 | 3.57E-07 | 1.35E-05 |
| ENSG00000105877 | DNAH11 | 2.167284 | 2.455905 | 32.16467 | 2.55E-06 | 6.44E-05 |
| ENSG00000229956 | NA | 2.231447 | 2.455852 | 32.43398 | 2.37E-06 | 6.07E-05 |
| ENSG00000115353 | TACR1 | 1.94303 | 2.452262 | 25.48091 | 1.60E-05 | 0.000311 |
| ENSG00000138834 | MAPK8IP3 | 1.243481 | 2.449964 | 12.40723 | 0.001276 | 0.012564 |
| ENSG00000176809 | LRRC37A3 | 1.38312 | 2.449776 | 15.74848 | 0.000368 | 0.004583 |
| ENSG00000185483 | ROR1 | 1.175386 | 2.449109 | 11.3265 | 0.001952 | 0.017572 |
| ENSG00000053524 | MCF2L2 | 2.786183 | 2.444535 | 55.90771 | 1.36E-08 | 1.24E-06 |
| ENSG00000157103 | SLC6A1 | 2.463355 | 2.443513 | 41.34852 | 2.74E-07 | 1.11E-05 |
| ENSG00000196876 | SCN8A | 2.45634 | 2.440487 | 36.58118 | 8.39E-07 | 2.67E-05 |
| ENSG00000135218 | CD36 | 1.965356 | 2.438231 | 25.03902 | 1.83E-05 | 0.00035 |
| ENSG00000228412 | LOC100506885 | 1.253309 | 2.437339 | 12.78401 | 0.001103 | 0.011209 |
| ENSG00000228412 | LNC-LBCS | 1.253309 | 2.437339 | 12.78401 | 0.001103 | 0.011209 |
| ENSG00000097096 | SYDE2 | 1.637885 | 2.436846 | 17.86627 | 0.000176 | 0.002461 |
| ENSG00000253846 | PCDHGA10 | 1.396701 | 2.435563 | 12.69899 | 0.00114 | 0.0115 |
| ENSG00000092421 | SEMA6A | 1.113663 | 2.43553 | 10.1207 | 0.003187 | 0.025641 |
| ENSG00000269934 | NA | 1.275267 | 2.434387 | 13.09882 | 0.000978 | 0.01018 |
| ENSG00000196839 | ADA | 1.291797 | 2.434069 | 9.33576 | 0.004714 | 0.034544 |
| ENSG00000198208 | RPS6KL1 | 1.359866 | 2.433183 | 10.23989 | 0.003093 | 0.025076 |
| ENSG00000228793 | LOC100507336 | 2.876033 | 2.43298 | 51.33223 | 3.29E-08 | 2.16E-06 |
| ENSG00000188603 | CLN3 | 1.138071 | 2.432829 | 9.367456 | 0.004369 | 0.032527 |
| ENSG00000228065 | NA | 3.08254 | 2.431367 | 62.79396 | 3.89E-09 | 5.15E-07 |
| ENSG00000153246 | PLA2R1 | 2.713843 | 2.430539 | 53.25707 | 2.25E-08 | 1.72E-06 |
| ENSG00000237975 | FLG-AS1 | 2.198215 | 2.426834 | 35.01151 | 1.23E-06 | 3.59E-05 |
| ENSG00000237807 | LOC100507516 | 1.777811 | 2.426747 | 20.27197 | 8.12E-05 | 0.001273 |
| ENSG00000188001 | TPRG1 | 1.845671 | 2.426066 | 23.29621 | 3.08E-05 | 0.000549 |
| ENSG00000162415 | ZSWIM5 | 1.401243 | 2.422843 | 15.17923 | 0.000452 | 0.005397 |
| ENSG00000182771 | GRID1 | 1.428959 | 2.421873 | 16.86773 | 0.000248 | 0.003256 |
| ENSG00000160111 | CPAMD8 | 1.526645 | 2.421812 | 16.52892 | 0.000279 | 0.003607 |
| ENSG00000117586 | TNFSF4 | 1.063722 | 2.421692 | 9.256906 | 0.004578 | 0.033804 |
| ENSG00000170485 | NPAS2 | 1.029376 | 2.421242 | 8.149261 | 0.007392 | 0.048926 |
| ENSG00000223522 | LOC100505716 | 1.202116 | 2.420086 | 9.167645 | 0.004785 | 0.034995 |
| ENSG00000101638 | ST8SIA5 | 2.990612 | 2.416996 | 56.27868 | 1.27E-08 | 1.16E-06 |
| ENSG00000278920 | NA | 2.353447 | 2.415818 | 34.94251 | 1.43E-06 | 4.03E-05 |
| ENSG00000197653 | DNAH10 | 1.663149 | 2.411468 | 23.08762 | 3.28E-05 | 0.000583 |
| ENSG00000103723 | AP3B2 | 1.779485 | 2.410402 | 26.46822 | 1.21E-05 | 0.000243 |
| ENSG00000105928 | GSDME | 1.26188 | 2.408015 | 12.89157 | 0.001058 | 0.010842 |
| ENSG00000236778 | INTS6-AS1 | 1.380016 | 2.407491 | 11.81477 | 0.001608 | 0.015118 |
| ENSG00000170846 | LOC93622 | 1.317383 | 2.407362 | 13.06594 | 0.00099 | 0.010271 |
| ENSG00000233382 | NKAPP1 | 1.244474 | 2.407011 | 12.3269 | 0.001316 | 0.012894 |
| ENSG00000140199 | SLC12A6 | 1.069625 | 2.40635 | 8.666718 | 0.005897 | 0.041112 |
| ENSG00000224924 | LINC00320 | 3.426628 | 2.405025 | 61.29929 | 6.02E-09 | 6.99E-07 |
| ENSG00000170927 | PKHD1 | 2.691537 | 2.400931 | 50.84173 | 3.63E-08 | 2.32E-06 |
| ENSG00000179869 | ABCA13 | 2.067095 | 2.399056 | 31.41886 | 3.09E-06 | 7.51E-05 |
| ENSG00000131711 | MAP1B | 2.062663 | 2.398964 | 31.9191 | 2.71E-06 | 6.76E-05 |
| ENSG00000198010 | DLGAP2 | 1.938705 | 2.398379 | 32.68201 | 2.23E-06 | 5.82E-05 |
| ENSG00000280048 | NA | 1.830985 | 2.397869 | 21.16458 | 6.47E-05 | 0.001051 |
| ENSG00000140090 | SLC24A4 | 2.034581 | 2.397572 | 30.5704 | 3.88E-06 | 9.06E-05 |
| ENSG00000168675 | LDLRAD4 | 1.961823 | 2.395469 | 22.66494 | 3.97E-05 | 0.000693 |
| ENSG00000131899 | LLGL1 | 1.25457 | 2.393368 | 11.22165 | 0.002036 | 0.018071 |
| ENSG00000249898 | NA | 1.673183 | 2.393231 | 14.78645 | 0.000628 | 0.007072 |
| ENSG00000078295 | ADCY2 | 2.793346 | 2.388978 | 54.4776 | 1.78E-08 | 1.46E-06 |
| ENSG00000188107 | EYS | 2.070236 | 2.384591 | 27.82193 | 8.51E-06 | 0.000177 |
| ENSG00000233974 | NA | 1.945081 | 2.383314 | 29.86238 | 4.69E-06 | 0.000107 |
| ENSG00000135407 | AVIL | 1.868262 | 2.380548 | 24.0758 | 2.43E-05 | 0.00045 |
| ENSG00000114656 | KIAA1257 | 1.470789 | 2.380194 | 15.07207 | 0.00047 | 0.005581 |
| ENSG00000106948 | AKNA | 1.405869 | 2.377695 | 13.18005 | 0.000948 | 0.009951 |
| ENSG00000163803 | PLB1 | 2.315906 | 2.370934 | 41.20826 | 2.83E-07 | 1.14E-05 |
| ENSG00000169554 | ZEB2 | 2.544813 | 2.369964 | 43.58784 | 1.66E-07 | 7.81E-06 |
| ENSG00000175147 | TMEM51-AS1 | 1.673393 | 2.36728 | 21.58621 | 5.22E-05 | 0.000876 |
| ENSG00000181333 | HEPHL1 | 1.142662 | 2.364006 | 10.08099 | 0.00324 | 0.026029 |
| ENSG00000136011 | STAB2 | 2.744342 | 2.358541 | 45.87973 | 1.01E-07 | 5.13E-06 |
| ENSG00000151067 | CACNA1C | 3.094081 | 2.3584 | 57.92063 | 9.33E-09 | 9.66E-07 |
| ENSG00000143851 | PTPN7 | 2.745505 | 2.356064 | 37.63323 | 7.12E-07 | 2.33E-05 |
| ENSG00000168386 | FILIP1L | 2.213054 | 2.355509 | 29.34641 | 5.39E-06 | 0.00012 |
| ENSG00000145147 | SLIT2 | 2.155876 | 2.355324 | 36.03642 | 9.58E-07 | 2.95E-05 |
| ENSG00000180354 | MTURN | 1.543956 | 2.350632 | 17.8482 | 0.000177 | 0.002474 |
| ENSG00000163376 | KBTBD8 | 1.10475 | 2.34815 | 9.790241 | 0.003657 | 0.028404 |
| ENSG00000075340 | ADD2 | 3.848357 | 2.345139 | 66.55367 | 2.41E-09 | 3.52E-07 |
| ENSG00000244128 | NA | 2.887028 | 2.343457 | 59.07392 | 7.55E-09 | 8.43E-07 |
| ENSG00000165029 | ABCA1 | 2.788297 | 2.342337 | 46.20208 | 9.45E-08 | 4.81E-06 |
| ENSG00000165899 | OTOGL | 2.275087 | 2.339846 | 30.66732 | 4.31E-06 | 9.98E-05 |
| ENSG00000022976 | ZNF839 | 1.454492 | 2.335766 | 17.25096 | 0.000217 | 0.002925 |
| ENSG00000003987 | MTMR7 | 1.474136 | 2.335308 | 17.23923 | 0.000218 | 0.00293 |
| ENSG00000112425 | EPM2A | 1.322412 | 2.334781 | 10.61114 | 0.002619 | 0.022055 |
| ENSG00000131378 | RFTN1 | 1.551765 | 2.334297 | 15.39635 | 0.000418 | 0.005048 |
| ENSG00000160767 | FAM189B | 1.230584 | 2.331907 | 8.959295 | 0.005199 | 0.037492 |
| ENSG00000286679 | LOC107985953 | 3.628917 | 2.331879 | 75.57186 | 4.80E-10 | 1.18E-07 |
| ENSG00000164465 | DCBLD1 | 1.117086 | 2.33163 | 8.383024 | 0.006672 | 0.045117 |
| ENSG00000175137 | SH3BP5L | 1.362056 | 2.331412 | 10.39131 | 0.002851 | 0.023583 |
| ENSG00000226471 | NA | 1.199422 | 2.331078 | 9.110768 | 0.004872 | 0.03554 |
| ENSG00000234948 | LINC01524 | 2.396254 | 2.328053 | 35.37575 | 1.13E-06 | 3.39E-05 |
| ENSG00000229425 | LOC101927745 | 2.908955 | 2.32741 | 47.9675 | 6.53E-08 | 3.63E-06 |
| ENSG00000229425 | LOC105369302 | 2.908955 | 2.32741 | 47.9675 | 6.53E-08 | 3.63E-06 |
| ENSG00000227110 | LMCD1-AS1 | 2.298214 | 2.325909 | 38.40389 | 5.42E-07 | 1.87E-05 |
| ENSG00000227110 | LOC101927394 | 2.298214 | 2.325909 | 38.40389 | 5.42E-07 | 1.87E-05 |
| ENSG00000237489 | C10orf143 | 1.769619 | 2.322451 | 22.27475 | 4.21E-05 | 0.000729 |
| ENSG00000229474 | PATL2 | 1.302545 | 2.319798 | 11.81423 | 0.001609 | 0.015118 |
| ENSG00000144810 | COL8A1 | 3.157928 | 2.314088 | 40.97131 | 1.28E-06 | 3.67E-05 |
| ENSG00000088756 | ARHGAP28 | 2.012149 | 2.308424 | 27.80902 | 8.25E-06 | 0.000172 |
| ENSG00000163406 | SLC15A2 | 1.788824 | 2.308188 | 19.55196 | 0.000105 | 0.001589 |
| ENSG00000124920 | MYRF | 1.778015 | 2.307398 | 19.62739 | 9.79E-05 | 0.001499 |
| ENSG00000135709 | KIAA0513 | 1.629604 | 2.304862 | 15.49775 | 0.000403 | 0.004908 |
| ENSG00000159173 | TNNI1 | 1.20886 | 2.303997 | 10.82522 | 0.002389 | 0.02052 |
| ENSG00000109265 | CRACD | 1.289212 | 2.303956 | 11.27407 | 0.001994 | 0.017805 |
| ENSG00000215182 | MUC5AC | 1.28539 | 2.303787 | 11.33944 | 0.001942 | 0.017536 |
| ENSG00000227354 | RBM26-AS1 | 1.336516 | 2.303263 | 10.58834 | 0.00263 | 0.022117 |
| ENSG00000225937 | PCA3 | 3.204129 | 2.299594 | 54.75165 | 1.69E-08 | 1.44E-06 |
| ENSG00000205038 | PKHD1L1 | 2.962085 | 2.297953 | 52.86217 | 2.44E-08 | 1.80E-06 |
| ENSG00000081248 | CACNA1S | 2.695952 | 2.297877 | 42.13622 | 2.30E-07 | 9.82E-06 |
| ENSG00000143603 | KCNN3 | 2.262216 | 2.297324 | 33.64647 | 1.74E-06 | 4.76E-05 |
| ENSG00000204929 | LOC101927533 | 2.295661 | 2.294949 | 33.33993 | 1.88E-06 | 5.05E-05 |
| ENSG00000225746 | NA | 2.387566 | 2.294437 | 39.07315 | 4.63E-07 | 1.65E-05 |
| ENSG00000232044 | NA | 2.323554 | 2.294161 | 35.61474 | 1.06E-06 | 3.24E-05 |
| ENSG00000163873 | GRIK3 | 2.2616 | 2.294117 | 35.30287 | 1.15E-06 | 3.42E-05 |
| ENSG00000246922 | UBAP1L | 1.947832 | 2.29334 | 28.56867 | 6.68E-06 | 0.000144 |
| ENSG00000130477 | UNC13A | 1.963358 | 2.292946 | 28.01894 | 7.78E-06 | 0.000163 |
| ENSG00000079841 | RIMS1 | 1.983552 | 2.291312 | 23.88854 | 2.57E-05 | 0.000471 |
| ENSG00000188677 | PARVB | 1.314357 | 2.288465 | 11.20048 | 0.002053 | 0.018207 |
| ENSG00000169876 | MUC17 | 3.789283 | 2.285028 | 65.72902 | 2.38E-09 | 3.52E-07 |
| ENSG00000179915 | NRXN1 | 4.038984 | 2.284939 | 71.80884 | 8.65E-10 | 1.81E-07 |
| ENSG00000230426 | LINC01036 | 3.006208 | 2.280721 | 43.19904 | 2.64E-07 | 1.10E-05 |
| ENSG00000197140 | ADAM32 | 2.409102 | 2.280581 | 36.62071 | 8.31E-07 | 2.65E-05 |
| ENSG00000188984 | AADACL3 | 2.717436 | 2.279905 | 47.3801 | 7.37E-08 | 3.98E-06 |
| ENSG00000118257 | NRP2 | 2.434122 | 2.2785 | 32.51552 | 2.32E-06 | 6.02E-05 |
| ENSG00000255346 | NOX5 | 2.181048 | 2.277281 | 33.49518 | 1.81E-06 | 4.90E-05 |
| ENSG00000214595 | EML6 | 1.753463 | 2.276304 | 23.66884 | 2.75E-05 | 0.000497 |
| ENSG00000135063 | FAM189A2 | 1.737352 | 2.276145 | 19.92239 | 8.89E-05 | 0.001378 |
| ENSG00000254561 | NA | 1.704675 | 2.275268 | 17.4709 | 0.000202 | 0.002746 |
| ENSG00000285106 | NA | 1.293067 | 2.274726 | 13.39105 | 0.000875 | 0.009348 |
| ENSG00000137878 | GCOM1 | 1.530901 | 2.273417 | 14.67584 | 0.000543 | 0.006286 |
| ENSG00000162814 | SPATA17 | 1.38188 | 2.27322 | 11.95752 | 0.00152 | 0.014491 |
| ENSG00000163686 | ABHD6 | 1.067912 | 2.272132 | 8.409316 | 0.006595 | 0.044707 |
| ENSG00000249715 | FER1L5 | 3.099859 | 2.266606 | 42.53699 | 3.16E-07 | 1.25E-05 |
| ENSG00000152689 | RASGRP3 | 2.765749 | 2.266319 | 50.76855 | 3.69E-08 | 2.32E-06 |
| ENSG00000172403 | SYNPO2 | 3.00844 | 2.266227 | 51.72028 | 3.05E-08 | 2.09E-06 |
| ENSG00000106477 | CEP41 | 3.642372 | 2.266154 | 66.38949 | 2.10E-09 | 3.26E-07 |
| ENSG00000242593 | NA | 2.548933 | 2.264877 | 48.03356 | 6.44E-08 | 3.61E-06 |
| ENSG00000233593 | LINC02609 | 2.784219 | 2.263788 | 39.94272 | 3.96E-07 | 1.45E-05 |
| ENSG00000249464 | LINC01091 | 2.24287 | 2.262954 | 30.0132 | 4.50E-06 | 0.000103 |
| ENSG00000170271 | FAXDC2 | 2.326407 | 2.262387 | 36.26294 | 9.07E-07 | 2.83E-05 |
| ENSG00000224897 | POT1-AS1 | 2.173975 | 2.261947 | 31.98217 | 2.67E-06 | 6.69E-05 |
| ENSG00000224897 | LOC101928283 | 2.173975 | 2.261947 | 31.98217 | 2.67E-06 | 6.69E-05 |
| ENSG00000106278 | PTPRZ1 | 1.704669 | 2.260168 | 19.80674 | 9.23E-05 | 0.001421 |
| ENSG00000162722 | TRIM58 | 1.278813 | 2.257904 | 9.969257 | 0.003395 | 0.026865 |
| ENSG00000286215 | NA | 2.913152 | 2.250934 | 46.68442 | 8.54E-08 | 4.44E-06 |
| ENSG00000079102 | RUNX1T1 | 3.598959 | 2.250588 | 68.15824 | 1.56E-09 | 2.64E-07 |
| ENSG00000234899 | SOX9-AS1 | 1.97874 | 2.247035 | 30.00808 | 4.51E-06 | 0.000103 |
| ENSG00000234899 | LOC102723517 | 1.97874 | 2.247035 | 30.00808 | 4.51E-06 | 0.000103 |
| ENSG00000272631 | NA | 1.346372 | 2.241196 | 12.16137 | 0.001404 | 0.013536 |
| ENSG00000042980 | ADAM28 | 3.670969 | 2.234965 | 65.1339 | 2.60E-09 | 3.69E-07 |
| ENSG00000091536 | MYO15A | 2.563468 | 2.23413 | 36.56519 | 8.48E-07 | 2.69E-05 |
| ENSG00000258628 | NA | 2.784974 | 2.23411 | 44.19377 | 1.46E-07 | 6.92E-06 |
| ENSG00000133958 | UNC79 | 2.830716 | 2.233944 | 55.04465 | 1.60E-08 | 1.39E-06 |
| ENSG00000130226 | DPP6 | 3.12704 | 2.23349 | 53.40694 | 2.19E-08 | 1.69E-06 |
| ENSG00000236008 | LINC01814 | 2.691417 | 2.232596 | 48.752 | 5.55E-08 | 3.19E-06 |
| ENSG00000161381 | PLXDC1 | 2.514845 | 2.232579 | 43.47939 | 1.70E-07 | 7.93E-06 |
| ENSG00000102359 | SRPX2 | 1.807971 | 2.230455 | 19.40688 | 0.000105 | 0.00159 |
| ENSG00000176771 | NCKAP5 | 1.841318 | 2.227994 | 22.45493 | 3.98E-05 | 0.000694 |
| ENSG00000175155 | YPEL2 | 1.199437 | 2.226778 | 9.205543 | 0.00468 | 0.034351 |
| ENSG00000198963 | RORB | 1.428626 | 2.225581 | 12.07774 | 0.00145 | 0.013916 |
| ENSG00000154217 | PITPNC1 | 1.205788 | 2.225084 | 9.941141 | 0.003434 | 0.027111 |
| ENSG00000134539 | KLRD1 | 2.95021 | 2.218159 | 48.95891 | 5.32E-08 | 3.07E-06 |
| ENSG00000286891 | NA | 2.897721 | 2.217291 | 53.44794 | 2.17E-08 | 1.69E-06 |
| ENSG00000182050 | MGAT4C | 3.090408 | 2.215964 | 50.62237 | 3.79E-08 | 2.36E-06 |
| ENSG00000164692 | COL1A2 | 2.205672 | 2.215844 | 30.14374 | 4.35E-06 | 0.0001 |
| ENSG00000176438 | SYNE3 | 2.264804 | 2.214391 | 25.77114 | 1.88E-05 | 0.000356 |
| ENSG00000134955 | SLC37A2 | 1.920615 | 2.213662 | 24.82066 | 1.95E-05 | 0.000368 |
| ENSG00000127329 | PTPRB | 1.728392 | 2.212992 | 17.58075 | 0.000199 | 0.00272 |
| ENSG00000282961 | PRNCR1 | 1.997743 | 2.212981 | 26.73817 | 1.12E-05 | 0.000227 |
| ENSG00000185261 | KIAA0825 | 1.706698 | 2.211366 | 18.39473 | 0.000147 | 0.00212 |
| ENSG00000196090 | PTPRT | 3.712066 | 2.204835 | 43.32965 | 1.54E-06 | 4.33E-05 |
| ENSG00000261272 | MUC22 | 2.854831 | 2.203111 | 42.47865 | 2.28E-07 | 9.80E-06 |
| ENSG00000183775 | KCTD16 | 3.282529 | 2.202622 | 53.84697 | 2.01E-08 | 1.61E-06 |
| ENSG00000113327 | GABRG2 | 2.807135 | 2.201255 | 50.18508 | 4.14E-08 | 2.51E-06 |
| ENSG00000144406 | UNC80 | 2.560582 | 2.200031 | 41.12167 | 2.89E-07 | 1.15E-05 |
| ENSG00000234680 | NA | 2.66244 | 2.199796 | 41.40675 | 2.71E-07 | 1.10E-05 |
| ENSG00000049192 | ADAMTS6 | 2.46197 | 2.198713 | 21.23937 | 0.000195 | 0.002675 |
| ENSG00000182177 | ASB18 | 1.977782 | 2.196422 | 19.09492 | 0.000135 | 0.001961 |
| ENSG00000099338 | CATSPERG | 1.744523 | 2.195065 | 20.51293 | 7.34E-05 | 0.001164 |
| ENSG00000233639 | PANTR1 | 3.680084 | 2.188601 | 54.24243 | 3.15E-08 | 2.12E-06 |
| ENSG00000178722 | C5orf64 | 3.581338 | 2.18649 | 59.66174 | 6.79E-09 | 7.73E-07 |
| ENSG00000128815 | WDFY4 | 3.084635 | 2.185714 | 57.11596 | 1.08E-08 | 1.04E-06 |
| ENSG00000115970 | THADA | 2.591 | 2.183886 | 40.39018 | 3.41E-07 | 1.31E-05 |
| ENSG00000132915 | PDE6A | 2.264507 | 2.181364 | 35.47057 | 1.10E-06 | 3.33E-05 |
| ENSG00000140470 | ADAMTS17 | 2.064324 | 2.179851 | 26.43575 | 1.22E-05 | 0.000244 |
| ENSG00000235831 | BHLHE40-AS1 | 1.673172 | 2.177257 | 18.32819 | 0.000151 | 0.002163 |
| ENSG00000160766 | GBAP1 | 1.267223 | 2.176338 | 8.189453 | 0.007263 | 0.048235 |
| ENSG00000261404 | LOC101928035 | 4.134251 | 2.172116 | 58.22479 | 1.78E-08 | 1.46E-06 |
| ENSG00000146192 | FGD2 | 2.931867 | 2.169323 | 39.4139 | 6.18E-07 | 2.09E-05 |
| ENSG00000104237 | RP1 | 2.787959 | 2.168707 | 47.40319 | 7.34E-08 | 3.98E-06 |
| ENSG00000104237 | LOC107984125 | 2.787959 | 2.168707 | 47.40319 | 7.34E-08 | 3.98E-06 |
| ENSG00000035664 | DAPK2 | 3.121529 | 2.167213 | 50.42274 | 3.95E-08 | 2.42E-06 |
| ENSG00000287277 | NA | 2.780564 | 2.166949 | 41.47593 | 2.66E-07 | 1.10E-05 |
| ENSG00000144908 | ALDH1L1 | 2.872382 | 2.166458 | 50.54222 | 3.86E-08 | 2.38E-06 |
| ENSG00000091592 | NLRP1 | 1.838611 | 2.164294 | 20.00719 | 8.65E-05 | 0.001347 |
| ENSG00000196482 | ESRRG | 2.44207 | 2.164064 | 33.11934 | 1.99E-06 | 5.29E-05 |
| ENSG00000114631 | PODXL2 | 1.226945 | 2.158048 | 9.653675 | 0.003872 | 0.029596 |
| ENSG00000228956 | NA | 3.121555 | 2.151179 | 54.85402 | 1.66E-08 | 1.42E-06 |
| ENSG00000204677 | FAM153CP | 2.639159 | 2.150304 | 37.24638 | 7.14E-07 | 2.33E-05 |
| ENSG00000156218 | ADAMTSL3 | 2.722752 | 2.149745 | 28.38816 | 1.37E-05 | 0.000272 |
| ENSG00000110799 | VWF | 2.232348 | 2.14881 | 32.15067 | 2.56E-06 | 6.45E-05 |
| ENSG00000111452 | ADGRD1 | 2.270116 | 2.148336 | 35.36629 | 1.13E-06 | 3.39E-05 |
| ENSG00000146021 | KLHL3 | 2.235025 | 2.147154 | 28.89591 | 6.10E-06 | 0.000133 |
| ENSG00000261200 | NA | 1.530374 | 2.14654 | 13.35053 | 0.000913 | 0.00964 |
| ENSG00000042062 | RIPOR3 | 1.629618 | 2.145625 | 17.61969 | 0.000192 | 0.002639 |
| ENSG00000174844 | DNAH12 | 3.88221 | 2.137105 | 64.32238 | 2.99E-09 | 4.14E-07 |
| ENSG00000147488 | ST18 | 2.700185 | 2.133603 | 41.43166 | 2.69E-07 | 1.10E-05 |
| ENSG00000271913 | LOC105378083 | 2.553353 | 2.132211 | 32.57755 | 2.29E-06 | 5.95E-05 |
| ENSG00000228590 | MIR4432HG | 2.606604 | 2.131887 | 39.7328 | 3.97E-07 | 1.45E-05 |
| ENSG00000158258 | CLSTN2 | 2.37083 | 2.131402 | 33.88629 | 1.64E-06 | 4.53E-05 |
| ENSG00000129682 | FGF13 | 2.177349 | 2.130287 | 27.647 | 8.63E-06 | 0.000179 |
| ENSG00000033122 | LRRC7 | 2.105406 | 2.128441 | 24.01055 | 2.48E-05 | 0.000456 |
| ENSG00000120664 | SPART-AS1 | 1.403289 | 2.126744 | 13.57025 | 0.000818 | 0.008819 |
| ENSG00000146267 | FAXC | 1.806521 | 2.126141 | 15.62603 | 0.000396 | 0.004843 |
| ENSG00000147234 | FRMPD3 | 1.491333 | 2.125974 | 14.68174 | 0.000541 | 0.006283 |
| ENSG00000261738 | MIR3976HG | 4.10446 | 2.120078 | 70.37902 | 1.09E-09 | 2.20E-07 |
| ENSG00000112038 | OPRM1 | 4.263166 | 2.118253 | 76.8796 | 3.93E-10 | 1.03E-07 |
| ENSG00000164398 | ACSL6 | 3.097057 | 2.116731 | 53.873 | 2.00E-08 | 1.61E-06 |
| ENSG00000112992 | NNT | 3.066259 | 2.115749 | 52.93001 | 2.40E-08 | 1.79E-06 |
| ENSG00000165186 | PTCHD1 | 2.396441 | 2.11491 | 33.86091 | 1.65E-06 | 4.54E-05 |
| ENSG00000151687 | ANKAR | 1.71534 | 2.109815 | 18.89295 | 0.000125 | 0.00184 |
| ENSG00000198929 | NOS1AP | 1.628081 | 2.108695 | 14.1312 | 0.000663 | 0.007395 |
| ENSG00000234663 | NA | 4.347745 | 2.10288 | 68.37743 | 1.51E-09 | 2.63E-07 |
| ENSG00000235770 | LINC00607 | 3.625345 | 2.101528 | 51.87928 | 4.87E-08 | 2.86E-06 |
| ENSG00000121446 | RGSL1 | 2.907752 | 2.101047 | 43.35716 | 1.75E-07 | 8.08E-06 |
| ENSG00000251372 | LINC00499 | 3.343702 | 2.099755 | 56.50892 | 1.21E-08 | 1.13E-06 |
| ENSG00000227906 | SNAP25-AS1 | 2.957185 | 2.099004 | 39.21384 | 5.63E-07 | 1.92E-05 |
| ENSG00000137491 | SLCO2B1 | 2.979726 | 2.098766 | 52.81691 | 2.46E-08 | 1.80E-06 |
| ENSG00000176584 | DMBT1P1 | 3.32599 | 2.09773 | 51.49961 | 3.18E-08 | 2.12E-06 |
| ENSG00000041982 | TNC | 2.921644 | 2.096896 | 45.68445 | 1.06E-07 | 5.30E-06 |
| ENSG00000165300 | SLITRK5 | 1.878469 | 2.09366 | 20.62974 | 7.07E-05 | 0.001131 |
| ENSG00000188227 | ZNF793 | 1.554356 | 2.092456 | 16.02183 | 0.000334 | 0.004205 |
| ENSG00000183625 | CCR3 | 1.8496 | 2.091936 | 18.97492 | 0.000121 | 0.001803 |
| ENSG00000123411 | IKZF4 | 1.518532 | 2.091569 | 14.10116 | 0.000671 | 0.00744 |
| ENSG00000174705 | SH3PXD2B | 1.247954 | 2.089452 | 9.790787 | 0.003656 | 0.028404 |
| ENSG00000140279 | DUOX2 | 3.51618 | 2.0818 | 61.29782 | 5.06E-09 | 6.20E-07 |
| ENSG00000184860 | SDR42E1 | 3.117252 | 2.08134 | 47.74697 | 6.83E-08 | 3.74E-06 |
| ENSG00000155875 | SAXO1 | 3.883315 | 2.080875 | 62.4659 | 4.12E-09 | 5.39E-07 |
| ENSG00000169436 | COL22A1 | 3.041875 | 2.08073 | 46.44481 | 8.98E-08 | 4.63E-06 |
| ENSG00000241369 | LINC01192 | 2.573912 | 2.07979 | 32.49054 | 2.40E-06 | 6.11E-05 |
| ENSG00000248441 | LINC01197 | 2.428955 | 2.079342 | 32.18631 | 2.53E-06 | 6.41E-05 |
| ENSG00000107611 | CUBN | 2.477872 | 2.07709 | 32.82849 | 2.14E-06 | 5.63E-05 |
| ENSG00000234380 | LINC01426 | 1.737874 | 2.076408 | 18.23967 | 0.000155 | 0.002215 |
| ENSG00000113319 | RASGRF2 | 1.960281 | 2.076049 | 16.86155 | 0.000304 | 0.003886 |
| ENSG00000164309 | CMYA5 | 4.022516 | 2.06498 | 68.56065 | 1.46E-09 | 2.59E-07 |
| ENSG00000155926 | SLA | 3.098946 | 2.064183 | 50.41969 | 3.95E-08 | 2.42E-06 |
| ENSG00000134516 | DOCK2 | 3.346308 | 2.062038 | 46.22865 | 9.40E-08 | 4.81E-06 |
| ENSG00000225791 | TRAM2-AS1 | 2.275522 | 2.060581 | 28.7974 | 6.27E-06 | 0.000137 |
| ENSG00000104043 | ATP8B4 | 2.235438 | 2.060283 | 30.65594 | 3.79E-06 | 8.88E-05 |
| ENSG00000182578 | CSF1R | 1.933655 | 2.059389 | 23.39469 | 2.99E-05 | 0.000536 |
| ENSG00000154783 | FGD5 | 3.946425 | 2.046547 | 68.70175 | 1.43E-09 | 2.59E-07 |
| ENSG00000260230 | FRRS1L | 3.780255 | 2.046119 | 54.99138 | 2.20E-08 | 1.69E-06 |
| ENSG00000100433 | KCNK10 | 3.412939 | 2.04496 | 57.96942 | 9.24E-09 | 9.66E-07 |
| ENSG00000149256 | TENM4 | 3.226334 | 2.044871 | 53.03214 | 2.36E-08 | 1.77E-06 |
| ENSG00000185518 | SV2B | 2.802489 | 2.044378 | 31.66772 | 3.99E-06 | 9.30E-05 |
| ENSG00000081277 | PKP1 | 2.917784 | 2.043901 | 40.21147 | 3.56E-07 | 1.35E-05 |
| ENSG00000236107 | SCN1A-AS1 | 3.105323 | 2.04389 | 48.32305 | 6.06E-08 | 3.45E-06 |
| ENSG00000236107 | LOC102724058 | 3.105323 | 2.04389 | 48.32305 | 6.06E-08 | 3.45E-06 |
| ENSG00000136960 | ENPP2 | 2.371252 | 2.043031 | 30.90777 | 3.54E-06 | 8.39E-05 |
| ENSG00000178568 | ERBB4 | 2.207467 | 2.042546 | 27.1508 | 9.93E-06 | 0.000204 |
| ENSG00000235903 | CPB2-AS1 | 2.27272 | 2.041939 | 29.33025 | 5.42E-06 | 0.00012 |
| ENSG00000184156 | KCNQ3 | 2.06578 | 2.040863 | 25.55054 | 1.57E-05 | 0.000306 |
| ENSG00000249550 | LINC01234 | 2.134908 | 2.040547 | 23.32118 | 3.05E-05 | 0.000545 |
| ENSG00000134297 | PLEKHA8P1 | 1.86598 | 2.037207 | 17.58636 | 0.000194 | 0.002666 |
| ENSG00000144285 | SCN1A | 3.4435 | 2.026801 | 44.64269 | 2.02E-07 | 9.11E-06 |
| ENSG00000104177 | MYEF2 | 3.562509 | 2.026564 | 51.17302 | 3.40E-08 | 2.20E-06 |
| ENSG00000143921 | ABCG8 | 3.065819 | 2.026317 | 54.55061 | 1.76E-08 | 1.46E-06 |
| ENSG00000146839 | ZAN | 2.339291 | 2.024984 | 26.64219 | 1.17E-05 | 0.000237 |
| ENSG00000183873 | SCN5A | 2.773885 | 2.02479 | 39.90139 | 3.82E-07 | 1.42E-05 |
| ENSG00000091622 | PITPNM3 | 2.160443 | 2.024646 | 14.79567 | 0.001138 | 0.011492 |
| ENSG00000285569 | NA | 2.521398 | 2.024508 | 35.91606 | 9.87E-07 | 3.03E-05 |
| ENSG00000250723 | NA | 2.235986 | 2.023968 | 29.15125 | 5.69E-06 | 0.000125 |
| ENSG00000101333 | PLCB4 | 2.507019 | 2.023846 | 26.28633 | 1.53E-05 | 0.000299 |
| ENSG00000139364 | TMEM132B | 2.699659 | 2.023709 | 38.58125 | 5.20E-07 | 1.81E-05 |
| ENSG00000081189 | MEF2C | 2.608398 | 2.023077 | 37.63967 | 6.50E-07 | 2.17E-05 |
| ENSG00000197565 | COL4A6 | 2.00269 | 2.021783 | 25.24348 | 1.72E-05 | 0.000331 |
| ENSG00000143469 | SYT14 | 1.803133 | 2.020277 | 19.30053 | 0.000109 | 0.001638 |
| ENSG00000278916 | CEP83-DT | 1.797601 | 2.019982 | 19.86234 | 9.06E-05 | 0.001399 |
| ENSG00000229205 | LINC00200 | 1.720006 | 2.019264 | 17.03199 | 0.000234 | 0.0031 |
| ENSG00000179796 | LRRC3B | 3.317264 | 2.009384 | 58.98257 | 9.42E-09 | 9.68E-07 |
| ENSG00000251209 | LINC00923 | 3.16647 | 2.006473 | 54.55813 | 1.75E-08 | 1.46E-06 |
| ENSG00000170959 | DCDC1 | 2.42059 | 2.005177 | 32.81929 | 2.15E-06 | 5.63E-05 |
| ENSG00000166206 | GABRB3 | 2.235266 | 2.005038 | 21.98341 | 5.88E-05 | 0.000963 |
| ENSG00000231999 | LRRC8C-DT | 1.917852 | 2.002516 | 21.75999 | 4.94E-05 | 0.00084 |
| ENSG00000159307 | SCUBE1 | 2.100205 | 2.002415 | 25.65548 | 1.52E-05 | 0.000299 |
| ENSG00000138347 | MYPN | 1.956392 | 2.002022 | 18.42169 | 0.000163 | 0.002318 |
| ENSG00000140297 | GCNT3 | 1.783012 | 2.00198 | 20.45431 | 7.48E-05 | 0.001184 |
| ENSG00000124493 | GRM4 | 1.638998 | 2.001477 | 14.48755 | 0.000581 | 0.006622 |
| ENSG00000139304 | PTPRQ | 1.516409 | 2.000857 | 14.36748 | 0.000608 | 0.006867 |
| ENSG00000224071 | NA | 4.204216 | 1.990673 | 61.14083 | 5.21E-09 | 6.30E-07 |
| ENSG00000127241 | MASP1 | 4.236219 | 1.990094 | 60.91765 | 5.69E-09 | 6.75E-07 |
| ENSG00000240405 | SAMMSON | 3.137045 | 1.987855 | 53.15606 | 2.30E-08 | 1.74E-06 |
| ENSG00000144712 | CAND2 | 3.108763 | 1.987317 | 49.46928 | 4.79E-08 | 2.83E-06 |
| ENSG00000111913 | RIPOR2 | 2.826861 | 1.98652 | 43.2056 | 1.81E-07 | 8.26E-06 |
| ENSG00000106772 | PRUNE2 | 2.307556 | 1.98649 | 21.6245 | 7.19E-05 | 0.001146 |
| ENSG00000177301 | KCNA2 | 1.944407 | 1.986024 | 18.20831 | 0.000175 | 0.002453 |
| ENSG00000235885 | LOC101927661 | 3.047288 | 1.986007 | 33.60989 | 2.50E-06 | 6.36E-05 |
| ENSG00000162946 | DISC1 | 2.020431 | 1.983916 | 20.86745 | 6.89E-05 | 0.001108 |
| ENSG00000204301 | NOTCH4 | 1.744022 | 1.983369 | 16.79874 | 0.000254 | 0.003324 |
| ENSG00000091137 | SLC26A4 | 1.641274 | 1.983302 | 14.82326 | 0.000514 | 0.006023 |
| ENSG00000162631 | NTNG1 | 1.464117 | 1.981946 | 11.43192 | 0.001872 | 0.017102 |
| ENSG00000253320 | NA | 1.581838 | 1.981632 | 14.93805 | 0.000493 | 0.005806 |
| ENSG00000169083 | AR | 1.722096 | 1.981577 | 17.07984 | 0.000231 | 0.003056 |
| ENSG00000249375 | CASC11 | 2.833267 | 1.969764 | 31.14828 | 5.40E-06 | 0.00012 |
| ENSG00000081052 | COL4A4 | 2.637225 | 1.967715 | 40.189 | 3.57E-07 | 1.35E-05 |
| ENSG00000254319 | LOC101927815 | 2.803573 | 1.967459 | 39.48506 | 4.21E-07 | 1.53E-05 |
| ENSG00000187955 | COL14A1 | 2.472358 | 1.966852 | 29.84626 | 4.71E-06 | 0.000107 |
| ENSG00000138696 | BMPR1B | 2.099031 | 1.96679 | 23.46911 | 2.92E-05 | 0.000525 |
| ENSG00000149557 | FEZ1 | 2.301183 | 1.965986 | 30.80607 | 3.64E-06 | 8.56E-05 |
| ENSG00000149557 | STT3A-AS1 | 2.301183 | 1.965986 | 30.80607 | 3.64E-06 | 8.56E-05 |
| ENSG00000261026 | NA | 2.528551 | 1.965628 | 30.43869 | 4.01E-06 | 9.35E-05 |
| ENSG00000273507 | NA | 2.314008 | 1.965498 | 31.20222 | 3.28E-06 | 7.87E-05 |
| ENSG00000173227 | SYT12 | 2.091871 | 1.965434 | 26.37685 | 1.24E-05 | 0.000248 |
| ENSG00000258526 | NA | 2.135161 | 1.965206 | 25.35343 | 1.67E-05 | 0.000321 |
| ENSG00000162949 | CAPN13 | 2.049873 | 1.964878 | 21.5667 | 5.25E-05 | 0.000881 |
| ENSG00000170500 | LONRF2 | 2.085646 | 1.964135 | 20.70673 | 6.90E-05 | 0.001108 |
| ENSG00000286071 | LOC105373170 | 3.527379 | 1.95159 | 40.68865 | 5.44E-07 | 1.87E-05 |
| ENSG00000111275 | ALDH2 | 4.157555 | 1.95154 | 53.41906 | 3.34E-08 | 2.18E-06 |
| ENSG00000081237 | PTPRC | 3.112696 | 1.949965 | 48.11545 | 6.33E-08 | 3.57E-06 |
| ENSG00000137809 | ITGA11 | 3.00784 | 1.949268 | 37.61396 | 8.60E-07 | 2.71E-05 |
| ENSG00000259070 | LINC00639 | 3.736732 | 1.949218 | 51.49964 | 3.18E-08 | 2.12E-06 |
| ENSG00000253301 | LINC01606 | 2.76337 | 1.948349 | 41.30735 | 2.77E-07 | 1.12E-05 |
| ENSG00000172578 | KLHL6 | 2.641509 | 1.947935 | 33.54831 | 1.78E-06 | 4.86E-05 |
| ENSG00000140009 | ESR2 | 2.909927 | 1.947543 | 38.79612 | 4.94E-07 | 1.74E-05 |
| ENSG00000143858 | SYT2 | 2.19925 | 1.947008 | 19.73434 | 0.000118 | 0.001758 |
| ENSG00000245526 | LINC00461 | 2.505951 | 1.946435 | 31.44234 | 3.08E-06 | 7.50E-05 |
| ENSG00000230102 | LINC02028 | 2.136616 | 1.945677 | 27.49629 | 9.00E-06 | 0.000186 |
| ENSG00000127954 | STEAP4 | 1.586273 | 1.945446 | 14.37346 | 0.000606 | 0.006866 |
| ENSG00000144331 | ZNF385B | 1.447464 | 1.94292 | 11.85267 | 0.001584 | 0.01494 |
| ENSG00000233928 | NA | 3.918604 | 1.933313 | 58.30609 | 1.06E-08 | 1.03E-06 |
| ENSG00000287516 | NA | 4.956874 | 1.931856 | 71.5531 | 9.01E-10 | 1.85E-07 |
| ENSG00000143127 | ITGA10 | 3.802501 | 1.930831 | 58.92967 | 7.75E-09 | 8.57E-07 |
| ENSG00000166923 | GREM1 | 3.590834 | 1.930813 | 57.38257 | 1.03E-08 | 1.00E-06 |
| ENSG00000153930 | ANKFN1 | 3.463851 | 1.929618 | 54.0278 | 1.94E-08 | 1.57E-06 |
| ENSG00000182308 | DCAF4L1 | 2.472356 | 1.928665 | 23.44523 | 3.91E-05 | 0.000684 |
| ENSG00000081138 | CDH7 | 3.128889 | 1.927666 | 41.48951 | 2.66E-07 | 1.10E-05 |
| ENSG00000127530 | OR7C1 | 2.944004 | 1.927305 | 39.02389 | 4.68E-07 | 1.66E-05 |
| ENSG00000166257 | SCN3B | 3.123421 | 1.927279 | 43.20601 | 1.81E-07 | 8.26E-06 |
| ENSG00000166394 | CYB5R2 | 1.8824 | 1.926603 | 18.16318 | 0.000159 | 0.002264 |
| ENSG00000069431 | ABCC9 | 2.101875 | 1.926221 | 27.0946 | 1.01E-05 | 0.000206 |
| ENSG00000038295 | TLL1 | 1.88096 | 1.925055 | 17.70528 | 0.000189 | 0.002618 |
| ENSG00000286125 | ZIM2-AS1 | 3.769142 | 1.910427 | 61.35901 | 5.01E-09 | 6.19E-07 |
| ENSG00000152377 | SPOCK1 | 3.01261 | 1.909243 | 43.68023 | 1.63E-07 | 7.68E-06 |
| ENSG00000163554 | SPTA1 | 2.633686 | 1.908754 | 21.49254 | 0.000132 | 0.001932 |
| ENSG00000196208 | GREB1 | 2.503993 | 1.908685 | 31.4715 | 3.05E-06 | 7.45E-05 |
| ENSG00000038945 | MSR1 | 2.852931 | 1.907982 | 40.37592 | 3.42E-07 | 1.31E-05 |
| ENSG00000172771 | EFCAB12 | 2.158252 | 1.907858 | 21.76303 | 4.97E-05 | 0.000842 |
| ENSG00000167077 | MEI1 | 2.095639 | 1.907345 | 24.96585 | 1.87E-05 | 0.000355 |
| ENSG00000197410 | DCHS2 | 2.778162 | 1.907017 | 31.76166 | 2.93E-06 | 7.21E-05 |
| ENSG00000002079 | NA | 2.429416 | 1.906325 | 29.41778 | 5.29E-06 | 0.000119 |
| ENSG00000137474 | MYO7A | 2.712385 | 1.905952 | 26.70133 | 1.44E-05 | 0.000285 |
| ENSG00000231057 | NA | 1.985057 | 1.904459 | 21.11655 | 6.05E-05 | 0.000989 |
| ENSG00000116299 | KIAA1324 | 3.258941 | 1.891146 | 39.10115 | 5.99E-07 | 2.03E-05 |
| ENSG00000253553 | NA | 3.437142 | 1.890424 | 50.22163 | 4.11E-08 | 2.50E-06 |
| ENSG00000154258 | ABCA9 | 3.740759 | 1.889663 | 55.13208 | 1.57E-08 | 1.38E-06 |
| ENSG00000279628 | NA | 3.406496 | 1.8893 | 42.08126 | 2.37E-07 | 1.00E-05 |
| ENSG00000278935 | NA | 3.364399 | 1.88917 | 45.39651 | 1.12E-07 | 5.57E-06 |
| ENSG00000080224 | EPHA6 | 2.982706 | 1.888866 | 40.34198 | 3.45E-07 | 1.31E-05 |
| ENSG00000203867 | RBM20 | 3.014532 | 1.888461 | 40.41295 | 3.39E-07 | 1.31E-05 |
| ENSG00000284977 | NA | 3.047564 | 1.88821 | 39.9238 | 3.80E-07 | 1.41E-05 |
| ENSG00000187908 | DMBT1 | 2.201205 | 1.888033 | 24.79876 | 1.96E-05 | 0.00037 |
| ENSG00000153363 | LINC00467 | 3.049104 | 1.887904 | 32.93721 | 2.31E-06 | 5.99E-05 |
| ENSG00000165633 | VSTM4 | 2.383511 | 1.887472 | 30.0104 | 4.50E-06 | 0.000103 |
| ENSG00000133687 | TMTC1 | 2.275826 | 1.886866 | 27.92469 | 7.98E-06 | 0.000167 |
| ENSG00000125675 | GRIA3 | 1.902906 | 1.885189 | 19.678 | 9.62E-05 | 0.001476 |
| ENSG00000274956 | NKAIN3-IT1 | 4.485451 | 1.870053 | 62.10387 | 4.39E-09 | 5.63E-07 |
| ENSG00000273540 | AGBL1 | 3.472322 | 1.869685 | 52.68001 | 2.52E-08 | 1.81E-06 |
| ENSG00000163492 | CCDC141 | 3.673004 | 1.86955 | 47.87679 | 6.65E-08 | 3.67E-06 |
| ENSG00000128833 | MYO5C | 3.074168 | 1.869429 | 44.68121 | 1.31E-07 | 6.36E-06 |
| ENSG00000224819 | NA | 2.783038 | 1.868609 | 31.19171 | 3.58E-06 | 8.45E-05 |
| ENSG00000174502 | SLC26A9 | 3.197257 | 1.868548 | 45.22252 | 1.17E-07 | 5.74E-06 |
| ENSG00000157680 | DGKI | 2.980222 | 1.868474 | 40.56766 | 3.28E-07 | 1.28E-05 |
| ENSG00000237505 | PKN2-AS1 | 2.634489 | 1.868008 | 26.80908 | 1.41E-05 | 0.00028 |
| ENSG00000225914 | HCG23 | 2.56212 | 1.8669 | 32.01266 | 2.65E-06 | 6.67E-05 |
| ENSG00000225914 | TSBP1-AS1 | 2.56212 | 1.8669 | 32.01266 | 2.65E-06 | 6.67E-05 |
| ENSG00000203930 | LINC00632 | 2.838714 | 1.866446 | 35.73158 | 1.03E-06 | 3.16E-05 |
| ENSG00000250241 | LOC101927359 | 2.277278 | 1.865418 | 26.47695 | 1.20E-05 | 0.000243 |
| ENSG00000070601 | FRMPD1 | 4.468234 | 1.85157 | 53.3226 | 3.73E-08 | 2.33E-06 |
| ENSG00000135778 | NTPCR | 2.745365 | 1.846773 | 36.9551 | 7.66E-07 | 2.47E-05 |
| ENSG00000002746 | HECW1 | 2.940519 | 1.846071 | 41.45444 | 2.68E-07 | 1.10E-05 |
| ENSG00000256654 | NA | 2.564408 | 1.846009 | 35.26762 | 1.16E-06 | 3.44E-05 |
| ENSG00000182256 | GABRG3 | 2.795756 | 1.84586 | 34.93088 | 1.26E-06 | 3.63E-05 |
| ENSG00000130649 | CYP2E1 | 2.151593 | 1.843318 | 20.73004 | 6.85E-05 | 0.001105 |
| ENSG00000137766 | UNC13C | 3.942896 | 1.827648 | 57.9276 | 9.31E-09 | 9.66E-07 |
| ENSG00000168631 | MUCL3 | 3.191343 | 1.827277 | 40.48026 | 3.34E-07 | 1.30E-05 |
| ENSG00000182648 | LINC01006 | 3.152348 | 1.826427 | 38.61715 | 5.15E-07 | 1.80E-05 |
| ENSG00000216863 | LY86-AS1 | 3.124868 | 1.82624 | 43.69245 | 1.63E-07 | 7.68E-06 |
| ENSG00000138741 | TRPC3 | 3.5469 | 1.825641 | 42.5238 | 2.10E-07 | 9.46E-06 |
| ENSG00000183454 | GRIN2A | 2.735359 | 1.825344 | 31.14757 | 3.33E-06 | 7.98E-05 |
| ENSG00000009694 | TENM1 | 3.644006 | 1.806314 | 53.43013 | 2.18E-08 | 1.69E-06 |
| ENSG00000229618 | NA | 3.367943 | 1.805335 | 48.15464 | 6.28E-08 | 3.56E-06 |
| ENSG00000226994 | NA | 3.399363 | 1.805016 | 52.70545 | 2.51E-08 | 1.81E-06 |
| ENSG00000152580 | IGSF10 | 3.607307 | 1.80499 | 52.81111 | 2.46E-08 | 1.80E-06 |
| ENSG00000006468 | ETV1 | 3.50903 | 1.804352 | 47.86191 | 6.67E-08 | 3.67E-06 |
| ENSG00000267586 | LINC00907 | 2.833232 | 1.803482 | 36.71804 | 8.12E-07 | 2.60E-05 |
| ENSG00000253100 | NA | 3.08747 | 1.803141 | 42.45225 | 2.14E-07 | 9.46E-06 |
| ENSG00000111728 | ST8SIA1 | 2.395602 | 1.802422 | 30.33113 | 4.13E-06 | 9.60E-05 |
| ENSG00000123612 | ACVR1C | 2.029253 | 1.800832 | 20.60197 | 7.14E-05 | 0.001138 |
| ENSG00000257522 | LOC102724934 | 3.646729 | 1.784989 | 50.78305 | 3.67E-08 | 2.32E-06 |
| ENSG00000152402 | GUCY1A2 | 3.63942 | 1.783599 | 49.26074 | 5.00E-08 | 2.92E-06 |
| ENSG00000142661 | MYOM3 | 3.060508 | 1.782525 | 39.66094 | 4.04E-07 | 1.47E-05 |
| ENSG00000139767 | SRRM4 | 3.011128 | 1.782204 | 38.46336 | 5.35E-07 | 1.85E-05 |
| ENSG00000166573 | GALR1 | 2.837491 | 1.781646 | 34.80809 | 1.30E-06 | 3.71E-05 |
| ENSG00000255545 | LOC283177 | 2.795453 | 1.781013 | 38.62233 | 5.15E-07 | 1.80E-05 |
| ENSG00000122012 | SV2C | 2.366362 | 1.780678 | 24.72504 | 2.00E-05 | 0.000376 |
| ENSG00000255087 | LOC101929473 | 3.336837 | 1.760965 | 43.04349 | 1.88E-07 | 8.53E-06 |
| ENSG00000235538 | NA | 3.222289 | 1.760442 | 40.65892 | 3.21E-07 | 1.26E-05 |
| ENSG00000114757 | PEX5L | 2.830156 | 1.760417 | 34.73558 | 1.32E-06 | 3.77E-05 |
| ENSG00000233008 | LOC101927560 | 3.01825 | 1.760192 | 42.24777 | 2.24E-07 | 9.79E-06 |
| ENSG00000233008 | LINC01725 | 3.01825 | 1.760192 | 42.24777 | 2.24E-07 | 9.79E-06 |
| ENSG00000186334 | SLC36A3 | 2.88094 | 1.759362 | 22.8064 | 9.96E-05 | 0.001517 |
| ENSG00000165084 | C8orf34 | 3.22476 | 1.759228 | 37.81422 | 6.24E-07 | 2.10E-05 |
| ENSG00000229727 | LOC100506274 | 2.641688 | 1.759118 | 25.75208 | 1.81E-05 | 0.000347 |
| ENSG00000250337 | PURPL | 2.453644 | 1.758501 | 27.14424 | 9.94E-06 | 0.000204 |
| ENSG00000188761 | BCL2L15 | 3.901433 | 1.73962 | 54.24099 | 1.86E-08 | 1.52E-06 |
| ENSG00000267252 | LINC01255 | 3.900775 | 1.739443 | 57.66274 | 9.78E-09 | 9.78E-07 |
| ENSG00000196440 | ARMCX4 | 3.759063 | 1.739143 | 41.37631 | 2.83E-07 | 1.14E-05 |
| ENSG00000239921 | LINC01471 | 3.015483 | 1.738984 | 34.80898 | 1.30E-06 | 3.71E-05 |
| ENSG00000253877 | LINC01608 | 3.047067 | 1.73849 | 37.33664 | 6.99E-07 | 2.30E-05 |
| ENSG00000182329 | KIAA2012 | 2.979193 | 1.737834 | 37.68839 | 6.43E-07 | 2.15E-05 |
| ENSG00000234350 | LOC101926913 | 2.643734 | 1.736609 | 28.78748 | 6.29E-06 | 0.000137 |
| ENSG00000235531 | MSC-AS1 | 2.599794 | 1.736518 | 29.36631 | 5.36E-06 | 0.00012 |
| ENSG00000152931 | PART1 | 2.635666 | 1.735975 | 31.97319 | 2.68E-06 | 6.69E-05 |
| ENSG00000131059 | BPIFA3 | 3.428823 | 1.71581 | 39.97662 | 3.75E-07 | 1.40E-05 |
| ENSG00000261371 | PECAM1 | 2.983254 | 1.715042 | 33.12817 | 1.99E-06 | 5.29E-05 |
| ENSG00000163075 | CFAP221 | 2.758685 | 1.715013 | 29.0876 | 5.79E-06 | 0.000127 |
| ENSG00000151490 | PTPRO | 3.503687 | 1.714916 | 42.11018 | 2.31E-07 | 9.84E-06 |
| ENSG00000185823 | NPAP1 | 3.222066 | 1.714673 | 40.76358 | 3.13E-07 | 1.24E-05 |
| ENSG00000081148 | IMPG2 | 2.448543 | 1.714143 | 29.36008 | 5.37E-06 | 0.00012 |
| ENSG00000133067 | LGR6 | 2.833684 | 1.714057 | 35.23232 | 1.17E-06 | 3.46E-05 |
| ENSG00000154736 | ADAMTS5 | 2.406739 | 1.71381 | 28.70623 | 6.43E-06 | 0.000139 |
| ENSG00000094661 | OR1I1 | 4.765114 | 1.693628 | 56.6474 | 1.18E-08 | 1.11E-06 |
| ENSG00000175356 | SCUBE2 | 3.873243 | 1.693245 | 51.71404 | 3.05E-08 | 2.09E-06 |
| ENSG00000232624 | C10orf126 | 4.659176 | 1.693063 | 58.27329 | 8.74E-09 | 9.31E-07 |
| ENSG00000204740 | MALRD1 | 3.431845 | 1.692329 | 46.66247 | 8.57E-08 | 4.44E-06 |
| ENSG00000238217 | LINC01877 | 3.683931 | 1.691908 | 47.19833 | 7.66E-08 | 4.08E-06 |
| ENSG00000204271 | SPIN3 | 2.471603 | 1.690359 | 26.80532 | 1.09E-05 | 0.000223 |
| ENSG00000130368 | MAS1 | 4.164102 | 1.669139 | 55.31832 | 1.52E-08 | 1.36E-06 |
| ENSG00000286619 | NA | 3.465393 | 1.66894 | 42.48624 | 2.12E-07 | 9.46E-06 |
| ENSG00000111058 | ACSS3 | 3.228426 | 1.668543 | 42.17436 | 2.28E-07 | 9.80E-06 |
| ENSG00000241163 | LINC00877 | 2.980175 | 1.667693 | 39.13131 | 4.57E-07 | 1.63E-05 |
| ENSG00000227373 | RABGAP1L-DT | 2.674399 | 1.666904 | 33.51868 | 1.80E-06 | 4.89E-05 |
| ENSG00000257746 | NA | 4.620145 | 1.645103 | 51.19619 | 3.38E-08 | 2.20E-06 |
| ENSG00000196778 | OR52K1 | 3.677086 | 1.645079 | 37.2076 | 8.53E-07 | 2.70E-05 |
| ENSG00000182568 | SATB1 | 3.107287 | 1.6449 | 35.04286 | 1.22E-06 | 3.57E-05 |
| ENSG00000140798 | ABCC12 | 2.938166 | 1.644885 | 33.84599 | 1.65E-06 | 4.54E-05 |
| ENSG00000180347 | ITPRID1 | 3.430385 | 1.644871 | 47.56368 | 7.10E-08 | 3.87E-06 |
| ENSG00000134830 | C5AR2 | 3.557945 | 1.643493 | 42.99752 | 1.89E-07 | 8.58E-06 |
| ENSG00000164509 | IL31RA | 2.858729 | 1.643332 | 36.2155 | 9.17E-07 | 2.85E-05 |
| ENSG00000287177 | NA | 2.858596 | 1.643317 | 36.10962 | 9.41E-07 | 2.91E-05 |
| ENSG00000223553 | NA | 2.327794 | 1.642296 | 25.76685 | 1.48E-05 | 0.00029 |
| ENSG00000251381 | LINC00958 | 4.621387 | 1.621016 | 54.63873 | 1.73E-08 | 1.46E-06 |
| ENSG00000196341 | OR8D1 | 3.313304 | 1.619519 | 35.53422 | 1.08E-06 | 3.28E-05 |
| ENSG00000242516 | LINC00960 | 2.721983 | 1.619231 | 32.3707 | 2.68E-06 | 6.69E-05 |
| ENSG00000162598 | C1orf87 | 3.177932 | 1.618273 | 35.4592 | 1.10E-06 | 3.33E-05 |
| ENSG00000152270 | PDE3B | 2.286363 | 1.617762 | 24.5048 | 2.14E-05 | 0.000399 |
| ENSG00000181847 | TIGIT | 2.895549 | 1.595507 | 30.15288 | 4.81E-06 | 0.000109 |
| ENSG00000073734 | ABCB11 | 3.723558 | 1.595245 | 45.21326 | 1.17E-07 | 5.74E-06 |
| ENSG00000136542 | GALNT5 | 3.307376 | 1.594687 | 42.47991 | 2.13E-07 | 9.46E-06 |
| ENSG00000143199 | ADCY10 | 5.417424 | 1.571975 | 63.50986 | 3.43E-09 | 4.65E-07 |
| ENSG00000258779 | LINC01568 | 3.969695 | 1.570515 | 44.29495 | 1.42E-07 | 6.80E-06 |
| ENSG00000148082 | SHC3 | 3.110503 | 1.57025 | 38.09682 | 6.61E-07 | 2.20E-05 |
| ENSG00000248858 | FLJ46284 | 3.927342 | 1.570002 | 42.21355 | 2.26E-07 | 9.80E-06 |
| ENSG00000148655 | LRMDA | 2.817142 | 1.569229 | 33.24255 | 1.93E-06 | 5.17E-05 |
| ENSG00000172164 | SNTB1 | 2.498253 | 1.567788 | 28.01216 | 7.79E-06 | 0.000163 |
| ENSG00000147465 | STAR | 5.303361 | 1.546246 | 57.73037 | 9.66E-09 | 9.75E-07 |
| ENSG00000234323 | LINC01505 | 4.049226 | 1.544413 | 49.19763 | 5.06E-08 | 2.94E-06 |
| ENSG00000159618 | ADGRG5 | 2.731624 | 1.542156 | 24.99159 | 1.88E-05 | 0.000356 |
| ENSG00000178965 | ERICH3 | 5.088193 | 1.436926 | 45.51357 | 1.09E-07 | 5.48E-06 |
| ENSG00000287611 | NA | 3.423484 | 1.408224 | 38.30169 | 5.55E-07 | 1.90E-05 |

### ANNEX B - downregulated genes

| **ENSEMBL** | **SYMBOL** | **logFC** | **logCPM** | F | **PValue** | **FDR** |
|---|---|---|---|---|---|---|
| ENSG00000059804 | SLC2A3 | -1.15784 | 8.833954 | 231.8851 | 1.76E-16 | 1.21E-12 |
| ENSG00000168209 | DDIT4 | -1.36402 | 8.299682 | 232.6411 | 2.11E-16 | 1.21E-12 |
| ENSG00000099194 | SCD | -1.7153 | 8.159075 | 279.6653 | 6.29E-16 | 1.81E-12 |
| ENSG00000113369 | ARRDC3 | -1.18036 | 8.278419 | 215.9896 | 4.92E-16 | 1.81E-12 |
| ENSG00000197930 | ERO1A | -1.15958 | 8.326384 | 193.8403 | 2.30E-15 | 5.30E-12 |
| ENSG00000109107 | ALDOC | -1.28908 | 7.174557 | 171.3087 | 1.30E-14 | 2.14E-11 |
| ENSG00000135245 | HILPDA | -1.1776 | 7.116894 | 144.1048 | 1.40E-13 | 1.78E-10 |
| ENSG00000134107 | BHLHE40 | -1.01835 | 7.603612 | 132.6048 | 4.25E-13 | 4.89E-10 |
| ENSG00000122884 | P4HA1 | -0.95461 | 7.741128 | 123.8935 | 1.04E-12 | 1.09E-09 |
| ENSG00000167772 | ANGPTL4 | -1.8026 | 5.473873 | 121.3981 | 1.36E-12 | 1.31E-09 |
| ENSG00000176171 | BNIP3 | -0.91461 | 8.115004 | 119.8006 | 1.62E-12 | 1.43E-09 |
| ENSG00000214049 | UCA1 | -0.79015 | 8.872124 | 116.6515 | 2.29E-12 | 1.88E-09 |
| ENSG00000102837 | OLFM4 | -1.21858 | 6.422984 | 113.1267 | 3.40E-12 | 2.61E-09 |
| ENSG00000130066 | SAT1 | -0.74583 | 9.601029 | 111.6821 | 4.01E-12 | 2.89E-09 |
| ENSG00000171401 | KRT13 | -0.7538 | 9.646573 | 110.687 | 4.50E-12 | 3.05E-09 |
| ENSG00000105220 | GPI | -0.75828 | 10.17994 | 109.4759 | 5.18E-12 | 3.31E-09 |
| ENSG00000102144 | PGK1 | -0.72253 | 10.57397 | 107.3301 | 6.67E-12 | 3.80E-09 |
| ENSG00000104765 | BNIP3L | -0.75047 | 8.661867 | 90.7738 | 5.39E-11 | 2.30E-08 |
| ENSG00000171345 | KRT19 | -0.62898 | 10.76351 | 81.11035 | 2.09E-10 | 6.68E-08 |
| ENSG00000148926 | ADM | -0.63742 | 8.983586 | 75.5252 | 4.83E-10 | 1.18E-07 |
| ENSG00000163435 | ELF3 | -0.67339 | 9.191021 | 74.67258 | 5.51E-10 | 1.29E-07 |
| ENSG00000162496 | DHRS3 | -0.60339 | 9.610711 | 74.19627 | 5.94E-10 | 1.37E-07 |
| ENSG00000122861 | PLAU | -0.6052 | 9.800976 | 73.33984 | 6.79E-10 | 1.50E-07 |
| ENSG00000173391 | OLR1 | -0.59716 | 10.07465 | 72.84583 | 7.34E-10 | 1.59E-07 |
| ENSG00000149573 | MPZL2 | -0.63115 | 8.393004 | 59.29357 | 7.25E-09 | 8.18E-07 |
| ENSG00000148346 | LCN2 | -0.60026 | 8.982236 | 55.26484 | 1.61E-08 | 1.40E-06 |
| ENSG00000164096 | C4orf3 | -0.62547 | 7.863159 | 53.80429 | 2.03E-08 | 1.61E-06 |
| ENSG00000135821 | GLUL | -0.5499 | 9.257118 | 52.32046 | 2.71E-08 | 1.91E-06 |
| ENSG00000111859 | NEDD9 | -0.52116 | 8.893757 | 52.16642 | 2.79E-08 | 1.96E-06 |
| ENSG00000134333 | LDHA | -0.50103 | 12.16499 | 50.80165 | 3.66E-08 | 2.32E-06 |
| ENSG00000152256 | PDK1 | -0.84419 | 6.364427 | 50.8339 | 3.64E-08 | 2.32E-06 |
| ENSG00000189067 | LITAF | -0.5118 | 10.82146 | 47.10307 | 7.82E-08 | 4.14E-06 |
| ENSG00000196586 | MYO6 | -0.70347 | 6.920511 | 46.9566 | 8.06E-08 | 4.25E-06 |
| ENSG00000169242 | EFNA1 | -0.58384 | 7.851598 | 44.86741 | 1.26E-07 | 6.13E-06 |
| ENSG00000148344 | PTGES | -0.73215 | 6.619588 | 44.55342 | 1.35E-07 | 6.51E-06 |
| ENSG00000067064 | IDI1 | -0.72555 | 6.747967 | 44.47078 | 1.37E-07 | 6.60E-06 |
| ENSG00000138413 | IDH1 | -0.55463 | 8.087772 | 43.47813 | 1.70E-07 | 7.93E-06 |
| ENSG00000165389 | SPTSSA | -0.6733 | 8.22927 | 48.49599 | 1.78E-07 | 8.17E-06 |
| ENSG00000154639 | CXADR | -0.51099 | 8.215517 | 42.45275 | 2.14E-07 | 9.46E-06 |
| ENSG00000109046 | WSB1 | -0.58411 | 7.750907 | 41.16709 | 2.86E-07 | 1.15E-05 |
| ENSG00000079459 | FDFT1 | -0.48537 | 8.693538 | 40.38371 | 3.42E-07 | 1.31E-05 |
| ENSG00000137145 | DENND4C | -0.54048 | 7.617222 | 40.38426 | 3.42E-07 | 1.31E-05 |
| ENSG00000143217 | NECTIN4 | -0.75691 | 6.275246 | 40.10107 | 3.65E-07 | 1.37E-05 |
| ENSG00000119801 | YPEL5 | -0.82106 | 6.221519 | 39.99915 | 3.73E-07 | 1.40E-05 |
| ENSG00000111640 | GAPDH | -0.46503 | 12.23166 | 39.73979 | 3.96E-07 | 1.45E-05 |
| ENSG00000164825 | DEFB1 | -0.6223 | 7.230159 | 39.38687 | 4.30E-07 | 1.55E-05 |
| ENSG00000124107 | SLPI | -0.72592 | 6.381975 | 39.25487 | 4.44E-07 | 1.59E-05 |
| ENSG00000163220 | S100A9 | -0.49229 | 9.607589 | 39.29201 | 4.64E-07 | 1.65E-05 |
| ENSG00000163516 | ANKZF1 | -0.72088 | 6.424774 | 38.31271 | 5.54E-07 | 1.90E-05 |
| ENSG00000265972 | TXNIP | -0.60292 | 8.215507 | 41.2744 | 5.59E-07 | 1.91E-05 |
| ENSG00000137393 | RNF144B | -0.46963 | 8.854575 | 37.39929 | 6.89E-07 | 2.28E-05 |
| ENSG00000168092 | PAFAH1B2 | -0.58319 | 6.984538 | 36.81816 | 7.92E-07 | 2.55E-05 |
| ENSG00000000971 | CFH | -0.41975 | 9.62772 | 36.26654 | 9.06E-07 | 2.83E-05 |
| ENSG00000100292 | HMOX1 | -0.62819 | 6.97861 | 36.18706 | 9.24E-07 | 2.86E-05 |
| ENSG00000101782 | RIOK3 | -0.62685 | 6.689671 | 35.60155 | 1.07E-06 | 3.24E-05 |
| ENSG00000107438 | PDLIM1 | -0.44736 | 8.660899 | 35.35542 | 1.13E-06 | 3.39E-05 |
| ENSG00000128422 | KRT17 | -0.44741 | 8.67074 | 35.21872 | 1.17E-06 | 3.47E-05 |
| ENSG00000100234 | TIMP3 | -0.57406 | 7.20052 | 35.18112 | 1.18E-06 | 3.49E-05 |
| ENSG00000129521 | EGLN3 | -0.69481 | 6.381465 | 35.15974 | 1.19E-06 | 3.49E-05 |
| ENSG00000137575 | SDCBP | -0.44208 | 8.942967 | 35.00523 | 1.24E-06 | 3.59E-05 |
| ENSG00000153292 | ADGRF1 | -0.58545 | 7.001635 | 33.91907 | 1.62E-06 | 4.51E-05 |
| ENSG00000272398 | CD24 | -0.56278 | 7.369848 | 33.84689 | 1.65E-06 | 4.54E-05 |
| ENSG00000083444 | PLOD1 | -0.45149 | 8.853091 | 33.20229 | 1.95E-06 | 5.21E-05 |
| ENSG00000196968 | FUT11 | -1.00886 | 5.348571 | 33.09802 | 2.00E-06 | 5.30E-05 |
| ENSG00000134215 | VAV3 | -0.62487 | 6.87337 | 32.9919 | 2.06E-06 | 5.42E-05 |
| ENSG00000111669 | TPI1 | -0.39252 | 11.52191 | 32.98016 | 2.06E-06 | 5.43E-05 |
| ENSG00000090013 | BLVRB | -0.46544 | 8.344513 | 32.5095 | 2.33E-06 | 6.02E-05 |
| ENSG00000188994 | ZNF292 | -0.60801 | 6.631877 | 32.43108 | 2.38E-06 | 6.07E-05 |
| ENSG00000164342 | TLR3 | -0.96285 | 5.326582 | 32.19926 | 2.52E-06 | 6.41E-05 |
| ENSG00000114796 | KLHL24 | -0.79447 | 5.672321 | 31.91763 | 2.72E-06 | 6.76E-05 |
| ENSG00000114023 | FAM162A | -0.61413 | 7.075794 | 32.24805 | 2.72E-06 | 6.76E-05 |
| ENSG00000185215 | TNFAIP2 | -0.69318 | 6.132508 | 31.708 | 2.87E-06 | 7.08E-05 |
| ENSG00000115548 | KDM3A | -0.56586 | 6.923362 | 31.33901 | 3.16E-06 | 7.65E-05 |
| ENSG00000139793 | MBNL2 | -0.57778 | 6.863266 | 31.31621 | 3.18E-06 | 7.67E-05 |
| ENSG00000033800 | PIAS1 | -0.53368 | 7.365935 | 31.29556 | 3.20E-06 | 7.69E-05 |
| ENSG00000112972 | HMGCS1 | -0.57234 | 6.841153 | 31.0965 | 3.37E-06 | 8.04E-05 |
| ENSG00000102024 | PLS3 | -0.39184 | 10.08083 | 31.08627 | 3.38E-06 | 8.05E-05 |
| ENSG00000120594 | PLXDC2 | -0.50122 | 7.479119 | 30.94701 | 3.51E-06 | 8.32E-05 |
| ENSG00000105856 | HBP1 | -0.56351 | 6.801036 | 30.26483 | 4.21E-06 | 9.76E-05 |
| ENSG00000100439 | ABHD4 | -0.53937 | 7.407033 | 30.12359 | 4.58E-06 | 0.000105 |
| ENSG00000112308 | C6orf62 | -0.41006 | 9.303033 | 29.78569 | 4.79E-06 | 0.000109 |
| ENSG00000177565 | TBL1XR1 | -0.42127 | 8.159461 | 29.51813 | 5.15E-06 | 0.000116 |
| ENSG00000166710 | B2M | -0.40303 | 11.62658 | 29.49358 | 5.18E-06 | 0.000117 |
| ENSG00000113161 | HMGCR | -0.46121 | 7.560814 | 29.30568 | 5.45E-06 | 0.000121 |
| ENSG00000213639 | PPP1CB | -0.44394 | 8.523717 | 29.09535 | 5.78E-06 | 0.000127 |
| ENSG00000197746 | PSAP | -0.35901 | 10.85938 | 29.01101 | 5.91E-06 | 0.000129 |
| ENSG00000112414 | ADGRG6 | -0.44278 | 7.563818 | 28.51209 | 6.78E-06 | 0.000145 |
| ENSG00000183421 | RIPK4 | -0.43489 | 9.65559 | 29.20734 | 6.85E-06 | 0.000146 |
| ENSG00000101871 | MID1 | -0.39024 | 8.716321 | 28.40281 | 6.99E-06 | 0.000149 |
| ENSG00000117650 | NEK2 | -0.47464 | 7.435631 | 28.16308 | 7.47E-06 | 0.000158 |
| ENSG00000137710 | RDX | -0.41449 | 8.350533 | 28.1688 | 7.46E-06 | 0.000158 |
| ENSG00000011638 | TMEM159 | -0.5585 | 6.537763 | 27.72004 | 8.45E-06 | 0.000176 |
| ENSG00000165685 | TMEM52B | -0.50487 | 7.351715 | 27.59302 | 8.76E-06 | 0.000182 |
| ENSG00000230937 | MIR205HG | -0.37621 | 9.017083 | 27.52199 | 8.94E-06 | 0.000185 |
| ENSG00000168300 | PCMTD1 | -0.76817 | 5.484484 | 26.9146 | 1.06E-05 | 0.000217 |
| ENSG00000074800 | ENO1 | -0.36992 | 11.98192 | 26.58816 | 1.17E-05 | 0.000236 |
| ENSG00000068697 | LAPTM4A | -0.41565 | 8.188767 | 26.16808 | 1.31E-05 | 0.000262 |
| ENSG00000143546 | S100A8 | -0.43151 | 7.880587 | 26.17069 | 1.31E-05 | 0.000262 |
| ENSG00000105612 | DNASE2 | -0.44591 | 7.460895 | 25.83438 | 1.45E-05 | 0.000286 |
| ENSG00000187446 | CHP1 | -0.39767 | 8.108093 | 25.79907 | 1.46E-05 | 0.000288 |
| ENSG00000204592 | HLA-E | -0.36847 | 9.174385 | 25.6897 | 1.51E-05 | 0.000296 |
| ENSG00000182054 | IDH2 | -0.46676 | 7.454315 | 25.369 | 1.66E-05 | 0.00032 |
| ENSG00000181467 | RAP2B | -0.53414 | 6.741679 | 25.14237 | 1.77E-05 | 0.00034 |
| ENSG00000134258 | VTCN1 | -0.82683 | 5.211585 | 24.995 | 1.85E-05 | 0.000353 |
| ENSG00000159399 | HK2 | -0.51586 | 7.29187 | 25.4779 | 1.85E-05 | 0.000354 |
| ENSG00000072682 | P4HA2 | -0.46712 | 7.256555 | 24.96711 | 1.87E-05 | 0.000355 |
| ENSG00000138640 | FAM13A | -0.50001 | 6.985115 | 24.92497 | 1.89E-05 | 0.000357 |
| ENSG00000134317 | GRHL1 | -0.65234 | 5.988319 | 24.54292 | 2.11E-05 | 0.000396 |
| ENSG00000132561 | MATN2 | -0.56621 | 6.43197 | 24.2348 | 2.32E-05 | 0.000429 |
| ENSG00000104549 | SQLE | -0.42676 | 7.715324 | 24.04898 | 2.45E-05 | 0.000452 |
| ENSG00000116209 | TMEM59 | -0.45207 | 8.136066 | 24.60202 | 2.55E-05 | 0.000468 |
| ENSG00000116747 | RO60 | -0.42531 | 7.850821 | 23.91546 | 2.55E-05 | 0.000468 |
| ENSG00000186480 | INSIG1 | -1.4753 | 3.93445 | 24.37306 | 2.61E-05 | 0.000476 |
| ENSG00000168615 | ADAM9 | -0.33321 | 10.46935 | 23.72204 | 2.70E-05 | 0.000491 |
| ENSG00000116133 | DHCR24 | -0.36334 | 9.090255 | 23.70857 | 2.71E-05 | 0.000493 |
| ENSG00000115339 | GALNT3 | -0.53814 | 6.731994 | 23.6743 | 2.74E-05 | 0.000497 |
| ENSG00000081923 | ATP8B1 | -0.54206 | 6.518827 | 23.43991 | 2.94E-05 | 0.000529 |
| ENSG00000005448 | WDR54 | -0.41829 | 7.453107 | 23.049 | 3.32E-05 | 0.000589 |
| ENSG00000143320 | CRABP2 | -0.46912 | 7.477963 | 23.0829 | 3.48E-05 | 0.000614 |
| ENSG00000197956 | S100A6 | -0.39123 | 8.491381 | 22.84401 | 3.53E-05 | 0.000621 |
| ENSG00000164754 | RAD21 | -0.39199 | 7.796662 | 22.56451 | 3.85E-05 | 0.000675 |
| ENSG00000155508 | CNOT8 | -0.43634 | 7.193288 | 22.44889 | 3.99E-05 | 0.000694 |
| ENSG00000183726 | TMEM50A | -0.43157 | 7.235208 | 22.21125 | 4.29E-05 | 0.000742 |
| ENSG00000167815 | PRDX2 | -0.35998 | 8.979658 | 22.14365 | 4.38E-05 | 0.000756 |
| ENSG00000124151 | NCOA3 | -0.37614 | 8.054831 | 21.93152 | 4.68E-05 | 0.000803 |
| ENSG00000091128 | LAMB4 | -0.3734 | 8.297197 | 21.82499 | 4.84E-05 | 0.000825 |
| ENSG00000086666 | ZFAND6 | -0.44921 | 7.239383 | 21.52492 | 5.32E-05 | 0.000891 |
| ENSG00000187210 | GCNT1 | -0.5313 | 6.278434 | 21.3313 | 5.66E-05 | 0.000939 |
| ENSG00000162819 | BROX | -0.38746 | 8.558425 | 21.39812 | 5.67E-05 | 0.00094 |
| ENSG00000077092 | RARB | -0.65238 | 5.601745 | 21.24388 | 5.81E-05 | 0.000957 |
| ENSG00000008282 | SYPL1 | -0.45044 | 7.782267 | 21.65186 | 6.29E-05 | 0.001025 |
| ENSG00000134762 | DSC3 | -0.45726 | 7.209959 | 20.94719 | 6.39E-05 | 0.00104 |
| ENSG00000109586 | GALNT7 | -0.42567 | 7.240761 | 20.79359 | 6.71E-05 | 0.001087 |
| ENSG00000112378 | PERP | -0.3565 | 8.671874 | 20.78421 | 6.73E-05 | 0.001089 |
| ENSG00000166750 | SLFN5 | -0.66811 | 5.531268 | 20.77581 | 6.75E-05 | 0.00109 |
| ENSG00000151135 | TMEM263 | -0.51074 | 6.303682 | 20.68155 | 6.96E-05 | 0.001116 |
| ENSG00000107968 | MAP3K8 | -0.73238 | 5.280491 | 20.66578 | 6.99E-05 | 0.00112 |
| ENSG00000044115 | CTNNA1 | -0.33151 | 9.071451 | 20.51206 | 7.34E-05 | 0.001164 |
| ENSG00000108395 | TRIM37 | -0.43634 | 6.862958 | 20.21647 | 8.08E-05 | 0.00127 |
| ENSG00000169252 | ADRB2 | -0.44531 | 6.881531 | 20.19911 | 8.12E-05 | 0.001273 |
| ENSG00000117394 | SLC2A1 | -0.36268 | 8.554886 | 20.18681 | 8.16E-05 | 0.001277 |
| ENSG00000139154 | AEBP2 | -0.60228 | 5.974854 | 20.16767 | 8.21E-05 | 0.001283 |
| ENSG00000125868 | DSTN | -0.33971 | 9.532875 | 20.15372 | 8.24E-05 | 0.001287 |
| ENSG00000198125 | MB | -1.3069 | 3.669424 | 19.97981 | 8.72E-05 | 0.001354 |
| ENSG00000130638 | ATXN10 | -0.32168 | 9.371649 | 19.8756 | 9.02E-05 | 0.001395 |
| ENSG00000131171 | SH3BGRL | -0.4957 | 6.391708 | 19.77317 | 9.33E-05 | 0.001433 |
| ENSG00000170348 | TMED10 | -0.32988 | 9.474789 | 19.62053 | 9.81E-05 | 0.0015 |
| ENSG00000111846 | GCNT2 | -0.44687 | 6.700027 | 19.59881 | 9.88E-05 | 0.001509 |
| ENSG00000037637 | FBXO42 | -0.45139 | 6.692258 | 19.59215 | 9.90E-05 | 0.00151 |
| ENSG00000134308 | YWHAQ | -0.30922 | 10.04401 | 19.53193 | 0.000101 | 0.001534 |
| ENSG00000115963 | RND3 | -0.37068 | 7.904819 | 19.34895 | 0.000107 | 0.001616 |
| ENSG00000175906 | ARL4D | -0.47237 | 7.239333 | 19.80495 | 0.000108 | 0.001621 |
| ENSG00000176974 | SHMT1 | -0.35046 | 8.016107 | 19.20729 | 0.000112 | 0.001681 |
| ENSG00000106460 | TMEM106B | -0.45493 | 6.736689 | 19.09202 | 0.000117 | 0.001744 |
| ENSG00000063046 | EIF4B | -0.35791 | 8.355726 | 18.98946 | 0.000121 | 0.001798 |
| ENSG00000180739 | S1PR5 | -0.47632 | 6.652191 | 18.98733 | 0.000121 | 0.001798 |
| ENSG00000104763 | ASAH1 | -0.3098 | 9.227851 | 18.95733 | 0.000122 | 0.001812 |
| ENSG00000204264 | PSMB8 | -0.37742 | 7.745241 | 18.94944 | 0.000122 | 0.001814 |
| ENSG00000107104 | KANK1 | -0.36851 | 7.813491 | 18.89023 | 0.000125 | 0.00184 |
| ENSG00000115738 | ID2 | -0.59231 | 6.242889 | 19.13483 | 0.000127 | 0.001868 |
| ENSG00000115825 | PRKD3 | -0.35956 | 8.21917 | 18.7547 | 0.000131 | 0.001911 |
| ENSG00000168143 | FAM83B | -0.51566 | 6.135548 | 18.69381 | 0.000133 | 0.001942 |
| ENSG00000109475 | RPL34 | -0.31171 | 8.94623 | 18.56958 | 0.000139 | 0.002015 |
| ENSG00000205302 | SNX2 | -0.33245 | 8.060655 | 18.54813 | 0.00014 | 0.002025 |
| ENSG00000111348 | ARHGDIB | -0.29318 | 10.35088 | 18.33867 | 0.00015 | 0.002158 |
| ENSG00000163931 | TKT | -0.32762 | 9.858621 | 18.29538 | 0.000152 | 0.002182 |
| ENSG00000213853 | EMP2 | -0.41202 | 7.177936 | 18.26898 | 0.000154 | 0.002198 |
| ENSG00000139697 | SBNO1 | -0.32718 | 8.190291 | 18.07686 | 0.000164 | 0.00232 |
| ENSG00000108256 | NUFIP2 | -0.52357 | 6.667604 | 18.45937 | 0.000166 | 0.00234 |
| ENSG00000156711 | MAPK13 | -0.39901 | 7.071738 | 18.03343 | 0.000166 | 0.002343 |
| ENSG00000116857 | TMEM9 | -0.32227 | 8.47362 | 18.01718 | 0.000167 | 0.002353 |
| ENSG00000116106 | EPHA4 | -0.5063 | 6.123533 | 17.91567 | 0.000173 | 0.002426 |
| ENSG00000170266 | GLB1 | -0.33868 | 7.843709 | 17.67613 | 0.000188 | 0.002601 |
| ENSG00000069275 | NUCKS1 | -0.34906 | 9.01759 | 17.92737 | 0.00019 | 0.002622 |
| ENSG00000141562 | NARF | -0.37742 | 7.34823 | 17.52588 | 0.000198 | 0.002712 |
| ENSG00000164292 | RHOBTB3 | -0.44161 | 6.773525 | 17.48584 | 0.000201 | 0.002737 |
| ENSG00000103811 | CTSH | -0.30585 | 8.751215 | 17.45376 | 0.000203 | 0.002757 |
| ENSG00000124766 | SOX4 | -0.49375 | 6.323487 | 17.35897 | 0.000209 | 0.002838 |
| ENSG00000137845 | ADAM10 | -0.29274 | 9.29322 | 17.34263 | 0.000211 | 0.002851 |
| ENSG00000090861 | AARS1 | -0.33403 | 8.033104 | 17.28188 | 0.000215 | 0.002901 |
| ENSG00000122729 | ACO1 | -0.34986 | 7.532698 | 17.15084 | 0.000225 | 0.003011 |
| ENSG00000221963 | APOL6 | -0.45355 | 6.497291 | 17.13622 | 0.000226 | 0.003017 |
| ENSG00000143756 | FBXO28 | -0.40505 | 6.878555 | 17.10833 | 0.000228 | 0.003034 |
| ENSG00000005483 | KMT2E | -0.3929 | 7.374044 | 16.91305 | 0.000244 | 0.003216 |
| ENSG00000105854 | PON2 | -0.4816 | 6.344692 | 16.90688 | 0.000245 | 0.003219 |
| ENSG00000163430 | FSTL1 | -0.29544 | 9.838067 | 16.88423 | 0.000247 | 0.003241 |
| ENSG00000116005 | PCYOX1 | -0.33915 | 7.722655 | 16.84639 | 0.00025 | 0.003273 |
| ENSG00000125304 | TM9SF2 | -0.31291 | 8.683165 | 16.75037 | 0.000259 | 0.003377 |
| ENSG00000164211 | STARD4 | -0.55569 | 5.827001 | 16.6283 | 0.00027 | 0.003516 |
| ENSG00000144476 | ACKR3 | -1.93272 | 2.674453 | 16.85704 | 0.000274 | 0.003556 |
| ENSG00000039319 | ZFYVE16 | -0.40101 | 6.845425 | 16.56088 | 0.000276 | 0.003575 |
| ENSG00000165410 | CFL2 | -0.41595 | 6.712764 | 16.55796 | 0.000277 | 0.003575 |
| ENSG00000113441 | LNPEP | -0.35716 | 7.568566 | 16.50727 | 0.000282 | 0.003631 |
| ENSG00000131238 | PPT1 | -0.29032 | 9.899524 | 16.4628 | 0.000286 | 0.003684 |
| ENSG00000105755 | ETHE1 | -0.33029 | 7.905777 | 16.30563 | 0.000302 | 0.003867 |
| ENSG00000163660 | CCNL1 | -0.39036 | 7.243065 | 16.25455 | 0.000308 | 0.003929 |
| ENSG00000205581 | HMGN1 | -0.35673 | 7.6375 | 16.12897 | 0.000322 | 0.00408 |
| ENSG00000136868 | SLC31A1 | -0.40162 | 6.73427 | 16.11729 | 0.000323 | 0.004092 |
| ENSG00000197563 | PIGN | -0.36782 | 7.247652 | 16.09898 | 0.000325 | 0.00411 |
| ENSG00000133789 | SWAP70 | -0.4286 | 6.596863 | 16.0341 | 0.000333 | 0.004191 |
| ENSG00000117448 | AKR1A1 | -0.34249 | 7.61461 | 15.97917 | 0.000339 | 0.00426 |
| ENSG00000169241 | SLC50A1 | -0.45206 | 6.399477 | 15.98022 | 0.000339 | 0.00426 |
| ENSG00000142619 | PADI3 | -0.37951 | 7.316828 | 15.86508 | 0.000353 | 0.004431 |
| ENSG00000113583 | C5orf15 | -0.36214 | 7.605891 | 15.84148 | 0.000356 | 0.004464 |
| ENSG00000071553 | ATP6AP1 | -0.29997 | 8.704483 | 15.77068 | 0.000365 | 0.004558 |
| ENSG00000150593 | PDCD4 | -0.56485 | 5.712161 | 15.7579 | 0.000367 | 0.004574 |
| ENSG00000123595 | RAB9A | -0.41263 | 6.845345 | 15.72774 | 0.000371 | 0.004603 |
| ENSG00000079739 | PGM1 | -0.32577 | 7.850683 | 15.58228 | 0.000391 | 0.004813 |
| ENSG00000136235 | GPNMB | -0.41488 | 6.856072 | 15.57904 | 0.000391 | 0.004813 |
| ENSG00000129691 | ASH2L | -0.36579 | 7.108508 | 15.54611 | 0.000396 | 0.004843 |
| ENSG00000185624 | P4HB | -0.29949 | 9.818064 | 15.54326 | 0.000396 | 0.004843 |
| ENSG00000230590 | FTX | -0.49851 | 6.347916 | 15.51929 | 0.000407 | 0.004949 |
| ENSG00000134291 | TMEM106C | -0.30571 | 8.994565 | 15.44398 | 0.000411 | 0.004983 |
| ENSG00000145730 | PAM | -0.41914 | 7.279823 | 15.75215 | 0.000413 | 0.005011 |
| ENSG00000105976 | MET | -0.31457 | 7.946485 | 15.41758 | 0.000415 | 0.00502 |
| ENSG00000103769 | RAB11A | -0.30261 | 8.393 | 15.36844 | 0.000422 | 0.005093 |
| ENSG00000182149 | IST1 | -0.29974 | 8.340385 | 15.34436 | 0.000426 | 0.005127 |
| ENSG00000070087 | PFN2 | -0.31733 | 8.450328 | 15.33578 | 0.000427 | 0.005137 |
| ENSG00000068745 | IP6K2 | -0.3661 | 7.293928 | 15.24376 | 0.000441 | 0.005289 |
| ENSG00000127955 | GNAI1 | -0.41694 | 6.696934 | 15.23902 | 0.000442 | 0.005292 |
| ENSG00000170949 | ZNF160 | -0.46928 | 6.314212 | 15.21798 | 0.000446 | 0.005327 |
| ENSG00000138660 | AP1AR | -0.4567 | 6.601198 | 15.19435 | 0.000452 | 0.005398 |
| ENSG00000100852 | ARHGAP5 | -0.4671 | 6.179261 | 15.17142 | 0.000453 | 0.005401 |
| ENSG00000173193 | PARP14 | -0.33984 | 7.472248 | 15.11063 | 0.000463 | 0.005515 |
| ENSG00000100811 | YY1 | -0.50965 | 6.408317 | 15.37498 | 0.000469 | 0.005575 |
| ENSG00000005889 | ZFX | -0.38024 | 7.005407 | 15.03763 | 0.000476 | 0.005635 |
| ENSG00000138376 | BARD1 | -0.38839 | 6.844784 | 15.03727 | 0.000476 | 0.005635 |
| ENSG00000138182 | KIF20B | -0.39553 | 6.884341 | 15.02183 | 0.000478 | 0.005661 |
| ENSG00000052802 | MSMO1 | -0.44346 | 6.565824 | 14.98913 | 0.000484 | 0.005717 |
| ENSG00000114268 | PFKFB4 | -0.65579 | 5.279733 | 14.9527 | 0.00049 | 0.005781 |
| ENSG00000129625 | REEP5 | -0.29455 | 8.385573 | 14.89848 | 0.0005 | 0.005878 |
| ENSG00000104419 | NDRG1 | -0.72666 | 4.714159 | 14.78786 | 0.000521 | 0.006095 |
| ENSG00000163605 | PPP4R2 | -0.53462 | 5.655945 | 14.72855 | 0.000532 | 0.00621 |
| ENSG00000164924 | YWHAZ | -0.26815 | 10.96624 | 14.66718 | 0.000544 | 0.006293 |
| ENSG00000164024 | METAP1 | -0.34141 | 7.367877 | 14.63945 | 0.00055 | 0.006345 |
| ENSG00000108479 | GALK1 | -0.43038 | 6.490019 | 14.62406 | 0.000553 | 0.006355 |
| ENSG00000196912 | ANKRD36B | -0.66064 | 5.046274 | 14.60153 | 0.000558 | 0.006382 |
| ENSG00000052344 | PRSS8 | -0.33515 | 7.921076 | 14.565 | 0.000565 | 0.006462 |
| ENSG00000132823 | OSER1 | -0.52127 | 5.999474 | 14.55212 | 0.000568 | 0.006486 |
| ENSG00000171792 | RHNO1 | -0.45323 | 6.256462 | 14.49365 | 0.00058 | 0.006619 |
| ENSG00000171346 | KRT15 | -0.40555 | 6.600064 | 14.41853 | 0.000596 | 0.006766 |
| ENSG00000175582 | RAB6A | -0.33845 | 7.402403 | 14.37017 | 0.000607 | 0.006867 |
| ENSG00000036054 | TBC1D23 | -0.34246 | 7.170514 | 14.36067 | 0.000609 | 0.006878 |
| ENSG00000125968 | ID1 | -0.59637 | 9.231141 | 22.40218 | 0.000636 | 0.007145 |
| ENSG00000152558 | TMEM123 | -0.35144 | 7.140009 | 14.21352 | 0.000643 | 0.00722 |
| ENSG00000039068 | CDH1 | -0.28507 | 8.237857 | 14.18225 | 0.000651 | 0.007297 |
| ENSG00000113712 | CSNK1A1 | -0.28177 | 8.968451 | 14.17695 | 0.000652 | 0.007304 |
| ENSG00000065154 | OAT | -0.28203 | 8.96546 | 14.14924 | 0.000659 | 0.007373 |
| ENSG00000133872 | SARAF | -0.32444 | 8.2036 | 14.16469 | 0.000663 | 0.007395 |
| ENSG00000169583 | CLIC3 | -0.59978 | 5.279283 | 14.13377 | 0.000663 | 0.007395 |
| ENSG00000115866 | DARS1 | -0.33298 | 7.350726 | 14.12032 | 0.000666 | 0.007409 |
| ENSG00000277443 | MARCKS | -0.9074 | 4.294405 | 14.12996 | 0.000666 | 0.007409 |
| ENSG00000170791 | CHCHD7 | -0.48121 | 5.958663 | 14.10127 | 0.000671 | 0.00744 |
| ENSG00000114439 | BBX | -0.30822 | 7.851592 | 14.06121 | 0.000681 | 0.007537 |
| ENSG00000136783 | NIPSNAP3A | -0.56112 | 5.835977 | 14.12257 | 0.00068 | 0.007537 |
| ENSG00000112078 | KCTD20 | -0.32405 | 7.388777 | 14.02786 | 0.000689 | 0.007616 |
| ENSG00000175265 | GOLGA8A | -0.85644 | 4.409945 | 14.00223 | 0.000696 | 0.007682 |
| ENSG00000006747 | SCIN | -0.34596 | 7.765297 | 13.98228 | 0.00072 | 0.007896 |
| ENSG00000164244 | PRRC1 | -0.29464 | 7.835125 | 13.90061 | 0.000723 | 0.007918 |
| ENSG00000168385 | SEPTIN2 | -0.26188 | 9.918813 | 13.83868 | 0.00074 | 0.008092 |
| ENSG00000142657 | PGD | -0.32663 | 7.574248 | 13.82874 | 0.000742 | 0.00811 |
| ENSG00000156273 | BACH1 | -0.46049 | 6.023274 | 13.8232 | 0.000744 | 0.008119 |
| ENSG00000156273 | GRIK1-AS2 | -0.46049 | 6.023274 | 13.8232 | 0.000744 | 0.008119 |
| ENSG00000266412 | NCOA4 | -0.28083 | 8.322721 | 13.82049 | 0.000745 | 0.00812 |
| ENSG00000151239 | TWF1 | -0.36915 | 7.352212 | 13.83376 | 0.000764 | 0.008315 |
| ENSG00000100320 | RBFOX2 | -0.32468 | 7.318136 | 13.70718 | 0.000777 | 0.008432 |
| ENSG00000234745 | HLA-B | -0.3001 | 9.122528 | 13.77161 | 0.000782 | 0.008479 |
| ENSG00000112697 | TMEM30A | -0.30954 | 7.815677 | 13.5095 | 0.000837 | 0.008997 |
| ENSG00000173230 | GOLGB1 | -0.36382 | 7.022057 | 13.45948 | 0.000853 | 0.009143 |
| ENSG00000197712 | FAM114A1 | -0.35153 | 6.886529 | 13.42877 | 0.000863 | 0.009241 |
| ENSG00000213799 | ZNF845 | -0.6385 | 5.356497 | 13.48304 | 0.000871 | 0.009318 |
| ENSG00000102893 | PHKB | -0.31343 | 7.625175 | 13.35291 | 0.000888 | 0.009452 |
| ENSG00000122545 | SEPTIN7 | -0.28096 | 8.628534 | 13.352 | 0.000888 | 0.009452 |
| ENSG00000100934 | SEC23A | -0.29098 | 7.941371 | 13.34431 | 0.000891 | 0.009463 |
| ENSG00000008394 | MGST1 | -0.28903 | 8.018152 | 13.3198 | 0.000899 | 0.009524 |
| ENSG00000126787 | DLGAP5 | -0.33246 | 7.546789 | 13.28266 | 0.000912 | 0.00964 |
| ENSG00000164181 | ELOVL7 | -0.60428 | 5.410173 | 13.27365 | 0.000915 | 0.009656 |
| ENSG00000160752 | FOPS | -0.34335 | 6.903445 | 13.23818 | 0.000927 | 0.009769 |
| ENSG00000142864 | SERBP1 | -0.26756 | 8.775746 | 13.22941 | 0.00093 | 0.009793 |
| ENSG00000145860 | RNF145 | -0.60582 | 6.226798 | 14.91002 | 0.000937 | 0.009846 |
| ENSG00000172893 | DHCR7 | -0.41962 | 6.730951 | 13.22637 | 0.000955 | 0.010009 |
| ENSG00000134049 | IER3IP1 | -0.41418 | 6.25572 | 13.14471 | 0.000961 | 0.010062 |
| ENSG00000142541 | RPL13A | -0.35197 | 9.94117 | 14.6931 | 0.000961 | 0.010062 |
| ENSG00000143742 | SRP9 | -0.30163 | 9.295124 | 13.35832 | 0.000967 | 0.010101 |
| ENSG00000162433 | AK4 | -0.31585 | 7.762883 | 13.08549 | 0.000983 | 0.010222 |
| ENSG00000110958 | PTGES3 | -0.25644 | 9.123353 | 13.078 | 0.000986 | 0.010237 |
| ENSG00000143622 | RIT1 | -0.48194 | 5.802076 | 13.04064 | 0.001 | 0.010361 |
| ENSG00000135677 | GNS | -0.29124 | 8.067972 | 12.95121 | 0.001035 | 0.010687 |
| ENSG00000158769 | F11R | -0.33755 | 7.023442 | 12.94807 | 0.001036 | 0.010687 |
| ENSG00000185650 | ZFP36L1 | -0.27541 | 9.005407 | 12.94553 | 0.001037 | 0.010687 |
| ENSG00000005893 | LAMP2 | -0.28581 | 8.770942 | 12.93265 | 0.001042 | 0.010728 |
| ENSG00000166224 | SGPL1 | -0.32187 | 7.290042 | 12.92677 | 0.001044 | 0.010735 |
| ENSG00000110492 | MDK | -0.36146 | 7.120474 | 12.91711 | 0.001048 | 0.010765 |
| ENSG00000159388 | BTG2 | -0.45514 | 6.002002 | 12.89727 | 0.001056 | 0.010837 |
| ENSG00000117335 | CD46 | -0.24991 | 9.38733 | 12.89488 | 0.001057 | 0.010838 |
| ENSG00000030582 | GRN | -0.26869 | 9.332016 | 12.85206 | 0.001075 | 0.010997 |
| ENSG00000135269 | TES | -0.31716 | 8.083936 | 12.90199 | 0.001081 | 0.011054 |
| ENSG00000075303 | SLC25A40 | -0.48659 | 5.801786 | 12.82581 | 0.001085 | 0.011079 |
| ENSG00000127483 | HP1BP3 | -0.3009 | 8.143539 | 12.82745 | 0.001085 | 0.011079 |
| ENSG00000133935 | ERG28 | -0.39349 | 6.587038 | 12.80215 | 0.001095 | 0.01116 |
| ENSG00000132424 | PNISR | -0.44438 | 6.210642 | 12.76382 | 0.001112 | 0.011286 |
| ENSG00000101843 | PSMD10 | -0.33177 | 7.191011 | 12.69573 | 0.001141 | 0.011504 |
| ENSG00000119986 | AVPI1 | -0.37422 | 6.935993 | 12.68924 | 0.001144 | 0.011513 |
| ENSG00000198898 | CAPZA2 | -0.3087 | 7.805567 | 12.6579 | 0.001158 | 0.011642 |
| ENSG00000000003 | TSPAN6 | -0.4264 | 6.244937 | 12.62269 | 0.001174 | 0.011791 |
| ENSG00000050130 | JKAMP | -0.30241 | 7.577437 | 12.59581 | 0.001186 | 0.011893 |
| ENSG00000137770 | CTDSPL2 | -0.35454 | 7.076438 | 12.58801 | 0.00119 | 0.011919 |
| ENSG00000092621 | PHGDH | -0.26426 | 8.603633 | 12.55367 | 0.001205 | 0.012007 |
| ENSG00000108061 | SHOC2 | -0.37745 | 6.656631 | 12.55327 | 0.001206 | 0.012007 |
| ENSG00000255302 | EID1 | -0.3685 | 6.706291 | 12.51817 | 0.001222 | 0.012157 |
| ENSG00000114978 | MOB1A | -0.25603 | 9.341349 | 12.50152 | 0.00123 | 0.012207 |
| ENSG00000184432 | COPB2 | -0.26706 | 8.785939 | 12.46234 | 0.001249 | 0.012351 |
| ENSG00000115380 | EFEMP1 | -0.281 | 8.20961 | 12.45827 | 0.001251 | 0.01236 |
| ENSG00000224892 | NA | -0.87124 | 3.925212 | 12.456 | 0.001252 | 0.01236 |
| ENSG00000091527 | CDV3 | -0.4177 | 6.214981 | 12.40971 | 0.001274 | 0.012562 |
| ENSG00000082258 | CCNT2 | -0.44531 | 6.225663 | 12.35339 | 0.001303 | 0.012776 |
| ENSG00000029363 | BCLAF1 | -0.30726 | 7.784646 | 12.32509 | 0.001317 | 0.012894 |
| ENSG00000117724 | CENPF | -0.25589 | 9.351653 | 12.31996 | 0.00132 | 0.012909 |
| ENSG00000204525 | HLA-C | -0.37093 | 7.413951 | 12.65482 | 0.001324 | 0.012937 |
| ENSG00000086598 | TMED2 | -0.27473 | 9.239709 | 12.27801 | 0.001341 | 0.013088 |
| ENSG00000168036 | CTNNB1 | -0.25075 | 9.627143 | 12.26297 | 0.001349 | 0.013153 |
| ENSG00000121236 | TRIM6 | -0.71599 | 4.463622 | 12.25156 | 0.001355 | 0.013183 |
| ENSG00000134996 | OSTF1 | -0.41936 | 6.309136 | 12.25067 | 0.001356 | 0.013183 |
| ENSG00000127125 | PPCS | -0.3877 | 6.520828 | 12.24643 | 0.001358 | 0.013194 |
| ENSG00000179750 | APOBEC3B | -0.42605 | 6.091157 | 12.19951 | 0.001383 | 0.013426 |
| ENSG00000100612 | DHRS7 | -0.32956 | 7.109442 | 12.17975 | 0.001394 | 0.013484 |
| ENSG00000100603 | SNW1 | -0.29852 | 7.485675 | 12.17296 | 0.001397 | 0.013509 |
| ENSG00000138735 | PDE5A | -0.37408 | 6.568716 | 12.16911 | 0.0014 | 0.013518 |
| ENSG00000054598 | FOXC1 | -0.76023 | 4.319722 | 12.16693 | 0.001401 | 0.013518 |
| ENSG00000075426 | FOSL2 | -0.53609 | 5.588413 | 12.09455 | 0.001441 | 0.013859 |
| ENSG00000215717 | TMEM167B | -0.43786 | 5.934422 | 12.09473 | 0.001441 | 0.013859 |
| ENSG00000116171 | SCP2 | -0.28186 | 7.696869 | 12.03658 | 0.001474 | 0.014118 |
| ENSG00000155304 | HSPA13 | -0.35952 | 6.9302 | 12.00952 | 0.00149 | 0.014245 |
| ENSG00000139163 | ETNK1 | -0.3606 | 6.552135 | 11.9647 | 0.001516 | 0.014462 |
| ENSG00000148700 | ADD3 | -0.52658 | 5.419494 | 11.91834 | 0.001544 | 0.014641 |
| ENSG00000283041 | NA | -0.34864 | 7.101585 | 11.90854 | 0.00155 | 0.014661 |
| ENSG00000092010 | PSME1 | -0.25454 | 8.928865 | 11.88611 | 0.001564 | 0.014769 |
| ENSG00000101911 | PRPS2 | -0.29206 | 7.74038 | 11.85419 | 0.001584 | 0.01494 |
| ENSG00000173267 | SNCG | -0.38903 | 6.338585 | 11.84335 | 0.00159 | 0.014982 |
| ENSG00000168710 | AHCYL1 | -0.53786 | 5.390666 | 11.83112 | 0.001598 | 0.015042 |
| ENSG00000145287 | PLAC8 | -0.38987 | 6.384618 | 11.80935 | 0.001612 | 0.015124 |
| ENSG00000171862 | PTEN | -0.53207 | 5.674146 | 11.79445 | 0.001623 | 0.015206 |
| ENSG00000198034 | RPS4X | -0.28662 | 9.976095 | 12.04789 | 0.001641 | 0.015342 |
| ENSG00000163466 | ARPC2 | -0.23253 | 10.1179 | 11.73242 | 0.001661 | 0.0155 |
| ENSG00000184445 | KNTC1 | -0.30111 | 7.219685 | 11.73323 | 0.001661 | 0.0155 |
| ENSG00000083307 | GRHL2 | -0.29246 | 7.717664 | 11.68285 | 0.001694 | 0.015772 |
| ENSG00000159176 | CSRP1 | -0.28226 | 7.938116 | 11.63857 | 0.001724 | 0.016009 |
| ENSG00000046604 | DSG2 | -0.25644 | 8.341973 | 11.56701 | 0.001774 | 0.016377 |
| ENSG00000171903 | CYP4F11 | -0.57179 | 5.257476 | 11.57287 | 0.00177 | 0.016377 |
| ENSG00000153214 | TMEM87B | -0.33868 | 6.764465 | 11.56251 | 0.001777 | 0.016393 |
| ENSG00000100504 | PYGL | -0.48254 | 5.659073 | 11.49886 | 0.001823 | 0.016746 |
| ENSG00000117984 | CTSD | -0.3389 | 8.367805 | 12.29236 | 0.001827 | 0.01677 |
| ENSG00000166681 | BEX3 | -0.28288 | 8.675676 | 11.52207 | 0.001845 | 0.016922 |
| ENSG00000165887 | ANKRD2 | -0.35029 | 6.625442 | 11.44653 | 0.001861 | 0.01703 |
| ENSG00000075420 | FNDC3B | -0.34303 | 7.182994 | 11.45339 | 0.001866 | 0.017059 |
| ENSG00000135862 | LAMC1 | -0.31683 | 8.73692 | 11.98632 | 0.001877 | 0.017132 |
| ENSG00000181789 | COPG1 | -0.25172 | 8.93111 | 11.41766 | 0.001883 | 0.017152 |
| ENSG00000198408 | OGA | -0.52786 | 5.748375 | 11.56141 | 0.001883 | 0.017152 |
| ENSG00000197766 | CFD | -0.47954 | 5.580851 | 11.40304 | 0.001894 | 0.017222 |
| ENSG00000179912 | R3HDM2 | -0.7057 | 4.46635 | 11.39106 | 0.001903 | 0.017287 |
| ENSG00000151092 | NGLY1 | -0.41891 | 6.058086 | 11.38099 | 0.00191 | 0.017343 |
| ENSG00000167754 | KLK5 | -0.27152 | 8.160626 | 11.35176 | 0.001933 | 0.017478 |
| ENSG00000035499 | DEPDC1B | -0.3706 | 6.76949 | 11.32965 | 0.00195 | 0.017567 |
| ENSG00000162704 | ARPC5 | -0.27288 | 8.826211 | 11.33226 | 0.00195 | 0.017567 |
| ENSG00000114120 | SLC25A36 | -0.34093 | 6.794438 | 11.32421 | 0.001954 | 0.017574 |
| ENSG00000018408 | WWTR1 | -0.4839 | 5.684232 | 11.29574 | 0.001976 | 0.01772 |
| ENSG00000000419 | DPM1 | -0.37869 | 6.513583 | 11.28893 | 0.001982 | 0.017755 |
| ENSG00000124343 | XG | -0.62402 | 4.810959 | 11.28266 | 0.001987 | 0.017758 |
| ENSG00000124343 | XGY2 | -0.62402 | 4.810959 | 11.28266 | 0.001987 | 0.017758 |
| ENSG00000103978 | TMEM87A | -0.34788 | 6.889809 | 11.26161 | 0.002004 | 0.017865 |
| ENSG00000117155 | SSX2IP | -0.30658 | 7.266015 | 11.25986 | 0.002005 | 0.017865 |
| ENSG00000101972 | STAG2 | -0.2825 | 7.532714 | 11.25061 | 0.002012 | 0.017903 |
| ENSG00000071189 | SNX13 | -0.37034 | 6.773733 | 11.23613 | 0.002024 | 0.017994 |
| ENSG00000113558 | SKP1 | -0.28465 | 8.118561 | 11.19902 | 0.002054 | 0.018207 |
| ENSG00000171159 | C9orf16 | -0.44981 | 6.045813 | 11.1694 | 0.002079 | 0.018396 |
| ENSG00000172296 | SPTLC3 | -0.55765 | 5.117594 | 11.14513 | 0.002099 | 0.018548 |
| ENSG00000122042 | UBL3 | -0.32727 | 6.844015 | 11.13981 | 0.002104 | 0.018573 |
| ENSG00000204642 | HLA-F | -0.48574 | 5.555378 | 11.09009 | 0.002146 | 0.018919 |
| ENSG00000197056 | ZMYM1 | -0.45675 | 5.726281 | 11.08659 | 0.002149 | 0.018931 |
| ENSG00000176046 | NUPR1 | -0.62739 | 4.833182 | 11.0837 | 0.002152 | 0.018939 |
| ENSG00000120756 | PLS1 | -0.37036 | 6.386263 | 11.0636 | 0.002169 | 0.019064 |
| ENSG00000120805 | ARL1 | -0.2895 | 7.319834 | 11.00483 | 0.002221 | 0.019402 |
| ENSG00000131966 | ACTR10 | -0.30191 | 7.395992 | 11.00627 | 0.00222 | 0.019402 |
| ENSG00000164930 | FZD6 | -0.26949 | 8.100149 | 10.97894 | 0.002245 | 0.019546 |
| ENSG00000196975 | ANXA4 | -0.3469 | 6.765715 | 10.92879 | 0.002291 | 0.019886 |
| ENSG00000115365 | LANCL1 | -0.33868 | 6.834973 | 10.90935 | 0.002309 | 0.020013 |
| ENSG00000118705 | RPN2 | -0.23069 | 9.949533 | 10.90179 | 0.002316 | 0.020044 |
| ENSG00000144747 | TMF1 | -0.39227 | 6.320766 | 10.89826 | 0.002319 | 0.020057 |
| ENSG00000182827 | ACBD3 | -0.52375 | 5.349111 | 10.89617 | 0.002321 | 0.020059 |
| ENSG00000115392 | FANCL | -0.46645 | 5.633518 | 10.85743 | 0.002358 | 0.020285 |
| ENSG00000120727 | PAIP2 | -0.42288 | 6.034622 | 10.85896 | 0.002356 | 0.020285 |
| ENSG00000114480 | GBE1 | -0.47976 | 5.725416 | 10.84697 | 0.002368 | 0.020355 |
| ENSG00000117859 | OSBPL9 | -0.28213 | 8.209017 | 10.83501 | 0.002392 | 0.020531 |
| ENSG00000005020 | SKAP2 | -0.42944 | 5.865678 | 10.75154 | 0.002461 | 0.021064 |
| ENSG00000089157 | RPLP0 | -0.22866 | 10.15316 | 10.74733 | 0.002465 | 0.021069 |
| ENSG00000170540 | ARL6IP1 | -0.26359 | 9.332655 | 10.80095 | 0.002464 | 0.021069 |
| ENSG00000056586 | RC3H2 | -0.51012 | 5.611919 | 10.74548 | 0.002467 | 0.021069 |
| ENSG00000128708 | HAT1 | -0.26607 | 8.034197 | 10.73277 | 0.00248 | 0.021163 |
| ENSG00000235109 | ZSCAN31 | -0.5232 | 5.242785 | 10.71677 | 0.002496 | 0.021269 |
| ENSG00000112984 | KIF20A | -0.24308 | 8.496206 | 10.67906 | 0.002535 | 0.021534 |
| ENSG00000137868 | STRA6 | -0.49732 | 5.317866 | 10.67246 | 0.002542 | 0.021576 |
| ENSG00000134602 | STK26 | -0.39285 | 6.153445 | 10.6556 | 0.002559 | 0.021709 |
| ENSG00000011405 | PIK3C2A | -0.33253 | 7.106922 | 10.6296 | 0.002586 | 0.021908 |
| ENSG00000085365 | SCAMP1 | -0.39303 | 6.539322 | 10.62983 | 0.002615 | 0.022055 |
| ENSG00000204386 | NEU1 | -0.30577 | 7.095352 | 10.59952 | 0.002618 | 0.022055 |
| ENSG00000102054 | RBBP7 | -0.23549 | 9.457194 | 10.5845 | 0.002634 | 0.022135 |
| ENSG00000124795 | DEK | -0.24242 | 9.013923 | 10.5743 | 0.002645 | 0.022211 |
| ENSG00000137942 | FNBP1L | -0.27255 | 7.611108 | 10.56619 | 0.002654 | 0.022269 |
| ENSG00000138674 | SEC31A | -0.25711 | 7.92361 | 10.5333 | 0.00269 | 0.022537 |
| ENSG00000197329 | PELI1 | -0.36147 | 6.733951 | 10.52611 | 0.002698 | 0.022587 |
| ENSG00000010704 | HFE | -0.39225 | 6.135131 | 10.5049 | 0.002721 | 0.022727 |
| ENSG00000158315 | RHBDL2 | -0.54522 | 5.133727 | 10.49693 | 0.00273 | 0.022759 |
| ENSG00000015475 | BID | -0.36133 | 6.998837 | 10.59413 | 0.00279 | 0.023221 |
| ENSG00000074695 | LMAN1 | -0.24118 | 8.450348 | 10.4317 | 0.002804 | 0.023324 |
| ENSG00000137509 | PRCP | -0.28879 | 7.426542 | 10.41169 | 0.002827 | 0.023466 |
| ENSG00000091640 | SPAG7 | -0.33749 | 6.622451 | 10.40677 | 0.002833 | 0.023496 |
| ENSG00000111328 | CDK2AP1 | -0.48443 | 5.636567 | 10.40165 | 0.002839 | 0.023514 |
| ENSG00000109184 | DCUN1D4 | -0.3163 | 7.274809 | 10.39079 | 0.002852 | 0.023583 |
| ENSG00000181885 | CLDN7 | -0.28266 | 8.201284 | 10.41661 | 0.002905 | 0.023945 |
| ENSG00000070831 | CDC42 | -0.25286 | 8.432163 | 10.33172 | 0.002922 | 0.024002 |
| ENSG00000177888 | ZBTB41 | -0.42779 | 6.18695 | 10.32528 | 0.00296 | 0.024237 |
| ENSG00000091136 | LAMB1 | -0.22446 | 9.250814 | 10.29091 | 0.002971 | 0.024294 |
| ENSG00000138698 | RAP1GDS1 | -0.32394 | 7.058798 | 10.29066 | 0.002972 | 0.024294 |
| ENSG00000103485 | QPRT | -0.46548 | 5.881373 | 10.28594 | 0.002977 | 0.024324 |
| ENSG00000165943 | MOAP1 | -0.56687 | 5.003368 | 10.25346 | 0.003017 | 0.024574 |
| ENSG00000049245 | VAMP3 | -0.30319 | 7.018251 | 10.23037 | 0.003046 | 0.024764 |
| ENSG00000144224 | UBXN4 | -0.2818 | 7.552247 | 10.23153 | 0.003045 | 0.024764 |
| ENSG00000126432 | PRDX5 | -0.24341 | 8.649911 | 10.22705 | 0.00305 | 0.024781 |
| ENSG00000165476 | REEP3 | -0.3958 | 6.173245 | 10.20594 | 0.003077 | 0.024962 |
| ENSG00000179454 | KLHL28 | -0.48163 | 5.330683 | 10.17859 | 0.003112 | 0.02521 |
| ENSG00000104904 | OAZ1 | -0.23934 | 8.939808 | 10.14969 | 0.003149 | 0.025441 |
| ENSG00000072042 | RDH11 | -0.32531 | 6.986183 | 10.13173 | 0.003173 | 0.025595 |
| ENSG00000115966 | ATF2 | -0.39539 | 6.301384 | 10.12728 | 0.003179 | 0.025606 |
| ENSG00000163683 | SMIM14 | -0.6448 | 4.538652 | 10.1289 | 0.003177 | 0.025606 |
| ENSG00000116679 | IVNS1ABP | -0.24199 | 8.670092 | 10.09421 | 0.003223 | 0.025905 |
| ENSG00000112343 | TRIM38 | -0.44754 | 5.695633 | 10.07599 | 0.003247 | 0.026046 |
| ENSG00000101846 | STS | -0.35062 | 6.525858 | 10.07087 | 0.003254 | 0.026064 |
| ENSG00000003096 | KLHL13 | -0.41069 | 6.089152 | 10.05331 | 0.003278 | 0.026165 |
| ENSG00000184661 | CDCA2 | -0.25962 | 8.016294 | 10.03544 | 0.003302 | 0.026341 |
| ENSG00000153561 | RMND5A | -0.41339 | 5.807213 | 10.01803 | 0.003326 | 0.02644 |
| ENSG00000069329 | VPS35 | -0.23256 | 8.728864 | 10.0156 | 0.003329 | 0.026449 |
| ENSG00000100316 | RPL3 | -0.23326 | 10.37983 | 9.989665 | 0.003365 | 0.026698 |
| ENSG00000141232 | TOB1 | -0.44373 | 5.821259 | 9.975056 | 0.003386 | 0.026824 |
| ENSG00000178802 | MPI | -0.38923 | 6.16629 | 9.968015 | 0.003396 | 0.026865 |
| ENSG00000162896 | PIGR | -0.56633 | 5.012342 | 9.952613 | 0.003418 | 0.027001 |
| ENSG00000108946 | PRKAR1A | -0.23061 | 9.036359 | 9.936493 | 0.003441 | 0.027145 |
| ENSG00000178966 | RMI1 | -0.44636 | 5.704332 | 9.93084 | 0.003449 | 0.02719 |
| ENSG00000175390 | EIF3F | -0.2712 | 8.022268 | 9.916394 | 0.00347 | 0.027277 |
| ENSG00000182220 | ATP6AP2 | -0.26465 | 8.378961 | 9.910373 | 0.003488 | 0.027351 |
| ENSG00000137872 | SEMA6D | -0.73314 | 4.174071 | 9.882704 | 0.003519 | 0.027533 |
| ENSG00000186806 | VSIG10L | -1.38337 | 2.407591 | 9.883854 | 0.003517 | 0.027533 |
| ENSG00000140941 | MAP1LC3B | -0.27327 | 7.293535 | 9.854425 | 0.00356 | 0.027803 |
| ENSG00000146409 | SLC18B1 | -0.57625 | 4.662924 | 9.854919 | 0.00356 | 0.027803 |
| ENSG00000164104 | HMGB2 | -0.22908 | 8.867906 | 9.835262 | 0.003589 | 0.028007 |
| ENSG00000150459 | SAP18 | -0.28131 | 7.643409 | 9.830353 | 0.003596 | 0.028027 |
| ENSG00000149418 | ST14 | -0.51025 | 5.376699 | 9.822266 | 0.003608 | 0.028103 |
| ENSG00000102580 | DNAJC3 | -0.28734 | 7.379253 | 9.810371 | 0.003626 | 0.028223 |
| ENSG00000055917 | PUM2 | -0.32315 | 6.622823 | 9.797142 | 0.003646 | 0.028361 |
| ENSG00000112742 | TTK | -0.2782 | 7.330925 | 9.784217 | 0.003666 | 0.028429 |
| ENSG00000116350 | SRSF4 | -0.28836 | 7.286853 | 9.783257 | 0.003668 | 0.028429 |
| ENSG00000134882 | UBAC2 | -0.33676 | 6.640934 | 9.774279 | 0.003681 | 0.02846 |
| ENSG00000085224 | ATRX | -0.28275 | 7.149886 | 9.735007 | 0.003742 | 0.028834 |
| ENSG00000119392 | GLE1 | -0.30473 | 7.258655 | 9.737748 | 0.003738 | 0.028834 |
| ENSG00000119787 | ATL2 | -0.31132 | 6.736962 | 9.736827 | 0.00374 | 0.028834 |
| ENSG00000165806 | CASP7 | -0.34003 | 6.741917 | 9.730232 | 0.00375 | 0.028873 |
| ENSG00000138085 | ATRAID | -0.26258 | 7.788332 | 9.714143 | 0.003775 | 0.02901 |
| ENSG00000115216 | NRBP1 | -0.29466 | 7.092295 | 9.688833 | 0.003816 | 0.02928 |
| ENSG00000153113 | CAST | -0.26012 | 7.529481 | 9.680143 | 0.003829 | 0.029347 |
| ENSG00000010256 | UQCRC1 | -0.21591 | 9.792916 | 9.661916 | 0.003859 | 0.029514 |
| ENSG00000103507 | BCKDK | -0.25552 | 7.790235 | 9.649536 | 0.003879 | 0.029628 |
| ENSG00000119048 | UBE2B | -0.39294 | 5.972813 | 9.644443 | 0.003887 | 0.029672 |
| ENSG00000130309 | COLGALT1 | -0.29012 | 7.505017 | 9.638448 | 0.003897 | 0.029727 |
| ENSG00000169752 | NRG4 | -0.77378 | 3.923526 | 9.615922 | 0.003934 | 0.02997 |
| ENSG00000085433 | WDR47 | -0.39475 | 6.16516 | 9.557092 | 0.004033 | 0.030619 |
| ENSG00000165280 | VCP | -0.21509 | 9.755609 | 9.522469 | 0.004092 | 0.030987 |
| ENSG00000130741 | EIF2S3 | -0.21407 | 9.221515 | 9.509961 | 0.004113 | 0.031092 |
| ENSG00000156052 | GNAQ | -0.46124 | 5.568388 | 9.509771 | 0.004114 | 0.031092 |
| ENSG00000136560 | TANK | -0.304 | 6.952799 | 9.4928 | 0.004143 | 0.031274 |
| ENSG00000165304 | MELK | -0.29254 | 8.133266 | 9.691856 | 0.004194 | 0.031593 |
| ENSG00000169504 | CLIC4 | -0.28407 | 7.343262 | 9.449366 | 0.00422 | 0.031748 |
| ENSG00000081154 | PCNP | -0.31527 | 6.85364 | 9.442348 | 0.004233 | 0.031801 |
| ENSG00000141380 | SS18 | -0.23494 | 8.336344 | 9.423737 | 0.004266 | 0.03201 |
| ENSG00000167842 | MIS12 | -0.45567 | 5.537194 | 9.409038 | 0.004293 | 0.032126 |
| ENSG00000121940 | CLCC1 | -0.36702 | 6.092729 | 9.406556 | 0.004297 | 0.032138 |
| ENSG00000090863 | GLG1 | -0.27474 | 7.762109 | 9.401852 | 0.004306 | 0.032161 |
| ENSG00000129515 | SNX6 | -0.29438 | 7.39594 | 9.397781 | 0.004313 | 0.032195 |
| ENSG00000038002 | AGA | -0.53581 | 4.993475 | 9.390778 | 0.004326 | 0.03227 |
| ENSG00000164329 | TENT2 | -0.32512 | 6.927129 | 9.374351 | 0.004356 | 0.032453 |
| ENSG00000124486 | USP9X | -0.3112 | 7.173352 | 9.375761 | 0.004372 | 0.032527 |
| ENSG00000139324 | TMTC3 | -0.29058 | 7.153747 | 9.335953 | 0.004427 | 0.032881 |
| ENSG00000177879 | AP3S1 | -0.35834 | 6.572487 | 9.335736 | 0.004428 | 0.032881 |
| ENSG00000134533 | RERG | -0.50886 | 5.365234 | 9.318493 | 0.00446 | 0.033094 |
| ENSG00000165502 | RPL36AL | -0.2712 | 7.488381 | 9.317465 | 0.004462 | 0.033094 |
| ENSG00000097033 | SH3GLB1 | -0.29581 | 7.030741 | 9.315189 | 0.004467 | 0.033105 |
| ENSG00000148660 | CAMK2G | -0.3597 | 6.322669 | 9.293514 | 0.004508 | 0.033389 |
| ENSG00000096696 | DSP | -0.2448 | 7.911735 | 9.288412 | 0.004518 | 0.033419 |
| ENSG00000122218 | COPA | -0.2347 | 8.851346 | 9.26578 | 0.004561 | 0.033698 |
| ENSG00000185551 | NR2F2 | -0.30455 | 6.718275 | 9.249622 | 0.004593 | 0.033887 |
| ENSG00000125430 | HS3ST3B1 | -0.33502 | 6.603451 | 9.224616 | 0.004642 | 0.034183 |
| ENSG00000165416 | SUGT1 | -0.30633 | 6.994908 | 9.222219 | 0.004646 | 0.034196 |
| ENSG00000083093 | PALB2 | -0.44572 | 5.866688 | 9.256117 | 0.00465 | 0.034203 |
| ENSG00000102243 | VGLL1 | -0.26575 | 7.669443 | 9.212082 | 0.004667 | 0.034278 |
| ENSG00000085719 | CPNE3 | -0.23875 | 8.243 | 9.172302 | 0.004746 | 0.03473 |
| ENSG00000126945 | HNRNPH2 | -0.24472 | 7.943366 | 9.144079 | 0.004804 | 0.03509 |
| ENSG00000133318 | RTN3 | -0.28245 | 7.005092 | 9.07069 | 0.004957 | 0.036108 |
| ENSG00000106484 | MEST | -0.25648 | 7.737958 | 9.027095 | 0.00505 | 0.036719 |
| ENSG00000130595 | TNNT3 | -1.26617 | 2.392634 | 9.027008 | 0.00505 | 0.036719 |
| ENSG00000145687 | SSBP2 | -0.36887 | 6.049236 | 9.023121 | 0.005058 | 0.036734 |
| ENSG00000091542 | ALKBH5 | -0.63948 | 4.70999 | 9.025044 | 0.00507 | 0.036767 |
| ENSG00000132356 | PRKAA1 | -0.29407 | 7.350971 | 9.007091 | 0.005109 | 0.036985 |
| ENSG00000095906 | NUBP2 | -0.27952 | 7.261187 | 8.962798 | 0.005191 | 0.037471 |
| ENSG00000108039 | XPNPEP1 | -0.29764 | 6.901749 | 8.954495 | 0.005209 | 0.037535 |
| ENSG00000124688 | MAD2L1BP | -0.36216 | 6.11976 | 8.949553 | 0.00522 | 0.037578 |
| ENSG00000143158 | MPC2 | -0.39576 | 5.838944 | 8.94291 | 0.005235 | 0.037662 |
| ENSG00000187840 | EIF4EBP1 | -0.32376 | 7.132784 | 9.054195 | 0.005239 | 0.037662 |
| ENSG00000115221 | ITGB6 | -0.54827 | 5.057784 | 8.93369 | 0.005256 | 0.037741 |
| ENSG00000135108 | FBXO21 | -0.25833 | 7.429561 | 8.929588 | 0.005265 | 0.03776 |
| ENSG00000164190 | NIPBL | -0.31924 | 6.848852 | 8.930643 | 0.005263 | 0.03776 |
| ENSG00000062194 | GPBP1 | -0.28365 | 7.087528 | 8.919184 | 0.005289 | 0.037882 |
| ENSG00000083312 | TNPO1 | -0.21326 | 8.900619 | 8.892645 | 0.00535 | 0.038174 |
| ENSG00000074201 | CLNS1A | -0.24485 | 7.885811 | 8.843761 | 0.005463 | 0.038828 |
| ENSG00000100281 | HMGXB4 | -0.31656 | 6.641544 | 8.843954 | 0.005463 | 0.038828 |
| ENSG00000141424 | SLC39A6 | -0.24548 | 7.798877 | 8.84714 | 0.005455 | 0.038828 |
| ENSG00000153130 | SCOC | -0.32844 | 7.08555 | 8.957338 | 0.005459 | 0.038828 |
| ENSG00000244038 | DDOST | -0.20605 | 9.760961 | 8.833793 | 0.005487 | 0.038935 |
| ENSG00000123562 | MORF4L2 | -0.22215 | 9.005897 | 8.827942 | 0.005501 | 0.039009 |
| ENSG00000197713 | RPE | -0.26902 | 7.332427 | 8.812211 | 0.005538 | 0.03925 |
| ENSG00000184743 | ATL3 | -0.23804 | 8.055773 | 8.809221 | 0.005545 | 0.039277 |
| ENSG00000129810 | SGO1 | -0.49646 | 5.482748 | 8.849647 | 0.005575 | 0.039439 |
| ENSG00000137563 | GGH | -0.27993 | 7.455765 | 8.782358 | 0.00561 | 0.039612 |
| ENSG00000019186 | CYP24A1 | -0.23235 | 8.600432 | 8.749439 | 0.00569 | 0.04013 |
| ENSG00000172992 | DCAKD | -0.37426 | 6.026522 | 8.734311 | 0.005727 | 0.040319 |
| ENSG00000178078 | STAP2 | -0.3267 | 6.595421 | 8.713736 | 0.005778 | 0.040615 |
| ENSG00000113732 | ATP6V0E1 | -0.23588 | 7.849418 | 8.709827 | 0.005788 | 0.040623 |
| ENSG00000166598 | HSP90B1 | -0.19928 | 10.1923 | 8.701579 | 0.005809 | 0.040719 |
| ENSG00000134294 | SLC38A2 | -0.21837 | 9.428574 | 8.698583 | 0.005816 | 0.040747 |
| ENSG00000182952 | HMGN4 | -0.31283 | 6.723656 | 8.6961 | 0.005823 | 0.040766 |
| ENSG00000213625 | LEPROT | -0.26163 | 7.565688 | 8.668342 | 0.005893 | 0.041108 |
| ENSG00000111530 | CAND1 | -0.22725 | 8.264574 | 8.661655 | 0.00591 | 0.041177 |
| ENSG00000100711 | ZFYVE21 | -0.35406 | 6.095112 | 8.654959 | 0.005927 | 0.041247 |
| ENSG00000105141 | CASP14 | -0.57605 | 4.59933 | 8.627154 | 0.005999 | 0.041685 |
| ENSG00000170242 | USP47 | -0.25195 | 7.546471 | 8.602354 | 0.006064 | 0.042062 |
| ENSG00000139218 | SCAF11 | -0.25334 | 7.931816 | 8.573076 | 0.006142 | 0.042507 |
| ENSG00000117632 | STMN1 | -0.21619 | 9.763908 | 8.550235 | 0.006203 | 0.042879 |
| ENSG00000110367 | DDX6 | -0.25945 | 7.403746 | 8.542505 | 0.006224 | 0.042997 |
| ENSG00000139644 | TMBIM6 | -0.19989 | 10.09635 | 8.538029 | 0.006236 | 0.043039 |
| ENSG00000102804 | TSC22D1 | -0.38003 | 6.111256 | 8.531901 | 0.006252 | 0.043063 |
| ENSG00000111911 | HINT3 | -0.91909 | 3.485809 | 8.529335 | 0.006259 | 0.043063 |
| ENSG00000132581 | SDF2 | -0.34058 | 6.480226 | 8.531073 | 0.006255 | 0.043063 |
| ENSG00000160446 | ZDHHC12 | -0.33761 | 6.436415 | 8.515676 | 0.006297 | 0.043191 |
| ENSG00000113811 | SELENOK | -0.38832 | 5.865087 | 8.487715 | 0.006374 | 0.043642 |
| ENSG00000167397 | VKORC1 | -0.40582 | 5.685924 | 8.486257 | 0.006378 | 0.043643 |
| ENSG00000023572 | GLRX2 | -0.45004 | 5.294626 | 8.469477 | 0.006425 | 0.043885 |
| ENSG00000131747 | TOP2A | -0.20052 | 10.14926 | 8.463272 | 0.006442 | 0.043952 |
| ENSG00000009844 | VTA1 | -0.30105 | 6.977167 | 8.455802 | 0.006463 | 0.044069 |
| ENSG00000107897 | ACBD5 | -0.3686 | 6.252236 | 8.453412 | 0.00647 | 0.044089 |
| ENSG00000172380 | GNG12 | -0.22684 | 8.891228 | 8.446091 | 0.00649 | 0.044204 |
| ENSG00000197415 | VEPH1 | -0.40208 | 5.738237 | 8.43854 | 0.006512 | 0.044297 |
| ENSG00000135842 | NIBAN1 | -0.29588 | 6.870526 | 8.414281 | 0.006581 | 0.044663 |
| ENSG00000128595 | CALU | -0.20679 | 9.632399 | 8.398889 | 0.006626 | 0.044876 |
| ENSG00000150991 | UBC | -0.22814 | 9.616161 | 8.420938 | 0.006628 | 0.044876 |
| ENSG00000189266 | PNRC2 | -0.2468 | 7.706119 | 8.390673 | 0.006649 | 0.044993 |
| ENSG00000084234 | APLP2 | -0.20344 | 9.319522 | 8.380263 | 0.00668 | 0.045145 |
| ENSG00000180304 | OAZ2 | -0.24633 | 7.792062 | 8.374528 | 0.006697 | 0.045232 |
| ENSG00000178035 | IMPDH2 | -0.22296 | 8.655718 | 8.364406 | 0.006726 | 0.045406 |
| ENSG00000176909 | MAMSTR | -1.14727 | 2.423625 | 8.344167 | 0.006786 | 0.045756 |
| ENSG00000021355 | SERPINB1 | -0.42707 | 5.486904 | 8.338768 | 0.006802 | 0.04581 |
| ENSG00000254999 | BRK1 | -0.23415 | 7.738817 | 8.333438 | 0.006818 | 0.045891 |
| ENSG00000153914 | SREK1 | -0.30948 | 6.934054 | 8.343805 | 0.006824 | 0.045904 |
| ENSG00000187109 | NAP1L1 | -0.23085 | 7.999512 | 8.319352 | 0.00686 | 0.046094 |
| ENSG00000168938 | PPIC | -0.24957 | 7.608262 | 8.313601 | 0.006877 | 0.046156 |
| ENSG00000198160 | MIER1 | -0.32273 | 6.728574 | 8.312188 | 0.006883 | 0.046165 |
| ENSG00000131844 | MCCC2 | -0.23797 | 7.632181 | 8.287119 | 0.006958 | 0.046586 |
| ENSG00000177854 | TMEM187 | -0.62315 | 4.332507 | 8.255052 | 0.007056 | 0.047101 |
| ENSG00000179889 | PDXDC1 | -0.23665 | 7.749177 | 8.254106 | 0.007059 | 0.047101 |
| ENSG00000179889 | LOC102724985 | -0.23665 | 7.749177 | 8.254106 | 0.007059 | 0.047101 |
| ENSG00000022277 | RTF2 | -0.29831 | 6.750915 | 8.215205 | 0.007181 | 0.047802 |
| ENSG00000117592 | PRDX6 | -0.22587 | 8.281357 | 8.208637 | 0.007202 | 0.047885 |
| ENSG00000048028 | USP28 | -0.33306 | 6.255504 | 8.194003 | 0.007248 | 0.048166 |
| ENSG00000245571 | FAM111A-DT | -0.49361 | 4.89525 | 8.168868 | 0.007329 | 0.048617 |
| ENSG00000255529 | POLR2M | -0.36742 | 5.969014 | 8.147608 | 0.007398 | 0.048934 |
| ENSG00000167552 | TUBA1A | -0.79704 | 3.676235 | 8.144885 | 0.007407 | 0.048964 |
| ENSG00000106392 | C1GALT1 | -0.358 | 6.037433 | 8.133031 | 0.007445 | 0.049115 |
| ENSG00000079332 | SAR1A | -0.20624 | 8.625344 | 8.117328 | 0.007497 | 0.04942 |
| ENSG00000101474 | APMAP | -0.2126 | 8.245078 | 8.112924 | 0.007512 | 0.04946 |
| ENSG00000196305 | IARS1 | -0.19629 | 9.894191 | 8.113976 | 0.007508 | 0.04946 |
| ENSG00000213290 | NA | -0.54987 | 4.803076 | 8.108088 | 0.007528 | 0.049537 |
| ENSG00000244754 | N4BP2L2 | -0.25067 | 7.378472 | 8.095762 | 0.007569 | 0.049779 |
| ENSG00000180957 | PITPNB | -0.23758 | 8.065741 | 8.090439 | 0.007587 | 0.049867 |
| ENSG00000197894 | ADH5 | -0.2357 | 8.220671 | 8.085926 | 0.007602 | 0.049938 |
| ENSG00000055732 | MCOLN3 | -0.40131 | 6.257801 | 8.23914 | 0.007618 | 0.049988 |

### REFERENCES

Andrews S. FastQC - A quality control tool for high throughput sequence data. http://www.bioinformatics.babraham.ac.uk/projects/fastqc/. 2010. https://doi.org/citeulike-article-id:11583827.
Balaj L, Lessard R, Dai L, Cho Y-J, Pomeroy SL, Breakefield XO, et al. Tumour microvesicles contain retrotransposon elements and amplified oncogene sequences. Nat Commun. 2011 Feb;2:180.
Berg JM. Zinc-finger proteins. Curr Opin Struct Biol. 1993 Aug;3(1):11-6.
Bó GA, Mapletoft RJ. Evaluation and classification of bovine embryos. 2013.
Bolger AM, Lohse M, Usadel B. Trimmomatic: A flexible trimmer for Illumina sequence data. Bioinformatics 2014. https://doi.org/10.1093/bioinformatics/btu170.
Bromer JG, Seli E. Assessment of embryo viability in assisted reproductive technology: shortcomings of current approaches and the emerging role of metabolomics. Curr Opin Obstet Gynecol. 2008 Jun;20(3):234-41.
Caballero I, Al Ghareeb S, Basatvat S, Sánchez-López JA, Montazeri M, Maslehat N, et al. Human Trophoblast Cells Modulate Endometrial Cells Nuclear Factor κB Response to Flagellin In Vitro. PLoS One. 2013;8(1).
Chen, Y. & Wang, X. miRDB: an online database for prediction of functional microRNA targets. Nucleic Acids Res. 48, D127-D131 (2019).
Cuman C, Van Sinderen M, Gantier MP, Rainczuk K, Sorby K, Rombauts L, et al. Human Blastocyst Secreted microRNA Regulate Endometrial Epithelial Cell Adhesion. EBioMedicine. 2015;2(10):1528-35.
Derrien T, Johnson R, Bussotti G, Tanzer A, Djebali S, Tilgner H, et al. The GENCODE v7 catalog of human long noncoding RNAs: analysis of their gene structure, evolution, and expression. Genome Res. 2012 Sep;22(9):1775-89.
Dissanayake K, Nõmm M, Lattekivi F, Ressaissi Y, Godakumara K, Lavrits A, et al. Individually cultured bovine embryos produce extracellular vesicles that have the potential to be used as non-invasive embryo quality markers. Theriogenology 2020;149:104-16. https://doi.org/10.1016/j.theriogenology.2020.03.008.
Dvo 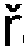 áčková M, Fajkus J. Visualization of the Nucleolus Using Ethynyl Uridine. Front Plant Sci [Internet]. 2018;9(February):1-8. Available from: http://journal.frontiersin.org/article/10.3389/fpls.2018.00177/full
Es-Haghi, M. et al. Specific trophoblast transcripts transferred by extracellular vesicles affect gene expression in endometrial epithelial cells and may have a role in embryo-maternal crosstalk. Cell Commun. Signal. 17, 146 (2019).
Faridani, O. R. et al. Single-cell sequencing of the small-RNA transcriptome. Nat. Biotechnol. 34, 1264-1266 (2016).
Feuerstein P, Cadoret V, Dalbies-Tran R, Guerif F, Bidault R, Royere D. Gene expression in human cumulus cells: one approach to oocyte competence. Hum Reprod. 2007 Dec;22(12):3069-77.
Gardner DK, Lane M, Stevens J, Schlenker T, Schoolcraft WB. Blastocyst score affects implantation and pregnancy outcome: towards a single blastocyst transfer. Fertil Steril. 2000 Jun;73(6):1155-8.
Goke J, Lu X, Chan Y-S, Ng H-H, Ly L-H, Sachs F, et al. Dynamic transcription of distinct classes of endogenous retroviral elements marks specific populations of early human embryonic cells. Cell Stem Cell. 2015 Feb;16(2):135-41.
Hagemann-Jensen, M., Abdullayev, I., Sandberg, R. & Faridani, O. R. Small-seq for single-cell small-RNA sequencing. Nat. Protoc. 13, 2407-2424 (2018)
Hansen TR, Austin KJ, Johnson GA. Transient ubiquitin cross-reactive protein gene expression in the bovine endometrium. Endocrinology 1997. https://doi.org/10.1210/endo.138.11.5655.
Hu T, Pi W, Zhu X, Yu M, Ha H, Shi H, et al. Long non-coding RNAs transcribed by ERV-9 LTR retrotransposon act in cis to modulate long-range LTR enhancer function. Nucleic Acids Res. 2017 May;45(8):4479-92.
Hubley R, Finn RD, Clements J, Eddy SR, Jones TA, Bao W, et al. The Dfam database of repetitive DNA families. Nucleic Acids Res. 2016;44(D1):D81-9.
Imbeault Michael, Helleboid Pierre-Yves, Trono Didier. KRAB zinc-finger proteins contribute to the evolution of gene regulatory networks. Nature [Internet]. 2017 Mar 8;543:550-4. Available from: http://www.nature.com/nature/journal/v543/n7646/pdf/nature21683.pdf
Jiang M, Zhang S, Yang Z, Lin H, Zhu J, Liu L, et al. Self-Recognition of an Inducible Host IncRNA by RIG-I Feedback Restricts Innate Immune Response. Cell [Internet]. 2018 May 3 [cited 2019 Apr 4];173(4):906-919.e13. Available from: http://www.ncbi.nlm.nih.gov/pubmed/29706547
Kelley D, Rinn J. Transposable elements reveal a stem cell-specific class of long noncoding RNAs. Genome Biol. 2012 Nov;13(11):R107.
Kim D, Paggi JM, Park C, Bennett C, Salzberg SL. Graph-based genome alignment and genotyping with HISAT2 and HISAT-genotype. Nat Biotechnol 2019. https://doi.org/10.1038/s41587-019-0201-4.
Kolde, R. pheatmap: Pretty Heatmaps. R package version 1.0.12. (2019).
Kornilov R, Puhka M, Mannerström B, et al. Efficient ultrafiltration-based protocol to deplete extracellular vesicles from fetal bovine serum. J Extracell Vesicles. 2018;7(1): 1422674. Published 2018 Jan 21. doi:10.1080/20013078.2017.1422674
van de Lavoir M-C, Diamond JH, Leighton PA, Mather-Love C, Heyer BS, Bradshaw R, et al. Germline transmission of genetically modified primordial germ cells. Nature. 2006 Jun;441(7094):766-9.
Liao Y, Smyth GK, Shi W. FeatureCounts: An efficient general purpose program for assigning sequence reads to genomic features. Bioinformatics 2014. https://doi.org/10.1093/bioinformatics/btt656.
Lloret-Llinares M, Karadoulama E, Chen Y, Wojenski LA, Villafano GJ, Bornholdt J, et al. The RNA exosome contributes to gene expression regulation during stem cell differentiation. Nucleic Acids Res [Internet]. 2018;46(21):11502-13. Available from: https://academic.oup.com/nar/advance-article/doi/10.1093/nar/gky817/5096074
Lokossou AG, Toudic C, Barbeau B. Implication of human endogenous retrovirus envelope proteins in placental functions. Viruses. 2014 Nov;6(11):4609-27.
Lu X, Sachs F, Ramsay L, Jacques P-E, Göke J, Bourque G, et al. The retrovirus HERVH is a long noncoding RNA required for human embryonic stem cell identity. Nat Struct &Amp; Mol Biol [Internet]. 2014 Mar 30;21:423. Available from: https://doi.org/10.1038/nsmb.2799
Lynch AM, Gibbs RS, Murphy JR, Byers TIM, Neville MC, Giclas PC, et al. Pregnancy and Spontaneous Preterm Birth. 2009;199(4):1-13.
Nõmm M, Porosk R, Parn P, Kilk K, Soomets U, Kõks S, et al. In vitro culture and non-invasive metabolic profiling of single bovine embryos. Reprod Fertil Dev 2019. https://doi.org/10.1071/RD17446.
Picelli, S. et al. Full-length RNA-seq from single cells using Smart-seq2. Nat. Protoc. 9, 171-181 (2014).
Quinn JJ, Chang HY. Unique features of long non-coding RNA biogenesis and function. Nat Rev Genet. 2016 Jan;17(1):47-62.
Robinson MD, McCarthy DJ, Smyth GK. edgeR: A Bioconductor package for differential expression analysis of digital gene expression data. Bioinformatics. 2009;26(1):139-40.
Rødgaard T, Heegaard PMH, Callesen H. Non-invasive assessment of in-vitro embryo quality to improve transfer success. Reprod Biomed Online [Internet]. 2015;31(5):585-92. Available from: http://dx.doi.org/10.1016/j.rbmo.2015.08.003
Sakkas D, Percival G, D'Arcy Y, Sharif K, Afnan M. Assessment of early cleaving in vitro fertilized human embryos at the 2-cell stage before transfer improves embryo selection. Fertil Steril. 2001 Dec;76(6):1150-6.
Sher G, Keskintepe L, Fisch JD, Acacio BA, Ahlering P, Batzofin J, et al. Soluble human leukocyte antigen G expression in phase I culture media at 46 hours after fertilization predicts pregnancy and implantation from day 3 embryo transfer. Fertil Steril. 2005 May;83(5):1410-3.
Smárason AK, Sargent IL, Starkey PM, Redman CWG. The effect of placental syncytiotrophoblast microvillous membranes from normal and pre-eclamptic women on the growth of endothelial cells in vitro. BJOG An Int J Obstet Gynaecol. 1993;100(10):943-9.
Soygur B, Moore H. Expression of Syncytin 1 (HERV-W), in the preimplantation human blastocyst, embryonic stem cells and trophoblast cells derived in vitro. Hum Reprod. 2016 Jul;31(7):1455-61.
Syed V, Hecht NB. Up-regulation and down-regulation of genes expressed in cocultures of rat sertoli cells and germ cells. Mol Reprod Dev. 1997;47(4):380-9.
Talukder AK, Rashid MB, Yousef MS, Kusama K, Shimizu T, Shimada M, et al. Oviduct epithelium induces interferon-tau in bovine Day-4 embryos, which generates an anti-inflammatory response in immune cells. Sci Rep 2018. https://doi.org/10.1038/s41598-018-26224-8.
Team RC. R: A Language and Environment for Statistical Computing. Vienna, Austria 2019.
Thery C, Witwer KW, Aikawa E, Alcaraz MJ, Anderson JD, Andriantsitohaina R, et al. Minimal information for studies of extracellular vesicles 2018 (MISEV2018): a position statement of the International Society for Extracellular Vesicles and update of the MISEV2014 guidelines. J Extracell Vesicles [Internet]. 2019;8(1):1535750. Available from: https://www.tandfonline.com/doi/full/10.1080/20013078.2018.1535750
Thery C, Amigorena S, Raposo G, Clayton A. Isolation and Characterization of Exosomes from Cell Culture Supernatants and Biological Fluids. Current Protocols in Cell Biology.2006, 30(1): 3.22.1- 3.22.29.
Tian W, Du Y, Ma Y, Gu L, Zhou J, Deng D. MALAT1-miR663a negative feedback loop in colon cancer cell functions through direct miRNA-IncRNA binding. Cell Death Dis [Internet]. 2018 [cited 2019 Apr 4];9(9). Available from: https://www.ncbi.nlm.nih.gov/pmc/articles/PMC6113222/
Travella S, Keller B. Down-regulation of gene expression by RNA-induced gene silencing. Methods Mol Biol. 2009;478:185-99.
Trono D. Transposable elements, polydactyl proteins, and the genesis of human-specific transcription networks. Cold Spring Harb Symp Quant Biol. 2016;80:281-8.
Trowsdale J, Betz AG. Mother's little helpers: Mechanisms of maternal-fetal tolerance. Nat Immunol. 2006;7(3):241-6.
Vargas A, Zhou S, Ethier-Chiasson M, Flipo D, Lafond J, Gilbert C, et al. Syncytin proteins incorporated in placenta exosomes are important for cell uptake and show variation in abundance in serum exosomes from patients with preeclampsia. FASEB J Off Publ Fed Am Soc Exp Biol. 2014 Aug;28(8):3703-19.
Vilella F, Moreno-Moya JM, Balaguer N, Grasso A, Herrero M, Martinez S, et al. Hsa-miR-30d, secreted by the human endometrium, is taken up by the pre-implantation embryo and might modify its transcriptome. Development [Internet]. 2015 Sep 15;142(18):3210 LP - 3221. Available from: http://dev.biologists.org/content/142/18/3210.abstract
Wang HM, Zhang X, Qian D, Lin HY, Li QL, Liu DL, et al. Effect of ubiquitin-proteasome pathway on mouse blastocyst implantation and expression of matrix metalloproteinases-2 and -9. Biol Reprod. 2004 Feb;70(2):481-7.
Wang SXY. The past, present, and future of embryo selection in in vitro fertilization: Frontiers in Reproduction Conference. Vol. 84, The Yale journal of biology and medicine. 2011. p. 487-90.
Wang J hua, Jiang D, Rao H yng, Zhao J min, Wang Y, Wei L. Absolute quantification of serum microRNA-122 and its correlation with liver inflammation grade and serum alanine aminotransferase in chronic hepatitis C patients. Int J Infect Dis [Internet]. 2015;30:e52-6. Available from: http://dx.doi.org/10.1016/j.ijid.2014.09.020
H. Wickham. ggplot2: Elegant Graphics for Data Analysis. (Springer-Verlag, 2016).
Yan J, Huang W, Huang X, Xiang W, Ye C, Liu J. A negative feedback loop between long noncoding RNA NBAT1 and Sox9 inhibits the malignant progression of gastric cancer cells. Biosci Rep [Internet]. 2018 Dec 21 [cited 2019 Apr 4];38(6):BSR20180882. Available from: http://www.ncbi.nlm.nih.gov/pubmed/30287498
Yu G, Wang LG, Han Y, He QY. ClusterProfiler: An R package for comparing biological themes among gene clusters. Omi A J Integr Biol 2012. https://doi.org/10.1089/omi.2011.0118.
Yu, G. & He, Q. Y. ReactomePA: An R/Bioconductor package for reactome pathway analysis and visualization. Mol. Biosyst. 12, 477-479 (2016).
Zhang X, Jafari N, Barnes RB, Confino E, Milad M, Kazer RR. Studies of gene expression in human cumulus cells indicate pentraxin 3 as a possible marker for oocyte quality. Fertil Steril. 2005 Apr;83 Suppl 1:1169-79.

## Claims

1. A method of predicting outcome of an embryo transfer in an *in vitro* fertilization (IVF) procedure, the method comprising:
contacting *in vitro* responder cells with extracellular vesicles isolated from an IVF embryo to be transferred into a female subject and/or with a conditioned medium from the IVF embryo;
determining, in the responder cells, an amount of at least one ribonucleic acid (RNA) transcript induced by embryonic RNA present in the extracellular vesicles and/or the conditioned medium; and
predicting pregnancy outcome of the embryo transfer based on the determined amount of the at least one RNA transcript by predicting a high likelihood for successful embryo transfer and pregnancy of the female subject based on a significant change in the amount of the at least one RNA transcript relative to a reference level of the at least one RNA transcript, and predicting a low likelihood for successful embryo transfer and pregnancy of the female subject based on a non-significant change in the amount of the at least one RNA transcript relative to the reference level.

2. The method according to claim 1, wherein
determining the amount of the at least one RNA transcript comprises determining an amount of downregulation or upregulation of the at least one RNA transcript in the responder cells induced by the extracellular vesicles and/or the conditioned medium; and
predicting the outcome of the embryo transfer comprises predicting the outcome of the embryo transfer based on the determined amount of downregulation or upregulation of the at least one RNA transcript.

3. A method of determining a quality of an *in vitro* fertilization (IVF) embryo, the method comprising:
contacting *in vitro* responder cells with extracellular vesicles isolated from the IVF embryo and/or with a conditioned medium from the IVF embryo;
determining, in the responder cells, an amount of at least one ribonucleic acid (RNA) transcript induced by embryonic RNA present in the extracellular vesicles and/or the conditioned medium; and
determining the quality of the IVF embryo based on the determined amount of the at least one RNA transcript by determining the IVF embryo to be good for intrauterine transfer into a female subject based on a significant change in the amount of the at least one RNA transcript relative to a reference level of the at least one RNA transcript, and determining the IVF embryo to be not good for intrauterine transfer into the female subject based on a non-significant change in the amount of the at least one RNA transcript relative to the reference level.

4. The method according to claim 3, wherein
determining the amount of the at least one RNA transcript comprises determining an amount of downregulation or upregulation of the at least one RNA transcript in the responder cells induced by the extracellular vesicles and/or the conditioned medium; and
determining the quality of the IVF embryo comprises determining the quality of the IVF embryo based on the determined amount of downregulation or upregulation of the at least one RNA transcript.

5. The method according to any of the claims 1 to 4, wherein the reference level represents an amount of the at least one RNA transcript in the responder cells prior to contacting the responder cells *in vitro* with the isolated EVs and/or the conditioned medium.

6. A method of selecting an embryo for an *in vitro* fertilization (IVF) procedure, the method comprising:
contacting *in vitro,* for each IVF embryo among M potential IVF embryos, responder cells with extracellular vesicles isolated from the IVF embryo and/or a conditioned medium from the IVF embryo, wherein M is an integer equal to or larger than two;
determining, for each IVF embryo among the M potential IVF embryos and in the responder cells, an amount of at least one ribonucleic acid (RNA) transcript induced by embryonic RNA present in the extracellular vesicles and/or the conditioned medium; and
selecting at least one IVF embryo among the multiple potential IVF embryos based on the respective determined amounts of the at least one RNA transcript by selecting the N IVF embryo(s) resulting in a largest downregulation of the at least one RNA transcript among the M potential IVF embryos, wherein N < M, or selecting the N IVF embryo(s) resulting in a largest upregulation of the at least one RNA transcript among the M potential IVF embryos.

7. The method according to claim 6, wherein
determining the amount of the at least one RNA transcript comprises determining, for each IVF embryo among the M IVF embryos, an amount of downregulation or upregulation of the at least one RNA transcript in the responder cells induced by the extracellular vesicles and/or the conditioned medium; and
selecting the at least one IVF embryo comprises selecting at least one IVF embryo among the M potential IVF embryos based on the respective determined amounts of downregulation or upregulations of the at least one RNA transcript.

8. The method according to any of the claims 1 to 7, wherein the responder cells are of a same species as the female subject and the IVF embryo.

9. The method according to any of the claims 1 to 7, wherein the responder cells are cells of reproductive lineage, preferably endometrial cells, and more preferably human endometrial RL95-2 cells.

10. The method according to any of the claims 1 to 9, wherein determining the amount of the at least one RNA transcript comprises determining, in the responder cells, the amount of at least one RNA transcript selected for the group consisting of a mucin 4 (*MUC4*) transcript, a *MUC3A* transcript, a *MUC16* transcript, a *MUC12* transcript, a zinc finger protein 81 (*ZNF81*) transcript, a Ras-related GTP-binding protein B (RRAGB) transcript, a mitochondrially encoded tRNA tryptophan (*MT-TW*) transcript, a Z95704.5 transcript, a mitochondrially encoded tRNA serine 1 (*MT-TSI*) transcript, an integrin, alpha E (*ITGAE*) transcript, a RP11-357C3.3 transcript, a transmembrane protein 154 (*TMEM154*) transcript, a caspase 14 (CASP14) transcript, a *ZNF765* transcript, a long intergenic non-protein coding RNA 478 (LINC00478) transcript, a mitochondrially encoded tRNA glutamine (*MT-TQ*) transcript, an ankyrin repeat domain-containing protein 44 (*ANKRD44*) transcript, and a zinc finger BED-type containing 3 - antisense RNA 1 (*ZBED3-AS1*) transcript, preferably selected among the group consisting of a transcript of an intronic region of LINC00478, a transcript of an exonic region of LINC00478 and a transcript of an exonic region of *ZNF81,* and more preferably selected from the group consisting of SEQ ID NO: 17 to 19 or an RNA sequence complementary to any of SEQ ID NO: 17 to 19.

11. The method according to any of the claims 1 to 9, wherein determining the amount of the at least one RNA transcript comprises determining, in the responder cells, the amount of at least one RNA transcript selected for the group consisting of an ER oxidoreductin 1 alpha (*ERO1A*) transcript, a stearoyl-CoA desaturase (SCD) transcript, a solute carrier family 2, facilitated glucose transporter member 3 (SLC2A3) transcript, an arrestin domain containing 3 (ARRDC3) transcript, a class E basic helix-loop-helix protein 40 (*BHLHE40*) transcript, an atypical chemokine receptor 3 (ACKR3) transcript, a hypoxia inducible lipid droplet-associated (*HILPDA*) transcript, a DNA-damage-inducible transcript 4 (*DDIT4*) transcript, an olfactomedin 4 (*OLFM4*) transcript, an OLFM3 transcript, a F-box-like/WD repeat-containing protein (*TBL1XR1*) transcript, a glucosamine (N-acetyl)-6-sulfatase (GMS) transcript, a N-myc downstream regulated 1 (*NDRG1*) transcript and an aldolase C, and fructose-bisphosphate (*ALDOC*) transcript.

12. The method according to any of the claims 1 to 9, wherein determining the amount of the at least one RNA transcript comprises:
determining, in the responder cells, the amount of at least one RNA transcript selected for the group consisting of a 2'-5' oligoadenylate synthase (*OAS1Y*) transcript, an interferon-induced GTP-binding protein (*MX1*) transcript, an interferon-induced protein with tetratricopeptide repeats 1 (*LOC100139670*) transcript, an interferon-stimulated gene 15 (*ISG15*)*,* an ENSBTAG00000051364 transcript, an ENSBTAG00000053545 transcript, a cytochrome P450, family 1, subfamily A, polypeptide 1 (*CYP1A1*) transcript, an alkB homolog 4, alpha-ketoglutarate dependent dioxygenase (*ALKBH4*) transcript, a MAP kinase-activating death domain protein (*MADD*) transcript, a Huntingtin-interacting protein 1-related protein (*HIP1R*)*,* a chromosome 28 C1 open reading frame 198 (*C28H1orf198*) transcript, a HID1 domain containing (*HID1*) transcript, a Cdc42 effector protein 1 (*CDC42EP1*) transcript, a protein unc-13 homolog D (*UNC13D*) transcript, an aldehyde dehydrogenase 16 family, member A1 (*ALDH16A1*) transcript, a calpain-1 catalytic subunit (*CAPN1*) transcript, a peroxidasin homolog (*PXDN*)*,* an ENSBTAG00000043565 transcript, a cleavage and polyadenylation specificity factor subunit 1 (*CPSF1*) transcript, a HGH1 homolog (*HGH1*) transcript, a Rho guanine nucleotide exchange factor 2 (*ARHGEF2*) transcript, a laminin subunit beta-3 (LAMB3) transcript, a follistatin-related protein 3 (*FSTL3*) transcript and a rhomboid family member 2 (*RHBDF2*) transcript, or
determining, in the responder cells, the amount of at least one RNA transcript selected for the group consisting of a 2'-5' oligoadenylate synthase (*OAS1Y*) transcript, an interferon-induced GTP-binding protein (*MX1*) transcript, an interferon-induced protein with tetratricopeptide repeats 1 (LOC100139670) transcript, an interferon-stimulated gene 15 (*ISG15*)*,* a MAP kinase-activating death domain protein *(MADD)* transcript, a Huntingtin-interacting protein 1-related protein (*HIP1R*)*,* a calpain-1 catalytic subunit (*CAPN1*) transcript, a HID1 domain containing (*HID1*) transcript, a Cdc42 effector protein 1 (*CDC42EP1*) transcript, a protein unc-13 homolog D (*UNC13D*) transcript, a peroxidasin homolog (*PXDN*)*,* an 1-acyl-sn-glycerol-3-phosphate acyltransferase alpha (*AGPAT1*) transcript, a Bcl-2 homologous antagonist/killer (*BAK1*) transcript, a large neutral amino acids transporter small subunit 2 (SLC7A8) transcript and a tissue transglutaminase (*TGM2*) transcript.

## Patentansprüche

1. Verfahren zum Vorhersagen eines Ergebnisses eines Embryotransfers in einer Prozedur zur In-vitro-Fertilisation (IVF), wobei das Verfahren Folgendes umfasst:
In-vitro-Inkontaktbringen von Responderzellen mit extrazellulären Vesikeln, die aus einem IVF-Embryo isoliert wurden, um in ein weibliches Subjekt übertragen zu werden, und/oder mit einem konditionierten Medium aus dem IVF-Embryo;
Bestimmen, in den Responderzellen, einer Menge von mindestens einem Ribonukleinsäure(RNA)-Transkript, das durch embryonale RNA induziert wird, die in den extrazellulären Vesikeln und/oder dem konditionierten Medium vorhanden ist; und
Vorhersagen eines Schwangerschaftsergebnisses des Embryotransfers auf Grundlage der bestimmten Menge des mindestens einen RNA-Transkripts durch Vorhersagen einer hohen Wahrscheinlichkeit für einen erfolgreichen Embryotransfer und eine Schwangerschaft des weiblichen Subjekts auf Grundlage einer signifikanten Änderung der Menge des mindestens einen RNA-Transkripts relativ zu einem Referenzspiegel des mindestens einen RNA-Transkripts und Vorhersagen einer geringen Wahrscheinlichkeit für einen erfolgreichen Embryotransfer und eine Schwangerschaft des weiblichen Subjekts auf Grundlage einer nicht signifikanten Änderung der Menge des mindestens einen RNA-Transkripts relativ zu dem Referenzspiegel.

2. Verfahren nach Anspruch 1, wobei
das Bestimmen der Menge des mindestens einen RNA-Transkripts Bestimmen einer Menge an Herunterregulierung oder Hochregulierung des mindestens einen RNA-Transkripts in den Responderzellen umfasst, das durch die extrazellulären Vesikel und/oder das konditionierte Medium induziert wird; und
das Vorhersagen des Ergebnisses des Embryotransfers Vorhersagen des Ergebnisses des Embryotransfers auf Grundlage der bestimmten Menge an Herabregulierung oder Hochregulierung des mindestens einen RNA-Transkripts umfasst.

3. Verfahren zum Bestimmen einer Qualität eines Embryos zur In-vitro-Fertilisation (IVF), wobei das Verfahren Folgendes umfasst:
In-vitro-Inkontaktbringen von Responderzellen mit extrazellulären Vesikeln, die aus dem IVF-Embryo isoliert wurden, und/oder mit einem konditionierten Medium aus dem IVF-Embryo;
Bestimmen, in den Responderzellen, einer Menge von mindestens einem Ribonukleinsäure(RNA)-Transkript, das durch embryonale RNA induziert wird, die in den extrazellulären Vesikeln und/oder dem konditionierten Medium vorhanden ist; und
Bestimmen der Qualität des IVF-Embryos auf Grundlage der bestimmten Menge des mindestens einen RNA-Transkripts durch Bestimmen, dass der IVF-Embryo für den intrauterinen Transfer in ein weibliches Subjekt gut ist, auf Grundlage einer signifikanten Änderung der Menge des mindestens einen RNA-Transkripts relativ zu einem Referenzspiegel des mindestens einen RNA-Transkripts und Bestimmen, dass der IVF-Embryo für den intrauterinen Transfer in das weibliche Subjekt nicht gut ist, auf Grundlage einer nicht signifikanten Änderung der Menge des mindestens einen RNA-Transkripts relativ zu dem Referenzspiegel.

4. Verfahren nach Anspruch 3, wobei
das Bestimmen der Menge des mindestens einen RNA-Transkripts Bestimmen einer Menge an Herunterregulierung oder Hochregulierung des mindestens einen RNA-Transkripts in den Responderzellen umfasst, das durch die extrazellulären Vesikel und/oder das konditionierte Medium induziert wird; und
das Bestimmen der Qualität des IVF-Embryos Bestimmen der Qualität des IVF-Embryos auf Grundlage der bestimmten Menge der Herunterregulierung oder Hochregulierung des mindestens einen RNA-Transkripts umfasst.

5. Verfahren nach einem der Ansprüche 1 bis 4, wobei der Referenzspiegel eine Menge des mindestens einen RNA-Transkripts in den Responderzellen vor dem In-In-vitro-*Inkontaktbringen* der Responderzellen mit den isolierten EVs und/oder dem konditionierten Medium darstellt.

6. Verfahren zum Auswählen eines Embryos für einen Prozedur zur In-vitro-Fertilisation (IVF), wobei das Verfahren Folgendes umfasst:
In-vitro-Inkontaktbringen, für jeden IVF-Embryo unter M potenziellen IVF-Embryonen, von Responderzellen mit extrazellulären Vesikeln, die aus dem IVF-Embryo isoliert wurden, und/oder einem konditionierten Medium aus dem IVF-Embryo, wobei M eine ganze Zahl größer gleich zwei ist;
Bestimmen, für jeden IVF-Embryo unter den M potenziellen IVF-Embryonen und in den Responderzellen, einer Menge von mindestens einem Ribonukleinsäure(RNA)-Transkript, das durch embryonale RNA induziert wird, die in den extrazellulären Vesikeln und/oder dem konditionierten Medium vorhanden ist; und
Auswählen mindestens eines IVF-Embryos aus den mehreren potenziellen IVF-Embryonen auf Grundlage der jeweiligen bestimmten Mengen des mindestens einen RNA-Transkripts durch Auswählen des/der *N* IVF-Embryos/Embryonen, was zu einer größten Herunterregulierung des mindestens einen RNA-Transkripts unter den *M* potenziellen IVF-Embryonen führt, wobei *N<M,* oder Auswählen des/der *N* IVF-Embryos/Embryonen, was zu einer größten Hochregulation des mindestens einen RNA-Transkripts unter den *M* potenziellen IVF-Embryonen führt.

7. Verfahren nach Anspruch 6, wobei
das Bestimmen der Menge des mindestens einen RNA-Transkripts Bestimmen, für jeden IVF-Embryo unter den *M* IVF-Embryonen, einer Menge an Herunterregulierung oder Hochregulierung des mindestens einen RNA-Transkripts in den Responderzellen umfasst, das durch die extrazellulären Vesikel und/oder das konditionierte Medium induziert wird; und
das Auswählen des mindestens einen IVF-Embryos Auswählen mindestens eines IVF-Embryos aus den *M* potenziellen IVF-Embryonen auf Grundlage der jeweiligen bestimmten Mengen an Herunterregulierung oder Hochregulierung des mindestens einen RNA-Transkripts umfasst.

8. Verfahren nach einem der Ansprüche 1 bis 7, wobei die Responderzellen von einer selben Spezies sind wie das weibliche Subjekt und der IVF-Embryo.

9. Verfahren nach einem der Ansprüche 1 bis 7, wobei die Responderzellen Zellen einer reproduktiven Abstammungslinie sind, vorzugsweise endometriale Zellen und bevorzugter menschliche endometriale RL95-2-Zellen.

10. Verfahren nach einem der Ansprüche 1 bis 9, wobei das Bestimmen der Menge des mindestens einen RNA-Transkripts Bestimmen der Menge von mindestens einem RNA-Transkript in den Responderzellen umfasst, ausgewählt aus der Gruppe bestehend aus einem Transkript für Mucin 4 (*MUC4*)*,* einem Transkript für *MUC3A,* einem Transkript für *MUC16,* einem Transkript für *MUC12,* einem Transkript für Zinkfinger-Protein 81 (*ZNF81*)*,* einem Transkript für Ras-bezogenes GTPbindendes Protein B (*RRAGB*), einem Transkript für mitochondrial codiertes tRNA-Tryptophan (*MT*-*TW*)*,* einem Transkript für Z95704.5, einem Transkript für mitochondrial codiertes tRNA-Serin 1 (*MT-TS1*), einem Transkript für Integrin alpha E (*ITGAE*)*,* einem Transkript für RP11-357C3.3, einem Transkript für Transmembranprotein 154 (*TMEM154*)*,* einem Transkript für Caspase 14 (*CASP14*)*,* einem Transkript für *ZNF765,* einem Transkript für lange intergene Nicht-Protein-codierende RNA 478 (LINC00478), einem Transkript für mitochondrial codiertes tRNA-Glutamin (*MT-TQ*), einem Transkript für Ankyrin-Repeat-Domänen-enthaltendes Protein 44 (*ANKRD44*) und einem Transkript für Zinkfinger-BED-Typenthaltende 3-Antisense-RNA-1 (*ZBED3-AS1*), vorzugsweise ausgewählt aus der Gruppe bestehend aus einem Transkript einer intronischen Region von LINC00478, einem Transkript einer exonischen Region von LINC00478 und einem Transkript einer exonischen Region von *ZNF81,* und bevorzugter ausgewählt aus der Gruppe bestehend aus SEQ ID NO: 17 bis 19 oder einer RNA-Sequenz, die zu einer beliebigen von SEQ ID NO: 17 bis 19 komplementär ist.

11. Verfahren nach einem der Ansprüche 1 bis 9, wobei das Bestimmen der Menge des mindestens einen RNA-Transkripts Bestimmen der Menge von mindestens einem RNA-Transkript in den Responderzellen umfasst, ausgewählt aus der Gruppe bestehend aus einem Transkript für ER-Oxidoreduktin 1 alpha (*ERO1A*)*,* einem Transkript für Stearoyl-CoA-Desaturase (*SCD*), einem Transkript für Solute-Carrier-Family-2-unterstützten Glukosetransporter 3 (*SLC2A3*), einem Transkript für Arrestin-Domäne enthaltend 3 (*ARRDC3*)*,* einem Transkript für Klasse-E-basisches Helix-Schleifen-Helix-Protein 40 (*BHLHE40*), einem Transkript für atypischen Chemokin-Rezeptor 3 (*ACKR3*), einem Transkript für Hypoxie-induzierbar Lipidtröpfchen-assoziiert (*HILPDA*)*,* einem Transkript für DNA-Schaden-induzierbares Transkript 4 (*DDIT4*)*,* einem Transkript für Olfactomedin 4 (*OLFM4*)*,* einem Transkript für OLFM3, einem Transkript für F-Box-ähnliches/WD-Repeatenthaltendes Protein (*TBL1XR1*), einem Transkript für Glucosamin-(N-Acetyl)-6-sulfatase (*GNS*), einem Transkript für N-myc-stromabwärts reguliert 1 (*NDRG1*) und einem Transkript für Aldolase C und Fructose-Bisphosphat (*ALDOC*)*.*

12. Verfahren nach einem der Ansprüche 1 bis 9, wobei das Bestimmen der Menge des mindestens einen RNA-Transkripts Folgendes umfasst:
Bestimmen der Menge mindestens eines RNA-Transkripts in den Responderzellen, ausgewählt aus der Gruppe bestehend aus einem Transkript für 2'-5'-Oligoadenylatsynthase (*OAS1Y*)*,* einem Transkript für Interferon-induziertes GTP-Bindungsprotein (*MX1*), einem Transkript für Interferon-induziertes Protein mit Tetratricopeptid-Repeats 1 (*LOC100139670*), einem Transkript für Interferon-stimuliertes Gen 15 (*ISG15*)*,* einem Transkript für ENSBTAG00000051364, einem Transkript für ENSBTAG00000053545, einem Transkript für Cytochrom P450, Familie 1, Unterfamilie A, Polypeptid 1 (*CYP1A1*), einem Transkript für alkB Homolog 4, alpha-Ketoglutarat-abhängige Dioxygenase (*ALKBH4*), einem Transkript für MAP-Kinase-aktivierendes Todesdomänenprotein (*MADD*)*,* einem Transkript für Huntingtin-interagierendes Protein-1-verwandtes Protein (*HIP1R*), einem Transkript für offenen Chromosom-28-C1-Leserahmen 198 (*C28H1orf198*), einem Transkript für HID1-Domänen-enthaltend (*HID1*), einem Transkript für Cdc42-Effektorprotein 1 (*CDC42EP1*)*,* einem Transkript für Protein-unc-13-Homolog D (*UNC13D*), einem Transkript für Aldehyddehydrogenase-16-Familie, Mitglied A1 (*ALDH16A1*)*,* einem Transkript für Calpain-1 katalytische Untereinheit (*CAPN1*), einem Transkript für Peroxidasin-Homolog (*PXDN*)*,* einem Transkript für ENSBTAG00000043565, einem Transkript für Spaltungs- und Polyadenylierungsspezifitätsfaktor Untereinheit 1 (*CPSF1*)*,* einem Transkript für HGH1-Homolog (*HGH1*)*,* einem Transkript für Rho-Guanin-Nukleotid-Austauschfaktor 2 (*ARHGEF2*)*,* einem Transkript für Laminin-Untereinheit beta-3 (LAMB3), einem Transkript für Follistatin-verwandtes Protein 3 (*FSTL3*) und einem Transkript für Rhomboidfamilienmitglied 2 (*RHBDF2*) oder
Bestimmen der Menge mindestens eines RNA-Transkripts in den Responderzellen, ausgewählt aus der Gruppe bestehend aus einem Transkript für 2'-5'-Oligoadenylatsynthase (*OAS1Y*)*,* einem Transkript für Interferon-induziertes GTP-Bindungsprotein (*MX1*), einem Transkript für Interferon-induziertes Protein mit Tetratricopeptid-Repeats 1 (*LOC100139670*), einem Transkript für Interferon-stimuliertes Gen 15 (*ISG15*), einem Transkript für MAP-Kinase-aktivierendes Todesdomänenprotein (*MADD*)*,* einem Transkript für Huntingtin-interagierendes Protein-1-verwandtes Protein (*HIP1R*), einem Transkript für Calpain-1 katalytische Untereinheit (*CAPN1*)*,* einem Transkript für HID1-Domänen-enthaltend (*HID1*), einem Transkript für Cdc42-Effektorprotein 1 (*CDC42EP1*)*,* einem Transkript für Protein-unc-13-Homolog D (*UNC13D*), einem Transkript für Peroxidasin-Homolog (*PXDN*)*,* einem Transkript für 1-Acyl-sn-glycerol-3-phosphat-acyltransferase alpha (*AGPAT1*)*,* einem Transkript für homologen Antagonisten/Killers von Bcl-2 (*BAK1*), einem Transkript für kleine Untereinheit 2 für Transporter von großen neutralen Aminosäuren (*SLC7A8*) und ein Transkript einer Gewebetransglutaminase (*TGM2*)*.*

## Revendications

1. Procédé de prédiction du résultat d'un transfert d'embryon dans une procédure de fécondation *in vitro* (FIV), le procédé comprenant :
la mise en contact de cellules répondeuses *in vitro* avec des vésicules extracellulaires isolées d'un embryon de FIV à transférer dans un sujet féminin et/ou avec un milieu conditionné provenant de l'embryon de FIV ;
la détermination, dans les cellules répondeuses, d'une quantité d'au moins un transcrit d'acide ribonucléique (ARN) induit par l'ARN embryonnaire présent dans les vésicules extracellulaires et/ou le milieu conditionné; et
la prédiction du résultat de grossesse du transfert d'embryon sur la base de la quantité déterminée de l'au moins un transcrit d'ARN en prédisant une probabilité élevée de transfert d'embryon et de grossesse réussis du sujet féminin sur la base d'un changement significatif de la quantité de l'au moins un transcrit d'ARN par rapport à un niveau de référence de l'au moins un transcrit d'ARN, et la prédiction d'une faible probabilité de transfert d'embryon et de grossesse réussis du sujet féminin sur la base d'un changement non significatif de la quantité de l'au moins un transcrit d'ARN par rapport au niveau de référence.

2. Procédé selon la revendication 1, dans lequel :
la détermination de la quantité de l'au moins un transcrit d'ARN comprend la détermination d'une quantité de régulation négative ou de régulation positive de l'au moins un transcrit d'ARN dans les cellules répondeuses induit par les vésicules extracellulaires et/ou le milieu conditionné ; et
la prédiction du résultat du transfert d'embryon comprend la prédiction du résultat du transfert d'embryon sur la base de la quantité déterminée de régulation négative ou de régulation positive de l'au moins un transcrit d'ARN.

3. Procédé de détermination d'une qualité d'un embryon de fécondation *in vitro* (FIV), le procédé comprenant :
la mise en contact de cellules répondeuses *in vitro* avec des vésicules extracellulaires isolées de l'embryon de FIV et/ou avec un milieu conditionné provenant de l'embryon de FIV ;
la détermination, dans les cellules répondeuses, d'une quantité d'au moins un transcrit d'acide ribonucléique (ARN) induit par l'ARN embryonnaire présent dans les vésicules extracellulaires et/ou le milieu conditionné; et
la détermination de la qualité de l'embryon de FIV sur la base de la quantité déterminée de l'au moins un transcrit d'ARN en déterminant l'embryon de FIV comme étant bon pour un transfert intra-utérin dans un sujet féminin sur la base d'un changement significatif de la quantité de l'au moins un transcrit d'ARN par rapport à un niveau de référence de l'au moins un transcrit d'ARN, et la détermination que l'embryon de FIV n'est pas bon pour un transfert intra-utérin dans le sujet féminin sur la base d'un changement non significatif de la quantité de l'au moins transcrit d'ARN par rapport au niveau de référence.

4. Procédé selon la revendication 3, dans lequel :
la détermination de la quantité de l'au moins un transcrit d'ARN comprend la détermination d'une quantité de régulation négative ou de régulation positive de l'au moins un transcrit d'ARN dans les cellules répondeuses induit par les vésicules extracellulaires et/ou le milieu conditionné ; et
la détermination de la qualité de l'embryon de FIV comprend la détermination de la qualité de l'embryon de FIV sur la base de la quantité déterminée de régulation négative ou de régulation positive de l'au moins un transcrit d'ARN.

5. Procédé selon l'une quelconque des revendications 1 à 4, dans lequel le niveau de référence représente une quantité de l'au moins un transcrit d'ARN dans les cellules répondeuses avant la mise en contact des cellules répondeuses *in vitro* avec les EV isolées et/ou le milieu conditionné.

6. Procédé de sélection d'un embryon pour une procédure de fécondation *in vitro* (FIV), le procédé comprenant :
la mise en contact *in vitro,* pour chaque embryon de FIV parmi M embryons de FIV potentiels, de cellules répondeuses avec des vésicules extracellulaires isolées de l'embryon de FIV et/ou un milieu conditionné provenant de l'embryon de FIV, dans lequel M est un nombre entier égal ou supérieur à deux ;
la détermination, pour chaque embryon de FIV parmi les M embryons de FIV potentiels et dans les cellules répondeuses, d'une quantité d'au moins un transcrit d'acide ribonucléique (ARN) induit par l'ARN embryonnaire présent dans les vésicules extracellulaires et/ou le milieu conditionné ; et
la sélection d'au moins un embryon de FIV parmi les multiples embryons de FIV potentiels sur la base des quantités déterminées respectives de l'au moins un transcrit d'ARN en sélectionnant le ou les N embryons de FIV, ce qui entraîne une régulation négative la plus importante de l'au moins un transcrit d'ARN parmi les M embryons de FIV potentiels, dans lequel N < M, ou la sélection du ou des N embryons de FIV, ce qui entraîne une régulation positive la plus importante de l'au moins un transcrit d'ARN parmi les M embryons de FIV potentiels.

7. Procédé selon la revendication 6, dans lequel :
la détermination de la quantité de l'au moins un transcrit d'ARN comprend la détermination, pour chaque embryon de FIV parmi les M embryons de FIV, d'une quantité de régulation négative ou de régulation positive de l'au moins un transcrit d'ARN dans les cellules répondeuses induit par les vésicules extracellulaires et/ou le milieu conditionné ; et
la sélection de l'au moins un embryon de FIV comprend la sélection d'au moins un embryon de FIV parmi les M embryons de FIV potentiels sur la base des quantités déterminées respectives de régulation négative ou de régulation positive de l'au moins un transcrit d'ARN.

8. Procédé selon l'une quelconque des revendications 1 à 7, dans lequel les cellules répondeuses sont de la même espèce que le sujet féminin et l'embryon de FIV.

9. Procédé selon l'une quelconque des revendications 1 à 7, dans lequel les cellules répondeuses sont des cellules de lignée reproductrice, de préférence des cellules endométriales, et plus préférablement des cellules RL95-2 de l'endomètre humain.

10. Procédé selon l'une quelconque des revendications 1 à 9, dans lequel la détermination de la quantité de l'au moins un transcrit d'ARN comprend la détermination, dans les cellules répondeuses, de la quantité d'au moins un transcrit d'ARN sélectionné dans le groupe constitué d'un transcrit de la mucine 4 (MUC4), d'un transcrit de la MUC3A, d'un transcrit de la MUC16, d'un transcrit de la MUC12, d'un transcrit de la protéine à doigt de zinc 81 (ZNF81), d'un transcrit de la protéine B de liaison au GTP liée à Ras (RRAGB), d'un transcrit du tryptophane d'ARNt codé par les mitochondries (MT-TW), d'un transcrit de Z95704.5, d'un transcrit d'ARNt sérine 1 codé par les mitochondries d'ARNt sérine 1, d'un transcrit de l'intégrine alpha E (ITGAE), d'un transcrit de RP11-357C3.3, d'un transcrit de la protéine transmembranaire 154 (TMEM154), d'un transcrit de la caspase 14 (CASP14), d'un transcrit de ZNF765, d'un long transcrit d'ARN 478 non codant pour une protéine intergénique (LINC00478), d'un transcrit d'ARNt glutamine codé par les mitochondries (MT-TQ), d'un transcrit de la protéine 44 contenant un domaine répété d'ankyrine (ANKRD44) et d'un transcrit contenant un doigt de zinc de type BED 3 - ARN antisens 1 (ZBED3-AS1), de préférence sélectionné dans le groupe constitué d'un transcrit d'une région intronique de LINC00478, d'un transcrit d'une région exonique de LINC00478 et d'un transcrit d'une région exonique de ZNF81, et plus préférablement sélectionné dans le groupe constitué de SEQ ID NO: 17 à 19 ou une séquence d'ARN complémentaire à l'une quelconque de SEQ ID NO: 17 à 19.

11. Procédé selon l'une quelconque des revendications 1 à 9, dans lequel la détermination de la quantité d'au moins un transcrit d'ARN comprend la détermination, dans les cellules répondeuses, de la quantité d'au moins un transcrit d'ARN sélectionné dans le groupe constitué d'un transcrit de l'ER oxydoréductine 1 alpha (ERO1A), d'un transcrit de la stéaroyl-CoA désaturase (SCO), d'un transcrit de la famille 2 des transporteurs de soluté, d'un transcrit du transporteur de glucose facilité 3 (SLC2A3), d'un transcrit contenant un domaine d'arrestine 3 (ARRDC3), d'un transcrit de la protéine hélice-boucle-hélice basique de classe E (BHLHE40), d'un transcrit du récepteur de chimiokine atypique 3 (ACKR3), d'un transcrit associé aux gouttelettes lipidiques inductibles par l'hypoxie (HILPDA), d'un transcrit 4 inductible par des dommages à l'ADN (DDIT4), d'un transcrit de l'olfactomédine 4 (OLFM4), d'un transcrit d'OLFM3, d'un transcrit de la protéine contenant une répétition de type boîte F/WD (TBL1XR1), d'un transcrit de la glucosamine (N-acétyl)-6-sulfatase (G/VS), d'un transcrit régulé en aval de N-myc 1 (NDRG1) et d'un transcrit de la fructose-bisphosphate aldolase C (ALDOC).

12. Procédé selon l'une quelconque des revendications 1 à 9, dans lequel la détermination de la quantité de l'au moins un transcrit d'ARN comprend :
la détermination, dans les cellules répondeuses, de la quantité d'au moins un transcrit d'ARN sélectionné dans le groupe constitué d'un transcrit de la 2-5' oligoadénylate synthase (OAS1Y), d'un transcrit de la protéine de liaison GTP induite par l'interféron (MX1), d'un transcrit de la protéine induite par l'interféron avec répétitions du tétratricopeptide 1 (LOC100139670), d'un gène stimulé par l'interféron 15 (ISG15), d'un transcrit d'ENSBTAG00000051364, d'un transcrit d'ENSBTAG00000053545, d'un cytochrome P450, famille 1, sous-famille A, d'un transcrit du polypeptide 1 (CYP1A), d'un transcrit de la dioxygénase dépendante de l'alpha-cétoglutarate homologue-2 de AlkB (ALKBH4), d'un transcrit de la protéine du domaine de la mort activant la MAP kinase (MADD), d'une protéine liée à la protéine 1 interagissant avec la huntingtine (HIP1R), d'un transcrit du cadre de lecture ouvert 198 du chromosome 28 C1 (C28H1orf198), d'un transcrit contenant un domaine HID1 (HID1), d'un transcrit de la protéine effectrice 1 Cdc42 (CDC42EP1), d'un transcrit de la protéine unc-13 homologue D (UNC13D), d'un transcrit de la famille d'aldéhyde déshydrogénase 16, membre A1 (ALDH16A1), d'un transcrit de la sous-unité catalytique de la calpaïne-1 (CAPN1), d'un homologue de peroxydasine (PXDN), d'un transcrit d'ENSBTAG00000043565, d'un transcrit de la sous-unité 1 du facteur de spécificité de clivage et de polyadénylation (CPSF1), d'un transcrit homologue de HGH1 (HGH1), d'un transcrit du facteur d'échange de nucléotide de guanine Rho 2 (ARHGEF2), d'un transcrit de la sous-unité bêta-3 de laminine (LAMB3), d'un transcrit de la protéine 3 liée à la follistatine (FSTL3) et d'un transcrit du membre de la famille rhomboïde 2 (RHBDF2), ou
la détermination, dans les cellules répondeuses, de la quantité d'au moins un transcrit d'ARN sélectionné dans le groupe constitué d'un transcrit de la 2-5' oligoadénylate synthase (OAS1Y), d'un transcrit de la protéine de liaison GTP induite par l'interféron (MX1), d'un transcrit de la protéine induite par l'interféron avec répétitions du tétratricopeptide 1 (LOC100139670), d'un gène stimulé par l'interféron 15 (ISG15), d'un transcrit de la protéine du domaine de la mort activant la MAP kinase (MADD), d'une protéine liée à la protéine 1 interagissant avec la huntingtine (HIP1R), d'un transcrit de la sous-unité catalytique de la calpaïne-1 (CAPN1), d'un transcrit contenant un domaine HID1 (HID1), d'un transcrit de la protéine effectrice 1 Cdc42 (CDC42EP1), d'un transcrit de la protéine unc-13 homologue D (UNC13D), d'un homologue de peroxydasine (PXDN), d'un transcrit de l'acyltransférase alpha de 1-acyl-sn-glycérol-3-phosphate (AGPAT1), d'un transcrit antagoniste/tueur homologue de Bcl-2 (BAK1), d'un transcrit de la petite sous-unité 2 de transporteur de larges acides aminés neutres (SLC7A8) et d'un transcrit de la transglutaminase tissulaire (TGM2).
